# EUROPEAN PATENT APPLICATION

(11) **EP 2 295 571 A1**
(43) Date of publication of application: **16.03.2011**
(21) Application number: 10010569.1
(22) Date of filing: 26.07.2006
(51) Int. Cl.: C12N 15/11, C12Q 1/68

(54) **Method of diagnosing small cell lung cancer**

(30) Priority: 27.07.2005 US 703192 P; 11.05.2006 US 799961 P
(62) Divisional of application: 06782127.2
(71) Applicant: Oncotherapy Science, Inc., Kawasaki-shi Kanagawa 213-0012 (JP)
(72) Inventor: Nakamura, Yusuke, Tokyo 113-8654 (JP); Daigo, Yataro, Tokyo 113-8654 (JP); Nakatsuru, Shuichi, Kawasaki-shi Kanagawa 213-0012 (JP)
(74) Representative: Vossius & Partner

(57) **Abstract**

Objective methods for detecting and diagnosing small cell lung cancer (SCLC) are described herein. In one embodiment, the diagnostic method involves determining the expression level of an SCLC-associated gene that discriminates between SCLC cells and normal cells. In another embodiment, the diagnostic method involves determining the expression level of an SCLC-associated gene that distinguishes two major histological types of lung cancer, non-small cell lung cancer (NSCLC) and SCLC. Finally, the present invention provides methods of screening for therapeutic agents useful in the treatment of small cell lung cancer, methods of treating small cell lung cancer and method for vaccinating a subject against small cell lung cancer. Furthermore, the present invention provides chemotherapy resistant lung cancer- or SCLC-associated genes as diagnostic markers and/or molecular targets for therapeutic agent for these cancers. These genes are up-regulated in chemoresistant lung cancer or SCLC. Accordingly, chemoresistant lung cancer or SCLC can be predicted using expression level of the genes as diagnostic markers. As the result, any adverse effects caused by ineffective chemotherapy can be avoided, and more suitable and effective therapeutic strategy can be selected.

SEQUENCE LISTING

<110>  ONCOTHERAPY SCIENCE, INC.
       THE UNIVERSITY OF TOKYO

<120>  METHOD OF DIAGNOSING SMALL CELL LUNG CANCER

<130>  ONC-A0518P

<150>  US 60/703,192
<151>  2005-07-27

<150>  US 60/799,961
<151>  2006-05-11

<160>  182

<170>  PatentIn version 3.3

<210>  1
<211>  21
<212>  DNA
<213>  Artificial

<220>
<223>  An artificially synthesized primer sequence for RT-PCR

<400>  1
gaggtgatag cattgctttc g                                                21

<210>  2
<211>  21
<212>  DNA
<213>  Artificial

<220>
<223>  An artificially synthesized primer sequence for RT-PCR

<400>  2
caagtcagtg tacaggtaag c                                                21

<210> 3
<211> 19
<212> DNA
<213> Artificial

<220>
<223> An artificially synthesized primer sequence for RT-PCR

<400> 3
ctgcgcgtac atgcgcact 19


<210> 4
<211> 22
<212> DNA
<213> Artificial

<220>
<223> An artificially synthesized primer sequence for RT-PCR

<400> 4
acttcatgct cctgaaaacc at 22


<210> 5
<211> 20
<212> DNA
<213> Artificial

<220>
<223> An artificially synthesized primer sequence for RT-PCR

<400> 5
ctcctttcct tgctgaggtg 20


<210> 6
<211> 20
<212> DNA
<213> Artificial

<220>
<223> An artificially synthesized primer sequence for RT-PCR

<400> 6
agctgtaggc cttgggaact 20

<210> 7
<211> 23
<212> DNA
<213> Artificial

<220>
<223> An artificially synthesized primer sequence for RT-PCR

<400> 7
ctgtataacg cgctcaccta tta 23

<210> 8
<211> 23
<212> DNA
<213> Artificial

<220>
<223> An artificially synthesized primer sequence for RT-PCR

<400> 8
tacacctttt actccctttt ccc 23

<210> 9
<211> 23
<212> DNA
<213> Artificial

<220>
<223> An artificially synthesized primer sequence for RT-PCR

<400> 9
aaagctagtg gcatacctca cag 23

<210> 10
<211> 22

<212>  DNA
<213>  Artificial

<220>
<223>  An artificially synthesized primer sequence for RT-PCR

<400>  10
cggtcagtga aaaacacatg at                                                22

<210>  11
<211>  23
<212>  DNA
<213>  Artificial

<220>
<223>  An artificially synthesized primer sequence for RT-PCR

<400>  11
aattgtggct ctcttccaag ttc                                               23

<210>  12
<211>  23
<212>  DNA
<213>  Artificial

<220>
<223>  An artificially synthesized primer sequence for RT-PCR

<400>  12
ggtactcttt tctccatttg gct                                               23

<210>  13
<211>  21
<212>  DNA
<213>  Artificial

<220>
<223>  An artificially synthesized primer sequence for RT-PCR

<400>  13

tccagaaatg gaatttgcct g 21

<210> 14
<211> 21
<212> DNA
<213> Artificial

<220>
<223> An artificially synthesized primer sequence for RT-PCR

<400> 14
ctgagggaaa agaaacccaa t 21

<210> 15
<211> 23
<212> DNA
<213> Artificial

<220>
<223> An artificially synthesized primer sequence for RT-PCR

<400> 15
gtcgtttcaa ccaggtagtt ttg 23

<210> 16
<211> 23
<212> DNA
<213> Artificial

<220>
<223> An artificially synthesized primer sequence for RT-PCR

<400> 16
cctattgcca aacacaatct ctc 23

<210> 17
<211> 22
<212> DNA
<213> Artificial

<220>
<223>  An artificially synthesized primer sequence for RT-PCR

<400>  17
agaaatgtgg atttcagcac ct                                                22

<210>  18
<211>  22
<212>  DNA
<213>  Artificial

<220>
<223>  An artificially synthesized primer sequence for RT-PCR

<400>  18
caataccaaa cacaacccaa ac                                                22

<210>  19
<211>  23
<212>  DNA
<213>  Artificial

<220>
<223>  An artificially synthesized primer sequence for RT-PCR

<400>  19
agcccaatct aagtattcct tgc                                               23

<210>  20
<211>  23
<212>  DNA
<213>  Artificial

<220>
<223>  An artificially synthesized primer sequence for RT-PCR

<400>  20
agtgacaacc agaaactttg cag                                               23

<210>  21
<211>  23
<212>  DNA
<213>  Artificial

<220>
<223>  An artificially synthesized primer sequence for RT-PCR

<400>  21
tccctgcaca gtaaagactt ttg                                                23


<210>  22
<211>  23
<212>  DNA
<213>  Artificial

<220>
<223>  An artificially synthesized primer sequence for RT-PCR

<400>  22
cagacataca ccagtcaaca gga                                                23


<210>  23
<211>  23
<212>  DNA
<213>  Artificial

<220>
<223>  An artificially synthesized primer sequence for RT-PCR

<400>  23
tttcagaggc tggagttaat ctg                                                23


<210>  24
<211>  23
<212>  DNA
<213>  Artificial

<220>

<223>  An artificially synthesized primer sequence for RT-PCR

<400>  24
ggattgatac agaacttgat gcc                                                23

<210>  25
<211>  23
<212>  DNA
<213>  Artificial

<220>
<223>  An artificially synthesized primer sequence for RT-PCR

<400>  25
ccagttactg tgtctatcgg gtc                                                23

<210>  26
<211>  23
<212>  DNA
<213>  Artificial

<220>
<223>  An artificially synthesized primer sequence for RT-PCR

<400>  26
agccatatgt agtcaagtgc cat                                                23

<210>  27
<211>  23
<212>  DNA
<213>  Artificial

<220>
<223>  An artificially synthesized primer sequence for RT-PCR

<400>  27
gcaaatcatt acaaggcaga cag                                                23

<210>  28

<211> 22
<212> DNA
<213> Artificial

<220>
<223> An artificially synthesized primer sequence for RT-PCR

<400> 28
aggatggcag gcttcctatt at 22


<210> 29
<211> 23
<212> DNA
<213> Artificial

<220>
<223> An artificially synthesized primer sequence for RT-PCR

<400> 29
tagggcaaca tggactgttt aag 23


<210> 30
<211> 23
<212> DNA
<213> Artificial

<220>
<223> An artificially synthesized primer sequence for RT-PCR

<400> 30
gctgtgtttt gtcatttagc tcc 23


<210> 31
<211> 25
<212> DNA
<213> Artificial

<220>
<223> An artificially synthesized primer sequence for RT-PCR

<400> 31
tctcctccca tatagaaggt actca 25

<210> 32
<211> 25
<212> DNA
<213> Artificial

<220>
<223> An artificially synthesized primer sequence for RT-PCR

<400> 32
cctcagaact ctcagtttat tcctg 25

<210> 33
<211> 23
<212> DNA
<213> Artificial

<220>
<223> An artificially synthesized primer sequence for RT-PCR

<400> 33
cacatgaaag agaaagaagt ggg 23

<210> 34
<211> 23
<212> DNA
<213> Artificial

<220>
<223> An artificially synthesized primer sequence for RT-PCR

<400> 34
aagtcaagat cgacaaacac tgc 23

<210> 35
<211> 22
<212> DNA

<213>  Artificial

<220>
<223>  An artificially synthesized primer sequence for RT-PCR

<400>  35
ttgcttccta atccctttgg tc                                                22


<210>  36
<211>  22
<212>  DNA
<213>  Artificial

<220>
<223>  An artificially synthesized primer sequence for RT-PCR

<400>  36
taagctgcat cttgatgcct tc                                                22


<210>  37
<211>  22
<212>  DNA
<213>  Artificial

<220>
<223>  An artificially synthesized primer sequence for RT-PCR

<400>  37
cctacttgtt ggagtccacg at                                                22


<210>  38
<211>  22
<212>  DNA
<213>  Artificial

<220>
<223>  An artificially synthesized primer sequence for RT-PCR

<400>  38
catgtcacat cttgatgcag tt                                                22

<210> 39
<211> 24
<212> DNA
<213> Artificial

<220>
<223> An artificially synthesized primer sequence for RT-PCR

<400> 39
ggacagttcc attcattagt tgtg 24

<210> 40
<211> 23
<212> DNA
<213> Artificial

<220>
<223> An artificially synthesized primer sequence for RT-PCR

<400> 40
ggcacttcat tgtatttgag gag 23

<210> 41
<211> 20
<212> DNA
<213> Artificial

<220>
<223> An artificially synthesized primer sequence for RT-PCR

<400> 41
gttgcctttt ggacctacca 20

<210> 42
<211> 25
<212> DNA
<213> Artificial

<220>
<223>  An artificially synthesized primer sequence for RT-PCR

<400>  42
caaccattta ccatgagatc attta                                          25


<210>  43
<211>  23
<212>  DNA
<213>  Artificial

<220>
<223>  An artificially synthesized primer sequence for RT-PCR

<400>  43
aagaggagac ctgaacatca aca                                            23


<210>  44
<211>  23
<212>  DNA
<213>  Artificial

<220>
<223>  An artificially synthesized primer sequence for RT-PCR

<400>  44
acgctatatt tggggcatag agt                                            23


<210>  45
<211>  23
<212>  DNA
<213>  Artificial

<220>
<223>  An artificially synthesized primer sequence for RT-PCR

<400>  45
caaagctgca tgtgtaggat gta                                            23

<210> 46
<211> 23
<212> DNA
<213> Artificial

<220>
<223> An artificially synthesized primer sequence for RT-PCR

<400> 46
ctcttgggaa ccagtacaga atg 23

<210> 47
<211> 21
<212> DNA
<213> Artificial

<220>
<223> An artificially synthesized primer sequence for RT-PCR

<400> 47
gagcaacagg ttggtgaaaa c 21

<210> 48
<211> 23
<212> DNA
<213> Artificial

<220>
<223> An artificially synthesized primer sequence for RT-PCR

<400> 48
gctgtatgta aatagcattg ggg 23

<210> 49
<211> 19
<212> DNA
<213> Artificial

<220>
<223> An artificially synthesized primer sequence for RT-PCR

<400>  49
gcaactctcc cgtcaaaca                                                    19

<210>  50
<211>  22
<212>  DNA
<213>  Artificial

<220>
<223>  An artificially synthesized primer sequence for RT-PCR

<400>  50
agatgccaat tcatgttctt cc                                                22

<210>  51
<211>  23
<212>  DNA
<213>  Artificial

<220>
<223>  An artificially synthesized primer sequence for RT-PCR

<400>  51
cggtgagact gatacagact tga                                               23

<210>  52
<211>  23
<212>  DNA
<213>  Artificial

<220>
<223>  An artificially synthesized primer sequence for RT-PCR

<400>  52
tatttctgta gcttccacat ccc                                               23

<210>  53
<211>  23

<212> DNA
<213> Artificial

<220>
<223> An artificially synthesized primer sequence for RT-PCR

<400> 53
aaccagagag aaagaggatc cag 23

<210> 54
<211> 23
<212> DNA
<213> Artificial

<220>
<223> An artificially synthesized primer sequence for RT-PCR

<400> 54
cagttggtgg ctatcaaatt agg 23

<210> 55
<211> 23
<212> DNA
<213> Artificial

<220>
<223> An artificially synthesized primer sequence for RT-PCR

<400> 55
ccaaatcaca acccaagata ctc 23

<210> 56
<211> 23
<212> DNA
<213> Artificial

<220>
<223> An artificially synthesized primer sequence for RT-PCR

<400> 56

caatgcttca ttctctgagt gct 23

<210> 57
<211> 20
<212> DNA
<213> Artificial

<220>
<223> An artificially synthesized primer sequence for RT-PCR

<400> 57
catctgtgga cacctcatgc 20

<210> 58
<211> 23
<212> DNA
<213> Artificial

<220>
<223> An artificially synthesized primer sequence for RT-PCR

<400> 58
ggaaatggta tggaataagc cag 23

<210> 59
<211> 23
<212> DNA
<213> Artificial

<220>
<223> An artificially synthesized primer sequence for RT-PCR

<400> 59
tccagaattg cttgttacgt agg 23

<210> 60
<211> 22
<212> DNA
<213> Artificial

<220>
<223>  An artificially synthesized primer sequence for RT-PCR

<400>  60
ggttctcaga gctgttttgc tt                                                22


<210>  61
<211>  23
<212>  DNA
<213>  Artificial

<220>
<223>  An artificially synthesized primer sequence for RT-PCR

<400>  61
tgatctgtct gctcctactc ctc                                               23


<210>  62
<211>  22
<212>  DNA
<213>  Artificial

<220>
<223>  An artificially synthesized primer sequence for RT-PCR

<400>  62
ctgtcccgta attgagagat gc                                                22


<210>  63
<211>  21
<212>  DNA
<213>  Artificial

<220>
<223>  An artificially synthesized primer sequence for RT-PCR

<400>  63
gtgctatgat cattgtaact t                                                 21

<210> 64
<211> 23
<212> DNA
<213> Artificial

<220>
<223> An artificially synthesized primer sequence for RT-PCR

<400> 64
gtaaatttct gaagtaatac ttt 23

<210> 65
<211> 22
<212> DNA
<213> Artificial

<220>
<223> An artificially synthesized primer sequence for RT-PCR

<400> 65
gacgtgcctc tcctactgtg ta 22

<210> 66
<211> 23
<212> DNA
<213> Artificial

<220>
<223> An artificially synthesized primer sequence for RT-PCR

<400> 66
aaagtccctc ttacctcgat ctg 23

<210> 67
<211> 23
<212> DNA
<213> Artificial

<220>

<223>  An artificially synthesized primer sequence for RT-PCR

<400>  67
cttctgacaa gcattcccta ttg                                              23

<210>  68
<211>  22
<212>  DNA
<213>  Artificial

<220>
<223>  An artificially synthesized primer sequence for RT-PCR

<400>  68
aatcaatccc tcgtattttc cc                                               22

<210>  69
<211>  23
<212>  DNA
<213>  Artificial

<220>
<223>  An artificially synthesized primer sequence for RT-PCR

<400>  69
ctgtcagggt catagtaggc att                                              23

<210>  70
<211>  22
<212>  DNA
<213>  Artificial

<220>
<223>  An artificially synthesized primer sequence for RT-PCR

<400>  70
ccaaagtcaa actcccattc at                                               22

<210>  71

<211>  20
<212>  DNA
<213>  Artificial

<220>
<223>  An artificially synthesized primer sequence for RT-PCR

<400>  71
cctgccaatt ctccttcatc                                                    20


<210>  72
<211>  22
<212>  DNA
<213>  Artificial

<220>
<223>  An artificially synthesized primer sequence for RT-PCR

<400>  72
catgcgccag taaatcagta ca                                                 22


<210>  73
<211>  20
<212>  DNA
<213>  Artificial

<220>
<223>  An artificially synthesized primer sequence for RT-PCR

<400>  73
gggtttgttt gctgcttttg                                                    20


<210>  74
<211>  19
<212>  DNA
<213>  Artificial

<220>
<223>  An artificially synthesized primer sequence for RT-PCR

<400> 74
cacaggggaa atggtggtt 19

<210> 75
<211> 20
<212> DNA
<213> Artificial

<220>
<223> An artificially synthesized primer sequence for RT-PCR

<400> 75
agccagcagt gagccagtat 20

<210> 76
<211> 20
<212> DNA
<213> Artificial

<220>
<223> An artificially synthesized primer sequence for RT-PCR

<400> 76
accacgcaca aggaattagg 20

<210> 77
<211> 20
<212> DNA
<213> Artificial

<220>
<223> An artificially synthesized primer sequence for RT-PCR

<400> 77
cccgtctgca actctctcac 20

<210> 78
<211> 20
<212> DNA

<213>  Artificial

<220>
<223>  An artificially synthesized primer sequence for RT-PCR

<400>  78
ctgaggttca gcgagggtaa                                                    20


<210>  79
<211>  23
<212>  DNA
<213>  Artificial

<220>
<223>  An artificially synthesized primer sequence for RT-PCR

<400>  79
cagatgctgg aggaagattc taa                                                23


<210>  80
<211>  22
<212>  DNA
<213>  Artificial

<220>
<223>  An artificially synthesized primer sequence for RT-PCR

<400>  80
aaagaaagag ggggaaacaa ag                                                 22


<210>  81
<211>  19
<212>  DNA
<213>  Artificial

<220>
<223>  An artificially synthesized primer sequence for RT-PCR

<400>  81
gaaagcacca gctcccgga                                                     19

<210>  82
<211>  24
<212>  DNA
<213>  Artificial

<220>
<223>  An artificially synthesized primer sequence for RT-PCR

<400>  82
gcttctacat ctcaaatcat gtcc                                              24


<210>  83
<211>  20
<212>  DNA
<213>  Artificial

<220>
<223>  An artificially synthesized primer sequence for RT-PCR

<400>  83
ggtggacacg gtcatctaca                                                   20


<210>  84
<211>  21
<212>  DNA
<213>  Artificial

<220>
<223>  An artificially synthesized primer sequence for RT-PCR

<400>  84
gaagcccgag aagatgggta t                                                 21


<210>  85
<211>  21
<212>  DNA
<213>  Artificial

<220>
<223>  An artificially synthesized primer sequence for RT-PCR

<400>  85
cttctccatt tctggagcca c                                                21


<210>  86
<211>  24
<212>  DNA
<213>  Artificial

<220>
<223>  An artificially synthesized primer sequence for RT-PCR

<400>  86
gccgttctaa tttagcttga agag                                             24


<210>  87
<211>  20
<212>  DNA
<213>  Artificial

<220>
<223>  An artificially synthesized primer sequence for RT-PCR

<400>  87
ggagaaactg caggacttgg                                                  20


<210>  88
<211>  22
<212>  DNA
<213>  Artificial

<220>
<223>  An artificially synthesized primer sequence for RT-PCR

<400>  88
caattttaat gtctgggttg gg                                               22

<210> 89
<211> 20
<212> DNA
<213> Artificial

<220>
<223> An artificially synthesized primer sequence for RT-PCR

<400> 89
catccagaag cacaagagca 20

<210> 90
<211> 22
<212> DNA
<213> Artificial

<220>
<223> An artificially synthesized primer sequence for RT-PCR

<400> 90
   tttccccttt taaacttccc tg 22

<210> 91
<211> 20
<212> DNA
<213> Artificial

<220>
<223> An artificially synthesized primer sequence for RT-PCR

<400> 91
ccctttgtca gaccctacca 20

<210> 92
<211> 23
<212> DNA
<213> Artificial

<220>
<223> An artificially synthesized primer sequence for RT-PCR

<400> 92
ttcagtaggc acacagttaa ccc 23

<210> 93
<211> 20
<212> DNA
<213> Artificial

<220>
<223> An artificially synthesized primer sequence for RT-PCR

<400> 93
actggaccac ccgaagatag 20

<210> 94
<211> 23
<212> DNA
<213> Artificial

<220>
<223> An artificially synthesized primer sequence for RT-PCR

<400> 94
ctacagcctg accacattct ttg 23

<210> 95
<211> 23
<212> DNA
<213> Artificial

<220>
<223> An artificially synthesized primer sequence for RT-PCR

<400> 95
aggacttggc tatcatttgg agt 23

<210> 96
<211> 23

<212>  DNA
<213>  Artificial

<220>
<223>  An artificially synthesized primer sequence for RT-PCR

<400>  96
caaagcaata cagcctttac cac                                                23


<210>  97
<211>  23
<212>  DNA
<213>  Artificial

<220>
<223>  An artificially synthesized primer sequence for RT-PCR

<400>  97
ccctaatgtc ccatgaagat aca                                                23


<210>  98
<211>  23
<212>  DNA
<213>  Artificial

<220>
<223>  An artificially synthesized primer sequence for RT-PCR

<400>  98
gccttagcaa gtcattttct gtc                                                23


<210>  99
<211>  23
<212>  DNA
<213>  Artificial

<220>
<223>  An artificially synthesized primer sequence for RT-PCR

<400>  99

tgagagtcct cagagggtat cag                                              23

<210>  100
<211>  23
<212>  DNA
<213>  Artificial

<220>
<223>  An artificially synthesized primer sequence for RT-PCR

<400>  100
cttgaagtca agagtcctgg tgt                                              23

<210>  101
<211>  23
<212>  DNA
<213>  Artificial

<220>
<223>  An artificially synthesized primer sequence for RT-PCR

<400>  101
ctcagaccta ccagtttccc ttt                                              23

<210>  102
<211>  23
<212>  DNA
<213>  Artificial

<220>
<223>  An artificially synthesized primer sequence for RT-PCR

<400>  102
gctttattta gggctaagct gga                                              23

<210>  103
<211>  23
<212>  DNA
<213>  Artificial

<220>
<223>  An artificially synthesized primer sequence for RT-PCR

<400>  103
actctcctac agagagccct gat                                              23


<210>  104
<211>  22
<212>  DNA
<213>  Artificial

<220>
<223>  An artificially synthesized primer sequence for RT-PCR

<400>  104
cagccaggat ttagtgccca gc                                               22


<210>  105
<211>  23
<212>  DNA
<213>  Artificial

<220>
<223>  An artificially synthesized primer sequence for RT-PCR

<400>  105
gaagatctcg tctgctcacc tta                                              23


<210>  106
<211>  23
<212>  DNA
<213>  Artificial

<220>
<223>  An artificially synthesized primer sequence for RT-PCR

<400>  106
cagacagatg gagaggctag aga                                              23

<210>  107
<211>  24
<212>  DNA
<213>  Artificial

<220>
<223>  An artificially synthesized primer sequence for RT-PCR

<400>  107
ccttaggtca gtaattgttg tgag                                              24


<210>  108
<211>  20
<212>  DNA
<213>  Artificial

<220>
<223>  An artificially synthesized primer sequence for RT-PCR

<400>  108
catatctctg gggtgcttgg                                                   20


<210>  109
<211>  23
<212>  DNA
<213>  Artificial

<220>
<223>  An artificially synthesized primer sequence for RT-PCR

<400>  109
ggcagacagg ggaagtaaga ata                                               23


<210>  110
<211>  23
<212>  DNA
<213>  Artificial

<220>

<223>  An artificially synthesized primer sequence for RT-PCR

<400>  110
ctgcatctca ccaaccaata act                                              23


<210>  111
<211>  23
<212>  DNA
<213>  Artificial

<220>
<223>  An artificially synthesized primer sequence for RT-PCR

<400>  111
atatatgagt tgctggggac ctt                                              23


<210>  112
<211>  23
<212>  DNA
<213>  Artificial

<220>
<223>  An artificially synthesized primer sequence for RT-PCR

<400>  112
tgctttggtc tgtacaaagt ctg                                              23


<210>  113
<211>  23
<212>  DNA
<213>  Artificial

<220>
<223>  An artificially synthesized primer sequence for RT-PCR

<400>  113
gggaacaatc ctagaaaaca ctg                                              23


<210>  114

<211>  24
<212>  DNA
<213>  Artificial

<220>
<223>  An artificially synthesized primer sequence for RT-PCR

<400>  114
gggaaggtca cattttacca ttag                                              24


<210>  115
<211>  22
<212>  DNA
<213>  Artificial

<220>
<223>  An artificially synthesized primer sequence for RT-PCR

<400>  115
caaaaaccag cttcttctct gg                                                22


<210>  116
<211>  23
<212>  DNA
<213>  Artificial

<220>
<223>  An artificially synthesized primer sequence for RT-PCR

<400>  116
caggaaagat cacaacctca ttc                                               23


<210>  117
<211>  23
<212>  DNA
<213>  Artificial

<220>
<223>  An artificially synthesized primer sequence for RT-PCR

<400>  117
tatcagtaac tgctccgtgt tca                                              23


<210>  118
<211>  23
<212>  DNA
<213>  Artificial

<220>
<223>  An artificially synthesized primer sequence for RT-PCR

<400>  118
ggtctgtcat tgaccaaaac atc                                              23


<210>  119
<211>  23
<212>  DNA
<213>  Artificial

<220>
<223>  An artificially synthesized primer sequence for RT-PCR

<400>  119
gaagattagg ggaaaagagg tca                                              23


<210>  120
<211>  23
<212>  DNA
<213>  Artificial

<220>
<223>  An artificially synthesized primer sequence for RT-PCR

<400>  120
cagagtccag tagagaatgc gat                                              23


<210>  121
<211>  23
<212>  DNA

<213>  Artificial

<220>
<223>  An artificially synthesized primer sequence for RT-PCR

<400>  121
ccctagtttt tgtagctgtc gaa                                                23


<210>  122
<211>  22
<212>  DNA
<213>  Artificial

<220>
<223>  An artificially synthesized primer sequence for RT-PCR

<400>  122
gatcacatgc caagaacaca at                                                 22


<210>  123
<211>  23
<212>  DNA
<213>  Artificial

<220>
<223>  An artificially synthesized primer sequence for RT-PCR

<400>  123
cttccattgg tatggttgtt acc                                                23


<210>  124
<211>  23
<212>  DNA
<213>  Artificial

<220>
<223>  An artificially synthesized primer sequence for RT-PCR

<400>  124
cccaattccc tactctcagc tat                                                23

<210> 125
<211> 23
<212> DNA
<213> Artificial

<220>
<223> An artificially synthesized primer sequence for RT-PCR

<400> 125
tgtgtctcat ctgtgaactg ctt 23


<210> 126
<211> 23
<212> DNA
<213> Artificial

<220>
<223> An artificially synthesized primer sequence for RT-PCR

<400> 126
ttcgtgttac ggtatatcct gct 23


<210> 127
<211> 23
<212> DNA
<213> Artificial

<220>
<223> An artificially synthesized primer sequence for RT-PCR

<400> 127
tattgggaaa agagaaggac cac 23


<210> 128
<211> 23
<212> DNA
<213> Artificial

<220>
<223> An artificially synthesized primer sequence for RT-PCR

<400> 128
agaagttggt tcatgtgtag gca 23

<210> 129
<211> 23
<212> DNA
<213> Artificial

<220>
<223> An artificially synthesized primer sequence for RT-PCR

<400> 129
gtgagaatat tcctcgtcac agc 23

<210> 130
<211> 23
<212> DNA
<213> Artificial

<220>
<223> An artificially synthesized primer sequence for RT-PCR

<400> 130
actgaagggg acaggaagac tac 23

<210> 131
<211> 23
<212> DNA
<213> Artificial

<220>
<223> An artificially synthesized primer sequence for RT-PCR

<400> 131
tgcctgagga tatagcagta agc 23

<210> 132
<211> 22
<212> DNA
<213> Artificial

<220>
<223> An artificially synthesized primer sequence for RT-PCR

<400> 132
ttctaagaag ggttctggct ca 22

<210> 133
<211> 23
<212> DNA
<213> Artificial

<220>
<223> An artificially synthesized primer sequence for RT-PCR

<400> 133
acacactaaa gcctgatgca gat 23

<210> 134
<211> 23
<212> DNA
<213> Artificial

<220>
<223> An artificially synthesized primer sequence for RT-PCR

<400> 134
cactgttagg cttgtaagac agc 23

<210> 135
<211> 20
<212> DNA
<213> Artificial

<220>
<223> An artificially synthesized primer sequence for RT-PCR

<400>  135
ccgtcagcag tgtgaagtct                                                    20


<210>  136
<211>  22
<212>  DNA
<213>  Artificial

<220>
<223>  An artificially synthesized primer sequence for RT-PCR

<400>  136
cctcctaagc agtcaacctt gt                                                 22


<210>  137
<211>  24
<212>  DNA
<213>  Artificial

<220>
<223>  An artificially synthesized primer sequence for RT-PCR

<400>  137
aaacaaacat acacttctcc tggc                                               24


<210>  138
<211>  25
<212>  DNA
<213>  Artificial

<220>
<223>  An artificially synthesized primer sequence for RT-PCR

<400>  138
cgtcacaaga agagacaata catac                                              25


<210>  139
<211>  23

<212>  DNA
<213>  Artificial

<220>
<223>  An artificially synthesized primer sequence for RT-PCR

<400>  139
atctggtttt taagggtctg agc                                              23


<210>  140
<211>  23
<212>  DNA
<213>  Artificial

<220>
<223>  An artificially synthesized primer sequence for RT-PCR

<400>  140
gcaagcgtaa gagactggtt tta                                              23


<210>  141
<211>  22
<212>  DNA
<213>  Artificial

<220>
<223>  An artificially synthesized primer sequence for RT-PCR

<400>  141
ccacacagag agatgtcacc tt                                               22


<210>  142
<211>  23
<212>  DNA
<213>  Artificial

<220>
<223>  An artificially synthesized primer sequence for RT-PCR

<400>  142

gatgaggaga gacgtgagag cta                                                23

<210>  143
<211>  21
<212>  DNA
<213>  Artificial

<220>
<223>  An artificially synthesized primer sequence for RT-PCR

<400>  143
aggaacatgt caggggctta c                                                  21

<210>  144
<211>  23
<212>  DNA
<213>  Artificial

<220>
<223>  An artificially synthesized primer sequence for RT-PCR

<400>  144
aagttcaact aacccccaaa gac                                                23

<210>  145
<211>  22
<212>  DNA
<213>  Artificial

<220>
<223>  An artificially synthesized primer sequence for RT-PCR

<400>  145
aaaaggtatg aacttttggg gg                                                 22

<210>  146
<211>  23
<212>  DNA
<213>  Artificial

<220>
<223>  An artificially synthesized primer sequence for RT-PCR

<400>  146
gcttgctctc tattggaggt aca                                                23

<210>  147
<211>  23
<212>  DNA
<213>  Artificial

<220>
<223>  An artificially synthesized primer sequence for RT-PCR

<400>  147
ggtcgtcttt atcccctata tgc                                                23

<210>  148
<211>  23
<212>  DNA
<213>  Artificial

<220>
<223>  An artificially synthesized primer sequence for RT-PCR

<400>  148
cagtgactct taaactgagc ggt                                                23

<210>  149
<211>  23
<212>  DNA
<213>  Artificial

<220>
<223>  An artificially synthesized primer sequence for RT-PCR

<400>  149
tctcctggac agtatgggtc taa                                                23

<210>  150
<211>  24
<212>  DNA
<213>  Artificial

<220>
<223>  An artificially synthesized primer sequence for RT-PCR

<400>  150
tgagcaggag atcttaattg acag                                          24


<210>  151
<211>  23
<212>  DNA
<213>  Artificial

<220>
<223>  An artificially synthesized primer sequence for RT-PCR

<400>  151
cactttacag aagcagaagt ggg                                           23


<210>  152
<211>  23
<212>  DNA
<213>  Artificial

<220>
<223>  An artificially synthesized primer sequence for RT-PCR

<400>  152
agctctaccc aggagaatac agg                                           23


<210>  153
<211>  23
<212>  DNA
<213>  Artificial

<220>

<223>  An artificially synthesized primer sequence for RT-PCR

<400>  153
aaagaggaac acactgggtg taa                                                23


<210>  154
<211>  23
<212>  DNA
<213>  Artificial

<220>
<223>  An artificially synthesized primer sequence for RT-PCR

<400>  154
aggagcctag agaagcaatc atc                                                23


<210>  155
<211>  23
<212>  DNA
<213>  Artificial

<220>
<223>  An artificially synthesized primer sequence for RT-PCR

<400>  155
gtgcaaggta agctgtcaaa aac                                                23


<210>  156
<211>  23
<212>  DNA
<213>  Artificial

<220>
<223>  An artificially synthesized primer sequence for RT-PCR

<400>  156
gaggtgtttt aaccagaaaa tcg                                                23


<210>  157

<211>  20
<212>  DNA
<213>  Artificial

<220>
<223>  An artificially synthesized primer sequence for RT-PCR

<400>  157
gcaggaaaga tcccaagtca                                                    20


<210>  158
<211>  22
<212>  DNA
<213>  Artificial

<220>
<223>  An artificially synthesized primer sequence for RT-PCR

<400>  158
agatgaacgg aacattgcac ac                                                 22


<210>  159
<211>  22
<212>  DNA
<213>  Artificial

<220>
<223>  An artificially synthesized primer sequence for RT-PCR

<400>  159
ggattccaaa cattttcgac ag                                                 22


<210>  160
<211>  22
<212>  DNA
<213>  Artificial

<220>
<223>  An artificially synthesized primer sequence for RT-PCR

<400> 160
gcaaatgcag tttctgccaa ta 22

<210> 161
<211> 22
<212> DNA
<213> Artificial

<220>
<223> An artificially synthesized primer sequence for RT-PCR

<400> 161
tgtgtgtata attgcaagcg ca 22

<210> 162
<211> 22
<212> DNA
<213> Artificial

<220>
<223> An artificially synthesized primer sequence for RT-PCR

<400> 162
tgctgaatta atgaggcacc aa 22

<210> 163
<211> 19
<212> DNA
<213> Artificial

<220>
<223> target sequence for siRNA

<400> 163
cgtacgcgga atacttcga 19

<210> 164
<211> 51
<212> DNA

<213>  Artificial

<220>
<223>  Artificially synthesized oligonucleotide for siRNA

<400>  164
tccccgtacg cggaatactt cgattcaaga gatcgaagta ttccgcgtac g                51


<210>  165
<211>  51
<212>  DNA
<213>  Artificial

<220>
<223>  Artificially synthesized oligonucleotide for siRNA

<400>  165
aaaacgtacg cggaatactt cgatctcttg aatcgaagta ttccgcgtac g                51


<210>  166
<211>  47
<212>  DNA
<213>  Artificial

<220>
<223>  siRNA hairpin design

<400>  166
cgtacgcgga atacttcgat tcaagagatc gaagtattcc gcgtacg                47


<210>  167
<211>  19
<212>  DNA
<213>  Artificial

<220>
<223>  target sequence for siRNA

<400>  167
gcgcgctttg taggattcg                19

<210> 168
<211> 51
<212> DNA
<213> Artificial

<220>
<223> Artificially synthesized oligonucleotide for siRNA

<400> 168
tcccgcgcgc tttgtaggat tcgttcaaga gacgaatcct acaaagcgcg c 51

<210> 169
<211> 51
<212> DNA
<213> Artificial

<220>
<223> Artificially synthesized oligonucleotide for siRNA

<400> 169
aaaagcgcgc tttgtaggat tcgtctcttg aacgaatcct acaaagcgcg c 51

<210> 170
<211> 47
<212> DNA
<213> Artificial

<220>
<223> siRNA hairpin design

<400> 170
gcgcgctttg taggattcgt tcaagagacg aatcctacaa agcgcgc 47

<210> 171
<211> 19
<212> DNA
<213> Artificial

<220>
<223>  target sequence for siRNA

<400>  171
tcaagcagga gctcatctg                                                    19


<210>  172
<211>  51
<212>  DNA
<213>  Artificial

<220>
<223>  Artificially synthesized oligonucleotide for siRNA

<400>  172
tccctcaagc aggagctcat ctgttcaaga gacagatgag ctcctgcttg a                51


<210>  173
<211>  51
<212>  DNA
<213>  Artificial

<220>
<223>  Artificially synthesized oligonucleotide for siRNA

<400>  173
aaaatcaagc aggagctcat ctgtctcttg aacagatgag ctcctgcttg a                51


<210>  174
<211>  47
<212>  DNA
<213>  Artificial

<220>
<223>  siRNA hairpin design

<400>  174
tcaagcagga gctcatctgt tcaagagaca gatgagctcc tgcttga                     47

<210> 175
<211> 4612
<212> DNA
<213> Homo sapiens

<220>
<221> CDS
<222> (250)..(2241)

<400> 175
   gcggccgcaa gcacgggggc gaatccccgc tgggtcgagg gcctgaacgg gagccaatcg 60
   agcagccgag gctactgcca atcacgcggc tccctccaat cccacccgtg ccatttccaa 120
   aatctcggtc ccactgtgca gctcaaatgt ggtgttcact ctgccaatcg ctggaggata 180
   gagtgggaac aggaataagc agagttaaga ggccaggaca aaagaagtta aagagcgccc 240
   aatacatac atg ttt ttg aag gcg ggc aga ggg aat aaa gtc ccc cca gtg 291
   Met Phe Leu Lys Ala Gly Arg Gly Asn Lys Val Pro Pro Val
   1 5 10
   agg gtc tat ggg cct gat tgt gta gtt ctg atg gag ccc cct ttg agc 339
   Arg Val Tyr Gly Pro Asp Cys Val Val Leu Met Glu Pro Pro Leu Ser
   15 20 25 30
   aag agg aac ccg cca gcg ctg aga tta gcg gat ttg gca acg gct cag 387
   Lys Arg Asn Pro Pro Ala Leu Arg Leu Ala Asp Leu Ala Thr Ala Gln
   35 40 45
   gtc cag ccg ctt cag aat atg aca ggc ttc ccg gcg ctg gcc ggc ccg 435
   Val Gln Pro Leu Gln Asn Met Thr Gly Phe Pro Ala Leu Ala Gly Pro
   50 55 60
   ccc gcc cac tcc caa ctc cgg gcc gcc gtc gcg cac ctc cgc ctg cgg 483
   Pro Ala His Ser Gln Leu Arg Ala Ala Val Ala His Leu Arg Leu Arg
   65 70 75
   gac ctg ggc gct gac ccc ggc gtg gcc acc act ccg ctc gga ccc gag 531
   Asp Leu Gly Ala Asp Pro Gly Val Ala Thr Thr Pro Leu Gly Pro Glu
   80 85 90
   cac atg gcc cag gcg agc acg ctg ggc ctc agc cct ccc tcc cag gcg 579
   His Met Ala Gln Ala Ser Thr Leu Gly Leu Ser Pro Pro Ser Gln Ala
   95 100 105 110
   ttc ccg gca cac ccg gag gct ccg gca gcc gcc gcc cgt gct gca gcc 627
   Phe Pro Ala His Pro Glu Ala Pro Ala Ala Ala Ala Arg Ala Ala Ala
   115 120 125
   ttg gtc gcg cac ccc ggc gcg ggc agc tac ccc tgc ggc ggg ggc agc 675
   Leu Val Ala His Pro Gly Ala Gly Ser Tyr Pro Cys Gly Gly Gly Ser
   130 135 140
   agt ggc gcg cag ccc tcc gcg ccc ccg ccc cca gcc cct cct ctt cct 723
   Ser Gly Ala Gln Pro Ser Ala Pro Pro Pro Pro Ala Pro Pro Leu Pro
   145 150 155
   ccc acc cct tca ccc cct ccc cct ccc ccg cct cct cct cct cct gcc 771
   Pro Thr Pro Ser Pro Pro Pro Pro Pro Pro Pro Pro Pro Pro Pro Ala
   160 165 170
   ctc tcg ggc tac acc acc acc aac agt ggc ggc ggc ggc agc agc ggc 819
   Leu Ser Gly Tyr Thr Thr Thr Asn Ser Gly Gly Gly Gly Ser Ser Gly
   175 180 185 190
   aaa ggc cac agc agg gac ttc gtc ctc cgg agg gac ctt tcc gcc acg 867
   Lys Gly His Ser Arg Asp Phe Val Leu Arg Arg Asp Leu Ser Ala Thr
   195 200 205
   gcc ccc gcg gcg gcc atg cac ggg gcc ccg ctc gga ggg gag cag cgg 915
   Ala Pro Ala Ala Ala Met His Gly Ala Pro Leu Gly Gly Glu Gln Arg
   210 215 220
   tcc ggc acc ggc tcc ccc cag cac ccg gcc ccg cct ccc cac tcg gcc 963
   Ser Gly Thr Gly Ser Pro Gln His Pro Ala Pro Pro Pro His Ser Ala
   225 230 235
   ggc atg ttc atc tcc gcc agc ggc acc tac gcg ggc ccg gac ggc agc 1011
   Gly Met Phe Ile Ser Ala Ser Gly Thr Tyr Ala Gly Pro Asp Gly Ser
   240 245 250
   ggc ggc ccg gcg ctc ttc ccc gcg ctg cac gac acg ccg ggg gcc cca 1059
   Gly Gly Pro Ala Leu Phe Pro Ala Leu His Asp Thr Pro Gly Ala Pro
   255 260 265 270
   ggc ggc cac ccg cac ccg ctc aac ggc cag atg cgc ctg ggg ctg gcg 1107
   Gly Gly His Pro His Pro Leu Asn Gly Gln Met Arg Leu Gly Leu Ala
   275 280 285
   gcg gca gcg gca gcc gcg gcg gct gag ctg tac ggc cgc gcc gaa ccg 1155
   Ala Ala Ala Ala Ala Ala Ala Ala Glu Leu Tyr Gly Arg Ala Glu Pro
   290 295 300
   ccc ttc gcg ccg cgc tct ggg gac gcg cac tac ggg gcg gtt gcg gcc 1203
   Pro Phe Ala Pro Arg Ser Gly Asp Ala His Tyr Gly Ala Val Ala Ala
   305 310 315
   gca gcg gcg gcc gcc ctg cac ggc tac gga gcc gtg aac tta aac ctg 1251
   Ala Ala Ala Ala Ala Leu His Gly Tyr Gly Ala Val Asn Leu Asn Leu
   320 325 330
   aac ctg gcg gct gcg gcg gcc gca gca gcg gcc ggg ccc ggg ccc cac 1299
   Asn Leu Ala Ala Ala Ala Ala Ala Ala Ala Ala Gly Pro Gly Pro His
   335 340 345 350
   ctg cag cac cac gcg ccg ccc ccg gcg ccg ccg ccg ccg ccg gcg ccc 1347
   Leu Gln His His Ala Pro Pro Pro Ala Pro Pro Pro Pro Pro Ala Pro
   355 360 365
   gcg cag cac ccg cac cag cac cac ccc cac ctc cca ggg gcg gct ggg 1395
   Ala Gln His Pro His Gln His His Pro His Leu Pro Gly Ala Ala Gly
   370 375 380
   gcc ttc ctg cgc tac atg cgg cag cca atc aag cag gag ctc atc tgc 1443
   Ala Phe Leu Arg Tyr Met Arg Gln Pro Ile Lys Gln Glu Leu Ile Cys
   385 390 395
   aag tgg atc gac ccc gac gag ctg gcc ggg ctg ccg ccg ccg ccg ccg 1491
   Lys Trp Ile Asp Pro Asp Glu Leu Ala Gly Leu Pro Pro Pro Pro Pro
   400 405 410
   ccg ccg ccg ccg ccg ccg cca ccg ccc ccg gcc ggc ggc gcc aag ccc 1539
   Pro Pro Pro Pro Pro Pro Pro Pro Pro Pro Ala Gly Gly Ala Lys Pro
   415 420 425 430
   tgc tcc aaa act ttc ggc acc atg cac gag ctg gtg aat cac gtc acg 1587
   Cys Ser Lys Thr Phe Gly Thr Met His Glu Leu Val Asn His Val Thr
   435                 440                 445
   gtg gag cac gtg gga ggc ccc gag cag agc agc cac gtc tgc ttc tgg     1635
   Val Glu His Val Gly Gly Pro Glu Gln Ser Ser His Val Cys Phe Trp
               450                 455                 460
   gag gac tgt ccg cgc gag ggc aag ccc ttc aag gcc aaa tac aag ctc     1683
   Glu Asp Cys Pro Arg Glu Gly Lys Pro Phe Lys Ala Lys Tyr Lys Leu
           465                 470                 475
   atc aac cac atc cgc gtg cac acc ggc gag aag ccc ttt ccc tgc cct     1731
   Ile Asn His Ile Arg Val His Thr Gly Glu Lys Pro Phe Pro Cys Pro
       480                 485                 490
   ttc ccc ggc tgc ggc aag gtc ttc gcg cgc tcc gag aac ctc aag atc     1779
   Phe Pro Gly Cys Gly Lys Val Phe Ala Arg Ser Glu Asn Leu Lys Ile
   495                 500                 505                 510
   cac aag cgt act cat aca ggg gaa aag cct ttc aaa tgt gaa ttt gat     1827
   His Lys Arg Thr His Thr Gly Glu Lys Pro Phe Lys Cys Glu Phe Asp
                   515                 520                 525
   ggc tgt gac agg aag ttt gcc aat agc agt gat cgg aag aaa cat tcc     1875
   Gly Cys Asp Arg Lys Phe Ala Asn Ser Ser Asp Arg Lys Lys His Ser
               530                 535                 540
   cat gtc cac acc agt gac aag ccc tac tac tgc aag att cga ggc tgt     1923
   His Val His Thr Ser Asp Lys Pro Tyr Tyr Cys Lys Ile Arg Gly Cys
           545                 550                 555
   gac aaa tcc tac act cac cca agc tcc ctg agg aag cac atg aag att     1971
   Asp Lys Ser Tyr Thr His Pro Ser Ser Leu Arg Lys His Met Lys Ile
       560                 565                 570
   cac tgc aag tcc ccg cca cct tct cca gga ccc ctt ggt tac tca tca     2019
   His Cys Lys Ser Pro Pro Pro Ser Pro Gly Pro Leu Gly Tyr Ser Ser
   575                 580                 585                 590
   gtg ggg act cca gtg ggc gcc ccc ttg tcc cct gtg ctg gac cca gcc     2067
   Val Gly Thr Pro Val Gly Ala Pro Leu Ser Pro Val Leu Asp Pro Ala
                   595                 600                 605
   agg agt cac tcc agc act ctg tcc cct cag gtg acc aac ctc aat gag     2115
   Arg Ser His Ser Ser Thr Leu Ser Pro Gln Val Thr Asn Leu Asn Glu
               610                 615                 620
   tgg tac gtt tgc cag gcc agt ggg gcc ccc agc cac ctc cac acc cct     2163
   Trp Tyr Val Cys Gln Ala Ser Gly Ala Pro Ser His Leu His Thr Pro
           625                 630                 635
   tcc agc aac gga acc acc tct gag act gaa gat gag gaa att tac ggg     2211
   Ser Ser Asn Gly Thr Thr Ser Glu Thr Glu Asp Glu Glu Ile Tyr Gly
       640                 645                 650
   aac cct gaa gtt gtg cgg acg ata cat tag aatttattat taataataat     2261
   Asn Pro Glu Val Val Arg Thr Ile His
   655                 660
   aagtgaaata ataagtggga gtccttggac cacatcctaa cctgagacaa tgccgagcct     2321
   gagacaaacc cgtgactcag acttgccacc gggtctaatt agccctattt attcagtatg     2381
   aaaccctatg gtgtttgtac atttaattaa tttaattaag atatttgggc tttttttttt     2441
   tttttttctt aaaaaacaaa caaaaaacaa ccaagctgga cttgtacatt gcaggaggat     2501
   ggggctgggg gcaaattgta ccaaggaaaa tgaatggaga gattagttaa tggcgataca     2561
   cactgccgat gcaatatata tatatatata tatacatata tatatatatt atttttttta     2621
   aaagggggag aaaaagagca ttaagtcaga acttaacaca gcaccaaggc cctctgcatt     2681
   tcccagagtg cctctcaaat gcctttgaca ccataccatg ggctgctttt gagcctcctt     2741
   gttggaccct aattctgcca aggcctcttg attgtaaacc acacacctgc tgcattgcca     2801
   acagatcctg ttccgtacct gtgtccaaaa acatttgtaa aaaccctttg agtttaatat     2861
   ttgtaatttt taatttccac tcttttatta ctgatcttag cttaatacaa tatttttata     2921
   caggattatt tcttcagtat cctactgtgt gattttaaaa aaagatgcag caaccttaat     2981
   atatctccat atcttgtgct actgtgattg ttcaagcaaa agtggagaga agaaaagctg     3041
   ctgcaaaaga caactgtgaa actgtgatat tttataaaat agaagaaatt caagtgcttt     3101
   ctttttccta tatgtttttt ttttttatct gaattctcag atactgcctc ctaactgtgt 3161
   ccaaacttct tgtgtaataa agagattctg ttttcgatcc taagttcttt gggatgccaa 3221
   cattcacagt caagtcttga ggaggtgtga tgatggcatc atgcctattt ttttggaaag 3281
   ctgttgtttt taaaacaggc caacacctct tttatactgt tgtatcagcc ttttaaaaag 3341
   tctatttttc aatgcctgaa actgcatttt aatgcatttt cttccacctg agcactgagc 3401
   acaccaaact ggaatccatt tgaaaatgac agtgtgtgaa gtgtatgatt tacattaaaa 3461
   gaggggaggg agttgccata catattaaaa atttttaaaa ggtttatagt taccaccaaa 3521
   cactgatgaa tgtgtgacct ttgccagagc tgtcaagcta ggataaaaaa ggtcaaggac 3581
   ctaggacaat aactcttagt cgatttattt tcggttggta caacacatct cctgtgcaaa 3641
   atgtagtcca tcagaaacat cctacagata cactaaagag cactaattta tccttagaga 3701
   ccccgaagac accccctccc cagggtttgt agaaatttgt tttgtgtgct gtgagtggtt 3761
   gatgtagtct tgtcattgtt aataacttgt atgtgaacac tattatttgt acagttgaat 3821
   taatttattt tcagacatca tccttttttt tttttctttc ctggaagagt tcaaagcaca 3881
   ccaaagaatt atattataca ttttggtgaa agattgtcat ttatgatcca tggtttattt 3941
   aaaaaaaaag ggaaagaaaa tggaaaaata tatttttaag cttacttgaa tgaacaacgt 4001
   aatgtgaaaa ccaagactct tcctgcatgt cttttttgca ttgtgttgat aagattatat 4061
   atagtttata gatatattat attactagta cagtgcatgg tgctgtcact tggaaagcct 4121
   ttcaatgttg tcttcagatt gttgtgatga atatgaaaca tgcagaccct cctttataaa 4181
   gaaaaagacc ttaaaacttg aatatgagat aattttacat tttaaaagtt tatttgattt 4241
   tcatattatt cactttcaaa gccctttcaa atagaaaagg tatgaacttt tggggggata 4301
   atttatgtat cgtaaactta ttagaacaaa atattcctga tgtataatga gttgttttat 4361
   ttatacaact ttttcaatgg tagtttgcac tattctttat tatgctacag gtttatttat 4421
   tatgaaacaa aggaatatgt attttatgta ttttaccatg cataggttaa ctctttgcca 4481
   cagatttatt ggttcttgat acacctaaaa taaaaaaaaa tgtgtacctc caatagagag 4541
   caagcaagaa tgattatgaa gtaacaaatt taataaaggt attcttgtta ttattgaaaa 4601
   aaaaaaaaaa a 4612

<210> 176
<211> 663
<212> PRT
<213> Homo sapiens

<400> 176

Met Phe Leu Lys Ala Gly Arg Gly Asn Lys Val Pro Pro Val Arg Val
1               5                   10                  15

Tyr Gly Pro Asp Cys Val Val Leu Met Glu Pro Pro Leu Ser Lys Arg
            20                  25                  30

Asn Pro Pro Ala Leu Arg Leu Ala Asp Leu Ala Thr Ala Gln Val Gln
        35                  40                  45

Pro Leu Gln Asn Met Thr Gly Phe Pro Ala Leu Ala Gly Pro Pro Ala
    50                  55                  60

His Ser Gln Leu Arg Ala Ala Val Ala His Leu Arg Leu Arg Asp Leu
65                  70                  75                  80

Gly Ala Asp Pro Gly Val Ala Thr Thr Pro Leu Gly Pro Glu His Met
                85                  90                  95

Ala Gln Ala Ser Thr Leu Gly Leu Ser Pro Pro Ser Gln Ala Phe Pro
            100                 105                 110

Ala His Pro Glu Ala Pro Ala Ala Ala Ala Arg Ala Ala Ala Leu Val
        115                 120                 125

Ala His Pro Gly Ala Gly Ser Tyr Pro Cys Gly Gly Gly Ser Ser Gly
    130                 135                 140

Ala Gln Pro Ser Ala Pro Pro Pro Pro Ala Pro Pro Leu Pro Pro Thr
145                 150                 155                 160

Pro Ser Pro Pro Pro Pro Pro Pro Pro Pro Pro Pro Pro Ala Leu Ser
                165                 170                 175

Gly Tyr Thr Thr Thr Asn Ser Gly Gly Gly Gly Ser Ser Gly Lys Gly
            180                 185                 190

His Ser Arg Asp Phe Val Leu Arg Arg Asp Leu Ser Ala Thr Ala Pro
        195                 200                 205

Ala Ala Ala Met His Gly Ala Pro Leu Gly Gly Glu Gln Arg Ser Gly
    210                 215                 220

Thr Gly Ser Pro Gln His Pro Ala Pro Pro Pro His Ser Ala Gly Met
225                 230                 235                 240

Phe Ile Ser Ala Ser Gly Thr Tyr Ala Gly Pro Asp Gly Ser Gly Gly
                245                 250                 255

Pro Ala Leu Phe Pro Ala Leu His Asp Thr Pro Gly Ala Pro Gly Gly
            260                 265                 270

His Pro His Pro Leu Asn Gly Gln Met Arg Leu Gly Leu Ala Ala Ala

275                 280                 285

Ala Ala Ala Ala Ala Ala Glu Leu Tyr Gly Arg Ala Glu Pro Pro Phe
    290                 295                 300

Ala Pro Arg Ser Gly Asp Ala His Tyr Gly Ala Val Ala Ala Ala Ala
305                 310                 315                 320

Ala Ala Ala Leu His Gly Tyr Gly Ala Val Asn Leu Asn Leu Asn Leu
                325                 330                 335

Ala Ala Ala Ala Ala Ala Ala Ala Ala Gly Pro Gly Pro His Leu Gln
            340                 345                 350

His His Ala Pro Pro Pro Ala Pro Pro Pro Pro Pro Ala Pro Ala Gln
        355                 360                 365

His Pro His Gln His His Pro His Leu Pro Gly Ala Ala Gly Ala Phe
    370                 375                 380

Leu Arg Tyr Met Arg Gln Pro Ile Lys Gln Glu Leu Ile Cys Lys Trp
385                 390                 395                 400

Ile Asp Pro Asp Glu Leu Ala Gly Leu Pro Pro Pro Pro Pro Pro Pro
                405                 410                 415

Pro Pro Pro Pro Pro Pro Pro Pro Ala Gly Gly Ala Lys Pro Cys Ser
            420                 425                 430

Lys Thr Phe Gly Thr Met His Glu Leu Val Asn His Val Thr Val Glu
        435                 440                 445

His Val Gly Gly Pro Glu Gln Ser Ser His Val Cys Phe Trp Glu Asp
    450                 455                 460

Cys Pro Arg Glu Gly Lys Pro Phe Lys Ala Lys Tyr Lys Leu Ile Asn
465                 470                 475                 480

His Ile Arg Val His Thr Gly Glu Lys Pro Phe Pro Cys Pro Phe Pro
                485                 490                 495

Gly Cys Gly Lys Val Phe Ala Arg Ser Glu Asn Leu Lys Ile His Lys
            500                 505                 510

Arg Thr His Thr Gly Glu Lys Pro Phe Lys Cys Glu Phe Asp Gly Cys
        515                 520                 525

Asp Arg Lys Phe Ala Asn Ser Ser Asp Arg Lys Lys His Ser His Val
    530                 535                 540

His Thr Ser Asp Lys Pro Tyr Tyr Cys Lys Ile Arg Gly Cys Asp Lys
545                 550                 555                 560

Ser Tyr Thr His Pro Ser Ser Leu Arg Lys His Met Lys Ile His Cys
                565                 570                 575

Lys Ser Pro Pro Pro Ser Pro Gly Pro Leu Gly Tyr Ser Ser Val Gly
            580                 585                 590

Thr Pro Val Gly Ala Pro Leu Ser Pro Val Leu Asp Pro Ala Arg Ser
        595                 600                 605

His Ser Ser Thr Leu Ser Pro Gln Val Thr Asn Leu Asn Glu Trp Tyr
    610                 615                 620

Val Cys Gln Ala Ser Gly Ala Pro Ser His Leu His Thr Pro Ser Ser
625                 630                 635                 640

Asn Gly Thr Thr Ser Glu Thr Glu Asp Glu Glu Ile Tyr Gly Asn Pro
                645                 650                 655

Glu Val Val Arg Thr Ile His
            660

<210>  177
<211>  20
<212>  DNA
<213>  Artificial

<220>
<223>  An artificially synthesized primer sequence for RT-PCR

<400>  177
agaactttgg ctccctttcc                                                   20

<210>  178
<211>  20
<212>  DNA
<213>  Artificial

<220>
<223>  An artificially synthesized primer sequence for RT-PCR

<400>  178
tgcatagttg cctggagatg                                                   20

<210>  179
<211>  20
<212>  DNA
<213>  Artificial

<220>

<223>  An artificially synthesized primer sequence for RT-PCR

<400>  179
gtccatgcca tgaatgagtg                                                    20


<210>  180
<211>  20
<212>  DNA
<213>  Artificial

<220>
<223>  An artificially synthesized primer sequence for RT-PCR

<400>  180
ctcttggcag atttgcatca                                                    20


<210>  181
<211>  20
<212>  DNA
<213>  Artificial

<220>
<223>  An artificially synthesized primer sequence for RT-PCR

<400>  181
cctctggtct ccccattaca                                                    20


<210>  182
<211>  20
<212>  DNA
<213>  Artificial

<220>
<223>  An artificially synthesized primer sequence for RT-PCR

<400>  182
ctgaggtgat gggttggtct                                                    20

## Description

### CROSS-REFERENCES TO RELATED APPLICATIONS

This application claims the benefit of U.S. Provisional Application Serial Nos. 60/703,192 filed July 27, 2005 and 60/799,961 filed May 11, 2006, the contents of each of which are hereby incorporated herein by reference in their entirety for all purposes.

### FIELD OF THE INVENTION

The present invention relates to methods of detecting, diagnosing, and providing a prognosis for small cell lung cancer as well as methods of treating and preventing small cell lung cancer.

### BACKGROUND OF THE INVENTION

Lung cancer is one of the most commonly fatal of human tumors. Many genetic alterations associated with development and progression of lung cancer have been reported, but the precise molecular mechanisms remain unclear (Sozzi, (2001) Eur J Cancer.;37 Suppl 7:S63-73). Small cell lung cancer (SCLC) comprises 15-20% of all lung cancers (Chute JP et al., (1999) J Clin Oncol.; 17:1794-801, Simon GR et al., (2003) Chest.; 123(1 Suppl):259S-271S). Although patients often respond favorably to multiagent chemotherapy, they relapse in a short time. Less than 5% of extensive-disease (ED) patients survive more than 5 years after initial diagnosis, and only 20% of patients with limited-stage disease (LD) are cured with combined modality therapy (Chute JP et al., (1999) J Clin Oncol.; 17:1794-801, Albain KS et al., (1991) J Clin Oncol.; 9:1618-26, Sandler AB et al., (2003) Semin Oncol.; 30:9-25). SCLC is categorized in a special class of lung tumors, including neuroendocrine tumors of the lung that share certain morphologic, ultrastructural, immunohistochemical and molecular characteristics. Certain paraneoplastic syndromes are distinctively associated with SCLC, for example, inappropriate secretion of antidiuretic hormone, ectopic Cushing's syndrome, and the Eaton-Lambert syndrome, however, detailed molecular characteristics of neuroendocrine tumors is still not well understood. Nonetheless, relatively high initial response rate of SCLC to chemotherapy suggests a potential for the development of effective, novel chemotherapeutic and targeted approaches (Sattler M & Salgia R, (2003) Semin Oncol.; 30:57-71, Wolff NC, et al., (2004) Clin Cancer Res. 10:3528-34).

The analysis of gene-expression profiles on cDNA microarrays enable performance of comprehensive analyses of gene expression in cancer cells, and can reveal detailed phenotypic and biological information about them (Golub TR et al., (1999) Science.; 286:531-7, Pomeroy SL et al., (2002) Nature.; 415:436-42, van't Veer LJ et al., (2002) Nature.; 415:530-6). Systematic analysis of expression levels among thousands of genes is also a useful approach to identification of unknown molecules involved in the pathways of lung carcinogenesis (Kikuchi T et al., (2003) Oncogene.;22:2192-205, Kakiuchi S et al., (2004) Hum Mol Genet.;13:3029-43 & (2003) Mol Cancer Res.;1:485-99, Zembutsu H, et al., (2003) Int J Oncol.;23:29-39), those discoveries can indicate targets for development of novel anti-cancer drugs and/or diagnostic markers (Suzuki C et al., (2003) Cancer Res.;63:7038-41, Ishikawa N et al., (2004) Clin Cancer Res.;10:8363-70).

### BRIEF SUMMARY OF THE INVENTION

Using comprehensive gene-expression profiles of 15 SCLCs purified by laser-microbeam microdissection (LMM) on a cDNA microarray containing 32,256 genes, the present inventors have identified a number of genes that are good candidates for development of therapeutic drugs or immunotherapy of lung cancers. The present inventors have also discovered that certain genes are expressed differently between the two most common histological types of lung cancer, non-small cell lung cancer (NSCLC) and SCLC. These results lend themselves to the application of "personalized therapy".

In order to identify the molecules involved in pulmonary carcinogenesis and those to be useful for novel diagnostic markers as well as targets for new drugs and immunotherapy, we constructed a screening system using the cDNA microarray. First, we used a cDNA microarray representing 32,256 genes to analyze the expression profiles of 15 small-cell lung cancers (SCLCs) purified by laser-microbeam microdissection (LMM). We have established a detailed genome-wide database for sets of genes that were significantly up- or down-regulated in SCLCs. 776 transcripts had at least 0.2-fold lower expression in more than 50% of SCLCs in comparison with the control (human lung), whereas 779 genes showed 5-fold higher expression in more than 50% of SCLCs. We confirmed 83 of their gene expression patterns in tumor and normal tissues using semi-quantitative RT-PCR and/or northern-blot analyses and that these genes are good candidates for development of novel therapeutic drugs or immunotherapy as well as tumor markers. Among these genes, we further characterized a Zic family member 5 (odd-paired homolog, Drosophila; *ZIC5*). Treatment of SCLC cells with small interfering RNAs (siRNAs) of *ZIC5* suppressed growth of the cancer cells. Additionally, a clustering algorithm applied to the expression data of 475 genes identified by random-permutation test easily distinguished two major histological types of lung cancer, non-small cell lung cancer (NSCLC) and SCLC. In particular, we obtained 34 genes which were expressed abundantly in SCLC, and some of which revealed the characteristics of certain neuronal functions including neurogenesis and neuroprotection. We also identified 68 genes that were abundantly expressed both in advanced SCLCs and advanced adenocarcinomas (ADCs), both of which had been obtained from patients with extensive chemotherapy treatment. Some of them are known to be transcription factors and/or gene expression regulators, for example, *TAF5L, TFCP2L4, PHF20, LMO4, TCF20, RFX2,* and *DKFZp547I048,* and some encode nucleotide-binding proteins, for example, *C9orf76, EHD3,* and *GIMAP4.* These data provide valuable information for identifying novel diagnostic systems and therapeutic target molecules for this type of cancer.

The present invention is based in part on the discovery of a pattern of gene expression that correlates with small cell lung cancer (SCLC). Genes that are differentially expressed in small cell lung cancer are collectively referred to herein as "SCLC nucleic acids," or "SCLC polynucleotides" and the corresponding encoded polypeptides are referred to as "SCLC polypeptides" or "SCLC proteins."

Accordingly, the present invention provides methods of diagnosing, providing a prognosis for or determining a predisposition to small cell lung cancer in a subject by determining an expression level of an SCLC-associated gene in a biological sample from a patient, for example, a tissue sample. The term "SCLC-associated gene" or "small cell lung cancer-associated gene" refers to a gene that is characterized by an expression level which differs in an SCLC cell as compared to the expression level of a normal cell. A normal cell is one obtained from lung tissue. In the context of the present invention, an SCLC-associated gene is a gene listed in Tables 2-3 (*i.e*., genes of SCLC Nos. 1-1555), or a gene having at least 90%, 95%, 96%, 97% 98%, or 99% sequence identity to a gene listed in tables 2-3 and the same function (*e.g*., homologs, genetic variants and polymorphisms). Algorithms known in the art can be used to determine the sequence identity of two or more nucleic acid sequences (*e.g*., BLAST, *see below*). An alteration or difference, *e.g.*, an increase or decrease in the level of expression of a gene as compared to a normal control expression level of the gene, indicates that the subject suffers from or is at risk of developing SCLC.

In the context of the present invention, the phrase "control level" refers to a protein expression level detected in a control sample and includes both a normal control level and a small cell lung cancer control level. A control level can be a single expression pattern from a single reference population or from a plurality of expression patterns. For example, the control level can be a database of expression patterns from previously tested cells. A "normal control level" refers to a level of gene expression detected in a normal, healthy individual or in a population of individuals known not to be suffering from small cell lung cancer. A normal individual is one with no clinical symptoms of small cell lung cancer. On the other hand, a "SCLC control level" refers to an expression profile of SCLC-associated genes found in a population suffering from SCLC.

An increase in the expression level of one or more SCLC-associated genes listed in Table 3 (*i.e.,* genes of SCLC Nos. 777-1555) detected in a test sample as compared to a normal control level indicates that the subject (from which the sample was obtained) suffers from or is at risk of developing SCLC. In contrast, a decrease in the expression level of one or more SCLC-associated genes listed in Table 2 (*i.e.,* genes of SCLC Nos. 1-776) detected in a test sample compared to a normal control level indicates said subject suffers from or is at risk of developing SCLC.

Alternatively, expression of a panel of SCLC-associated genes in a sample can be compared to an SCLC control level of the same panel of genes. A similarity between a sample expression and SCLC control expression indicates that the subject (from which the sample was obtained) suffers from or is at risk of developing SCLC.

According to the present invention, gene expression level is deemed "altered" or "to differ" when gene expression is increased or decreased 10%, 25%, 50% as compared to the control level. Alternatively, an expression level is deemed "increased" or "decreased" when gene expression is increased or decreased by at least 0.1, at least 0.2, at least 1, at least 2, at least 5, or at least 10 or more fold as compared to a control level. Expression is determined by detecting hybridization, *e.g.*, on an array, of an SCLC-associated gene probe to a gene transcript of the tissue sample from a patient.

In the context of the present invention, the tissue sample from a patient is any tissue obtained from a test subject, *e.g.*, a patient known to or suspected of having SCLC. For example, the tissue can contain an epithelial cell. More particularly, the tissue can be an epithelial cell from a lung cancer.

The present invention also provides an SCLC reference expression profile, comprising a gene expression level of two or more of SCLC-associated genes listed in Tables 2-3. Alternatively, the SCLC reference expression profile can comprise the levels of expression of two or more of SCLC-associated genes listed in Table 2, or SCLC-associated genes listed in Table 3.

The present invention further provides methods of identifying an agent that inhibits or enhances the expression or activity of one or more SCLC-associated genes, *e.g.* one or more SCLC-associated genes listed in Tables 2-3, by contacting a test cell expressing one or more SCLC-associated genes with a test compound and determining the expression level or activity of the SCLC-associated gene or the activity of its gene product. The test cell can be an epithelial cell, for example, an epithelial cell obtained from a lung cancer. A decrease in the expression level of an up-regulated SCLC-associated gene or the activity of its gene product as compared to a level or activity detected in absence of the test compound indicates that the test agent is an inhibitor of the SCLC-associated gene and can be used to reduce a symptom of SCLC, *e.g.* the expression of one or more SCLC-associated genes listed in Table 3. Alternatively, an increase in the expression level of a down-regulated SCLC-associated gene or the activity of its gene product as compared to an expression level or activity detected in absence of the test compound indicates that the test agent is an enhancer of expression or function of the SCLC-associated gene and can be used to reduce a symptom of SCLC, *e.g.*, the under-expression of one or more SCLC-associated genes listed in Table 2.

The present invention also provides a kit comprising a detection reagent which binds to one or more SCLC nucleic acids or SCLC polypeptides. Also provided is an array of nucleic acids that binds to one or more SCLC nucleic acids.

Therapeutic methods of the present invention include methods of treating or preventing SCLC in a subject including the step of administering to the subject an antisense composition (*i.e*., a composition comprising one or more antisense oligonucleotides). In the context of the present invention, the antisense composition reduces the expression of the specific target gene. For example, the antisense composition can contain one or more nucleotides which are complementary to one or more SCLC-associated gene sequences selected from the group consisting of the SCLC-associated genes listed in Table 3. Alternatively, the present method can include the steps of administering to a subject a small interfering RNA (siRNA) composition (*i.e.,* a composition comprising one or more siRNA oligonucleotides). In the context of the present invention, the siRNA composition reduces the expression of one or more SCLC nucleic acids selected from the group consisting of the SCLC-associated genes listed in Table 3, for example, ZIC5. In yet another method, the treatment or prevention of SCLC in a subject can be carried out by administering to a subject a ribozyme composition (*i.e*., a composition comprising one or more ribozymes). In the context of the present invention, the nucleic acid-specific ribozyme composition reduces the expression of one or more SCLC nucleic acids selected from the group consisting of the SCLC-associated genes listed in Table 3. Thus, in some embodiments of the present invention, one or more SCLC-associated genes listed in Table 3 are therapeutic targets of small cell lung cancer. Other therapeutic methods include those in which a subject is administered a compound that increases the expression of one or more of the SCLC-associated genes listed in Table 2 or the activity of a polypeptide encoded by one or more of the SCLC-associated genes listed in Table 2.

The present invention also includes vaccines and vaccination methods. For example, methods of treating or preventing SCLC in a subject can involve administering to the subject a vaccine composition comprising one or more polypeptides encoded by one or more nucleic acids selected from the group consisting of SCLC-associated genes listed in Table 3 or immunologically active fragments of such polypeptides. In the context of the present invention, an immunologically active fragment is a polypeptide that is shorter in length than the full-length naturally-occurring protein yet which induces an immune response analogous to that induced by the full-length protein. For example, an immunologically active fragment should be at least 8 residues in length and capable of stimulating an immune cell, for example, a T cell or a B cell. Immune cell stimulation can be measured by detecting cell proliferation, elaboration of cytokines (*e.g.*, IL-2), or production of an antibody. *See, for example,* Harlow and Lane, Using Antibodies: A Laboratory Manual, 1998, Cold Spring Harbor Laboratory Press; and Coligan, et al., Current Protocols in Immunology, 1991-2006, John Wiley & Sons.

The present invention is also based in part on the surprising discovery that inhibiting expression of ZIC5 is effective in inhibiting the cellular growth of various cancer cells, including those involved in SCLC. The inventions described in this application are based in part on this discovery.

The invention provides methods for inhibiting cell growth. Among the methods provided are those comprising contacting a cell with a composition comprising one or more small interfering RNA oligonucleotides (siRNA) that inhibit expression of ZIC5. The invention also provides methods for inhibiting tumor cell growth in a subject. Such methods include administering to a subject a composition comprising one or more small interfering RNAs (siRNA) that hybridizes specifically to a sequence from ZIC5. Another aspect of the invention provides methods for inhibiting the expression of the ZIC5 gene in a cell of a biological sample. Expression of the gene can be inhibited by introduction of one or more double stranded ribonucleic acid (RNA) molecules into the cell in amounts sufficient to inhibit expression of the ZIC5 gene. Another aspect of the invention relates to products including nucleic acid sequences and vectors as well as to compositions comprising them, useful, for example, in the provided methods. Among the products provided are siRNA molecules having the property to inhibit expression of the ZIC5 gene when introduced into a cell expressing said gene. Among such molecules are those that comprise a sense strand and an antisense strand, wherein the sense strand comprises a ribonucleotide sequence corresponding to a ZIC5 target sequence, and wherein the antisense strand comprises a ribonucleotide sequence which is complementary to said sense strand. The sense and the antisense strands of the molecule hybridize to each other to form a double-stranded molecule.

The invention features methods of inhibiting cell growth. Cell growth is inhibited by contacting a cell with a composition of a small interfering RNA (siRNA) of ZIC5. The cell is further contacted with a transfection-enhancing agent. The cell is provided *in vitro, in vivo* or *ex vivo.* The subject is a mammal, *e.g.*, a human, non-human primate, mouse, rat, dog, cat, horse, or cow. The cell is a lung epithelial cell. Alternatively, the cell is a tumor cell (*i.e.,* cancer cell), for example, a carcinoma cell or an adenocarcinoma cell. For example, the cell is a small cell lung cancer cell. By inhibiting cell growth is meant that the treated cell proliferates at a lower rate or has decreased viability than an untreated cell. Cell growth is measured by proliferation assays known in the art.

The present invention is also based in part on the discovery of a pattern of gene expression that correlates with chemotherapy-resistant lung cancer. The term . "chemotherapy" generally refers to a treatment of a disease using specific chemical agents. In the present invention, a subject of chemotherapy can be a cancer cell or tissue, preferably a lung cancer cell or tissue. Herein, "chemotherapeutic agent" refers to a pharmaceutical agent generally used for treating cancer, particularly lung cancer. The chemotherapeutic agents for treating cancer include, for example, cisplatin, carboplatin, etoposide, vincristine, cyclophosphamide, doxorubicin, ifosfamide, paclitaxel, gemcitabine, and docetaxel. More specifically, the chemotherapeutic agents of the present invention include platinum-based anti-cancer agents, including cisplatin and carboplatin. The term "chemotherapy-resistant" particularly means a cancer cell or tissue is not affected by chemotherapeutic agents that are selectively destructive to typical malignant cells or tissues.

Genes that are differentially expressed in chemotherapy-resistant lung cancer are collectively referred to herein as "chemotherapy-resistant lung cancer nucleic acids" or "chemotherapy-resistant lung cancer polynucleotides" and the corresponding encoded polypeptides are referred to as "chemotherapy-resistant lung cancer polypeptides" or "chemotherapy-resistant lung cancer proteins." Accordingly, the present invention further provides methods of diagnosing chemotherapy-resistant lung cancer or a predisposition for developing chemotherapy-resistant lung cancer in a subject by determining a level of expression of a chemotherapy-resistant lung cancer-associated gene in a biological sample from a patient. The term "chemotherapy-resistant lung cancer-associated gene" refers to a gene that is characterized by an expression level which differs in a chemotherapy-resistant lung cancer cell as compared to a chemotherapy-sensitive lung cancer cell. In the context of the present invention, a chemotherapy-resistant lung cancer-associated gene is a gene listed in Table 5. Alternatively, in the present invention, chemotherapy-resistant SCLC associated gene is a gene listed in Table 6. An increase in the sample expression level as compared to a control level of the gene indicates that the subject suffers from or is at risk of developing chemotherapy-resistant lung cancer or SCLC.

In this context of the present invention, the phrase "control level" refers to a level of gene expression detected in an individual or in a population suffering from chemotherapy-sensitive lung cancer or SCLC.

An increase in the expression level of one or more chemotherapy-resistant lung cancer-associated genes listed in Table 5 detected in a test sample as compared to a control level indicates that the subject (from which the sample was obtained) suffers from or is at risk of developing chemotherapy-resistant lung cancer. Similarly, an increase in the expression level of one or more chemotherapy-resistant SCLC-associated genes listed in Table 6 detected in a test sample as compared to a control level indicates that the subject (from which the sample was obtained) suffers from or is at risk of developing chemotherapy-resistant SCLC.

According to the present invention, chemotherapy-resistant lung cancer (or SCLC)-associated gene expression level is deemed "increased" when gene expression is increased at least about 10%, 25%, 50% as compared to a normal control level. Expression is determined by detecting hybridization, *e.g.*, on an array, of a chemotherapy-resistant lung cancer (or SCLC)-associated gene probe to a gene transcript of the tissue sample from a patient

In the context of the present invention, the tissue sample from a patient is any tissue obtained from a test subject, *e.g.*, a patient known to or suspected of having chemotherapy-resistant lung cancer or SCLC.

The present invention also provides a chemotherapy-resistant lung cancer (or SCLC) reference expression profile, comprising a gene expression level of two or more of chemotherapy-resistant lung cancer -associated genes listed in Tables 5-6. Alternatively, when the chemotherapy-resistant lung cancer is small cell lung cancer, the chemotherapy-resistant lung cancer reference expression profile comprise the levels of expression of two or more of chemotherapy-resistant lung cancer-associated genes listed in Table 6.

The present invention further provides methods of identifying an agent that inhibits or enhances the expression or activity of a chemotherapy-resistant lung cancer (or SCLC)-associated gene, *e.g.* a chemotherapy-resistant lung cancer (or SCLC)-associated gene listed in Tables 5-6, by contacting a test cell expressing a chemotherapy-resistant lung cancer (or SCLC)-associated gene with a test compound and determining the expression level or activity of the gene or the activity of its gene product. The test cell can be a cell obtained from a chemotherapy-resistant lung cancer (or SCLC). A decrease in the expression level of an up-regulated chemotherapy-resistant lung cancer (or SCLC)-associated gene or the activity of its gene product as compared to an expression level or activity detected in absence of the test compound indicates that the test compound is an inhibitor of the chemotherapy-resistant lung cancer (or SCLC)-associated gene and can be used to reduce a symptom of chemotherapy-resistant lung (or SCLC) cancer, *e.g.* the expression of one or more chemotherapy-resistant lung cancer (or SCLC)-associated genes listed in Tables 5-6.

The present invention also provides kits comprising a detection reagent which binds to one or more chemotherapy-resistant lung cancer (or SCLC) nucleic acids or chemotherapy-resistant lung cancer (or SCLC) polypeptides. Also provided are arrays of nucleic acids that binds to one or more chemotherapy-resistant lung cancer (or SCLC) nucleic acids.

Therapeutic methods of the present invention include methods of treating or preventing chemotherapy-resistant lung cancer (or SCLC) in a subject including the step of administering to the subject an antisense composition comprising one or more antisense oligonuleotides. In the context of the present invention, the antisense composition reduces the expression of the specific target gene. For example, the antisense composition can contain one or more nucleotides which are complementary to a chemotherapy-resistant lung cancer (or SCLC)-associated gene sequence selected from the group consisting of the chemotherapy-resistant lung cancer (or SCLC)-associated genes listed in Tables 5-6. Alternatively, the present method can include the steps of administering to a subject a small interfering RNA (siRNA) composition comprising one or more siRNA oligonucleotides. In the context of the present invention, the siRNA composition reduces the expression of one or more chemotherapy-resistant lung cancer (or SCLC) nucleic acids selected from the group consisting of the chemotherapy-resistant lung cancer (or SCLC)-associated genes listed in Tables 5-6. In yet another method, the treatment or prevention of chemotherapy-resistant lung cancer (or SCLC) in a subject can be carried out by administering to a subject a ribozyme composition comprising one or more ribozymes. In the context of the present invention, the nucleic acid-specific ribozyme composition reduces the expression of one or more chemotherapy-resistant lung cancer (or SCLC) nucleic acids selected from the group consisting of the chemotherapy-resistant lung cancer (or SCLC)-associated genes listed in Tables 5-6. Thus, in the present invention, chemotherapy-resistant lung cancer (or SCLC)-associated genes listed in Tables 5-6 are preferable therapeutic target of the chemotherapy-resistant lung cancer (or SCLC).

The present invention also includes vaccines and vaccination methods. For example, methods of treating or preventing chemotherapy-resistant lung cancer (or SCLC) in a subject can involve administering to the subject a vaccine containing one or more polypeptides encoded by one or more nucleic acids selected from the group consisting of chemotherapy-resistant lung cancer (or SCLC)-associated genes listed in Tables 5-6 or an immunologically active fragment of such a polypeptide. In the context of the present invention, an immunologically active fragment is a polypeptide that is shorter in length than the full-length naturally-occurring protein yet which induces an immune response analogous to that induced by the full-length protein. For example, an immunologically active fragment should be at least 8 residues in length and capable of stimulating an immune cell, for example, a T cell or a B cell. Immune cell stimulation can be measured by detecting cell proliferation, elaboration of cytokines (*e.g.*, IL-2), or production of an antibody.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference herein in their entirety. In case of conflict, the present specification, including definitions, will control. In addition, the materials, methods, and examples are illustrative only and not intended to be limiting.

One advantage of the methods described herein is that the disease is identified prior to detection of overt clinical symptoms of small cell lung cancer. Other features and advantages of the invention will be apparent from the following detailed description, and from the claims.

### BRIEF DESCRIPTION OF THE DRAWINGS

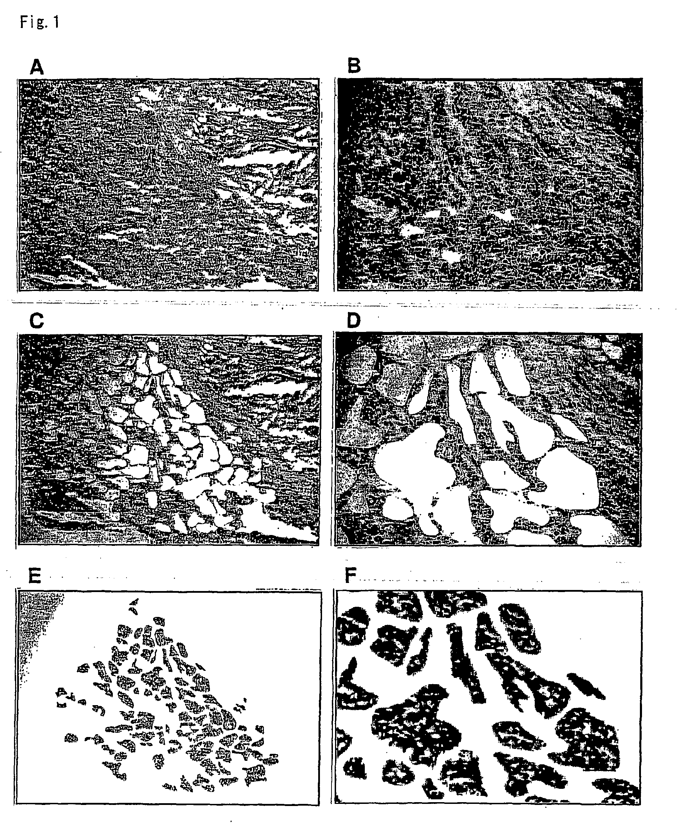

Figure 1 shows images illustrating laser-microbeam microdissection (LMM) of two representative SCLCs. The upper panels *(**A, B**)* show the samples before dissection; the middle *(**C, D**),* the same sections after microdissection (H.E. stain x 400). The microdissected cancer cells captured on the collecting cap were also shown in the bottom panels (***E, F***).

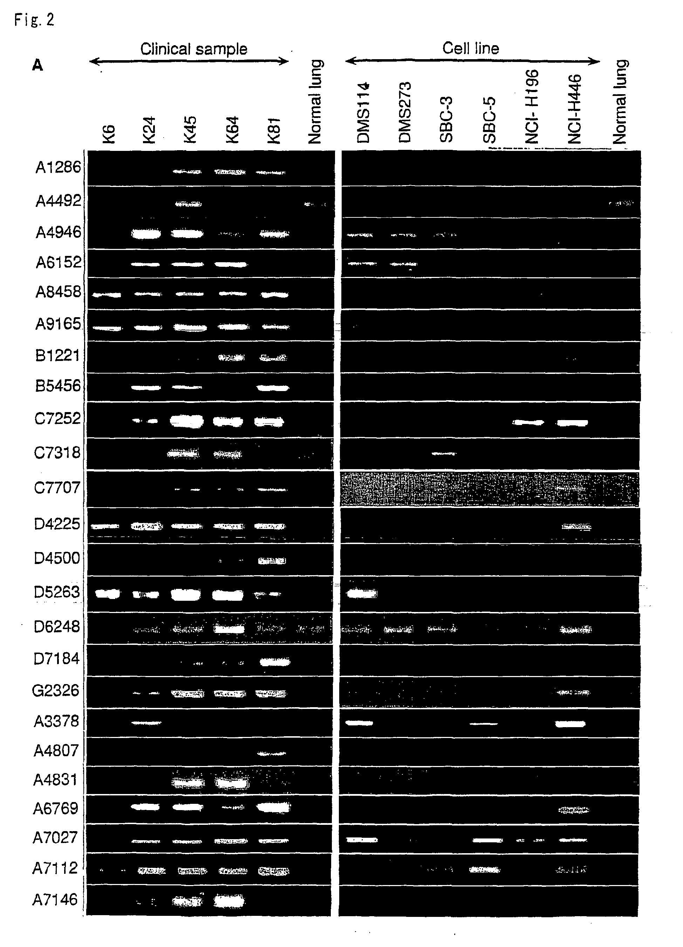

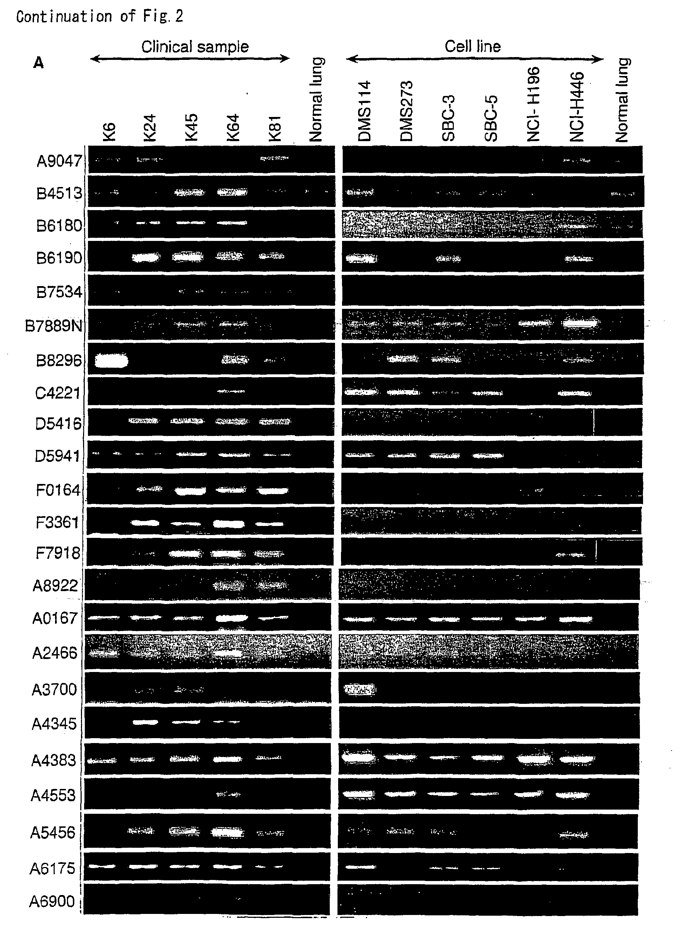

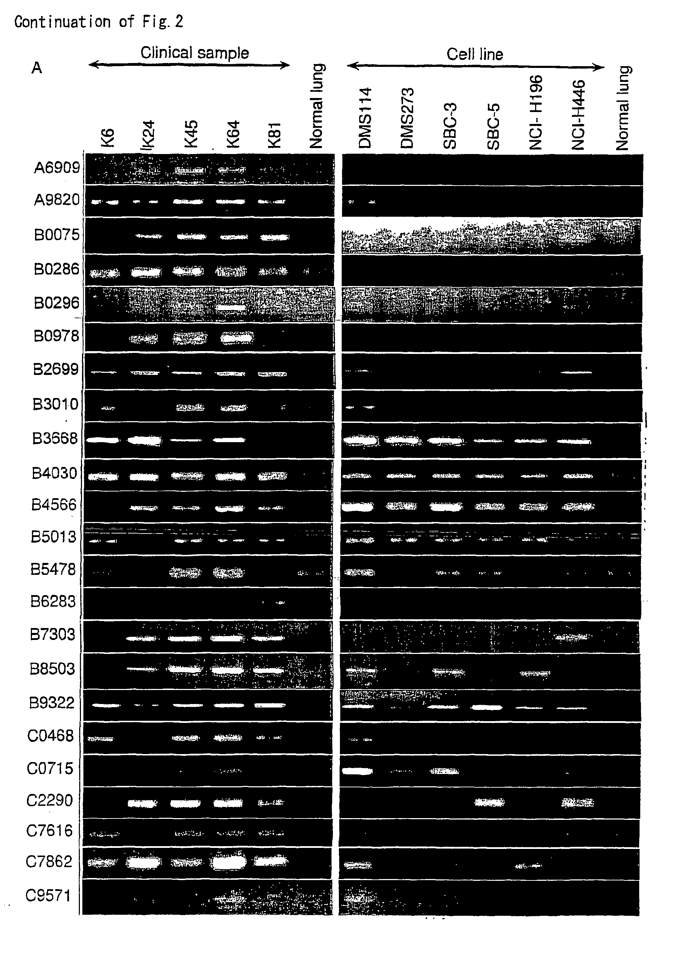

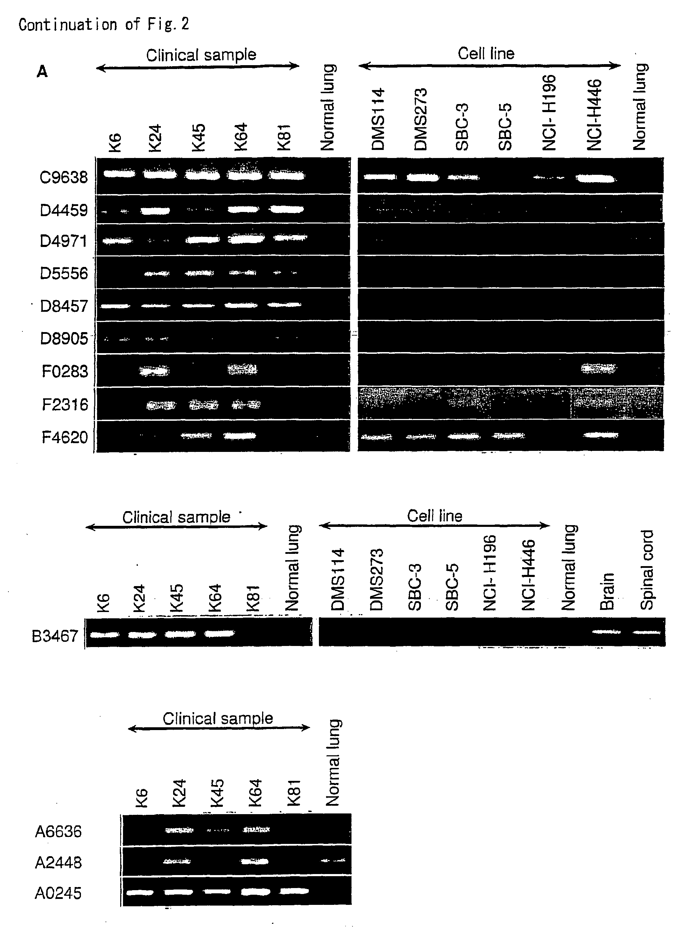

Figure 2A shows semi-quantitative RT-PCR of the 83 candidate genes, ***B*** shows immunohistochemical staining of representative samples from the SCLCs and normal lung tissue examined, using antibodies for 2 candidate protein markers, A6636(SCAMP5) and A0245(CDC20) (x 100, x 200).

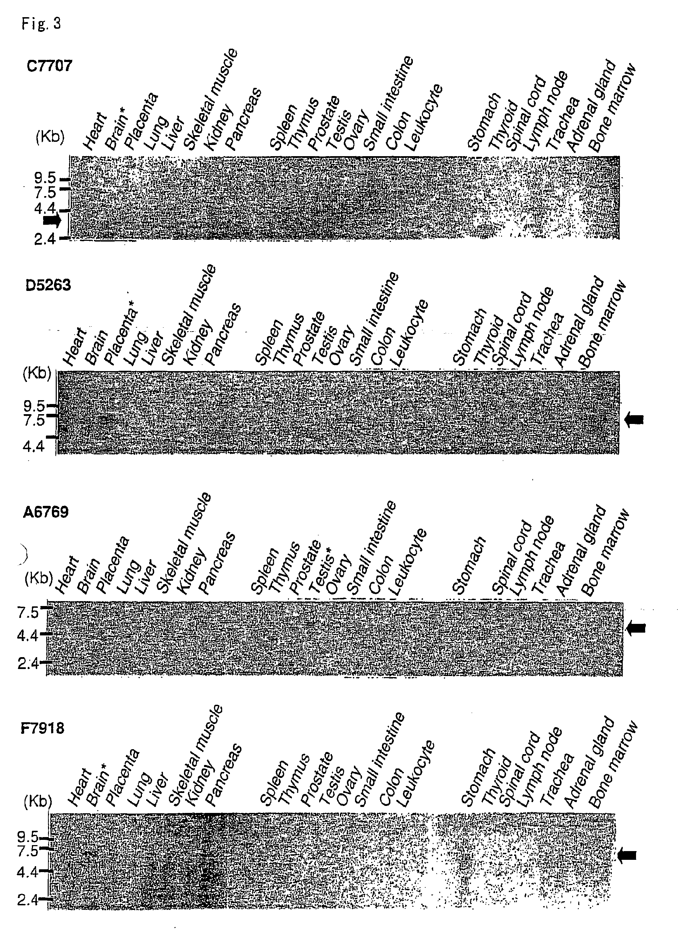

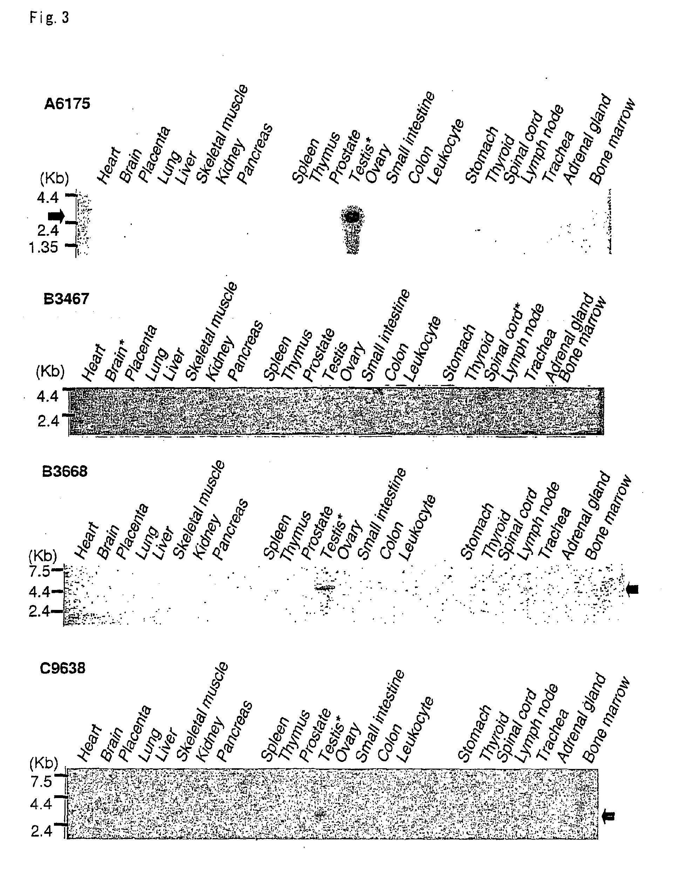

Figure 3 shows expression of the 8 candidate genes in normal organs using multiple tissue northern-blot.

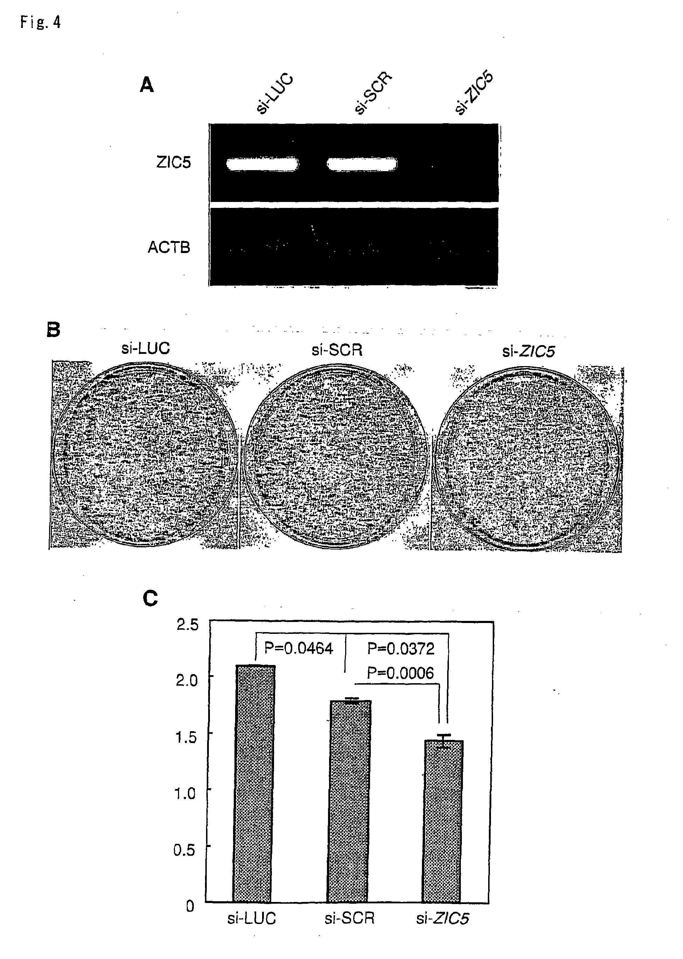

Figure 4. shows a knockdown effect of siRNA on ZIC5 in LC319 cells. A *ZIC5* siRNA expression vectors (si-*ZIC5*) and a Luciferase siRNA expression vector (si-LUC) and a Scramble siRNA expression vector (si-SCR) as negative controls were transfected into LC319 cells. ***A***, The knockdown effect on the ZIC5 transcript was validated by RT-PGR, with *ACTB* expression as a quantitative control. ***B**, **C***, si*-ZIC5* revealed strong knockdown effect, while si-LUC and si-SCR did not show any effect on the level of the *ZIC5* transcript. Transfection with si-ZIC5 vector resulted in reduction of the number of colonies (***B***), and numbers of viable cells (***C***), compared with the cells transfected with si-LUC or si-SCR.

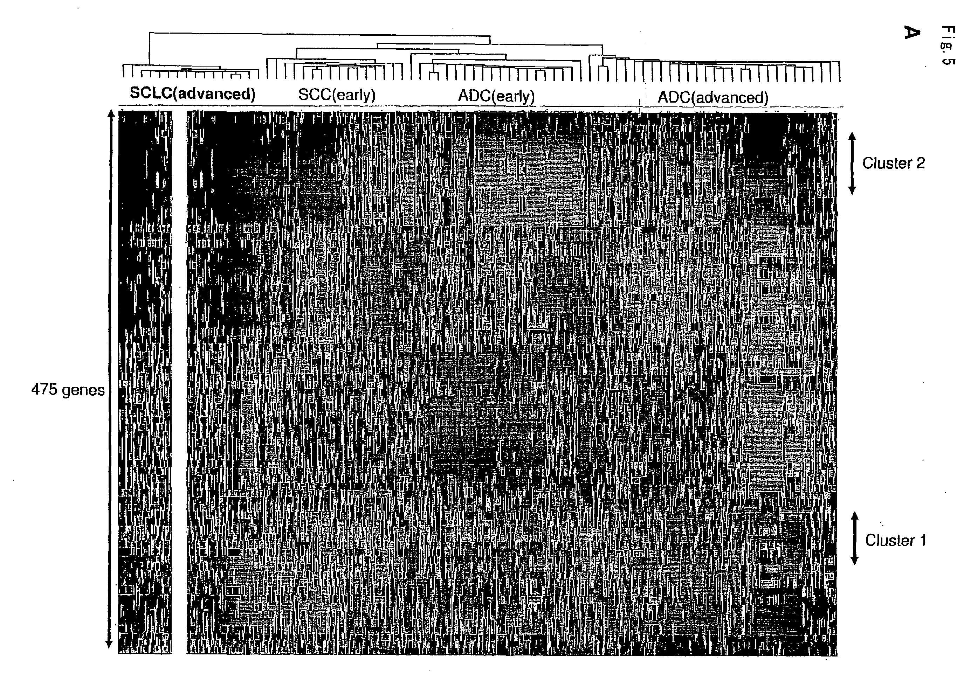

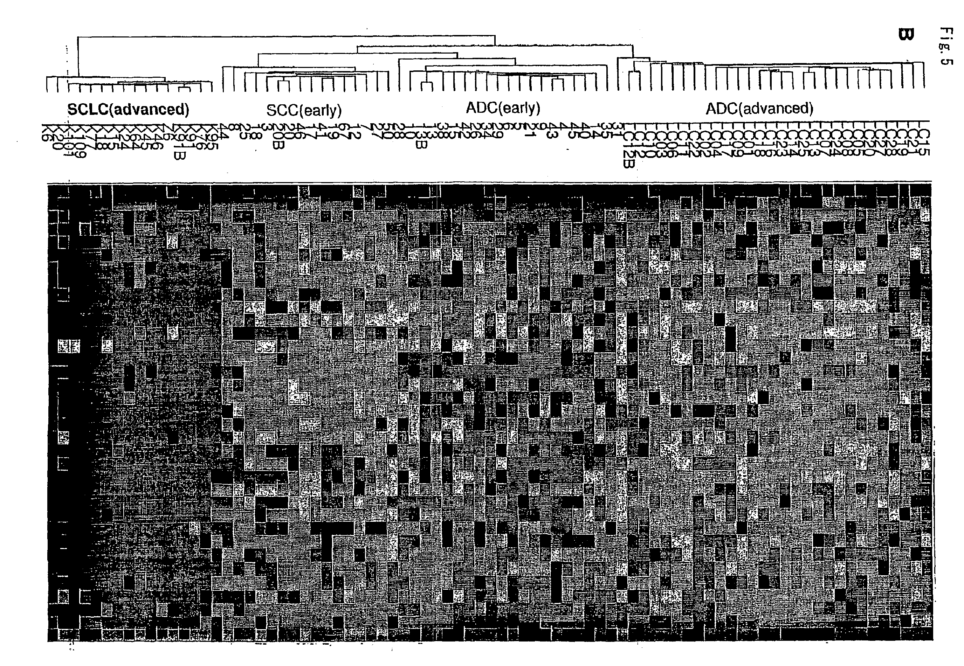

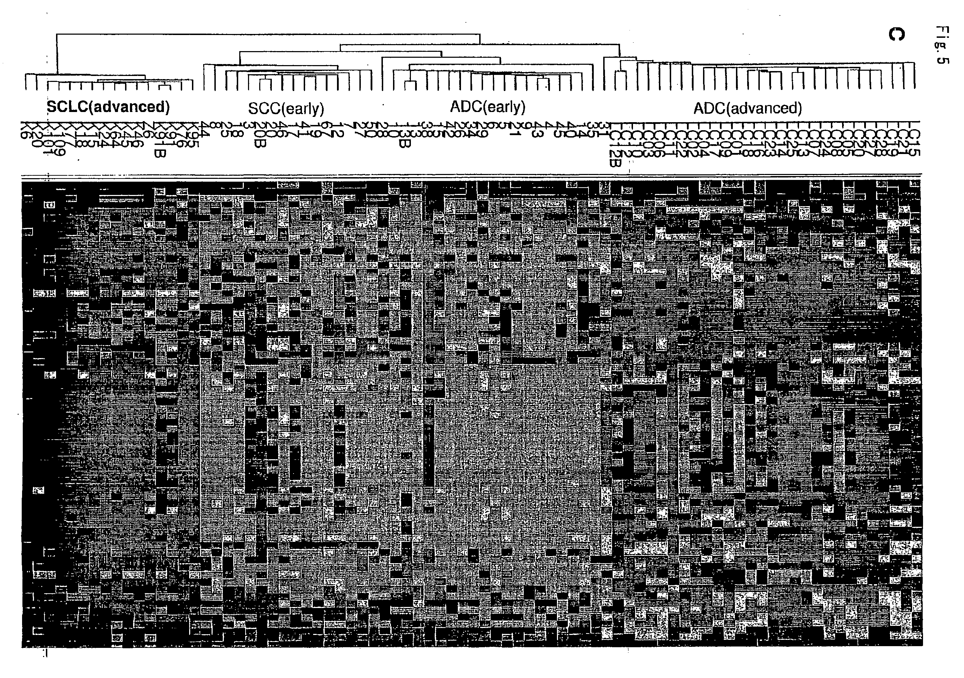

Figure 5 shows a supervised cluster analysis of SCLCs and NSCLCs (adenocarcinomas). ***A***, dendrogram of two-dimensional hierarchical clustering analysis of genes across samples from 77 lung cancer cases. The color of each well represents with red and green indicating transcript levels respectively above and below the median for that gene across all samples. Black, unchanged expression; gray, no detectable expression. In the horizontal axis representing 77 lung cancers, 15 advanced SCLCs, 35 early stage NSCLCs (20 ADCs and 15 SCCs) and 27 advanced ADCs were separated in four trunks. In the vertical axis the 475 genes were clustered in different branches according to similarities in relative expression ratio. ***B***, The cluster-1 includes 34 genes which expressed more abundantly in SCLCs than in NSCLCs. The four duplicated cases (No. 13, 20, K91, and LC12) that were labelled and hybridized in independent experiments were clustered most closely within the same group. The identical genes spotted on different positions on the slide glasses were also clustered into the adjacent rows. ***C***, The cluster-2 includes 68 genes which commonly expressed in advanced SCLCs and NSCLCs, both of which had been treated with chemotherapy.

### DETAILED DESCRIPTION OF THE INVENTION

The words "a", "an" and "the" as used herein mean "at least one" unless otherwise specifically indicated.

Generally small cell lung cancer cells exist as a solid mass having a highly inflammatory reaction and containing various cellular components. Therefore, previous published microarray data are likely to reflect heterogenous profiles.

With these issues in view, the purified populations of small cell lung cancer cells were prepared by a method of laser-microbeam microdissection (LMM), and analyzed genome-wide gene-expression profiles of 15 SCLCs, using a cDNA microarray representing 32,256 genes. These data not only provides important information about small cell lung carcinogenesis, but also facilitates the identification of candidate genes whose products serve as diagnostic markers and/or as molecular targets for treatment of patients with small cell lung cancer and providing clinically relevant information.

The present invention is based, in part, on the discovery of changes in expression patterns of multiple nucleic acids between epithelial cells and carcinomas of patients with SCLC. The differences in gene expression were identified using a comprehensive cDNA microarray system.

The gene-expression profiles of cancer cells from 15 SCLCs were analyzed using a cDNA microarray representing 32,256 genes coupled with laser microdissection. By comparing expression patterns between cancer cells from patients diagnosed with SCLC and normal cells purely selected with Laser Microdissection, 776 genes (shown in Table 2) were identified as being commonly down-regulated in SCLC cells. Similarly, 779 genes (shown in Table 3) were also identified as commonly up-regulated in SCLC cells. In addition, selection was made of candidate molecular markers useful to detect cancer-related proteins in serum or sputum of patients, and some targets useful for development of signal-suppressing strategies in human SCLC were discovered. Among them, Tables 2 and 3 provide a list of genes whose expression is altered between SCLC and normal tissue.

The differentially expressed genes identified herein find diagnostic utility as markers of SCLC and as SCLC gene targets, the expression of which can be altered to treat or alleviate a symptom of SCLC.

The genes whose expression level is modulated (*i.e.*, increased or decreased) in SCLC patients are summarized in Tables 2-3 and are collectively referred to herein as "SCLC-associated genes," "SCLC nucleic acids" or "SCLC polynucleotides" and the corresponding encoded polypeptides are referred to as "SCLC polypeptides" or "SCLC proteins." Unless indicated otherwise, "SCLC" refers to any of the sequences disclosed herein (*e.g.*, SCLC-associated genes listed in Tables 2-3). Genes that have been previously described are presented along with a database accession number.

By measuring expression of the various genes in a sample of cells, SCLC can be diagnosed. Similarly, measuring the expression of these genes in response to various agents can identify agents for treating SCLC.

The present invention involves determining (*e.g.*, measuring) the expression of at least one, and up to all the SCLC-associated genes listed in Tables 2-3. Using sequence information provided by the GenBank™ database entries for known sequences, the SCLC-associated genes can be detected and measured using techniques well known to one of ordinary skill in the art. For example, sequences within the sequence database entries corresponding to SCLC-associated genes, can be used to construct probes for detecting RNA sequences corresponding to SCLC-associated genes in, *e.g.*, Northern blot hybridization analyses. Probes typically include at least 10, at least 20, at least 50, at least 100, or at least 200 nucleotides of a reference sequence. As another example, the sequences can be used to construct primers for specifically amplifying the SCLC nucleic acid in, *e.g.*, amplification-based detection methods, for example, reverse-transcription based polymerase chain reaction.

Expression level of one or more of SCLC-associated genes in a test cell population, *e.g.*, a tissues sample from a patient, is then compared to the expression level(s) of the same gene(s) in a reference population. The reference cell population includes a plurality of cells for which the compared parameter is known, *i.e*., lung cancer cells (*e.g.,* SCLC cells) or normal lung cells.

Whether or not a pattern of gene expression in a test cell population as compared to a reference cell population indicates SCLC or a predisposition thereto depends upon the composition of the reference cell population. For example, if the reference cell population is composed of normal lung cells, a similarity in gene expression pattern between the test cell population and the reference cell population indicates the test cell population is not SCLC. Conversely, if the reference cell population is made up of SCLC cells, a similarity in gene expression profile between the test cell population and the reference cell population indicates that the test cell population includes SCLC cells.

A level of expression of an SCLC marker gene in a test cell population is considered "altered" or "to differ" if it varies from the expression level of the corresponding SCLC marker gene in a reference cell population by more than 1.1, more than 1.5, more than 2.0, more than 5.0, more than 10.0 or more fold.

Differential gene expression between a test cell population and a reference cell population can be normalized to a control nucleic acid, *e.g.* a housekeeping gene. For example, a control nucleic acid is one which is known not to differ depending on the cancerous or non-cancerous state of the cell. The expression level of a control nucleic acid can be used to normalize signal levels in the test and reference populations. Exemplary control genes include, but are not limited to, *e.g.*, β-actin, glyceraldehyde 3- phosphate dehydrogenase and ribosomal protein P1.

The test cell population can be compared to multiple reference cell populations. Each of the multiple reference populations can differ in the known parameter. Thus, a test cell population can be compared to a first reference cell population known to contain, *e.g.*, SCLC cells, as well as a second reference population known to contain, *e.g.*, normal lung cells. The test cell can be included in a tissue or cell sample from a subject known to contain, or suspected of containing, SCLC cells.

The test cell is obtained from a bodily tissue or a bodily fluid, *e.g.*, biological fluid (blood or sputum, for example). For example, the test cell can be purified from lung tissue. Preferably, the test cell population comprises an epithelial cell. The epithelial cell is preferably from a tissue known to be or suspected to be a lung cancer.

Cells in the reference cell population can be from a tissue type similar to that of the test cell. Optionally, the reference cell population is a cell line, *e.g.* an SCLC cell line (*i.e.*, a positive control) or a normal lung cell line (*i.e*., a negative control). Alternatively, the control cell population can be from a database of molecular information from cells for which the assayed parameter or condition is known.

The subject is preferably a mammal. Exemplary mammals include, but are not limited to, *e.g.*, a human, non-human primate, mouse, rat, dog, cat, horse, or cow.

Expression of the genes disclosed herein can be determined at the protein or nucleic acid level, using methods known in the art. For example, Northern hybridization analysis, using probes which specifically recognize one or more of these nucleic acid sequences can be used to determine gene expression. Alternatively, gene expression can be measured using reverse-transcription-based PCR assays, *e.g.*, using primers specific for the differentially expressed gene sequences. Expression can also be determined at the protein level, *i.e.,* by measuring the level of a polypeptides encoded by a gene described herein, or the biological activity thereof. Such methods are well known in the art and include, but are not limited to, *e.g.*, immunoassays that utilize antibodies to proteins encoded by the genes. The biological activities of the proteins encoded by the genes are generally well known. *See,* Sambrook and Russell, Molecular Cloning: A Laboratory Manual, 3rd Edition, 2001, Cold Spring Harbor Laboratory Press; Ausubel, Current Protocols in Molecular Biology, 1987-2006, John Wiley and Sons; and Harlow and Lane, Using Antibodies: A Laboratory Manual, 1998, Cold Spring Harbor Laboratory Press.

### Diagnosing small cell lung cancer:

In the context of the present invention, SCLC is diagnosed by measuring the expression level of one or more SCLC nucleic acids from a test population of cells, (*i.e.*, a biological sample from a patient). Preferably, the test cell population contains epithelial cells, *e.g.*, cells obtained from lung tissue. Gene expression can also be measured from blood or other bodily fluids, for example, saliva or sputum. Other biological samples can be used for measuring protein levels. For example, the protein level in blood or serum from a subject to be diagnosed can be measured by immunoassay or other conventional biological assay.

Expression of one or more SCLC-associated genes, *e.g.*, genes listed in Tables 2-3, is determined in the test cell population or biological sample and compared to the normal control expression level associated with the one or more SCLC-associated gene(s) assayed. A normal control level is an expression profile of one or more SCLC-associated genes typically found in a population known not to be suffering from SCLC. An alteration or difference (*e.g.*, an increase or decrease) in the level of expression in the tissue sample from a patient of one or more SCLC-associated gene indicates that the subject is suffering from or is at risk of developing SCLC. For example, a decrease in expression of one or more down-regulated SCLC-associated genes listed in Table 2 in the test population as compared to the normal control level indicates that the subject is suffering from or is at risk of developing SCLC. Conversely, an increase in the expression of one or more up-regulated SCLC-associated genes listed in Table 3 in the test population as compared to the normal control level indicates that the subject is suffering from or is at risk of developing SCLC.

Alteration of one or more of the SCLC-associated genes in the test population as compared to the normal control level indicates that the subject suffers from or is at risk of developing SCLC. For example, alteration of at least 1%, at least 5%, at least 25%, at least 50%, at least 60%, at least 70%, at least 80%, at least 90% or more of the panel of SCLC-associated genes (genes listed in Tables 2-3) indicates that the subject suffers from or is at risk of developing SCLC.

The expression levels of SCLC-associated genes in a biological sample can be estimated by quantifying mRNA corresponding to or protein encoded by SCLC-associated genes. Quantification methods for mRNA are known to those skilled in the art. For example, the levels of mRNAs corresponding to SCLC-associated genes can be estimated by Northern blotting or RT-PCR. Since the nucleotide sequences of SCLC-associated genes are known, anyone skilled in the art can design the nucleotide sequences for probes or primers to quantify SCLC-associated genes. For example, oligonucleotides comprising the nucleotide sequence listed in table 1 can be used as SCLC-associated gene specific primer sets.

Also the expression level of the SCLC-associated genes can be analyzed based on the activity or quantity of protein encoded by the genes. A method for determining the quantity of the protein encoded by SCLC-associated genes is shown below. For example, immunoassay methods are useful for the determination of proteins in biological materials. Any biological materials can be used as the biological sample for the determination of the protein or its activity so long as the marker gene (SCLC-associated genes) is expressed in the sample of a lung cancer patient. For example, epithelial cells from lung tissue. However, bodily fluids including blood and sputum also can be analyzed. On the other hand, a suitable method can be selected for the determination of the activity of a protein encoded by SCLC-associated genes according to the activity of a protein to be analyzed.

Expression levels of SCLC-associated genes in a test biological sample are estimated and compared with expression levels in a normal sample (*e.g.*, a sample from a non-diseased subject). When such a comparison shows that the expression level of the genes in the test sample is higher (SCLC-associated genes shown in table 3, *i.e.* 777-1555) or lower (SCLC-associated genes shown in table 2, *i.e.* 1-776) than those in the normal sample, the subject is judged to be affected with or predisposed to SCLC. The expression level of SCLC-associated genes in the biological samples from a normal subject and subject to be diagnosed can be determined at the same time. Alternatively, normal ranges of the expression levels can be determined by a statistical method based on the results obtained by analyzing the expression level of the genes in samples previously collected from a control group of individuals known not to have SCLC. A result obtained by comparing the sample of a subject is compared with the normal range; when the result does not fall within the normal range, the subject is judged to be affected with or is at risk of developing SCLC.

In the present invention, a diagnostic agent for diagnosing cell proliferative disease, including SCLC, is also provided. The diagnostic agent of the present invention comprises a compound that binds to a polynucleotide or a polypeptide of SCLC-associated genes. Preferably, an oligonucleotide that hybridizes to a polynucleotide of a SCLC-associated gene or an antibody that binds to a polypeptide encoded by a SCLC-associated gene can be used as such a compound.

The present methods of diagnosing SCLC can be applied for assessing the efficacy of treatment of SCLC in a subj ect. According to the method, a biological sample, including a test cell population, is obtained from a subject undergoing treatment for SCLC. The method for assessment can be conducted according to conventional methods of diagnosing SCLC.

If desired, biological samples are obtained from the subject at various time points before, during or after the treatment The expression level of SCLC-associated genes, in the test biological sample is then determined and compared to a control level, for example, from a reference cell population which includes cells whose state of SCLC (*i.e.,* cancerous cell or non-cancerous cell) is known. The control level is determined in a biological sample that has not been exposed to the treatment.

If the control level is from a biological sample which contains no cancerous cells, a similarity between the expression level in the test biological sample from a subject and the control level indicates that the treatment is efficacious. A difference between the expression level of the SCLC-associated genes in the test biological sample from a subject and the control level indicates a less favorable clinical outcome or prognosis.

### Identifying agents that inhibit or enhance SCLC-associated gene expression:

An agent that inhibits the expression of one or more SCLC-associated genes or the activity of their gene products can be identified by contacting a test cell population expressing one or more SCLC-associated up-regulated genes with a test agent and then determining the expression level of the SCLC-associated gene(s) or the activity of their gene products. A decrease in the level of expression of the one or more SCLC-associated genes or in the level of activity of its gene product in the presence of the agent as compared to the expression or activity level in the absence of the test agent indicates that the agent is an inhibitor of one or more SCLC-associated up-regulated genes and useful in inhibiting SCLC.

Alternatively, an agent that enhances the expression of one or more SCLC-associated down-regulated genes or the activity of its gene product can be identified by contacting a test cell population expressing one or more SCLC-associated genes with a test agent and then determining the expression level or activity of the SCLC-associated down-regulated gene(s). An increase in the level of expression of one or more SCLC-associated genes or in the level of activity of their gene products as compared to the expression or activity level in the absence of the test agent indicates that the test agent augments expression of one or more SCLC-associated down-regulated genes or the activity of their gene products.

The test cell population can be comprised of any cells expressing the SCLC-associated genes. For example, the test cell population can contain epithelial celsl, for example, cells from lung tissue. Furthermore, the test cell population can be an immortalized cell line from a carcinoma cell. Alternatively, the test cell population can be cells which have been transfected with one or more SCLC-associated genes or which have been transfected with a regulatory sequence (*e.g.* promoter sequence) from one or more SCLC-associated genes operably linked to a reporter gene.

The agent can be, for example, an inhibitory oligonucleotide (*e.g.*, an antisense oligonucleotide, an siRNA, a ribozyme), an antibody, a polypeptide, a small organic molecule. Screening for agents can be carried out using high throughput methods, by simultaneously screening a plurality of agents using multiwell plates (*e.g.*, 96-well, 192-well, 384-well, 768-well, 1536-well). Automated systems for high throughput screening are commercially available from, for example, Caliper Life Sciences, Hopkinton, MA. Small organic molecule libraries available for screening can be purchased, for example, from Reaction Biology Corp., Malvern, PA; TimTec, Newark, DE.

### Assessing efficacy of treatment of SCLC in a subject.

The differentially expressed SCLC-associated genes identified herein also allow for the course of treatment of SCLC to be monitored. In this method, a test cell population is provided from a subject undergoing treatment for SCLC. If desired, test cell populations are obtained from the subject at various time points, before, during, and/or after treatment. Expression of one or more of the SCLC-associated genes in the test cell population is then determined and compared to a reference cell population which includes cells whose SCLC state is known. In the context of the present invention, the reference cell population has not been exposed to the treatment of interest.

If the reference cell population contains no SCLC cells, a similarity in the expression of one or more SCLC-associated genes in the test cell population and the reference cell population indicates that the treatment of interest is efficacious. However, a difference in the expression of one or more SCLC-associated genes in the test cell population and a normal control reference cell population indicates a less favorable clinical outcome or prognosis. Similarly, if the reference cell population contains SCLC cells, a difference between the expression of one or more SCLC-associated genes in the test cell population and the reference cell population indicates that the treatment of interest is efficacious, while a similarity in the expression of one or more SCLC-associated genes in the test population and a small cell lung cancer control reference cell population indicates a less favorable clinical outcome or prognosis.

Additionally, the expression level of one or more SCLC-associated genes determined in a biological sample from a subject obtained after treatment (*i.e.*, post-treatment levels) can be compared to the expression level of the one or more SCLC-associated genes determined in a biological sample from a subject obtained prior to treatment onset (*i.e*., pretreatment levels). If the SCLC-associated gene is an up-regulated gene, a decrease in the expression level in a post-treatment sample indicates that the treatment of interest is efficacious while an increase or maintenance in the expression level in the post-treatment sample indicates a.less favorable clinical outcome or prognosis. Conversely, if the SCLC-associated gene is a down-regulated gene, an increase in the expression level in a post-treatment sample indicates that the treatment of interest is efficacious while a decrease or maintenance in the expression level in the post-treatment sample indicates a less favorable clinical outcome or prognosis.

As used herein, the term "efficacious" indicates that the treatment leads to a reduction in the expression of a pathologically up-regulated gene, an increase in the expression of a pathologically down-regulated gene or a decrease in size, prevalence, or metastatic potential of lung cancer in a subject. When a treatment of interest is applied prophylactically, the term "efficacious" means that the treatment retards or prevents a small cell lung cancer from forming or retards, prevents, or alleviates a symptom of clinical SCLC. Assessment of small cell lung tumors can be made using standard clinical protocols.

In addition, efficaciousness can be determined in association with any known method for diagnosing or treating SCLC. SCLC can be diagnosed, for example, by identifying symptomatic anomalies, *e.g.*, weight loss, abdominal pain, back pain, anorexia, nausea, vomiting and generalized malaise, weakness, and jaundice.

### Selecting a therapeutic agent for treating SCLC that is appropriate for a particular individual:

Differences in the genetic makeup of individuals can result in differences in their relative abilities to metabolize various drugs. An agent that is metabolized in a subject to act as an anti-SCLC agent can manifest itself by inducing a change in a gene expression pattern in the subject's cells from that characteristic of a cancerous state to a gene expression pattern characteristic of a non-cancerous state. Accordingly, the differentially expressed SCLC-associated genes disclosed herein allow for a therapeutic or prophylactic inhibitor of SCLC to be tested in a test cell population from a selected subject in order to determine if the agent is a suitable inhibitor of SCLC in the subject.

To identify an inhibitor of SCLC that is appropriate for a specific subject, a test cell population from the subject is exposed to a therapeutic agent, and the expression of one or more of SCLC-associated genes listed in Tables 2-3 is determined.

In the context of the methods of the present invention, the test cell population' contains SCLC cells expressing one or more SCLC-associated genes. Preferably, the test cell population contains epithelial cells. For example, a test cell population can be incubated in the presence of a candidate agent and the pattern of gene expression (*i.e.*, expression profile) of the test cell population can be measured and compared to one or more reference expression profiles, *e.g.*, an SCLC reference expression profile or a non-SCLC reference expression profile.

A decrease in expression of one or more of the SCLC-associated genes listed in Table 3 or an increase in expression of one or more of the SCLC-associated genes listed in Table 2 in a test cell population relative to expression in a reference cell population containing SCLC indicates that the agent has therapeutic use.

In the context of the present invention, the test agent can be any compound or composition. Exemplary test agents include, but are not limited to, immunomodulatory agents (*e.g.*, antibodies), inhibitory oligonuceotides (*e.g.*, antisense oligonucleodies, short-inhibitory oligonucleotides and ribozymes) and small organic compounds.

### Screening assays for identifying therapeutic agents:

The differentially expressed SCLC-associated genes disclosed herein can also be used to identify candidate therapeutic agents for treating SCLC. The methods of the present invention involve screening a candidate therapeutic agent to determine if the test agent can convert an expression profile of one or more SCLC-associated genes, including SCLC 1-1555, characteristic of an SCLC state to a gene expression pattern characteristic of an SCLC state. ,

In the present invention, SCLC 1-1555 are useful for screening of therapeutic agents for treating or preventing SCLC.

In one embodiment, a test cell population is exposed to a test agent or a plurality of test agents (sequentially or in combination) and the expression of one or more of SCLC 1-1555 in the cells is measured. The expression profile of the SCLC-associated gene(s) assayed in the test cell population is compared to expression profile of the same SCLC-associated gene(s) in a reference cell population that is not exposed to the test agent.

An agent capable of stimulating the expression of an under-expressed gene or suppressing the expression of an overexpressed gene has clinical benefit. Such agents can be further tested for the ability to prevent SCLC in animals or test subjects.

In a further embodiment, the present invention provides methods for screening candidate agents which are useful agents in the treatment of SCLC. As discussed in detail above, by controlling the expression levels of one or more marker genes or the activities of their gene products, one can control the onset and progression of SCLC. Thus, candidate agents, which are useful agents in the treatment of SCLC, can be identified through screening methods that use such expression levels and activities of as indices of the cancerous or non-cancerous state. In the context of the present invention, such screening can comprise, for example, the following steps:
a) contacting a test compound with a polypeptide encoded by a polynucleotide selected from the group consisting of SCLC 1-1555,
b) detecting the binding activity between the polypeptide and the test compound; and
c) selecting the test compound that binds to the polypeptide.

The one or more SCLC polypeptides encoded by the marker genes to be used for screening can be a recombinant polypeptide or a protein from the nature or a partial peptide thereof. The polypeptide to be contacted with a test compound can be, for example, a purified polypeptide, a soluble protein, a form bound to a carrier or a fusion protein fused with other polypeptides.

Many methods are known to those skilled in the art can be used for screening for proteins that bind to the one or more SCLC polypeptides encoded by the marker genes. Screening can be conducted by, for example, immunoprecipitation methods, specifically, in the following manner. The one or more marker genes are expressed in host (*e.g.*, animal) cells and so on by inserting the gene to an expression vector for foreign genes, for example, pSV2neo, pcDNA I, pcDNA3.1, pCAGGS and pCD8. The promoter to be used for the expression can be any promoter that can be used commonly and include, for example, the SV40 early promoter (Rigby in Williamson (ed.), (1982) Genetic Engineering, vol. 3. Academic Press, London, 83-141.), the EF-α promoter (Kim et al., (1990) Gene 91: 217-23.), the CAG promoter (Niwa et al., (1991) Gene 108: 193-9.), the RSV LTR promoter (Cullen, (1987) Methods in Enzymology 152: 684-704.) the SRα promoter (Takebe et al., (1988) Mol Cell Biol 8: 466-72.), the CMV immediate early promoter (Seed and Aruffo, (1987) Proc Natl Acad Sci USA 84: 3365-9.), the SV40 late promoter (Gheysen and Fiers, (1982) J Mol Appl Genet 1: 385-94.), the Adenovirus late promoter (Kaufman et al., (1989) Mol Cell Biol 9: 946-58.), the HSV TK promoter and so on. The introduction of the gene into host cells to express a foreign gene can be performed according to any methods, for example, the electroporation method (Chu et al., (1987) Nucleic Acids Res 15: 1311-26.), the calcium phosphate method (Chen and Okayama, (1987) Mol Cell Biol 7: 2745-52.), the DEAE dextran method (Lopata et al., (1984) Nucleic Acids Res 12: 5707-17.; Sussman and Milman, (1984) Mol Cell Biol 4: 1641-3.), the Lipofectin method (Derijard B, (1994) Cell 76: 1025-37.; Lamb et al., (1993) Nature Genetics 5: 22-30.: Rabindran et al., (1993) Science 259: 230-4.) and so on. The one or more SCLC polypeptides encoded by the marker genes can be expressed as a fusion protein comprising a recognition site (epitope) of a monoclonal antibody by introducing the epitope of the monoclonal antibody, whose specificity has been revealed, to the N- or C- terminus of the polypeptide. A commercially available epitope-antibody system can be used (Experimental Medicine 13: 85-90 (1995)). Vectors which can express a fusion protein with, for example, β-galactosidase, maltose binding protein, glutathione S-transferase, green florescence protein (GFP) and so on by the use of its multiple cloning sites are commercially available.

A fusion protein prepared by introducing only small epitopes consisting of several to a dozen amino acids so as not to change the property of the polypeptide by the fusion is also reported. Epitopes, including polyhistidine (His-tag), influenza aggregate HA, human c-myc, FLAG, Vesicular stomatitis virus glycoprotein (VSV-GP), T7 gene 10 protein (T7-tag), human simple herpes virus glycoprotein (HSV-tag), E-tag (an epitope on monoclonal phage) and such, and monoclonal antibodies recognizing them can be used as the epitope-antibody system for screening proteins binding to the polypeptide encoded by marker genes (Experimental Medicine 13: 85-90 (1995)).

In immunoprecipitation, an immune complex is formed by adding these antibodies to cell lysate prepared using an appropriate detergent The immune complex consists of an SCLC polypeptide encoded by the marker genes, a polypeptide comprising the binding ability with the polypeptide, and an antibody. Immunoprecipitation can be also conducted using antibodies against an SCLC polypeptide encoded by the marker genes, besides using antibodies against the above epitopes, which antibodies can be prepared as described above.

An immune complex can be precipitated, for example by Protein A sepharose or Protein G sepharose when the antibody is a mouse IgG antibody. If the one or more SCLC polypeptides encoded by the marker genes are prepared as a fusion protein with an epitope, for example GST, an immune complex can be formed in the same manner as in the use of the antibody against the polypeptide, using a substance specifically binding to these epitopes, for example, glutathione-Sepharose 4B.

Immunoprecipitation can be performed by following or according to, for example, the methods in the literature (Harlow and Lane, Antibodies, 511-52, Cold Spring Harbor Laboratory publications, New York (1988); and Harlow and Lane, Using Antibodies, Cold Spring Harbor Laboratory, New York (1998)).

SDS-PAGE is commonly used for analysis of immunoprecipitated proteins and the bound protein can be analyzed by the molecular weight of the protein using gels with an appropriate concentration. Since the protein bound to a SCLC polypeptide encoded by the marker genes is difficult to detect by a common staining method, for example, Coomassie staining or silver staining, the detection sensitivity for the protein can be improved by culturing cells in culture medium containing radioactive isotope, ³⁵S-methionine or ³⁵S-cystein, labeling proteins in the cells, and detecting the proteins. The target protein can be purified directly from the SDS-polyacrylamide gel and its sequence can be determined, when the molecular weight of a protein has been revealed.

As a method for screening proteins binding to a SCLC polypeptide encoded by the marker genes using the polypeptide, for example, West-Western blotting analysis (Skolnik et al., (1991) Cell 65: 83-90.) can be used. Specifically, a protein binding to a SCLC polypeptide encoded by the marker genes can be obtained by preparing a cDNA library from cells, tissues, organs (for example, tissues including testis or ovary), or cultured cells (*e.g.*, DMS 114, DMS273, SBC-3, SBC-5, NCI-H196, and NCI-H446) expected to express a protein binding to a SCLC polypeptide encoded by the marker genes using a phage vector (*e.g.*, ZAP), expressing the protein on LB-agarose, fixing the protein expressed on a filter, reacting the purified and the labeled polypeptide with the above filter, and detecting the plaques expressing proteins bound to the polypeptide encoded by the marker genes according to the label. The one or more SCLC polypeptides encoded by the marker genes can be labeled by utilizing the binding between biotin and avidin, or by utilizing an antibody that specifically binds to a SCLC polypeptide encoded by the marker genes, or a peptide or polypeptide (for example, GST) that is fused to a SCLC polypeptide encoded by the marker genes. Methods using radioisotope or fluorescence and such can be also used.

Alternatively, in another embodiment of the screening methods of the present invention, a two-hybrid system utilizing cells can be used ("MATCHMAKER Two-Hybrid system", "Mammalian MATCHMAKER Two-Hybrid Assay Kit", "MATCHMAKER one-Hybrid system" (Clontech); "HybriZAP Two-Hybrid Vector System" (Stratagene); the references "Dalton and Treisman, (1992) Cell 68: 597-612.", "Fields and Sternglanz, (1994) Trends Genet 10: 286-92.").

In the two-hybrid system, the polypeptide of the invention is fused to the SRF-binding region or GAL4-binding region and expressed in yeast cells. A cDNA library is prepared from cells expected to express a protein binding to the polypeptide of the invention, such that the library, when expressed, is fused to the VP 16 or GAL4 transcriptional activation region. The cDNA library is then introduced into the above yeast cells and the cDNA from the library is isolated from the positive clones detected (when a protein binding to the polypeptide of the invention is expressed in yeast cells, the binding of the two activates a reporter gene, making positive clones detectable). A protein encoded by the cDNA can be prepared by introducing the cDNA isolated above to *E*. *coli* and expressing the protein.

As a reporter gene, for example, Ade2 gene, lacZ gene, CAT gene, luciferase gene and such can be used in addition to the HIS3 gene.

A compound binding to one or more SCLC polypeptides encoded by the marker genes can also be screened using affinity chromatography. For example, an SCLC polypeptide encoded by the marker genes can be immobilized on a carrier of an affinity column, and a test compound, containing a protein capable of binding to a SCLC polypeptide encoded by the marker genes, is applied to the column. A test compound herein can be, for example, cell extracts, cell lysates, *etc.* After loading the test compound, the column is washed, and compounds bound to the polypeptide can be prepared.

When the test compound is a protein, the amino acid sequence of the obtained protein is analyzed, an oligo DNA is synthesized based on the sequence, and cDNA libraries are screened using the oligo DNA as a probe to obtain a DNA encoding the protein.

A biosensor using the surface plasmon resonance phenomenon can be used as a mean for detecting or quantifying the bound compound in the present invention. When such a biosensor is used, the interaction between the polypeptide of the invention and a test compound can be observed real-time as a surface plasmon resonance signal, using only a minute amount of polypeptide and without labeling (for example, BIAcore, Pharmacia). Therefore, it is possible to evaluate the binding between one or more SCLC polypeptides encoded by the marker genes and a test compound using a biosensor, for example, BIAcore.

The methods of screening for molecules that bind when the immobilized SCLC polypeptides encoded by the marker genes are exposed to synthetic chemical compounds, or natural substance banks or a random phage peptide display library, and the methods of screening using high-throughput based on combinatorial chemistry techniques (Wrighton et al., (1996) Science 273: 458-64.; Verdine, (1996) Nature 384: 11-3.) to isolate not only proteins but chemical compounds that bind to the protein (including agonist and antagonist) are well known to one skilled in the art.

Alternatively, the present invention provides methods of screening for a compound for treating or preventing SCLC using one or more polypeptides encoded by the marker genes comprising the steps as follows:
- a): contacting a test compound with a polypeptide encoded by a polynucleotide selected from the group consisting of SCLC 1-1555;
- b): detecting the biological activity of the polypeptide of step (a); and
- c): selecting a compound that i) suppresses the biological activity of the polypeptide encoded by the polynucleotide selected from the group consisting of SCLC 777- 1555 as compared to the biological activity detected in the absence of the test compound, or ii) enhances the biological activity of the polypeptide encoded by the polynucleotide selected from the group consisting of SCLC 1-776 as compared to the biological activity detected in the absence of the test compound.

A polypeptide for use in the screening methods of the present invention can be obtained as a recombinant protein using the nucleotide sequence of the marker gene. Any polypeptides can be used for screening so long as they comprise the biological activity of the polypeptide encoded by the marker genes. Based on the information regarding the marker gene and its encoded polypeptide, one skilled in the art can select any biological activity of the polypeptide as an index for screening and any suitable measurement method to assay for the selected biological activity.

The compound isolated by this screening is a candidate for agonists or antagonists of the polypeptide encoded by the marker genes. The term "agonist" refers to molecules that activate the function of the polypeptide by binding thereto. Likewise, the term "antagonist" refers to molecules that inhibit the function of the polypeptide by binding thereto. Moreover, a compound isolated by this screening will inhibit or stimulate the *in vivo* interaction of the polypeptide encoded by the marker genes with molecules (including DNAs and proteins).

When the biological activity to be detected in the present method is cell proliferation, it can be detected, for example, by preparing cells which express the polypeptide encoded by the marker genes, culturing the cells in the presence of a test compound, and determining the speed of cell proliferation, measuring the cell cycle and such, as well as by measuring the colony forming activity as described in the Examples.

In a further embodiment, the present invention provides methods for screening compounds for treating or preventing SCLC. As discussed in detail above, by controlling the expression levels of the marker genes, one can control the onset and progression of SCLC. Thus, compounds that can be used in the treatment or prevention of SCLC can be identified through screenings that use the expression levels of marker genes as indices. In the context of the present invention, such screening can comprise, for example, the following steps:
- a): contacting a candidate compound with a cell expressing one or more marker genes, wherein the one or more marker genes are selected from the group consisting of SCLC 1-1555; and
- b): selecting the candidate compound that reduces the expression level of one or more marker genes selected from the group consisting of SCLC 777-1555, or elevates the expression level of one or more marker genes selected from the group consisting of SCLC 1-776, as compared to an expression level dtected in the absence of the candidate compound.

Cells expressing a marker gene include, for example, cell lines established from SCLC; such cells can be used for the above screening of the present invention (*e.g.*, DMS114, DMS273, SBC-3, SBC-5, NCI-H196, and NCI-H446). The expression level can be estimated by methods well known to one skilled in the art. In the methods of screening, compounds that reduce or enhance the expression level of one or more marker genes find use for the treatment or prevention of SCLC.

Alternatively, the screening methods of the present invention can comprise the following steps:
- a): contacting a candidate compound with a cell into which a vector comprising the transcriptional regulatory region of one or more marker genes and a reporter gene that is expressed under the control of the transcriptional regulatory region has been introduced, wherein the one or more marker genes are selected from the group consisting of SCLC 1-1555
- b): measuring the expression or activity of said reporter gene; and
- c): selecting the candidate compound that reduces the expression or activity level of said reporter gene when said one or more marker genes are up-regulated marker genes selected from the group consisting of SCLC 777-1555 as compared to an expression level in the absence of the candidate compound, or that enhances the expression level of said reporter gene when said one or more marker genes are down-regulated marker genes selected from the group consisting of SCLC 1-776, as compared to an expression level detected in the absence of the candidate compound.

Suitable reporter genes and host cells are well known in the art. The reporter construct required for the screening can be prepared by using the transcriptional regulatory region of a marker gene. When the transcriptional regulatory region of a marker gene has been known to those skilled in the art, a reporter construct can be prepared by using the previous sequence information. When the transcriptional regulatory region of a marker gene remains unidentified, a nucleotide segment containing the transcriptional regulatory region can be isolated from a genome library based on the nucleotide sequence information of the marker gene.

Examples of supports that can be used for binding proteins include insoluble polysaccharides, including agarose, cellulose and dextran; and synthetic resins, including polyacrylamide, polystyrene and silicon; preferably commercially available beads and plates (*e.g.*, multi-well plates, biosensor chip, *etc*.) prepared from the above materials can be used. When using beads, they can be filled into a column.

The binding of a protein to a support can be conducted according to routine methods, for example, chemical bonding and physical adsorption. Alternatively, a protein can be bound to a support via antibodies specifically recognizing the protein. Moreover, binding of a protein to a support can be also conducted by means of avidin and biotin.

The binding between proteins is carried out in buffer, for example, but are not limited to, phosphate buffer and Tris buffer, as long as the buffer does not inhibit the binding between the proteins.

In the present invention, a biosensor using the surface plasmon resonance phenomenon can be used as a mean for detecting or quantifying the bound protein. When such a biosensor is used, the interaction between the proteins can be observed real-time as a surface plasmon resonance signal, using only a minute amount of polypeptide and without labeling (for example, BIAcore, Pharmacia).

Alternatively, polypeptide encoded by the marker genes can be labeled, and the label of the bound protein can be used to detect or measure the bound protein. Specifically, after pre-labeling one of the proteins, the labeled protein is contacted with the other protein in the presence of a test compound, and then bound proteins are detected or measured according to the label after washing.

Labeling substances for example radioisotope (*e.g.*, ³H, ¹⁴C, ³²P, ³³P ³⁵S ¹²⁵I, ¹³¹I), enzymes (*e.g.*, alkaline phosphatase, horseradish peroxidase, β-galactosidase, β-glucosidase), fluorescent substances (*e.g.*, fluorescein isothiosyanete (FITC), rhodamine) and biotin/avidin, can be used for the labeling of a protein in the present method. When the protein is labeled with radioisotope, the detection or measurement can be carried out by liquid scintillation. Alternatively, proteins labeled with enzymes can be detected or measured by adding a substrate of the enzyme to detect the enzymatic change of the substrate, for example, detecting the generation of color, with absorptiometer. Further, in case where a fluorescent substance is used as the label, the bound protein can be detected or measured using fluorophotometer.

In case of using an antibody in the present screening, the antibody is preferably labeled with one of the labeling substances mentioned above, and detected or measured based on the labeling substance. Alternatively, the antibody against the polypeptide encoded by the marker genes or actin can be used as a primary antibody to be detected with a secondary antibody that is labeled with a labeling substance. Furthermore, the antibody bound to the protein in the screening of the present invention can be detected or measured using protein G or protein A column.

Any test compound, for example, cell extracts, cell culture supernatant, products of fermenting microorganism, extracts from marine organism, plant extracts, purified or crude proteins, peptides, non-peptide compounds, synthetic micromolecular compounds and natural compounds can be used in the screening methods of the present invention. In the present invention, the test compound can be also obtained using any of the numerous approaches in combinatorial library methods known in the art, including (1) biological libraries, (2) spatially addressable parallel solid phase or solution phase libraries, (3) synthetic library methods requiring deconvolution, (4) the "one-bead one-compound" library method and (5) synthetic library methods using affinity chromatography selection. The biological library methods using affinity chromatography selection is limited to peptide libraries, while the other four approaches are applicable to peptide, non-peptide oligomer or small molecule libraries of compounds (Lam (1997) Anticancer Drug Des. 12: 145-67). Examples of methods for the synthesis of molecular libraries can be found in the art (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Hem. Int. Ed. Engl. 33: 2061; Gallop et al. (1994) J. Med. Chem. 37: 1233-51). Libraries of compounds can be presented in solution (see Houghten (1992) Bio/Techn.iques 13: 412-21) or on beads (Lam (1991) Nature 354: 82-4), chips (Fodor (1993) Nature 364: 555-6), bacteria (US Pat. No. 5,223,409), spores (US Pat. No. 5,571,698; 5,403,484, and 5,223,409), plasmids (Cull et al. (1992) Proc. Natl. Acad. Sci. USA 89: 1865-9) or phage (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; US Pat. Application 2002103360).

A compound isolated by the screening methods of the present invention can be used to inhibit or stimulate the activity of one or more SCLC polypeptides encoded by the marker genes, for treating or preventing diseases attributed to, for example, cell proliferative diseases, for example, SCLC. A compound in which a part of the structure of the compound obtained by the present screening methods of the present invention is converted by addition, deletion and/or replacement, is included in the compounds obtained by the screening methods of the present invention.

### Pharmaceutical compositions for treating or preventing SCLC

The present invention provides compositions for treating or preventing SCLC comprising any of the compounds selected by the screening methods of the present invention.

When administrating a compound isolated by the methods of the present invention as a pharmaceutical for humans and other mammals, including mice, rats, guinea-pigs, rabbits, cats, dogs, sheep, pigs, cattle, monkeys, baboons, and chimpanzees, the isolated compound can be directly administered or can be formulated into a dosage form using known pharmaceutical preparation methods. For example, according to the need, the drugs can be taken orally, as sugar-coated tablets, capsules, elixirs and microcapsules, or non-orally, in the form of injections of sterile solutions or suspensions with water or any other pharmaceutically acceptable liquid. For example, the compounds can be mixed with pharmaceutically acceptable carriers or media, specifically, sterilized water, physiological saline, plant-oils, emulsifiers, suspending agents, surfactants, stabilizers, flavoring agents, excipients, vehicles, preservatives, binders, and such, in a unit dose form required for generally accepted drug implementation. The amount of active ingredient contained in such a preparation makes a suitable dosage within the indicated range acquirable.

Examples of additives that can be admixed into tablets and capsules include, but are not limited to, binders, including gelatin, corn starch, tragacanth gum and arabic gum; excipients, including crystalline cellulose; swelling agents, including corn starch, gelatin and alginic acid; lubricants, including magnesium stearate; sweeteners, including sucrose, lactose or saccharin; and flavoring agents, including peppermint, Gaultheria adenothrix oil and cherry. When the unit-dose form is a capsule, a liquid carrier, including an oil, can be further included in the above ingredients. Sterile composites for injection can be formulated following normal drug implementations using vehicles, including distilled water, suitable for injection.

Physiological saline, glucose, and other isotonic liquids, including adjuvants, including D-sorbitol, D-mannose, D-mannitol, and sodium chloride, can be used as aqueous solutions for injection. These can be used in conjunction with suitable solubilizers, including alcohol, for example, ethanol; polyalcohols, including propylene glycol and polyethylene glycol; and non-ionic surfactants, including Polysorbate 80 (TM) and HCO-50.

Sesame oil or soy-bean oil can be used as an oleaginous liquid, can be used in conjunction with benzyl benzoate or benzyl alcohol as a solubilizer, and can be formulated with a buffer, including phosphate buffer and sodium acetate buffer; a pain-killer, including procaine hydrochloride; a stabilizer, including benzyl alcohol and phenol; and/or an antioxidant A prepared injection can be filled into a suitable ampoule.

Methods well known to those skilled in the art can be used to administer the pharmaceutical composition of the present invention to patients, for example as an intraarterial, intravenous, or percutaneous injection or as an intranasal, transbronchial, intramuscular or oral administration. The dosage and method of administration vary according to the body-weight and age of a patient and the administration method; however, one skilled in the art can routinely select a suitable method of administration. If said compound is encodable by a DNA, the DNA can be inserted into a vector for gene therapy and the vector administered to a patient to perform the therapy. The dosage and method of administration vary according to the body-weight, age, and symptoms of the patient; however, one skilled in the art can suitably select them.

For example, although the dose of a compound that binds to a protein of the present invention and regulates its activity depends on the symptoms, the dose is generally about 0.1 mg to about 100 mg per day, preferably about 1.0 mg to about 50 mg per day and more preferably about 1.0 mg to about 20 mg per day, when administered orally to a normal adult human (weighing about 60 kg).

When administering the compound parenterally, in the form of an injection to a normal adult human (weighing about 60 kg), although there are some differences according to the patient, target organ, symptoms and method of administration, it is convenient to intravenously inject a dose of about 0.01 mg to about 30 mg per day, preferably about 0.1 to about 20 mg per day and more preferably about 0.1 to about 10 mg per day. In the case of other animals, the appropriate dosage amount can be routinely calculated by converting to 60 kgs of body-weight.

### Assessing the prognosis of a subject with small cell lung cancer:

The present invention also provides methods of assessing the prognosis of a subject with SCLC including the step of comparing the expression of one or more SCLC-associated genes in a test cell population to the expression of the same SCLC-associated genes in a reference cell population from patients over a spectrum of disease stages. By comparing the gene expression of one or more SCLC-associated genes in the test cell population and the reference cell population(s), or by comparing the pattern of gene expression over time in test cell populations from the subject, the prognosis of the subject can be assessed.

For example, an increase in the expression of one or more of up-regulated SCLC-associated genes, including those listed in Table 3, as compared to expression in a normal control or a decrease in the expression of one or more of down-regulated SCLC-associated genes, including those listed in Table 2, as compared to expression in a normal control indicates less favorable prognosis. Conversely, a similarity in the expression of one or more of SCLC-associated genes listed in Tables 2-3 as compared to expression in a normal control indicates a more favorable prognosis for the subject. Preferably, the prognosis of a subject can be assessed by comparing the expression profile of the one or more genes selected from the group consisting of genes listed in Tables 2 and 3.

### Discriminating the SCLC and NSCLC

Also provided are methods of discriminating the SCLC and NSCLC by comparing the expression level of one or more marker gene listed in table 4. An increase in expression level of one or more marker gene listed in table 4 compared to an expression level of NSCLC indicates that the subject is suffering from SCLC. In the context of the present invention, the phrase "expression level of NSCLC" refers to an expression level of one or more marker gene or protein encoded thereby detected in a sample from a NSCLC patient. In some embodiments, the expression level of NSCLC serves as control level. The control level can be a single expression pattern from a single reference population or from a plurality of expression patterns. For example, the control level can be a database of expression patterns from previously tested NSCLC control samples. In the present invention, a preferable NSCLC control sample can be NSCLC tissue or cells identified with a standard diagnostic method *e.g.* histopathological test. Alternatively, an "expression level of NSCLC" refers to a level of gene expression detected in a sample from a NSCLC patient or in samples from a population of individuals known to be suffering from NSCLC.

### Identification of Genes Related to Chemoresistance.

In the present invention, up-regulated genes in advanced SCLC and advanced NSCLC were identified, and compared with chemotherapy-sensitive lung cancer. These chemotherapy-resistant lung cancer associated genes are listed in Table 5 (SLC 1590 to 1657). One or more of SLC 1590-1657 are useful for diagnostic marker to determine chemoresistant lung cancer including SCLC and NSCLC.

Accordingly, in some embodiments, the present invention provides methods of diagnosing chemotherapy resistant lung cancer or a predisposition for developing chemotherapy resistant lung cancer in a subject, comprising determining a level of expression of one or more chemotherapy resistant lung cancer-associated genes selected from the group consisting of the genes listed in Table 5 in a biological sample from a patient, wherein an increase in said sample expression level as compared to a control level of said gene indicates that said subject suffers from or is at risk of developing chemotherapy resistant lung cancer. In the present invention, the control level can be obtained from chemotherapy sensitive lung cancer sample. For example, the control level can be determined from an expression profile of SLC 1590 to 1657 in a cell or tissue obtained from chemotherapy sensitive lung cancer subject. Alternatively, a cell or tissue obtained from chemotherapy sensitive lung cancer can be used as a control sample.

The chemotherapy-resistant lung cancer associated genes listed in Table 5 are also useful as therapeutic targets for treating or preventing chemoresistant lung cancer. Thus, the present invention further provides methods of screening for a compound for treating or preventing chemotherapy resistant lung cancer. Alternatively, the present invention also provides methods of treating or preventing chemotherapy resistant lung cancer in a subject. In the present invention, the screening or therapeutic method for the SCLC described in the specification can be applied to those for chemotherapy resistant lung cancer, using one or more of SLC 1590 to 1657 as target genes.

For instance, the therapeutic methods of the present invention can include the step of decreasing the expression, activity, or both, of one or more gene products of genes whose expression is aberrantly increased ("up-regulated" or "over-expressed" gene) in chemotherapy resistant lung cancer. Expression can be inhibited in any of several ways known in the art. For example, expression can be inhibited by administering to the subject a nucleic acid that inhibits, or antagonizes the expression of the over-expressed gene or genes, *e.g.*, an antisense oligonucleotide or small interfering RNA which disrupts expression of the over-expressed gene or genes.

Furthermore, in the present invention, up-regulated genes in advanced SCLC compared with chemotherapy-sensitive SCLC were identified. These chemotherapy-resistant SCLC associated genes are listed in Table 6 (SLC 1658 to 1663). The genes listed in table 6 are selected from up-regulated genes listed in table 3, as chemotherapy-resistant SCLC associated genes. SCLC 1658 to 1663 are useful for diagnostic marker to determine chemoresistant SCLC.

Accordingly, in some embodiments, the present invention provides methods of diagnosing chemotherapy resistant SCLC or a predisposition for developing chemotherapy resistant SCLC in a subject, comprising determining a level of expression of one or more chemotherapy resistant lung cancer-associated genes selected from the group consisting of the genes listed in Table 6 in a biological sample from a patient, wherein an increase in said sample expression level as compared to a control level of said gene indicates that said subject suffers from or is at risk of developing chemotherapy resistant SCLC. In the present invention, the control level can be obtained from a chemotherapy sensitive SCLC sample. For example, the control level can be determined from expression profile of SLC 1658 to 1663 in a cell or tissue obtained from chemotherapy sensitive SCLC subject. Alternatively, a cell or tissue obtained from a chemotherapy sensitive SCLC subject can be used as control sample to provide the control level.

The chemotherapy-resistant lung cancer associated genes listed in Table 6 are also useful for therapeutic target for treating or preventing chemoresistant SCLC. Thus, the present invention further provides methods of screening for a compound for treating or preventing chemotherapy resistant SCLC. Alternatively, the present invention also provides methods of treating or preventing chemotherapy resistant lung cancer in a subject. In the present invention, the screening or therapeutic method for the SCLC described in the specification can be applied to those for chemotherapy resistant SCLC, using SLC 1658 to 1663 as target genes.

### Kits:

The present invention also includes an SCLC-detection reagent, *e.g.*, a nucleic acid that specifically binds to or identifies one or more SCLC nucleic acids, including oligonucleotide sequences which are complementary to a portion of an SCLC nucleic acid, or an antibody that binds to one or more proteins encoded by an SCLC nucleic acid. The detection reagents can be packaged together in the form of a kit. For example, the detection reagents can be packaged in separate containers, *e.g.*, a nucleic acid or antibody (either bound to a solid matrix or packaged separately with reagents for binding them to the matrix), a control reagent (positive and/or negative), and/or a detectable label. Instructions (*e.g.*, written, tape, VCR, CD-ROM, *etc.*) for carrying out the assay can also be included in the kit. The assay format of the kit can be a Northern hybridization or a sandwich ELISA, both of which are known in the art. *See, for example,* Sambrook and Russell, Molecular Cloning: A Laboratory Manual, 3rd Edition, 2001, Cold Spring Harbor Laboratory Press; and Harlow and Lane, *Using Antibodies, supra.*

For example, an SCLC detection reagent can be immobilized on a solid matrix, for example, a porous strip, to form at least one SCLC detection site. The measurement or detection region of the porous strip can include a plurality of sites, each containing a nucleic acid. A test strip can also contain sites for negative and/or positive controls. Alternatively, control sites can be located on a separate strip from the test strip. Optionally, the different detection sites can contain different amounts of immobilized nucleic acids, *i.e.,* a higher amount in the first detection site and lesser amounts in subsequent sites. Upon the addition of test sample, the number of sites displaying a detectable signal provides a quantitative indication of the amount of SCLC present in the sample. The detection sites can be configured in any suitably detectable shape and are typically in the shape of a bar or dot spanning the width of a test strip.

Alternatively, the kit can contain a nucleic acid substrate array comprising one or more nucleic acids. The nucleic acids on the array specifically identify one or more nucleic acid sequences represented by the SCLC-associated genes listed in Tables 2-3. The expression of 2, 3, 4, 5, 6, 7, 8, 9, 10, 15, 20, 25, 40 or 50 or more of the nucleic acids represented by the SCLC-associated genes listed in Tables 2-3 can be identified by virtue of the level of binding to an array test strip or chip. The substrate array can be on, *e.g.*, a solid substrate, for example, a "chip" described in U.S. Patent No. 5,744,305, the contents of which are hereby incorporated herein by reference in its entirety. Array substrates of use in the present methods are commercially available, for example, from Affymetrix, Santa Clara, CA.

### Arrays and pluralities:

The present invention also includes a nucleic acid substrate array comprising one or more nucleic acids. The nucleic acids on the array specifically correspond to one or more nucleic acid sequences represented by the SCLC-associated genes listed in Tables 2-3. The level of expression of 2, 3, 4, 5, 6, 7, 8, 9, 10, 15, 20, 25, 40 or 50 or more of the nucleic acids represented by the SCLC-associated genes listed in Tables 2-3 can be identified by detecting nucleic acid binding to the array.

The present invention also includes an isolated plurality (*i.e*., a mixture of two or more nucleic acids) of nucleic acids. The nucleic acids can be in a liquid phase or a solid phase, *e.g.*, immobilized on a solid support, for example, a nitrocellulose membrane. The plurality includes one or more of the nucleic acids represented by the SCLC-associated genes listed in Tables 2-3. In various embodiments, the plurality includes 2, 3, 4, 5, 6, 7, 8, 9, 10, 15, 20, 25, 40 or 50 or more of the nucleic acids represented by the SCLC-associated genes listed in Tables 2-3.

### Methods of inhibiting small cell lung cancer:

The present invention further provides a method for treating or alleviating a symptom of SCLC in a subject by decreasing the expression of one or more of the SCLC-associated genes listed in Table 3 (or the activity of its gene product) or increasing the expression of one or more of the SCLC-associated genes listed in Table 2 (or the activity of its gene product). Suitable therapeutic compounds can be administered prophylactically or therapeutically to a subject suffering from or at risk of (or susceptible to) developing SCLC. Such subjects can be identified using standard clinical methods or by detecting an aberrant level of expression of one or more of the SCLC-associated genes listed in Tables 2-3 or aberrant activity of its gene product. In the context of the present invention, suitable therapeutic agents include, for example, inhibitors of cell cycle regulation, cell proliferation, and protein kinase activity.

The therapeutic methods of the present invention includes the step of increasing the expression, activity, or both of one or more gene products of genes whose expression is decreased ("down-regulated" or "under-expressed" genes) in an SCLC cell relative to normal cells of the same tissue type from which the SCLC cells are retrieved. In these methods, the subject is treated with an effective amount of a compound that increases the amount of one or more of the under-expressed (down-regulated) genes in the subject. Administration can be systemic or local. Suitable therapeutic compounds include a polypeptide product of an under-expressed gene, a biologically active fragment thereof, and a nucleic acid encoding an under-expressed gene and having expression control elements permitting expression in the SCLC cells; for example, an agent that increases the level of expression of such a gene endogenous to the SCLC cells (*i.e*., which up-regulates the expression of the under-expressed gene or genes). Administration of such compounds counters the effects of an aberrantly under-expressed gene or genes in the subject's lung cells and improves the clinical condition of the subject.

Alternatively, the therapeutic methods of the present invention can include the step of decreasing the expression, activity, or both, of one or more gene products of genes whose expression is aberrantly increased ("up-regulated" or "over-expressed" gene) in lung cells. Expression can be inhibited in any of several ways known in the art. For example, expression can be inhibited by administering to the subject a nucleic acid that inhibits, or antagonizes the expression of the over-expressed gene or genes, *e.g.*, an antisense oligonucleotide or small interfering RNA which disrupts expression of the over-expressed gene or genes.

### Inhibitory Nucleic Acids:

As noted above, inhibitory nucleic acids (*e.g.*, antisense oligonucleotides, siRNAs, ribozymes) complementary to the nucleotide sequence of the SCLC-associated genes listed in Table 3 can be used to reduce the expression level of the genes. For example, inhibitory nucleic acids complementary to the SCLC-associated genes listed in Table 3 that are up-regulated in small cell lung cancer are useful for the treatment of small cell lung cancer. Specifically, the inhibitory nucleic acids of the present invention can act by binding to the SCLC-associated genes listed in Table 3, or mRNAs corresponding thereto, thereby inhibiting the transcription or translation of the genes, promoting the degradation of the mRNAs, and/or inhibiting the expression of proteins encoded by the SCLC-associated genes listed in Table 3, thereby, inhibiting the function of the proteins. The term "inhibitory nucleic acids" as used herein encompasses both nucleotides that are entirely complementary to the target sequence and those having a mismatch of one or more nucleotides, so long as the inhibitory nucleic acids can specifically hybridize to the target sequences. The inhibitory nucleic acids of the present invention include polynucleotides that have a sequence identity of at least 70%, 75%, 80%, 85%, 90%, 95%, 96%, 97%, 98%, 99% or higher over a span of at least 15 continuous nucleotides. Algorithms known in the art can be used to determine the sequence identity of two or more nucleic acid sequences.

One useful algorithm is BLAST 2.0, originally described in Altschul et al., (1990) J. Mol. Biol. 215: 403-10. Software for performing BLAST analyses is publicly available through the National Center for Biotechnology Information (available on the World Wide Web at ncbi.nlm.nih.gov). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see, Henikoff& Henikoff (1989) Proc. Natl. Acad. Sci. USA 89: 10915).

An additional example of a useful sequence alignment algorithm is PILEUP. PILEUP creates a multiple sequence alignment from a group of related sequences using progressive, pairwise alignments. It can also plot a tree showing the clustering relationships used to create the alignment PILEUP uses a simplification of the progressive alignment method of Feng & Doolittle, (1987) J. Mol. Evol. 35: 351-60. The method used is similar to the method described by Higgins & Sharp, (1989) CABIOS 5:151-3. The program can align, *e.g.*, up to 300 sequences of a maximum length of 5,000 letters. The multiple alignment procedure begins with the pairwise alignment of the two most similar sequences, producing a cluster of two aligned sequences. This cluster can then be aligned to the next most related sequence or cluster of aligned sequences. Two clusters of sequences can be aligned by a simple extension of the pairwise alignment of two individual sequences. The final alignment is achieved by a series of progressive, pairwise alignments. The program can also be used to plot a dendogram or tree representation of clustering relationships. The program is run by designating specific sequences and their amino acid or nucleotide coordinates for regions of sequence comparison. For example, in order to determine conserved amino acids in a monomer domain family or to compare the sequences of monomer domains in a family, the sequence of the invention, or coding nucleic acids, are aligned to provide structure-function information.

The antisense nucleic acids of the present invention act on cells producing the proteins encoded by SCLC-associated marker genes by binding to the DNAs or mRNAs encoding the proteins, inhibiting their transcription or translation, promoting the degradation of the mRNAs, and inhibiting the expression of the proteins, thereby resulting in the inhibition of the protein function.

An antisense nucleic acid of the present invention can be made into an external preparation, including a liniment or a poultice, by admixing it with a suitable base material which is inactive against the nucleic acid.

Also, as needed, the antisense nucleic acids of the present invention can be formulated into tablets, powders, granules, capsules, liposome capsules, injections, solutions, nose-drops and freeze-drying agents by adding excipients, isotonic agents, solubilizers, stabilizers, preservatives, pain-killers, and such. These can be prepared by following known methods.

The antisense nucleic acids of the present invention can be given to the patient by direct application onto the ailing site or by injection into a blood vessel so that it will reach the site of ailment. An antisense-mounting medium can also be used to increase durability and membrane-permeability. Examples include, but are not limited to, liposomes, poly-L-lysine, lipids, cholesterol, lipofectin or derivatives of these.

The dosage of the inhibitory nucleic acid derivative of the present invention can be adjusted suitably according to the patient's condition and used in desired amounts. For example, a dose range of 0.1 to 100 mg/kg, preferably 0.1 to 50 mg/kg can be administered.

The antisense nucleic acids of the present invention inhibit the expression of a protein of the present invention and are thereby useful for suppressing the biological activity of the protein of the invention. In addition, expression-inhibitors, comprising antisense nucleic acids of the present invention, are useful in that they can inhibit the biological activity of a protein of the present invention.

The methods of the present invention can be used to alter the expression in a cell of an up-regulated SCLC-associated gene, *e.g.*, up-regulation resulting from the malignant transformation of the cells. Binding of the siRNA to a transcript corresponding to one of the SCLC-associated genes listed in Table 3 in the target cell results in a reduction in the protein production by the cell.

The antisense nucleic acids of present invention include modified oligonucleotides. For example, thioated oligonucleotides can be used to confer nuclease resistance to an oligonucleotide.

Also, an siRNA against a marker gene can be used to reduce the expression level of the marker gene. Herein, term "siRNA" refers to a double stranded RNA molecule which prevents translation of a target mRNA. Standard techniques for introducing siRNA into the cell can be used, including those in which DNA is a template from which RNA is transcribed. In the context of the present invention, the siRNA comprises a sense nucleic acid sequence and an anti-sense nucleic acid sequence against an up-regulated marker gene, including an SCLC-associated gene listed in Table 3. The siRNA is constructed such that a single transcript has both the sense and complementary antisense sequences from the target gene, *e.g.*, a hairpin.

An siRNA of an SCLC-associated gene, including those listed in Table 3, hybridizes to target mRNA and thereby decreases or inhibits production of the polypeptides encoded by SCLC-associated gene listed in Table 3 by associating with the normally single-stranded mRNA transcript, thereby interfering with translation and thus, expression of the protein. Thus, siRNA molecules of the invention can be defined by their ability to hybridize specifically to mRNA or cDNA listed in Table 3 under stringent conditions. For the purposes of this invention the terms "hybridize" or "hybridize specifically" are used to refer the ability of two nucleic acid molecules to hybridize under "stringent hybridization conditions." The phrase "stringent hybridization conditions" refers to conditions under which a nucleic acid molecule will hybridize to its target sequence, typically in a complex mixture of nucleic acids, but not detectably to other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions can also be achieved with the addition of destabilizing agents, for example, formamide. For selective or specific hybridization, a positive signal is at least two times background, preferably 10 times background hybridization. Exemplary stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 50°C.

In the context of the present invention, an siRNA is preferably less than 500, 200, 100, 50, or 25 nucleotides in length. More preferably an siRNA is about 19 to about 25 nucleotides in length. In order to enhance the inhibition activity of the siRNA, one or more uridine ("u") nucleotides can be added to 3' end of the antisense strand of the target sequence. The number of "u's" to be added is at least 2, generally 2 to 10, preferably 2 to 5. The added "u's" form a single strand at the 3'end of the antisense strand of the siRNA.

An siRNA of an SCLC-associated gene, including those listed in Table 3, can be directly introduced into the cells in a form that is capable of binding to the mRNA transcripts. In these embodiments, the siRNA molecules of the invention are typically modified as described above for antisense molecules. Other modifications are also possible, for example, cholesterol-conjugated siRNAs have shown improved pharmacological properties. Song, et al., Nature Med. 9:347-51 (2003). Alternatively, a DNA encoding the siRNA can be carried in a vector.

Vectors can be produced, for example, by cloning an SCLC-associated gene target sequence into an expression vector having operatively-linked regulatory sequences flanking the sequence in a manner that allows for expression (by transcription of the DNA molecule) of both strands (Lee, N.S. et al., (2002) Nature Biotechnology 20 : 500-5.). An RNA molecule that is antisense strand for mRNA of an SCLC-associated gene is transcribed by a first promoter (*e.g.*, a promoter sequence 3' of the cloned DNA) and an RNA molecule that is the sense strand for the mRNA of an SCLC-associated gene is transcribed by a second promoter (*e.g.*, a promoter sequence 5' of the cloned DNA). The sense and antisense strands hybridize *in vivo* to generate siRNA constructs for silencing of the SCLC-associated gene. Alternatively, the two constructs can be utilized to create the sense and antisense strands of a siRNA construct. Cloned SCLC-associated genes can encode a construct having secondary structure, *e.g.*, hairpins, wherein a single transcript has both the sense and complementary antisense sequences from the target gene.

A loop sequence consisting of an arbitrary nucleotide sequence can be located between the sense and antisense sequence in order to form the hairpin loop structure. Thus, the present invention also provides siRNA having the general formula 5'-[A]-[B]-[A']-3', wherein [A] is a ribonucleotide sequence corresponding to a sequence that specifically hybridizes to an mRNA or a cDNA listed in Table 3. In preferred embodiments, [A] is a ribonucleotide sequence corresponding to a sequence of gene selected from Table 3, [B] is a ribonucleotide sequence consisting of about 3 to about 23 nucleotides, and [A'] is a ribonucleotide sequence consisting of the complementary sequence of [A]. The region [A] hybridizes to [A'], and then a loop consisting of region [B] is formed. The loop sequence can be preferably 3 to 23 nucleotide in length. The loop sequence, for example, can be selected from the following sequences (found on the worldwide web at ambion.com/techlib/tb/tb_506.html). Furthermore, loop sequence consisting of 23 nucleotides also provides active siRNA (Jacque, J.M. et al., (2002) Nature 418 : 435-8.). CCC, CCACC or CCACACC: Jacque, J. M. et al., (2002) Nature, 418: 435-8. UUCG: Lee, N.S. et al., (2002) Nature Biotechnology 20 : 500-5; Fruscoloni, P. et al., (2003) Proc. Natl. Acad. Sci. USA 100: 1639-44.

UUCAAGAGA: Dykxhoorn, D. M. et al., (2003) Nature Reviews Molecular Cell Biology 4: 457-67.

For example, a preferable siRNA having hairpin loop structure of the present invention is shown below. Accordingly, in some embodiments, the loop sequence [B] can be selected from group consisting of, CCC, UUCG, CCACC, CCACACC, and UUCAAGAGA. A preferable loop sequence is UUCAAGAGA ("ttcaagaga" in DNA).

For ZIC5-siRNA:
UCAAGCAGGAGCUCAUCUG-[B]-CAGAUGAGCUCCUGCUUGA (for target sequence of SEQ ID NO: 171)

The nucleotide sequence of suitable siRNAs can be designed using an siRNA design computer program available on the worldwide web at ambion.com/techlib/ misc/siRNA_finder.html). The computer program selects nucleotide sequences for siRNA synthesis based on the following protocol.

### Selection of siRNA Target Sites:

1. Beginning with the AUG start codon of the object transcript, scan downstream for AA dinucleotide sequences. Record the occurrence of each AA and the 3' adjacent 19 nucleotides as useful siRNA target sites. Tuschl, et al. (1999) Genes Dev 13: 3191-7, don't recommend against designing siRNA to the 5' and 3' untranslated regions (UTRs) and regions near the start codon (within 75 bases) as these can be richer in regulatory protein binding sites. UTR-binding proteins and/or translation initiation complexes can interfere with binding of the siRNA endonuclease complex.
2. Compare the target sites to the human genome database and eliminate from consideration any target sequences with significant sequence identity to other coding sequences. The sequence identity search can be performed using BLAST (Altschul SF, et al., (1997) Nucleic Acids Res. 25(17):3389-402.; (1990) J Mol Biol. 215(3):403-10.), which can be found on the NCBI server at ncbi.nlm.nih.gov/BLAST/.
3. Select qualifying target sequences for synthesis. Using the Ambion algorithm, preferably several target sequences can be selected along the length of the gene to evaluate.

Also included in the invention are isolated nucleic acid molecules that include the nucleic acid sequence of target sequences, for example, nucleotides of SEQ ID NO: 171 or a nucleic acid molecule that is complementary to the nucleic acid sequence of nucleotides of SEQ ID NO: 171. As used herein, an "isolated nucleic acid" is a nucleic acid removed from its original environment (*e.g*., the natural environment if naturally occurring) and thus, synthetically altered from its natural state: In the present invention, isolated nucleic acid includes DNA, RNA, and derivatives thereof. When the isolated nucleic acid is RNA or derivatives thereof, base "t" should be replaced with "u" in the nucleotide sequences. As used herein, the term "complementary" refers to Watson-Crick or Hoogsteen base pairing between nucleotides units of a nucleic acid molecule, and the term "binding" means the physical or chemical interaction between two nucleic acids or compounds or associated nucleic acids or compounds or combinations thereof. Complementary nucleic acid sequences hybridize under appropriate conditions to form stable duplexes containing few or no mismatches. For the purposes of this invention, two sequences having 5 or fewer mismatches are considered to be complementary. Furthermore, the sense strand and antisense strand of the isolated nucleotide of the present invention, can form double stranded nucleotide or hairpin loop structure by the hybridization. In a preferred embodiment, such duplexes contain no more than 1 mismatch for every 10 matches. In an especially preferred embodiment, where the strands of the duplex are fully complementary, such duplexes contain no mismatches. The nucleic acid molecule is less than 4612 nucleotides in length for ZIC5. For example, the nucleic acid molecule is less than about 500, about 200, or about 75 nucleotides in length. Also included in the invention is a vector containing one or more of the nucleic acids described herein, and a cell containing the vectors. The isolated nucleic acids of the present invention are useful for siRNA against ZIC5, or DNA encoding the siRNA. When the nucleic acids are used for siRNA or coding DNA thereof, the sense strand is preferably longer than about 19 nucleotides, and more preferably longer than 21 nucleotides.

The invention is based in part on the discovery that the gene encoding ZIC5 is over-expressed in small cell lung cancer (SCLC) compared to non-cancerous lung cells. The cDNA of ZIC5 is 4612 nucleotides in length. The nucleic acid and polypeptide sequences of ZIC5 are shown in SEQ ID NO: 175 and 176.

Transfection of siRNAs comprising SEQ ID NO: 171 resulted in a growth inhibition of SCLC cell lines. *ZIC5* was identified as an up-regulated gene in SCLC and was a cancer-testis antigen activated in the great majority of SCLCs, and plays a pivotal role in cell growth/survival, as demonstrated by northern-blot analysis and siRNA experiments. This gene encodes a protein of 663 amino acids with five C2H2 ZNF domains. This molecule is structurally a nucleic acid binding Zinc ion binding protein.

### Structure of siRNA compositions

The present invention also provides methods for inhibiting cell growth, *i.e.,* cancer cell growth by inhibiting expression of ZIC5. Expression of ZIC5 is inhibited, for example, by one or more small interfering RNA (siRNA) oligonucleotides that specifically target the ZINC5 gene. ZIC5 targets include, for example, nucleotides of SEQ ID NO: 171.

The regulatory sequences flanking the SCLC-associated gene sequences can be identical or different, such that their expression can be modulated independently, or in a temporal or spatial manner. siRNAs are transcribed intracellularly by cloning the SCLC-associated gene templates, respectively, into a vector containing, *e.g*., a RNA polymerase III transcription unit from the small nuclear RNA (snRNA) U6 or the human H1 RNA promoter. For introducing the vector into the cell, transfection-enhancing agent can be used. FuGENE (Roche diagnostics), Lipofectamine 2000 (Invitrogen), Oligofectamine (Invitrogen), and Nucleofector (Wako pure Chemical) are useful as the transfection-enhancing agent.

Oligonucleotides complementary to various portions of ZIC5 mRNA were tested *in vitro* for their ability to decrease production of ZIC5 in tumor cells (*e.g*., using the lung cancer cell line, for example, a small cell lung cancer (SCLC) cell line) according to standard methods. A reduction in ZIC5 gene product in cells contacted with the candidate siRNA composition compared to cells cultured in the absence of the candidate composition is detected using specific antibodies of ZIC5 or other detection strategies. Sequences which decreased production of ZIC5 in *in vitro* cell-based or cell-free assays are then tested for their inhibitory effects on cell growth. Sequences which inhibited cell growth in *in vitro* cell-based assay are tested *in vivo* in rats or mice to confirm decreased ZIC5 production and decreased tumor cell growth in animals with malignant neoplasms.

### Methods of treating malignant tumors

Patients with tumors characterized as over-expressing ZIC5 are treated by administering siRNA of ZIC5. siRNA therapy is used to inhibit expression of ZIC5 in patients suffering from or at risk of developing, for example, small cell lung cancer (SCLC). Such patients are identified by standard methods of the particular tumor type. Small cell lung cancer (SCLC) is diagnosed for example, by computed tomography (CT), magnetic resonance imaging (MRI), endoscopic retrograde cholangiopancreatography (ERCP), magnetic resonance cholangiopancreatography (MRCP), or ultrasound. Treatment is efficacious if the treatment leads to clinical benefit including, a reduction in expression of ZIC5, or a decrease in size, prevalence, or metastatic potential of the tumor in the subject. When treatment is applied prophylactically, "efficacious" means that the treatment retards or prevents tumors from forming or prevents or alleviates a clinical symptom of the tumor. Efficaciousness is determined in association with any known method for diagnosing or treating the particular tumor type. *See,* Harrison's Principles of Internal Medicine, Kasper, et al., eds, 2005, McGraw-Hill.

siRNA therapy is carried out by administering to a patient one or more siRNA oligonucleotides by standard vectors encoding the siRNAs of the invention and/or gene delivery systems, for example, by delivering the synthetic siRNA molecules. Typically, synthetic siRNA molecules are chemically stabilized to prevent nuclease degradation *in vivo.* Methods for preparing chemically stabilized RNA molecules are well known in the art. Typically, such molecules comprise modified backbones and nucleotides to prevent the action of ribonucleases. Other modifications are also possible, for example, cholesterol-conjugated siRNAs have shown improved pharmacological properties (Song et al., (2003) Nature Med. 9:347-51.). Suitable gene delivery systems can include liposomes, receptor-mediated delivery systems, or viral vectors including herpes viruses, retroviruses, adenoviruses and adeno-associated viruses, among others. A therapeutic nucleic acid composition is formulated in a pharmaceutically acceptable carrier. The therapeutic composition can also include a gene delivery system as described above. Pharmaceutically acceptable carriers are biologically compatible vehicles which are suitable for administration to an animal, *e.g*., physiological saline. A therapeutically effective amount of a compound is an amount which is capable of producing a medically desirable result, for example, reduced production of a ZIC5 gene product, reduction of cell growth, *e.g*., proliferation, or a reduction in tumor growth in a treated animal.

Parenteral administration, including intravenous, subcutaneous, intramuscular, and intraperitoneal delivery routes, can be used to deliver siRNA compositions of ZIC5. For treatment of lung tumors, direct infusion into the pulmonary artery, is useful.

Dosages for any one patient depends upon many factors, including the patient's size, body surface area, age, the particular nucleic acid to be administered, sex, time and route of administration, general health, and other drugs being administered concurrently. Dosage for intravenous administration of nucleic acids is from approximately 10⁶ to 10²² copies of the nucleic acid molecule.

The polynucleotides are administered by standard methods, for example, by injection into the interstitial space of tissues, for example, muscles or skin, introduction into the circulation or into body cavities or by inhalation or insufflation. Polynucleotides are injected or otherwise delivered to the animal with a pharmaceutically acceptable liquid carrier, which is aqueous or partly aqueous. The polynucleotides are associated with a liposome (*e.g*., a cationic or anionic liposome). The polynucleotide includes genetic information necessary for expression by a target cell, for example, promoters.

The inhibitory oligonucleotides of the invention inhibit the expression of one or more of the polypeptides of the invention and is thereby useful for suppressing the biological activity of one or more of the polypeptides of the invention. Also, expression-inhibitors, comprising the antisense oligonucleotides or siRNAs of the invention, are useful in the point that they can inhibit the biological activity of one or more of the polypeptides of the invention. Therefore, a composition comprising comprising one or more of the antisense oligonucleotides or siRNAs of the present invention is useful in treating a small cell lung cancer.

### Antibodies:

Alternatively, function of one or more gene products of the genes over-expressed in SCLC can be inhibited by administering a compound that binds to or otherwise inhibits the function of the gene products. For example, the compound is an antibody which binds to the over-expressed gene product or gene products.

The present invention refers to the use of antibodies, particularly antibodies against a protein encoded by an up-regulated marker gene, or a fragment of such an antibody. As used herein, the term "antibody" refers to an immunoglobulin molecule having a specific structure, that interacts (*i.e*., binds) only with the antigen that was used for synthesizing the antibody (i. e., the gene product of an up-regulated marker) or with an antigen closely related thereto. Furthermore, an antibody can be a fragment of an antibody or a modified antibody, so long as it binds to one or more of the proteins encoded by the marker genes. For instance, the antibody fragment can be Fab, F(ab')₂, Fv, or single chain Fv (scFv), in which Fv fragments from H and L chains are ligated by an appropriate linker (Huston J. S. et al., (1988) Proc. Natl. Acad. Sci. U.S.A. 85:5879-83.). More specifically, an antibody fragment can be generated by treating an antibody with an enzyme, including papain or pepsin. Alternatively, a gene encoding the antibody fragment can be constructed, inserted into an expression vector, and expressed in an appropriate host cell *(see,* for example, Co M. S. et al., (1994) J. Immunol. 152:2968-76; Better M. and Horwitz A. H. (1989) Methods Enzymol. 178:476-96.; Pluckthun A. and Skerra A. (1989) Methods Enzymol. 178:497-515.; Lamoyi E. (1986) Methods Enzymol. 121:652-63.; Rousseaux J. et al., (1986) Methods Enzymol. 121:663-9.; Bird R. E. and Walker B. W. (1991) Trends Biotechnol. 9:132-7.).

An antibody can be modified by conjugation with a variety of molecules, for example, polyethylene glycol (PEG). The present invention provides such modified antibodies. The modified antibody can be obtained by chemically modifying an antibody. Such modification methods are conventional in the field.

Alternatively, an antibody can comprise a chimeric antibody having a variable region from a nonhuman antibody and a constant region from a human antibody, or a humanized antibody, comprising a complementarity determining region (CDR) from a nonhuman antibody, a frame work region (FR) and a constant region from a human antibody. Such antibodies can be prepared by using known technologies. Humanization can be performed by substituting rodent CDRs or CDR sequences for the corresponding sequences of a human antibody *(see, e.g.,* Verhoeyen et al., (1988) Science 239:1534-6). Accordingly, such humanized antibodies are chimeric antibodies, wherein substantially less than an intact human variable domain has been substituted by the corresponding sequence from a non-human species.

Fully human antibodies comprising human variable regions in addition to human framework and constant regions can also be used. Such antibodies can be produced using various techniques known in the art. For example in vitro methods involve use of recombinant libraries of human antibody fragments displayed on bacteriophage (*e.g*., Hoogenboom & Winter, (1992) J. Mol. Biol. 227:381-8)., Similarly, human antibodies can be made by introducing of human immunoglobulin loci into transgenic animals, *e.g*., mice in which the endogenous immunoglobulin genes have been partially or completely inactivated. This approach is described, *e.g*., in U.S. Patent Nos. 6,150,584; 5,545,807; 5,545,806; 5,569,825; 5,625,126; 5,633,425; 5,661,016. Such antibodies can be prepared by using known technologies.

Cancer therapies directed at specific molecular alterations that occur in cancer cells have been validated through clinical development and regulatory approval of anti-cancer drugs, for example, trastuzumab (Herceptin) for the treatment of advanced breast cancer, imatinib methylate (Gleevec) for chronic myeloid leukemia, gefitinib (Iressa) for non-small cell lung cancer (NSCLC), and rituximab (anti-CD20 mAb) for B-cell lymphoma and mantle cell lymphoma (Ciardiello F and Tortora G. (2001) Clin Cancer Res.;7:2958-70. Review.; Slamon DJ et al., (2001) N Engl J Med.;344:783-92.; Rehwald U et al., (2003) Blood.;101:420-4; Fang G, et al. (2000). Blood, 96,2246-53.). These drugs are clinically effective and better tolerated than traditional anti-cancer agents because they target only transformed cells. Hence, such drugs not only improve survival and quality of life for cancer patients, but also validate the concept of molecularly targeted cancer therapy. Furthermore, targeted drugs can enhance the efficacy of standard chemotherapy when used in combination with it (Gianni L. (2002) Oncology, 63 Suppl 1, 47-56.; Klejman A, et al. (2002). Oncogene, 21, 5868-76.). Therefore, future cancer treatments will involve combining conventional drugs with target-specific agents aimed at different characteristics of tumor cells, for example, angiogenesis and invasiveness.

These modulatory methods can be performed *ex vivo* or *in vitro (e.g.,* by culturing the cell with the agent) or, alternatively, *in vivo* (*e.g.,* by administering the agent to a subject). The methods involve administering a protein or combination of proteins or a nucleic acid molecule or combination of nucleic acid molecules as therapy to counteract aberrant expression of the differentially expressed genes or aberrant activity of their gene products.

Diseases and disorders that are characterized by increased (relative to a subject not suffering from the disease or disorder) expression levels or biological activities of genes and gene products, respectively, can be treated with therapeutics that antagonize (i. e., reduce or inhibit) activity of the over-expressed gene or genes. Therapeutics that antagonize activity can be administered therapeutically or prophylactically.

Accordingly, therapeutics that can be utilized in the context of the present invention include, *e.g*., (i) a polypeptide of the over-expressed or under-expressed gene or genes, or analogs, derivatives, fragments or homologs thereof; (ii) antibodies to the over-expressed gene or gene products; (iii) nucleic acids encoding the over-expressed or under-expressed gene or genes; (iv) antisense nucleic acids or nucleic acids that are "dysfunctional" (*i.e*., due to a heterologous insertion within the nucleic acids of one or more over-expressed gene or genes); (v) small interfering RNA (siRNA); or (vi) modulators (*i.e*., inhibitors, agonists and antagonists that alter the interaction between an over-expressed or under-expressed polypeptide and its binding partner). The dysfunctional antisense molecules are utilized to "knockout" endogenous function of a polypeptide by homologous recombination *(see, e.g.,* Capecchi, (1989) Science 244: 1288-92.).

Diseases and disorders that are characterized by decreased (relative to a subject not suffering from the disease or disorder) biological activity can be treated with therapeutics that increase (*i.e*., are agonists to) activity. Therapeutics that up-regulate activity can be administered in a therapeutic or prophylactic manner. Therapeutics that can be utilized include, but are not limited to, a polypeptide (or analogs, derivatives, fragments or homologs thereof) or an agonist that increases bioavailability.

Increased or decreased levels can be readily detected by quantifying peptide and/or RNA, by obtaining a patient tissue sample (*e.g*., from biopsy tissue) and assaying it *in vitro* for RNA or peptide levels, structure and/or activity of the expressed peptides (or mRNAs of a gene whose expression is altered). Methods that are well-known within the art include, but are not limited to, immunoassays (*e.g*., by Western blot analysis, immunoprecipitation followed by sodium dodecyl sulfate (SDS) polyacrylamide gel electrophoresis, immunocytochemistry, *etc.)* and/or hybridization assays to detect expression of mRNAs (*e.g*., Northern assays, dot blots, in situ hybridization, etc.).

Prophylactic administration occurs prior to the manifestation of overt clinical symptoms of disease, such that a disease or disorder is prevented or, alternatively, delayed in its progression.

Therapeutic methods of the present invention can include the step of contacting a cell with an agent that modulates one or more of the activities of the gene products of the differentially expressed genes. Examples of agent that modulates protein activity include, but are not limited to, nucleic acids, proteins, naturally-occurring cognate ligands of such proteins, peptides, peptidomimetics, and other small molecule. For example, a suitable agent can stimulate one or more protein activities of one or more differentially under-expressed genes.

### Vaccinating against small cell lung cancer:

The present invention also relates to methods of treating or preventing small cell lung cancer in a subject comprising the step of administering to said subject a vaccine comprising one or more polypeptides encoded by one or more nucleic acids selected from the group consisting of the SCLC-associated genes listed in Table 3 (*i.e*., up-regulated genes), immunologically active fragment(s) *(i.e.,* an epitope) of said polypeptides, or polynucleotide(s) encoding such a polypeptide(s) or fragment(s) thereof. Administration of the one or more polypeptides induces an anti-tumor immunity in a subject To induce anti-tumor immunity, one or more polypeptides encoded by one or more nucleic acids selected from the group consisting of the SCLC-associated genes listed in Table 3, immunologically active fragment(s) of said polypeptides, or polynucleotide(s) encoding such polypeptide(s) or fragment(s) thereof is administered to a subject in need thereof. The polypeptides or the immunologically active fragments thereof are useful as vaccines against SCLC. In some cases, the proteins or fragments thereof can be administered in a form bound to the T cell receptor (TCR) or presented by an antigen presenting cell (APC), including macrophage, dendritic cell (DC), or B-cells. Due to the strong antigen presenting ability of DC, the use of DC is most preferable among the APCs.

Identification of immunologically active fragments (*i.e*., epitopes) is well known in the art. B-cell epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding (*i.e*., conformationally determined) are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. *See, e.g.,* Epitope Mapping Protocols in Methods in Molecular Biology, Vol. 66, Glenn E. Morris, Ed. (1996). Antibodies that recognize the same epitope can be identified in a simple immunoassay showing the ability of one antibody to block the binding of another antibody to a target antigen (*e.g*., a competitive ELISA or solid phase radioimmunoassay. (SPRIA)). T cells recognize continuous epitopes of about nine amino acids for CD8 cells or about 13-15 amino acids for CD4 cells. T cells that recognize the epitope can be identified by in vitro assays that measure antigen-dependent proliferation, as determined by 3H-thymidine incorporation by primed T cells in response to an epitope (Burke et al., J. Inf. Dis. 170, 1110-9 (1994)), by antigen-dependent killing (cytotoxic T lymphocyte assay, Tigges et al., J. Immunol. (1996) 156:3901-10) or by cytokine secretion. Methods for determining immunogenic epitopes are described, for example, in Reineke, et al., Curr Top Microbiol Immunol (1999) 243:23-36; Mahler, et al., Clin Immunol (2003) 107:65-79; Anthony and Lehmann, Methods (2003) 29:260-9; Parker and Tomer, Methods Mol Biol (2000) 146:185-201; DeLisser, Methods Mol Biol (1999) 96:11-20; Van de Water, et al., Clin Immunol Immunopathol (1997) 85:229-35; Carter, Methods Mol Biol (1994) 36:207-23; and Pettersson, Mol Biol Rep (1992) 16:149-53.

In the present invention, a vaccine against SCLC refers to a substance that has the ability to induce anti-tumor immunity upon inoculation into animals. According to the present invention, polypeptides encoded by the SCLC-associated genes listed in Table 3, or fragments thereof, are HLA-A24 or HLA-A*0201 restricted epitopes peptides that can induce potent and specific immune response against SCLC cells expressing the SCLC-associated genes listed in Table 3. Thus, the present invention also encompasses methods of inducing anti-tumor immunity using the polypeptides. In general, anti-tumor immunity includes immune responses including as follows:
- induction of cytotoxic lymphocytes against tumors,
- induction of antibodies that recognize tumors, and
- induction of anti-tumor cytokine production.

Therefore, when a certain protein induces any one of these immune responses upon inoculation into an animal, the protein is determined to have anti-tumor immunity inducing effect. The induction of the anti-tumor immunity by a protein can be detected by observing in *vivo* or *in vitro* the response of the immune system in the host against the protein.

For example, a method for detecting the induction of cytotoxic T lymphocytes is well known. Specifically, a foreign substance that enters the living body is presented to T cells and B cells by the action of antigen presenting cells (APCs). T cells that respond to the antigen presented by the APCs in an antigen specific manner differentiate into cytotoxic T cells (or cytotoxic T lymphocytes; CTLs) due to stimulation by the antigen, and then proliferate (this is referred to as activation of T cells). Therefore, CTL induction by a certain peptide can be evaluated by presenting the peptide to a T cell via an APC, and detecting the induction of CTLs. Furthermore, APCs have the effect of activating CD4+ T cells, CD8+ T cells, macrophages, eosinophils, and NK cells. Since CD4+ T cells and CD8+ T cells are also important in anti-tumor immunity, the anti-tumor immunity-inducing action of the peptide can be evaluated using the activation effect of these cells as indicators. *See,* Coligan, *Current Protocols in Immunology, supra.*

A method for evaluating the inducing action of CTLs using dendritic cells (DCs) as the APC is well known in the art. DCs are representative APCs having the strongest CTL-inducing action among APCs. In this method, the test polypeptide is initially contacted with DCs, and then the DCs are contacted with T cells. Detection of T cells having cytotoxic effects against the cells of interest after the contact with DC shows that the test polypeptide has an activity of inducing the cytotoxic T cells. Activity of CTLs against tumors can be detected, for example, using the lysis of ⁵¹Cr-labeled tumor cells as the indicator. Alternatively, the method of evaluating the degree of tumor cell damage using ³H-thymidine uptake activity or LDH (lactose dehydrogenase)-release as the indicator is also well known.

Apart from DCs, peripheral blood mononuclear cells (PBMCs) can also be used as the APC. The induction of CTLs has been reported to be enhanced by culturing PBMCs in the presence of GM-CSF and IL-4. Similarly, CTLs have been shown to be induced by culturing PBMCs in the presence of keyhole limpet hemocyanin (KLH) and IL-7.

Test polypeptides confirmed to possess CTL-inducing activity by these methods are deemed to be polypeptides having DC activation effect and subsequent CTL-inducing activity. Therefore, polypeptides that induce CTLs against tumor cells are useful as vaccines against tumors. Furthermore, APCs that have acquired the ability to induce CTLs against tumors through contact with the polypeptides are also useful as vaccines against tumors. Furthermore, CTLs that have acquired cytotoxicity due to presentation of the polypeptide antigens by APCs can be also used as vaccines against tumors. Such therapeutic methods for tumors, using anti-tumor immunity due to APCs and CTLs, are referred to as cellular immunotherapy.

Generally, when using a polypeptide for cellular immunotherapy, efficiency of the CTL-induction is known to be increased by combining a plurality of polypeptides having different structures and contacting them with DCs. Therefore, when stimulating DCs with protein fragments, it is advantageous to use a mixture of multiple types of fragments.

Alternatively, the induction of anti-tumor immunity by a polypeptide can be confirmed by observing the induction of antibody production against tumors. For example, when antibodies against a polypeptide are induced in a laboratory animal immunized with the polypeptide, and when growth of tumor cells is suppressed by those antibodies, the polypeptide is deemed to have the ability to induce anti-tumor immunity.

Anti-tumor immunity is induced by administering the vaccine of this invention, and the induction of anti-tumor immunity enables treatment and prevention of SCLC. Therapy against cancer or prevention of the onset of cancer includes any of the following steps, including inhibition of the growth of cancerous cells, involution of cancer, and suppression of the occurrence of cancer. A decrease in mortality and morbidity of individuals having cancer, decrease in the levels of tumor markers in the blood, alleviation of detectable symptoms accompanying cancer, and such are also included in the therapy or prevention of cancer. Such therapeutic and preventive effects are preferably statistically significant. For example, in observation, at a significance level of 5% or less, wherein the therapeutic or preventive effect of a vaccine against cell proliferative diseases is compared to a control without vaccine administration. For example, Student's t-test, the Mann-Whitney U-test, or ANOVA can be used for statistical analysis.

The above-mentioned protein having immunological activity or a vector encoding the protein can be combined with an adjuvant. An adjuvant refers to a compound that enhances the immune response against the protein when administered together (or successively) with the protein having immunological activity. Exemplary adjuvants include, but are not limited to, cholera toxin, salmonella toxin, alum, and such, but are not limited thereto. Furthermore, the vaccine of this invention can be combined appropriately with a pharmaceutically acceptable carrier. Examples of such carriers include sterilized water, physiological saline, phosphate buffer, culture fluid, and such. Furthermore, the vaccine can contain as necessary, stabilizers, suspensions, preservatives, surfactants, and such. The vaccine can be administered systemically or locally, for example, through intradermal, intramuscular, subcutaneous, transdermal, buccal, or intranasal routes. Vaccine administration can.be performed by single administration, or boosted by multiple administrations.

When using an APC or CTL as the vaccine of this invention, tumors can be treated or prevented, for example, by the *ex vivo* method. More specifically, PBMCs of the subject receiving treatment or prevention are collected, the cells are contacted with the polypeptide *ex vivo,* and following the induction of APCs or CTLs, the cells can be administered to the subject. APCs can be also induced by introducing a vector encoding the polypeptide into PBMCs *ex vivo.* APCs or CTLs induced *in vitro* can be cloned prior to administration. By cloning and growing cells having high activity of damaging target cells, cellular immunotherapy can be performed more effectively. Furthermore, APCs and CTLs isolated in this manner can be used for cellular immunotherapy not only against individuals from whom the cells are retrieved, but also against similar types of tumors from other individuals.

General methods for developing vaccines are described, for example, in Vaccine Protocols, Robinson and Cranage, Eds., 2003, Humana Press; Marshall, Vaccine Handbook: A Practical Guide for Clinicians, 2003, Lippincott Williams & Wilkins; and Vaccine Delivery Strategies, Dietrich, et al., Eds., 2003, Springer Verlag.

### Pharmaceutical compositions for inhibiting SCLC:

Furthermore, a pharmaceutical composition for treating or preventing a cell proliferative disease; including cancer, for example, SCLC, comprising a pharmaceutically effective amount of the polypeptide of the present invention is provided. The pharmaceutical composition can be used for raising anti-tumor immunity.

In the context of the present invention, suitable pharmaceutical formulations include those suitable for oral, rectal, nasal, topical (including buccal and sub-lingual), vaginal or parenteral (including intramuscular, sub-cutaneous and intravenous) administration, or for administration by inhalation or insufflation. Preferably, administration is intravenous. The formulations are optionally packaged in discrete dosage units.

Pharmaceutical formulations suitable for oral administration include capsules, cachets or tablets, each containing a predetermined amount of active ingredient. Suitable formulations also include powders, granules, solutions, suspensions and emulsions. The active ingredient is optionally administered as a bolus electuary or paste. Tablets and capsules for oral administration can contain conventional excipients, including binding agents, fillers, lubricants, disintegrant and/or wetting agents. A tablet can be made by compression or molding, optionally with one or more formulational ingredients. Compressed tablets can be prepared by compressing in a suitable machine the active ingredients in a free-flowing form, including a powder or granules, optionally mixed with a binder, lubricant, inert diluent, lubricating, surface active and/or dispersing agent. Molded tablets can be made by molding in a suitable machine a mixture of the powdered compound moistened with an inert liquid diluent. The tablets can be coated according to methods well known in the art. Oral fluid preparations can be in the form of, for example, aqueous or oily suspensions, solutions, emulsions, syrups or elixirs, or can be presented as a dry product for constitution with water or other suitable vehicle before use. Such liquid preparations can contain conventional additives, including suspending agents, emulsifying agents, non-aqueous vehicles (which can include edible oils), and/or preservatives. The tablets can optionally be formulated so as to provide slow or controlled release of the active ingredient therein. A package of tablets can contain one tablet to be taken on each of the month.

Formulations suitable for parenteral administration include aqueous and non-aqueous sterile injection solutions, optionally contain anti-oxidants, buffers, bacteriostats and solutes which render the formulation isotonic with the blood of the intended recipient; as well as aqueous and non-aqueous sterile suspensions including suspending agents and/or thickening agents. The formulations can be presented in unit dose or multi-dose containers, for example as sealed ampoules and vials, and can be stored in a freeze-dried (lyophilized) condition, requiring only the addition of the sterile liquid carrier, for example, saline, water-for-injection, immediately prior to use. Alternatively, the formulations can be presented for continuous infusion. Extemporaneous injection solutions and suspensions can be prepared from sterile powders, granules and tablets of the kind previously described.

Formulations suitable for rectal administration include suppositories with standard carriers including cocoa butter or polyethylene glycol. Formulations suitable for topical administration in the mouth, for example, buccally or sublingually, include lozenges, containing the active ingredient in a flavored base including sucrose and acacia or tragacanth, and pastilles, comprising the active ingredient in a base including gelatin and glycerin or sucrose and acacia. For intra-nasal administration, the compounds of the invention can be used as a liquid spray, a dispersible powder, or in the form of drops. Drops can be formulated with an aqueous or non-aqueous base also comprising one or more dispersing agents, solubilizing agents and/or suspending agents.

For administration by inhalation the compounds can be conveniently delivered from an insufflator, nebulizer, pressurized packs or other convenient means of delivering an aerosol spray. Pressurized packs can comprise a suitable propellant including dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount.

Alternatively, for administration by inhalation or insufflation, the compounds can take the form of a dry powder composition, for example a powder mix of the compound and a suitable powder base, for example, lactose or starch. The powder composition can be presented in unit dosage form, for example, as capsules, cartridges, gelatin or blister packs, from which the powder can be administered with the aid of an inhalator or insufflators.

Other formulations include implantable devices and adhesive patches which release a therapeutic agent.

When desired, the above described formulations, adapted to give sustained release of the active ingredient, can be employed. The pharmaceutical compositions can also contain other active ingredients, including antimicrobial agents, immunosuppressants and/or preservatives.

It should be understood that in addition to the ingredients particularly mentioned above, the formulations of this invention can include other agents conventional in the art with regard to the type of formulation in question. For example, formulations suitable for oral administration can include flavoring agents.

Preferred unit dosage formulations contain an effective dose, as recited below, or an appropriate fraction thereof, of the active ingredient.

For each of the aforementioned conditions, the compositions, *e.g*., polypeptides and organic compounds, can be administered orally or via injection at a dose ranging from about 0.1 to about 250 mg/kg per day. The dose range for adult humans is generally from about 5 mg to about 17.5 g/day, preferably about 5 mg to about 10 g/day, and most preferably about 100 mg to about 3 g/day. Tablets or other unit dosage forms of presentation provided in discrete units can conveniently contain an amount which is effective at such dosage or as a multiple of the same, for instance, units containing about 5 mg to about 500 mg, usually from about 100 mg to about 500 mg.

The dose employed will depend upon a number of factors, including the age and sex of the subject, the precise disorder being treated, and its severity. Also the route of administration can vary depending upon the condition and its severity. In any event, appropriate and optimum dosages can be routinely calculated by those skilled in the art, taking into consideration the above-mentioned factors.

Aspects of the present invention are described in the following examples, which are not intended to limit the scope of the invention described in the claims. The following examples illustrate the identification and characterization of genes differentially expressed in SCLC cells.

### EXAMPLE

### Materials and Methods

### Cell Lines

The human SCLC cell lines used in this Example were as follows: DMS 114, DMS273, SBC-3, SBC-5, NCI-H196, and NCI-H446. All cells were grown in monolayers in appropriate medium supplemented with 10% fetal calf serum (FCS) and were maintained at 37°C in atmospheres of humidified air with 5% CO₂.

### Patients and Tissue Samples

Advanced SCLC tissue samples (stage IV) were obtained with informed consent from post-mortem materials (15 individuals). Individual institutional Ethical Committees approved this study and the use of all clinical materials. All cancer tissues had been confirmed histologically as SCLC by the pathologists. Patient profiles were obtained from medical records. The pathological stage was determined according to the classification of the Union Internationale Contre le Cancer (Travis WD et al., World Health Organization International Histological classification of tumours 1999). Clinical stage was determined according to the staging system introduced by the Veterans Administration Lung Study Group (Zelen M, (1973) Cancer Chemother Rep 3.; 4:31-42). 14 of the 15 cases had been treated with chemotherapy. All samples were immediately frozen and embedded in TissueTek OCT medium (Sakura, Tokyo, Japan) and stored at -80°C until used for microarray analysis.

### Laser-microbeam Microdissection, Extraction of RNA, and T7-based RNA Amplification

Cancer cells were selectively collected from the preserved samples using laser-microbeam microdissection (Kakiuchi S et al., Hum Mol Genet. 2004; 13(24):3029-43 & Mol Cancer Res. 2003;1(7):485-99). To check the quality of RNAs, total RNA extracted from the residual tissue of each case were electrophoresed under the degenerative agarose gel, and confirmed their quality by a presence of ribosomal RNA bands. Extraction of total RNA and T7-based amplification were performed as described previously (Kakiuchi S et al., (2004) Hum Mol Genet;13:3029-43 & (2003) Mol Cancer Res. 2003;1:485-99). As a control probe, normal human lung poly(A) RNA (CLONTECH) was amplified in the same way; 2.5 µg aliquots of amplified RNAs (aRNAs) from each cancerous tissue and from the control were reversely transcribed in the presence of Cy5-dCTP and Cy3-dCTP, respectively.

### cDNA Microarrays

The "genome-wide" cDNA microarray system containing 32,256 cDNAs selected from the UniGene database of the National Center for Biotechnology Information (NCBI) was used for this analysis. Fabrication of the microarray, hybridization, washing, and detection of signal intensities were described previously (Kikuchi T et al., (2003) Oncogene.;22:2192-205, Kakiuchi S et al., (2004) Hum Mol Genet.;13:3029-43 & (2003) Mol Cancer Res.;1:485-99, Ochi K et al., (2004) Int J Oncol.;24:647-55.).

### Data Analysis

Signal intensities of Cy3 and Cy5 from the 32,256 spots were quantified and analyzed by substituting backgrounds, using ArrayVision software (Imaging Research Inc., Ontario, Canada). Subsequently, the fluorescent intensities of Cy5 (tumor) and Cy3 (control) for each target spot were adjusted so that the mean Cy5/Cy3 ratio of 52 housekeeping genes on the array was equal to one. Because data from low signal intensities are less reliable, we determined a cutoff value on each slide as described previously (Kakiuchi S et al., (2003) Mol Cancer Res.;1:485-99) and excluded genes from further analysis when both Cy3 and Cy5 dyes yielded signal intensities lower than the cutoff. For other genes, we calculated the Cy5/Cy3 ratio using the raw data of each sample.

### Semi-quantitative RT-PCR

We selected highly up-regulated genes and examined their expression levels by means of semi-quantitative RT-PCR experiments. A total of 3 µg aliquot of aRNA from each sample was reversely transcribed to single-stranded cDNAs using random primer (Roche) and Superscript II (Invitrogen). Each cDNA mixture was diluted for subsequent PCR amplification with the primer sets (Table 1) that were prepared for the target DNA- or beta-actin (*ACTB*)-specific reactions. Expression of *ACTB* served as an internal control. PCR reactions were optimized for the number of cycles to ensure product intensity within the linear phase of amplification.

**Table 1 Primer sequences for Semi-quantitative RT-PCR**

| LMMID | primer F | SEQ ID NO | primer R | SEQ ID NO |
|---|---|---|---|---|
| ACTB | GAGGTGATAGCATTGCTTCG | 1 | CAAGTCAGTGTACAGGTAAGC | 2 |
| A1286 | CTGCGCGTACATGCGCACT | 3 | ACTTCATGCTCCTGAAAACCAT | 4 |
| A4492 | CTCCTTTCCTTGCTGAGGTG | 5 | AGCTGTAGGCCTTGGGAACT | 6 |
| A4946 | CTGTATAACGCGCTCACCTATTA | 7 | TACACCTTTTACTCCCTTTTCCC | 8 |
| A6152 | AAAGCTAGTGGCATACCTCACAG | 9 | CGGTCAGTGAAAAACACATGAT | 10 |
| A8458 | AATTGTGGCTCTCTTCCAAGTTC | 11 | GGTACTCTTTTCTCCATTTGGCT | 12 |
| A9165 | TCCAGAAATGGAATTTGCCTG | 13 | CTGAGGGAAAAGAAACCCAAT | 14 |
| B1221 | GTCGTTTCAACCAGGTAGTTTTG | 15 | CCTATTGCCAAACACAATCTCTC | 16 |
| B5456 | AGAAATGTGGATTTCAGCACCT | 17 | CAATACCAAACACAACCCAAAC | 18 |
| C7252 | AGCCCAATCTAAGTATTCCTTGC | 19 | AGTGACAACCAGAAACTTTGCAG | 20 |
| C7318 | TCCCTGCACAGTAAAGACTTTTG | 21 | CAGACATACACCAGTCAACAGGA | 22 |
| C7707 | TTTCAGAGGCTGGAGTTAATCTG | 23 | GGATTGATACAGAACTTGATGCC | 24 |
| D4225 | CCAGTTACTGTGTCTATCGGGTC | 25 | AGCCATATGTAGTCAAGTGCCAT | 26 |
| D4500 | GCAAATCATTACAAGGCAGACAG | 27 | AGGATGGCAGGCTTCCTATTAT | 28 |
| D5263 | TAGGGCAACATGGACTGTTTAAG | 29 | GCTGTGTTTTGTCATTTAGCTCC | 30 |
| D6248 | TCTCCTCCCATATAGAAGGTACTCA | 31 | CCTCAGAACTCTCAGTTTATTCCTG | 32 |
| D7184 | CACATGAAAGAGAAAGAAGTGGG | 33 | AAGTCAAGATCGACAAACACTGC | 34 |
| G2326 | TTGCTTCCTAATCCCTTTGGTC | 35 | TAAGCTGCATCTTGATGCCTTC | 36 |
| A3378 | CCTACTTGTTGGAGTCCACGAT | 37 | CATGTCACATCTTGATGCAGTT | 38 |
| A4807 | GGACAGTTCCATTCATTAGTTGTG | 39 | GGCACTTCATTGTATTTGAGGAG | 40 |
| A4831 | GTTGCCTTTTGGACCTACCA | 41 | CAACCATTTACCATGAGATCATTTA | 42 |
| A6769 | AAGAGGAGACCTGAACATCAACA | 43 | ACGCTATATTTGGGGCATAGAGT | 44 |
| A7027 | CAAAGCTGCATGTGTAGGATGTA | 45 | CTCTTGGGAACCAGTACAGAATG | 46 |
| A7112 | GAGCAACAGGTTGGTGAAAAC | 47 | GCTGTATGTAAATAGCATTGGGG | 48 |
| A7146 | GCAACTCTCCCGTCAAACA | 49 | AGATGCCAATTCATGTTCTTCC | 50 |
| A9047 | CGGTGAGACTGATACAGACTTGA | 51 | TATTTCTGTAGCTTCCACATCCC | 52 |
| B4513 | AACCAGAGAGAAAGAGGATCCAG | 53 | CAGTTGGTGGCTATCAAATTAGG | 54 |
| B6180 | CCAAATCACAACCCAAGATACTC | 55 | CAATGCTTCATTCTCTGAGTGCT | 56 |
| B6190 | CATCTGTGGACACCTCATGC | 57 | GGAAATGGTATGGAATAAGCCAG | 58 |
| B7534 | TCCAGAATTGCTTGTTACGTAGG | 59 | GGTTCTCAGAGCTGTTTTGCTT | 60 |
| B7889N | TGATCTGTCTGCTCCTACTCCTC | 61 | CTGTCCCGTAATTGAGAGATGC | 62 |
| B8296 | GTGCTATGATCATTGTAACTT, | 63 | GTAAATTTCTGAAGTAATACTTT | 64 |
| C4221 | GACGTGCCTCTCCTACTGTGTA | 65 | AAAGTCCCTCTTACCTCGATCTG | 66 |
| D5416 | CTTCTGACAAGCATTCCCTATTG | 67 | AATCAATCCCTCGTATTTTCCC | 68 |
| D5941 | CTGTCAGGGTCATAGTAGGCATT | 69 | CCAAAGTCAAACTCCCATTCAT | 70 |
| F0164 | CCTGCCAATTCTCCTTCATC | 71 | CATGCGCCAGTAAATCAGTACA | 72 |
| F3361 | GGGTTTGTTTGCTGCTTTTG | 73 | CACAGGGGAAATGGTGGTT | 74 |
| F7918 | AGCCAGCAGTGAGCCAGTAT | 75 | ACCACGCACAAGGAATTAGG | 76 |
| A8922 | CCCGTCTGCAACTCTCTCAC | 77 | CTGAGGTTCAGCGAGGGTAA | 78 |
| A0167 | CAGATGCTGGAGGAAGATTCTAA | 79 | AAAGAAAGAGGGGGAAACAAAG | 80 |
| A2466 | GAAAGCACCAGCTCCCGGA | 81 | GCTTCTACATCTCAAATCATGTCC | 82 |
| A3700 | GGTGGACACGGTCATCTACA | 83 | GAAGCCCGAGAAGATGGGTAT | 84 |
| A4345 | CTTCTCCATTTCTGGAGCCAC | 85 | GCCGTTCTAATTTAGCTTGAAGAG | 86 |
| A4383 | GGAGAAACTGCAGGACTTGG | 87 | CAATTTTAATGTCTGGGTTGGG | 88 |
| A4553 | CATCCAGAAGCACAAGAGCA | 89 | TTTCCCCTTTTAAACTTCCCTG | 90 |
| A5456 | CCCTTTGTCAGACCCTACCA | 91 | TTCAGTAGGCACACAGTTAACCC | 92 |
| A6175 | ACTGGACCACCCGAAGATAG | 93 | CTACAGCCTGACCACATTCTTTG | 94 |
| A6900 | AGGACTTGGCTATCATTTGGAGT | 95 | CAAAGCAATACAGCCTTTACCAC | 96 |
| A6909 | CCCTAATGTCCCATGAAGATACA | 97 | GCCTTAGCAAGTCATTTTCTGTC | 98 |
| A9820 | TGAGAGTCCTCAGAGGGTATCAG | 99 | CTTGAAGTCAAGAGTCCTGGTGT | 100 |
| B0075 | CTCAGACCTACCAGTTTCCCTTT | 101 | GCTTTATTTAGGGCTAAGCTGGA | 102 |
| B0286 | ACTCTCCTACAGAGAGCCCTGAT | 103 | CAGCCAGGATTTAGTGCCCAGC | 104 |
| B0296 | GAAGATCTCGTCTGCTCACCTTA | 105 | CAGACAGATGGAGAGGCTAGAGA | 106 |
| B0978 | CCTTAGGTCAGTAATTGTTGTGAG | 107 | CATATCTCTGGGGTGCTTGG | 108 |
| B2699 | GGCAGACAGGGGAAGTAAGAATA | 109 | CTGCATCTCACCAACCAATAACT | 110 |
| B3010 | ATATATGAGTTGCTGGGGACCTT | 111 | TGCTTTGGTCTGTACAAAGTCTG | 112 |
| B3467 | GGGAACAATCCTAGAAAACACTG | 113 | GGGAAGGTCACATTTTACCATTAG | 114 |
| B3668 | CAAAAACCAGCTTCTTCTCTGG | 115 | CAGGAAAGATCACAACCTCATTC | 116 |
| B4030 | TATCAGTAACTGCTCCGTGTTCA | 117 | GGTCTGTCATTGACCAAAACATC | 118 |
| B4566 | GAAGATTAGGGGAAAAGAGGTCA | 119 | CAGAGTCCAGTAGAGAATGCGAT | 120 |
| B5013 | CCCTAGTTTTTGTAGCTGTCGAA | 121 | GATCACATGCCAAGAACACAAT | 122 |
| B5478 | CTTCCATTGGTATGGTTGTTACC | 123 | CCCAATTCCCTACTCTCAGCTAT | 124 |
| B6283 | TGTGTCTCATCTGTGAACTGCTT | 125 | TTCGTGTTACGGTATATCCTGCT | 126 |
| B7303 | TATTGGGAAAAGAGAAGGACCAC | 127 | AGAAGTTGGTTCATGTGTAGGCA | 128 |
| B8503 | GTGAGAATATTCCTCGTCACAGC | 129 | ACTGAAGGGGACAGGAAGACTAC | 130 |
| B9322 | TGCCTGAGGATATAGCAGTAAGC | 131 | TTCTAAGAAGGGTTCTGGCTCA | 132 |
| C0468 | ACACACTAAAGCCTGATGCAGAT | 133 | CACTGTTAGGCTTGTAAGACAGC | 134 |
| C0715 | CCGTCAGCAGTGTGAAGTCT | 135 | CCTCCTAAGCAGTCAACCTTGT | 136 |
| C2290 | AAACAAACATACACTTCTCCTGGC | 137 | CGTCACAAGAAGAGACAATACATAC | 138 |
| C7616 | ATCTGGTTTTTAAGGGTCTGAGC | 139 | GCAAGCGTAAGAGACTGGTTTTA | 140 |
| C7862 | CCACACAGAGAGATGTCACCTT | 141 | GATGAGGAGAGACGTGAGAGCTA | 142 |
| C9571 | AGGAACATGTCAGGGGCTTAC | 143 | AAGTTCAACTAACCCCCAAAGAC | 144 |
| C9638 | AAAAGGTATGAACTTTTGGGGG | 145 | GCTTGCTCTCTATTGGAGGTACA | 146 |
| D4459 | GGTCGTCTTTATCCCCTATATGC | 147 | CAGTGACTCTTAAACTGAGCGGT | 148 |
| D4971 | TCTCCTGGACAGTATGGGTCTAA | 149 | TGAGCAGGAGATCTTAATTGACAG | 150 |
| D5556 | CACTTTACAGAAGCAGAAGTGGG | 151 | AGCTCTACCCAGGAGAATACAGG | 152 |
| D8457 | AAAGAGGAACACACTGGGTGTAA | 153 | AGGAGCCTAGAGAAGCAATCATC | 154 |
| D8905 | GTGCAAGGTAAGCTGTCAAAAAC | 155 | GAGGTGTTTTAACCAGAAAATCG | 156 |
| F0283 | GCAGGAAAGATCCCAAGTCA | 157 | AGATGAACGGAACATTGCACAC | 158 |
| F2316 | GGATTCCAAACATTTTCGACAG | 159 | GCAAATGCAGTTTCTGCCAATA | 160 |
| F4620 | TGTGTGTATAATTGCAAGCGCA | 161 | TGCTGAATTAATGAGGCACCAA | 162 |
| A6636 | AGAACTTTGGCTCCCTTTCC | 177 | TGCATAGTTGCCTGGAGATG | 178 |
| A2448 | GTCCATGCCATGAATGAGTG | 179 | CTCTTGGCAGATTTGCATCA | 180 |
| A0245 | CCTCTGGTCTCCCCATTACA | 181 | CTGAGGTGATGGGTTGGTCT | 182 |

### Immunohistochemical analysis

To confirm the differential protein expression of 2 candidate markers, A6636(SCAMPS) and A0245(CDC20), which were highly up-regulated in SCLC, we stained clinical tissue sections using ENVISION+ Kit/HRP (DakoCytomation). Briefly, after endogenous peroxidase and protein blocking reactions, anti-human SCAMP5 polyclonal antibody (Medical & Biological Laboratories, Aichi, Japan) or anti-human CDC20 monoclonal antibody (Santa Cruz Biotechnology, CA) was added, and then HRP-labeled anti-rabbit or anti-mouse IgG as the secondary antibody. Substrate-chromogen was then added and the specimens were counterstained with hematoxylin.

### Northern-blot Analysis

Human multiple-tissue blots (BD Biosciences Clontech, Palo Alto, CA) were hybridized with a ³²P-labeled PCR product of individual genes. The cDNA probes were prepared by RT-PCR. Pre-hybridization, hybridization, and washing were performed according to the supplier's recommendations. The blots were autoradiographed with intensifying screens at -80°C for 168 hours.

### RNA Interference Assay

To evaluate the biological functions of ZIC5 in cancer cells, we used a psiH1BX3.0 vector for expression of short-hairpin RNA against the target gene, as described previously (Suzuki et al., (2003) Cancer Res, 63: 7038-7041, Kato et al., (2005) Cancer Res. 2005; 65:5638-46. 2005). The H1 promoter was cloned into upstream of the gene-specific sequence (19-nucleotide sequence from the target transcript, separated from the reverse complement of the same sequence by a short spacer, TTCAAGAGA), with five thymidines as a termination signal and a neo-cassette for selection by Geneticin (Sigma). The target sequences of the synthetic oligonucleotides for RNAi were as follows: control 1 (Luciferase (LUC): *Photinus pyralis* luciferase gene), 5'-CGTACGCGGAATACTTCGA-3' (SEQ ID NO: 163); control 2 (Scramble (SCR): chloroplast Euglena gracilis gene coding for 5S and 16S rRNAs), 5'-GCGCGCTTTGTAGGATTCG-3' (SEQ ID NO: 167); si-ZIC5, 5'-TCAAGCAGGAGCTCATCTG-3' (SEQ ID NO: 171).

The insert sequences were as follows:
LUC control:
   TCCCCGTACGCGGAATACTTCGATTCAAGAGATCGAAGTATTCCGCGTACG (SEQ ID NO: 164), and
   AAAACGTACGCGGAATACTTCGATCTCTTGAATCGAAGTATTCCGCGTACG (SEQ ID NO: 165);
SCR control:
   TCCCGCGCGCTTTGTAGGATTCGTTCAAGAGACGAATCCTACAAAGCGCGC (SEQ ID NO: 168), and
   AAAAGCGCGCTTTGTAGGATTCGTCTCTTGAACGAATCCTACAAAGCGCGC (SEQ ID NO: 169);
ZIC5:
   TCCCTCAAGCAGGAGCTCATCTGTTCAAGAGACAGATGAGCTCCTGCTTGA (SEQ ID NO: 172), and
   AAAATCAAGCAGGAGCTCATCTGTCTCTTGAACAGATGAGCTCCTGCTTGA (SEQ ID NO: 173).

LC319 cells were plated onto 10-cm dishes (1.5x 10⁶ cells per dish), and transfected with psiH1BX vectors that included the target sequences for LUC, SCR, and ZIC5, using Lipofectamine 2000 (Invitrogen), according to the manufacturers' instructions. Cells were selected in medium containing 1 mg/ml of geneticin (Invitrogen) for 7 days and harvested after 4 days for RT-PCR analysis of knockdown effects on individual genes. Primers for these RT-PCR experiments were the same as those described above. After 7 days of incubation, these cells were stained by Giemsa solution to assess colony formation, and cell numbers were assessed by 3-(4,5-dimethylthiazol-2-yl)-2,5-diphenyltetrazolium bromide (MTT) assay.

### Cluster Analysis and Identification of Genes discriminating SCLCs from NSCLC (adenocarcinoma)

We applied a hierarchical clustering method to both genes and tumors. To obtain reproducible clusters for classification of the 15 SCLC, and an independent set of 62 NSCLC (20 early stage ADC, 15 early stage SCC, and 27 advanced ADC) samples analyzed previously using a cDNA microarray containing a subset (27,648 genes) of 32,256 genes on our present microarray-system (data from Kikuchi T, et al., (2003) Oncogene 22: 2192-205.; Kakiuchi S, et al., (2004) Hum Mol Genet 13: 3029-43., and our unpublished data for 4608 gene-expression in the same set of 35 early stage NSCLC), we selected genes from them for which valid data were obtained in 80% of the experiments, and whose expression ratios varied by standard deviations of more than 1.7. The analysis was performed using web-available software ("Cluster" and "TreeView") written by M. Eisen (http://rana.1b1.gov/index.htm) (Ball CA, et al., Nucleic Acids Res. 2005;33(Database issue):D580-2, Gollub J, et al., Nucleic Acids Res. 2003;31(1):94-6, Sherlock G, et al., Nucleic Acids Res. 2001;29(1):152-5). Before applying the clustering algorithm, we log-transformed the fluorescence ratio for each spot and then median-centered the data for each sample to remove experimental biases.

### Results

### Isolation of SCLC Cells using LMM

To obtain precise expression profiles of SCLC cells, we employed LMM to avoid contamination of the samples by non-cancerous cells. Fig. 1 shows microscopic images of representative cancers before *(A, B)* and after microdissection *(C, D)* and dissected cancer cells (E, F).

### Identification of Genes Commonly Down-/up-regulated in SCLCs

We identified up/down-regulated genes common to SCLC according to the following criteria: (1) genes for which we were able to obtain expression data in more than 50% (at least eight of the 15 cases) of the cancers examined; and (2) genes whose expression ratio was more than 5.0 or less than 0.2 in SCLC at least 50% of the informative cases. A total of 776 genes commonly down-regulated in SCLC are listed in Table 2, while 779 genes commonly up-regulated are in Table 3.

Among them, 83 genes confirmed their gene expression pattern in tumor and normal tissues using semi-quantitative RT-PCR (Fig. 2A) and/or northern-blot analyses (Fig. 3).

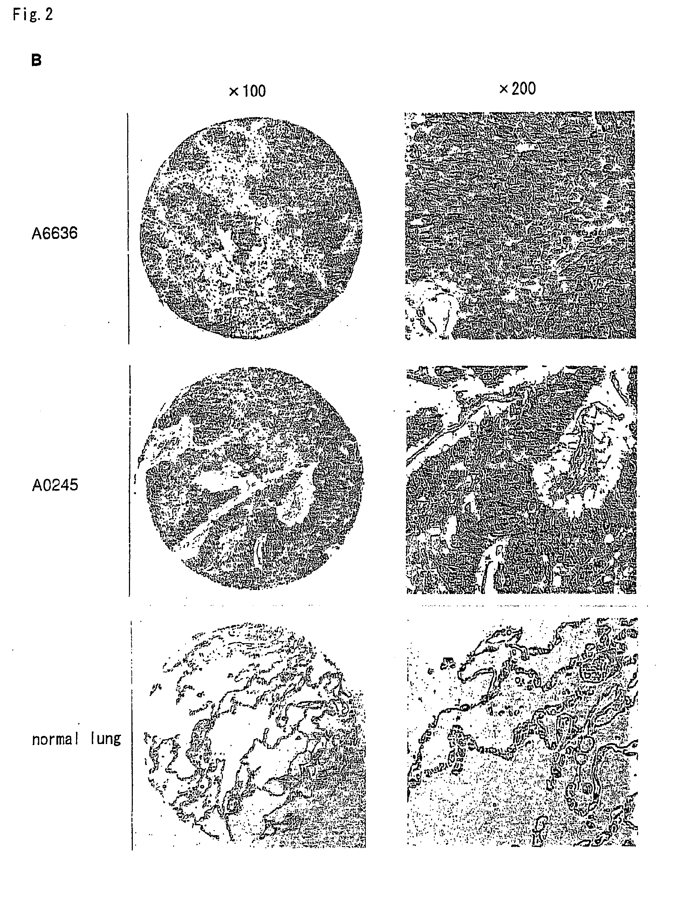

To further validate the data at the protein level, we carried out immunohistochemical analysis using the paired tumor and normal tissue sections using antibodies for A6636 (SCAMP5) or A0245 (CDC20) (Fig. 2B). Both proteins were confirmed to be expressed abundantly in SCLCs, but were hardly detectable in normal lung.

### Effect of ZIC5-Small Interfering RNAs on Growth of LC319 Cells

We constructed a siRNA expression vector specific to the sequences of *ZIC5* highly expressed in lung cancers and transfected them into LC319 cell lines that endogenously express high levels of *ZIC5* mRNA. A knockdown effect was confirmed by RT-PCR when we used si*-ZIC5* constructs (Fig. 4A). Colony-formation assays (Fig. 4B) and MTT assays (Fig. 4C) using LC319 revealed a drastic reduction in the number of cells transfected with si-ZIC5.

**Table 2 Down-regulated genes in SCLC**

| Asignment NO | LMMID | GenBank ID | Symbol | Gene name |
|---|---|---|---|---|
| 1 | A0148 | M16038 | LYN | V-yes-1 Yamaguchi sarcoma viral related oncogene homolog |
| 2 | A0192 | M62829 | EGR1 | Early growth response 1 |
| 3 | A0423 | X00129 | RBP4 | Retinol binding protein 4, plasma |
| 4 | A0764 | L10320 | FBP1 | Fructose-1,6-bisphosphatase 1 |
| 5 | A0906 | AB209196 | RNH | Ribonuclease/angiogenin inhibitor |
| 6 | A1378 | NM_000362 | TIMP3 | Tissue inhibitor of metalloproteinase 3 (Sorsby fundus dystrophy, pseudoinflammatory) |
| 7 | A1406 | L07555 | CD69 | CD69 antigen (p60, early T-cell activation antigen) |
| 8 | A1137 | L20688 | ARHGDIB | Rho GDP dissociation inhibitor (GDI) beta |
| 9 | A1445 | M27492 | IL1R1 | Interleukin 1 receptor, type I |
| 10 | A1813 | L36033 | CXCL12 | Chemokine (C-X-C motif) ligand 12 (stromal cell-derived factor 1) |
| 11 | A2188 | J02770 | IF | I factor (complement) |
| 12 | A2480 | NM_004484 | GPC3 | Glypican 3 |
| 13 | A2508 | X03350 | ADH1B | Alcohol dehydrogenase IB (class I), beta polypeptide |
| 14 | A2542 | J02874 | FABP4 | Fatty acid binding protein 4, adipocyte |
| 15 | A3037 | BC030975 | IL1RL1 | Interleukin 1 receptor-like 1 |
| 16 | A3536 | J03040 | SPARC | Secreted protein, acidic, cysteine-rich (osteonectin) |
| 17 | A3867 | AF013249 | LAIR1 | Leukocyte-associated Ig-like receptor 1 |
| 18 | A4224 | BC045651 | P2RY5 | Purinergic receptor P2Y, G-protein coupled, 5 |
| 19 | A4233 | BC078170 | WNT2 | Wingless-type MMTV integration site family member 2 |
| 20 | A5084 | CR614015 | CD14 | CD14 antigen |
| 21 | A5853 | N72866 | MIFF | Microphthalmia-associated transcription factor |
| 22 | A0854 | AB209583 | PLCB2 | Phospholipase C, beta 2 |
| 23 | A0797 | J04162 | FCGR3B | Fc fragment of IgG, low affinity IIIb, receptor (CD16b) |
| 24 | A1113 | BC003512 | MSLN | Mesothelin |
| 25 | A1150 | NM_000560 | CD53 | CD53 antigen |
| 26 | A1187 | NM_001343 | DAB2 | Disabled homolog 2, mitogen-responsive phosphoprotein (Drosophila) |
| 27 | A1764 | NM_002526 | NT5E | 5'-nucleotidase, ecto (CD73) |
| 28 | A1860 | NM_001014448 | CPZ | Carboxypeptidase Z |
| 29 | A2125 | BC022312 | C4BPA | Complement component 4 binding protein, alpha |
| 30 | A2523 | D21238 | GLRX | Glutaredoxin (thioltransferase) |
| 31 | A2964 | BQ219660 | GNG11 | Guanine nucleotide binding protein (G protein), gamma 11 |
| 32 | A2942 | BG685644 | IGLC2 | Immunoglobulin lambda variable 3-21 |
| 33 | A3613 | CR608325 | PLA2G7 | Phospholipase A2, group VII (platelet-activating factor acetylhydrolase, plasma) |
| 34 | A3322 | M_001620 | MGC5395 | AHNAK nucleoprotein (desmoyokin) |
| 35 | A3739 | NM 000090 | COL3A1 | Collagen, type III, alpha 1 (Ehlers-Danlos syndrome type IV, autosomal dominant) |
| 36 | A3778 | BC050277 | PELO | Integrin, alpha 1 |
| 37 | A4486 | AF059617 | PLK2 | Polo-like kinase 2 (Drosophila) |
| 38 | A4246 | CN479900 | CHIT1 | Chitinase 1 (chitotriosidase) |
| 39 | A4630 | U89281 | RODH | Hydroxysteroid (17-beta) dehydrogenase 6 |
| 40 | A5870 | NM_024829 | FLJ22662 | Hypothetical protein FLJ22662 |
| 41 | A5937 | BC028315 | GABARAPL1 | GABA(A) receptor-associated protein like 1 |
| 42 | A0417 | L03840 | FGFR4 | Fibroblast growth factor receptor 4 |
| 43 | A0657 | U37283 | MFAP5 | Microfibrillar associated protein 5 |
| 44 | A0765 | BC004102 | ALDH3A1 | Aldehyde dehydrogenase 3 family, memberA1 |
| 45 | A0878 | L13288 | VIPR1 | Vasoactive intestinal peptide receptor 1 |
| 46 | A1387 | BC038588 | AEBP1 | AE binding protein 1 |
| 47 | A1414 | NM_001855 | COL15A1 | Collagen, type XV, alpha 1 |
| 48 | A1516 | U24488 | TNXB | Tenascin XB |
| 49 | A1815 | NM_002664 | PLEK | Pleckstrin |
| 50 | A1951 | AL833268 | MEF2C | MADS box transcription enhancer factor 2, polypeptide C (myocyte enhancer factor 2C) |
| 51 | A2049 | BC062358 | IGHM | Immunoglobulin heavy constant mu |
| 52 | A2487 | D10923 | GPR109B | G protein-coupled receptor 109B |
| 53 | A2811 | X00570 | APOC1 | Apolipoprotein C-I |
| 54 | A2747 | NM_004347 | CASP5 | Caspase 5, apoptosis-related cysteine protease |
| 55 | A3250 | BC066956 | VIM | Vimentin |
| 56 | A3333 | AK223210 | CD79B | CD79B antigen (immunoglobulin-associated beta) |
| 57 | A3360 | NM_031850 | AGTR1 | Angiotensin II receptor, type 1 |
| 58 | A3154 | BC012160 | TNFRSF7 | Tumor necrosis factor receptor superfamily, member 7 |
| 59 | A3429 | M28696 | FCGR2B | Fc fragment of IgG, low affinity IIb, receptor (CD32) |
| 60 | A3631 | NM_005908 | MANBA | Mannosidase, beta A, lysosomal |
| 61 | A6250 | NM_006144 GZMA | lymphocyte-associated | Granzyme A (granzyme 1, cytotoxic T-serine esterase 3) |
| 62 | A3829 | NM_002182 | IL1RAP | Interleukin 1 receptor accessory protein |
| 63 | A4440 | NM_182643 | DLC1 | Deleted in liver cancer 1 |
| 64 | A4579 | L29394 | HP | Haptoglobin |
| 65 | A4234 | AI079183 | 1FI30 | Interferon, gamma-inducible protein 30 |
| 66 | A4641 | J02854 | MYL9 | Myosin, light polypeptide 9, regulatory |
| 67 | A4890 | M16973 | C8B | Complement component 8, beta polypeptide |
| 68 | A5154 | NM_004120 | GBP2 | Guanylate binding protein 2, interferon-inducible |
| 69 | A5991 | BX537522 | FLJ34077 | Weakly similar to zinc finger protein 195 |
| 70 | A0212 | M77349 | TGFBI | Transforming growth factor, beta-induced, 68kDa |
| 71 | A0571 | X64652 | RBMS1 | RNA binding motif, single stranded interacting protein 1 |
| 72 | A1032 | M87790 | IGLC2 | Immunoglobulin lambda variable 3-21 |
| 73 | A1455 | M58603 | NFKB1 | Nuclear factor of kappa light polypeptide gene enhancer in B-cells 1 (p105) |
| 74 | A2202 | AJ001016 | RAMP3 | Receptor (calcitonin) activity modifying protein 3 |
| 75 | A2408 | CR590167 | CD74 | CD74 antigen (invariant polypeptide of major histocompatibility complex, class II antigen-associated) |
| 76 | A2467 | AF035752 | CAV2 | Caveolin 2 |
| 77 | A2182 | CR749540 | CIR | Complement component 1, r subcomponent |
| 78 | A2418 | M96789 | GJA4 | Gap junction protein, alpha 4, 37kDa (connexin 37) |
| 79 | A2530 | CA310505 | APOD | Apolipoprotein D |
| 80 | A2644 | BC062476 | ADH1C | Alcohol dehydrogenase 1C (class I), gamma polypeptide |
| 81 | A2852 | X83006 | LCN2 | Lipocalin 2 (oncogene 24p3) |
| 82 | A3044 | BC092518 | IGHG3 | Immunoglobulin heavy constant mu |
| 83 | A2802 | CR592117 | CASP1 | Caspase 1, apoptosis-related cysteine protease (interleukin 1, beta, convertase) |
| 84 | A2972 | X72475 | | HRV Fab 027-VL |
| 85 | A3214 | X17042 | PRG1 | Proteoglycan 1, secretory granule |
| 86 | A3324 | BC057792 | CA4 | Carbonic anhydrase IV |
| 87 | A3412 | NM 000552 | VWF | Von Willebrand factor |
| 88 | A3875 | BC025717 | CCRL2 | Chemokine (C-C motif) receptor-like 2 |
| 89 | A4601 | BC016758 | HCLS1 | Hematopoietic cell-specific Lyn substrate 1 |
| 90 | A0084 | BC075838 | LAMB3 | Laminin, beta 3 |
| 91 | A0694 | M91211 | AGER | Advanced glycosylation end product-specific receptor |
| 92 | A0970 | BX648382 | SLA | Src-like-adaptor |
| 93 | A0593 | NM-002290 | LAMA4 | Laminin, alpha 4 |
| 94 | A1269 | NM_003841 | TNFRSF10C | Tumor necrosis factor receptor superfamily, member 10c, decoy without an intracellular domain |
| 95 | A1108 | D26579 | ADAM8 | A disintegrin and metalloproteinase domain 8 |
| 96 | A1617 | NM_133378 | TTN | Titin |
| 97 | A2084 | BM709336 | AIF1 | Allograft inflammatory factor 1 |
| 98 | A2115 | BQ949386 | FCGR1A | Fc fragment of IgG, high affinity Ia, receptor (CD64) |
| 99 | A2510 | X04481 | C2 | Complement component 2 |
| 100 | A2286 | NM_000118 | ENG | Endoglin (Osler-Rendu-Weber syndrome 1) |
| 101 | A2626 | NM_004137 | KCN1V1B1 | Potassium large conductance calcium-activated channel, subfamily M, beta member 1 |
| 102 | A2926 | X96719 | CLECSF2 | C-type lectin domain family 2, member B |
| 103 | A2638 | U20158 | LCP2 | Lymphocyte cytosolic protein 2 (SH2 domain containing leukocyte protein of 76kDa) |
| 104 | A6234 | NM000667 | ADH1A | Alcohol dehydrogenase 1A (class I), alpha polypeptide |
| 105 | A6248 | BC005312 | HLA-DRB1 | Major histocompatibility complex, class II, DR beta 4 |
| 106 | A3733 | X04665 | THBS1 | Thrombospondin 1 |
| 107 | A4545 | BC056898 | PLS3 | Plastin 3 (T isoform) |
| 108 | A4581 | M28204 | HLA-B | Major histocompatibility complex, class I, B |
| 109 | A5027 | U89165 | NRGN | Neurogranin (protein kinase C substrate, RC3) |
| 110 | A5155 | NM_000418 | IL4R | Interleukin 4 receptor |
| 111 | A0460 | X55656 | HBG2 | Hemoglobin, gamma G |
| 112 | A0025 | AF022184 | KLF4 | Kruppel-like factor 4 (gut) |
| 113 | A0383 | M13690 | SERPING1 | Serine (or cysteine) proteinase inhibitor, clade G (C1 inhibitor), member 1, (angioedema, hereditary) |
| 114 | A0791 | X63556 | FBN1 | Fibrillin 1 (Marfan syndrome) |
| 115 | A1064 | S55551 | TPSB2 | Tryptase alpha/beta 1 |
| 116 | A1193 | U52682 | IRF4 | Interferon regulatory factor 4 |
| 117 | A1254 | AF002986 | GPR171 | G protein-coupled receptor 171 |
| 118 | A1423 | L38486 | MFAP4 | Microfibrillar-associated protein 4 |
| 119 | A1456 | M59305 | NPR3 | Natriuretic peptide receptor C/guanylate cyclase C (atrionatriuretic peptide receptor C) |
| 120 | A1301 | AF039018 | PDLIM3 | PDZ and LIM domain 3 |
| 121 | A1431 | L43821 | NEDD9 | Neural precursor cell expressed, developmentally down-regulated 9 |
| 122 | A1736 | NM_001456 . | FLNA | Filamin A, alpha (actin binding protein 280) |
| 123 | A1708 | X85337 | MYLK | Myosin, light polypeptide kinase |
| 124 | A2075 | L02321 | GSTM5 | Glutathione S-transferase M5 |
| 125 | A2292 | X16832 | CTSH | Cathepsin H |
| 126 | A2388 | BC000574 | PCOLCE | Procollagen C-endopeptidase enhancer |
| 127 | A2557 | NM_001928 | DF | D component of complement (adipsin) |
| 128 | A2664 | BC033820 | FGL2 | Fibrinogen-like 2 |
| 129 | A3178 | M29696 | IL7R | Interleukin 7 receptor |
| 130 | A3297 | X01410 | | T cell receptor beta chain VB3 JB2.3 (TCRBV3D2J2S3) |
| 131 | A3903 | AF026692 | SFRP4 | Secreted frizzled-related protein 4 |
| 132 | A3688 | NM 006866 | LILRA2 | Leukocyte immunoglobulin-like receptor, subfamily A (with TM domain), member 2 |
| 133 | A4026 | NM 004982 | KCNJ8 | Potassium inwardly-rectifying channel, subfamily J, member 8 |
| 134 | A4559 | AK055599 | CTSL | Cathepsin L |
| 135 | A4664 | My 5153 . | TGM2 | Transglutaminase 2 (C polypeptide, protein-glutamine-gamma-glutamyltransferase) |
| 136 | A4766 | AF001434 | EHD1 | EH-domain containing 1 |
| 137 | A5015 | NM_001451 | FOXF1 | Forkhead box F1 |
| 138 | A0323 | X03438 | CSF3 | Colony stimulating factor 3 (granulocyte) |
| 139 | A0399 | NM_001912 | CTSL | Cathepsin L |
| 140 | A0941 | NM_002922 | RGS1 | Regulator of G-protein signalling 1 |
| 141 | A0707 | NM_000677 | ADORA3 | Adenosine A3 receptor |
| 142 | A1051 | BM662950 | FCER1G | Fc fragment of IgE, high affinity I, receptor for, gamma polypeptide |
| 143 | A1237 | Z29678 | MITF | Microphthalmia-associated transcription factor |
| 144 | A1217 | NM_002198 | IRF1 | Interferon regulatory factor 1 |
| 145 | A1450 | M33906 | HLA-DQA1 | Major histocompatibility complex, class II, DQ alpha 1 |
| 146 | A1718 | S81914 | IER3 | Immediate early response 3 |
| 147 | A1693 | X94991 | ZYX | Zyxin |
| 148 | A1760 | BC039065 | ADH6 | Alcohol dehydrogenase 6 (class V) |
| 149 | A2142 | NM_002087 | GRN | Granulin |
| 150 | A2366 | NM_000700 | ANXA1 | Annexin A1 |
| 151 | A2224 | NM 004469 | FIGF | C-fos induced growth factor (vascular endothelial growth factor D) |
| 152 | A2287 | AK127945 | HYAL2 | Hyaluronoglucosaminidase 2 |
| 153 | A2545 | BC033873 | BST2 | Bone marrow stromal cell antigen 2 |
| 154 | A2952 | D84143 | IGLC2 | Immunoglobulin lambda variable 3-21 |
| 155 | A2877 | NM_014485 | PGDS | Prostaglandin D2 synthase, hematopoietic |
| 156 | A3870 | AF013611 | CTSW | Cathepsin W (lymphopain) |
| 157 | A4043 | NM 000304 | PMP22 | Peripheral myelin protein 22 |
| 158 | A4509 | M31732 | BCL3 | B-cell CLL/lymphoma 3 |
| 159 | A4200 | AA989127 | HLA-C | Major histocompatibility complex, class I, C |
| 160 | A4236 | BM662200 | IFITM1 | Interferon induced transmembrane protein 1 (9-27) |
| 161 | A4453 | AF027299 | EPB41L2 | Erythrocyte membrane protein band 4.1-like 2 |
| 162 | A5022 | AF035528 | SMAD6 | SMAD, mothers against DPP homolog 6 (Drosophila) |
| 163 | A5176 | NM_022791 | MMP19 | Matrix metalloproteinase 19 |
| 164 | A0340 | X51345 | JUNB | Jun B proto-oncogene |
| 165 | A0753 | L10918 | CCR1 | Chemokine (C-C motif) receptor 1 |
| 166 | A1057 | M37766 | CD48 | CD48 antigen (B-cell membrane protein) |
| 167 | A0760 | L05568 | SLC6A4 | Solute carrier family 6 (neurotransmitter transporter, serotonin), member 4 |
| 168 | A1599 | X16150 | SELL | Selectin L (lymphocyte adhesion molecule 1) |
| 169 | A1797 | D00244 | PLAU | Plasminogen activator, urokinase |
| 170 | A2043 | BC005330 | TFPI2 | Tissue factor pathway inhibitor 2 |
| 171 | A2504 | NM_001710 | BF | B-factor, properdin |
| 172 | A3292 | CA430295 | FOLR3 | Folate receptor 3 (gamma) |
| 173 | A4130 | AA421322 | IGLC2 | Immunoglobulin lambda variable 3-21 1 |
| 174 | A4469 | AF044896 | Clorf38 | Chromosome 1 open reading frame 38 |
| 175 | A4702 | NM_014890 | DOC1 | Downregulated in ovarian cancer 1 |
| 176 | A5978 | BQ003596 | GJA5 | Gap junction protein, alpha 5, 40kDa (connexin 40) |
| 177 | A0325 | X03663 | CSF1R | Colony stimulating factor 1 receptor, formerly McDonough feline sarcoma viral (v-fms) oncogene homolog |
| 178 | A0357 | X15606 | ICAM2 | Intercellular adhesion molecule 2 |
| 179 | A0300 | BC063685 | VEGFC | Vascular endothelial growth factor C |
| 180 | A0401 | NM_005143 | HP. | Haptoglobin |
| 181 | A0456 | CR594071 | SERPINA1 | Serine (or cysteine) proteinase inhibitor, clade A (alpha 1 antiproteinase, antitrypsin), member 1 |
| 182 | A0711 | NM 004288 | PSCDBP | Pleckstrin homology, Sec7 and coiled-coil domains, binding protein |
| 183 | A1610 | NM_002084 | GPX3 | Glutathione peroxidase 3 (plasma) |
| 184 | A1754 | AB119995 | CES1 | Carboxylesterase 1 serine esterase 1) (monocyte/macrophage serine esterase 1) |
| 185 | A1730 | X9991 | CDH5 | Cadherin 5, type 2, VE-cadherin (vascular epithelium) |
| 186 | A1761 | K01171 | HLA-DRA | Major histocompatibility complex, class II, DR alpha |
| 187 | A2336 | BC032528 | LTA4H | Leukotriene A4 hydrolase |
| 188 | A2288 | Ask 12763 6 | IFNGR1 | Interferon gamma receptor 1 |
| 189 | A2403 | NM 001773 | CD34 | CD34 antigen |
| 190 | A2742 | NM_002272 | KRT4 | Keratin 4 |
| 191 | A3009 | BC009799 | AREG | Amphiregulin (schwannoma-derived growth factor) |
| 192 | A3061 | U07643 | LTF | Lactotransferrin |
| 193 | A2715 | BC035802 | GZMK | Gramme K (serine protease, granzyme 3; tryptase II) |
| 194 | A2751 | M68874 | PLA2G4A | Phospholipase A2, group IVA (cytosolic, calcium-dependent) |
| 195 | A3015 | NM 201442 | C1S | Complement component 1, s subcomponent |
| 196 | A3099 | M19722 | FGR | Gardner-Rasheed feline sarcoma viral (v-fgr) oncogene homolog |
| 197 | A3224 | J04132 | CD3Z | CD3Z antigen, zeta polypeptide (TiT3 complex) |
| 198 | A3313 | BQ188934 | DEFA1 | Defensin, alpha 1, myeloid-related sequence |
| 199 | A3402 | CR616287 | SERPINB9 | Serine (or cysteine) proteinase inhibitor, clade B (ovalbumin), member 9 |
| 200 | A4036 | BM667019 | HBG2 | Hemoglobin, gamma G |
| 201 | A4709 | BC016952 | CYR61 | Cysteine-rich, angiogenic inducer, 61 |
| 202 | A4970 | AF062075 | LPXN | Leupaxin |
| 203 | A5868 | BC037733 | SLC40A1 | Solute carrier family 40 (iron-regulated transporter), member 1 |
| 204 | A0438 | BC035625 | EGR2 | Early growth response 2 (Krox-20 homolog, Drosophila) |
| 205 | A0568 | BC038239 | TIE1 | Tyrosine kinase with immunoglobulin-like and EGF-like domains 1 |
| 206 | A0578 | NM 004417 | DUSP1 | Dual specificity phosphatase 1 |
| 207 | A0652 | L11015 | LTB | Lymphotoxin beta (TNF superfamily, member 3) |
| 208 | A0930 | X51420 | TYRP1 | Tyrosinase-related protein 1 |
| 209 | A1404 | K02770 | IL1B | Interleukin 1, beta |
| 210 | A1710 | NM_002133 | HMOX1 | Heme oxygenase (decycling) 1 |
| 211 | A1748 | U29089 | PRELP | Proline arginine-rich end leucine-rich repeat protein |
| 212 | A2353 | AK130133 | GLB1 | Galactosidase, beta 1 |
| 213 | A2359 | BX648013 | TAP1 | Transporter 1, ATP-binding cassette, sub-family B (MDR/TAP) |
| 214 | A2836 | BQ926240 | TNN12 | Troponin I, skeletal, fast |
| 215 | A3003 | BC058928 | ANPEP | Alanyl (membrane) aminopeptidase (aminopeptidase N, aminopeptidase M, microsomal aminopeptidase, CD13, p150) |
| 216 | A3027 | M28827 | CD1C | CD1C antigen, c polypeptide |
| 217 | A3054 | U01839 | FY | Duffy blood group |
| 218 | A3409 | BC069275 | STK22B | Testis-specific serine kinase 2 |
| 219 | A3299 | BM696587 | CRYAB | Crystallin, alpha B |
| 220 | A3628 | U59299 | SLC16A5 | Solute carrier family 16 (monocarboxylic acid transporters), member 5 |
| 221 | A4373 | M87434 | OAS2 | 2'-5'-oligoadenylate synthetase 2, 69/71kDa |
| 222 | A4819 | D17408 | CNN1 | Calponin 1, basic, smooth muscle |
| 223 | A4983 | X12830 | IL6R | Interleukin 6 receptor |
| 224 | A0122 | L17075 | ACVRL1 | Activin A receptor type II-like 1 |
| 225 | A0158 | M28526 | PECAM1 | Platelet/endothelial cell adhesion molecule (CD31 antigen) |
| 226 | A0451 | V00497 | HBB | Hemoglobin, beta |
| 227 | A0090 | BC040499 | TGFBR2 | Transforming growth factor, beta receptor II (70/80kDa) growth factor, beta receptor |
| 228 | A087S | L13740 | NR4A1 | Nuclear receptor subfamily 4, group A, member 1 |
| 229 | A1046 | AF266280 | LGALS3 | Lectin, galactoside-binding, soluble, 3 (galectin 3) |
| 230 | A0597 | X72760 | LAMB2 | Laminin, beta 2 (laminin S) |
| 231 | A0821 | NM_002164 | INDO | Indoleamine-pyrrole 2,3 dioxygenase |
| 232 | A0884 | U15085 | HLA-DMB | Major histocompatibility complex, class II, DM beta |
| 233 | A1179 | U18728 | LUM | Lumican |
| 234 | A1147 | NM_000129 | F13A1 | Coagulation factor XIII, A1 polypeptide |
| 235 | A1364 | CD013971 | CYP3A7 | Cytochrome P450, family 3, subfamily A, polypeptide 7 |
| 236 | A1886 | BC029261 | MYOC | Myocilin, trabecular meshwork inducible glucocorticoid response . |
| 237 | A2112 | BQ182722 | PLA2G2A | Phospholipase A2, group IIA (platelets, synovial fluid) |
| 238 | A1932 | J03037 | CA2 | Carbonic anhydrase II |
| 239 | A2404 | M15395 | ITGB2 | Integrin, beta 2 (antigen CD18 (p95), lymphocyte function-associated antigen 1; macrophage antigen 1 (mac-1) beta subunit) |
| 240 | A2548 | X67698 | NPC2 | Niemann-Pick disease, type C2 |
| 241 | A2675 | NM 005907 | MAN1A1 | Mannosidase, alpha, class 1A, member 1 |
| 242 | A2822 | BQ015859 | CSTA | Cystatin A (stefin A) |
| 243 | A3073 | NM 002192 | INHBA | Inhibin, beta A (activin A, activin AB alpha polypeptide) - |
| 244 | A3338 | M93056 | SERPINB1 | Serine (or cysteine) proteinase inhibitor, clade B (ovalbumin), member 1 |
| 245 | A3127 | D29642 | ARHGAP25 | Rho GTPase activating protein 25 |
| 246 | A3288 | BU626950 | TEYD1 | Tissue inhibitor of metalloproteinase 1 (erythroid potentiating activity, collagenase inhibitor) |
| 247 | A3730 | AB191261 | FN1 | Fibronectin 1 |
| 248 | A6251 | M25460 | 1FNB | Interferon, beta 1, fibroblast |
| 249 | A4111 | BC033040 | SLC1A1 | Solute carrier family 1 (neuronal/epithelial high affinity glutamate transporter, system Xag), member 1 |
| 250 | A3738 | NM 002332 | LRP1 | Low density lipoprotein-related protein 1 (alpha-2-macroglobulin receptor) |
| 251 | A4202 | BC053578 | GSTA1 | Glutathione S-transferase A1 |
| 252 | A0184 | NM 000426 | LAMA2 muscular | Laminin, alpha 2 (merosin, congenital dystrophy) |
| 253 | A0611 | BC037236 | DUSP6 | Dual specificity phosphatase 6 |
| 254 | A0931 | NM_001774 | CD37 | CD37 antigen |
| 255 | A1496 | NM_000104 | CYPIB1 | Cytochrome P450, family 1, subfamily B, polypeptide 1 |
| 256 | A1739 | J02761 | SFTPB | Surfactant, pulmonary-associated protein B |
| 257 | A2534 | M21119 | LYZ | Lysozyme (renal amyloidosis) |
| 258 | A3079 | J04599 | BGN | Biglycan |
| 259 | A3416 | BC033583 | CD2 | CD2 antigen (p50), sheep red blood cell receptor |
| 260 | A4608 | NM_001814 | CTSC | Cathepsin C |
| 261 | A4794 | AF064493 | LDB2 | LIM domain binding 2 |
| 262 | A4830 | NM_004557 | NOTCH4 | Notch homolog 4 (Drosophila) |
| 263 | A5083 | AK125193 | L1PA | Lipase A, lysosomal acid, cholesterol esterase (Wolman disease) |
| 264 | A5690 | AB028952 | SYNPO | Synaptopodin |
| 265 | A7233 | AA742701 | LCP1 | Lymphocyte cytosolic protein 1 (L-plastin) |
| 266 | A7978 | BC025176 | CYP3A5 | Cytochrome P450, family 3, subfamily A, polypeptide 43 |
| 267 | A7678 | U32331 | DKK3 | Dickkopf homolog 3 (Xenopus laevis) |
| 268 | A8600 | CR749355 | GIMAP6 | GTPase, IMAP family member 6 |
| 269 | A9850 | A1090386 | BAZ2A | Fucosyltransferase 1 (galactoside 2-alpha-L-fucosyltransferase) |
| 270 | B0565 | AF240635 | PCDH12 | Protocadherin 12 |
| 271 | B4100 | CR603708 | PON2 | Paraoxonase 2 |
| 272 | B6764 | M14338 | PROS1 | Protein S (alpha) |
| 273 | B9201 | BX647427 | WIFI | WNT inhibitory factor 1 |
| 274 | A6458 | AK127289 | SLCO2B1 | Solute carrier organic anion transporter family, member 2B1 |
| 275 | A6717 | AF495910 | SYNE1 | Spectrin repeat containing, nuclear envelope 1 |
| 276 | A6807 | NM_138711 | PPARG | Peroxisome proliferative activated receptor, gamma |
| 277 | A8898 | AF378756 | TENS1 | Tensin-like SH2 domain containing 1 |
| 278 | A9983 | NM_152703 | C7orf6 | Chromosome 7 open reading frame 6 |
| 279 | B2020 | BQ012846 | IL1RL1 | Interleukin 1 receptor-like 1 |
| 280 | B3746 | NM_003013 | SFRP2 | Secreted frizzled-related protein 2 |
| 281 | B3759 | BC092449 | MGC27165 | Hypothetical protein MGC27165 |
| 282 | B3894 | BC001356 | IFI35 | Interferon-induced protein 35 |
| 283 | C4126 | BC033887 | HRB2 | HIV-1 rev binding protein 2 |
| 284 | A6545 | NM_004613 | TGM2 | Transglutaminase 2 (C polypeptide, protein-glutamine-gamma-glutamyltransferase) |
| 285 | A8162 | AL832955 | TNFAIP9 | Tumor necrosis factor, alpha-induced protein 9 |
| 286 | A8796 | AB209591 | SLC7A7 | Solute carrier family 7 (cationic amino acid transporter, y+ system), member 7 |
| 287 | B2148 | M61900 | | |
| 288 | B4076 | NM_000165 | GJA1 | Gap junction protein, alpha 1,43kDa (connexin 43) |
| 289 | A7293 | NM 012302 | LPHN2 | Latrophilin 2 |
| 290 | A7454 | AF007162 | CRYAB | Crystallin, alpha B |
| 291 | A8148 | BU608708 | APOL1 | Apolipoprotein L, 1 |
| 292 | B0695 | A1208582, | MGC33414 | Zinc finger protein 683 |
| 293 | B3988 | NM_152243 | CDC42EP1 | CDC42 effector protein_(Rho GTPase binding) 1 |
| 294 | B4053 | NM_000499 | CYP1A1 | Cytochrome P450, family 1, subfamily A, polypeptide 1 |
| 295 | B4278 | AI198543 | DOCK6 | Dedicator of cytokinesis 6 |
| 296 | B4525 | D38169 | ITPKC | Inositol 1,4,5-trisphosphate 3-kinase C |
| 297 | A6504 | AB011146 | KIAA0574 | KIAA0574 protein |
| 298 | A7689 | X00457 | HLA-DPA1 | Major histocompatibility complex, class II, DP alpha 1 |
| 299 | A8639 | AI368204 | ENPP3 | Ectonucleotide pyrophosphatase/phosphodiesterase 3 |
| 300 | B0232 | BC060858 | SOCS3 | Suppressor of cytokine signaling 3 |
| 301 | B3940 | K02765 | C3, | Complement component 3 |
| 302 | B4602 | NM_005556 | KRT7 | Keratin 7 |
| 303 | B4077 | NM_004099 | STOM | Stomatin |
| 304 | C4884 | AA036952 | Gup1 | GRINL1A complex upstream protein |
| 305 | A6719 | AI302184 | SQRDL | Sulfide quinone reductase-like (yeast) |
| 306 | A7265 | NM_000847 | GSTA3 | Glutathione S-transferase A3 |
| 307 | A8155 | CD242398 | LOC51255 | Hypothetical protein LOC51255 |
| 308 | B2641 | BX094063 | PIN4 | Protein (peptidyl-prolyl cis/trans isomerase) NIMA-interacting, 4 (parvulin) |
| 309 | B0241 | BC056414 | PLVAP | Plasmalemma vesicle associated protein |
| 310 | B0878 | NM_005797 | EVA1 | Epithelial V-like antigen 1 |
| 311 | B1531 | BC063304 | NPR1 | Natriuretic peptide receptor A/guanylate cyclase A (atrionatriuretic peptide receptor A) |
| 312 | B2663 | BC009978 | ACTC | Actin, alpha, cardiac muscle |
| 313 | B4085 | NM 198098 | AQP1 | Aquaporin 1 (channel-forming integral protein, 28kDa) |
| 314 | B6287 | U66680 | | |
| 315 | A8204 | BX648041 | NEDD9 | Neural precursor cell expressed, 9 developmentally down-regulated 9 |
| 316 | A7286 | NM 021201 | MS4A7 | Membrane-spanning 4-domains, subfamily A, member 7 |
| 317 | A8142 | CF528794 | MS4A7 | Membrane-spanning 4-domains, subfamily A, member 7 |
| 318 | A8531 | BX537531 | FBLN5 | Fibulin 5 |
| 319 | A8648 | X54101 | GNLY | Granulysin |
| 320 | B4606 | BU634437 | FZD4 | Frizzled homolog 4 (Drosophila) |
| 321 | B5155 | W84893 | AGTRL1 | Angiotensin II receptor-like 1 |
| 322 | B5224 | NM_013374 | PDCD6IP | Programmed cell death 6 interacting protein |
| 323 | A6409 | AK091288 | C9orf19 | Chromosome 9 open reading frame 19 |
| 324 | A7411 | BC035028 | SERPIND1 | Serine (or cysteine) proteinase inhibitor, clade D (heparin cofactor), member 1 |
| 325 | A7429 | X17033 | ITGA2 | Integrin, alpha 2 (CD49B, alpha 2 subunit of VLA-2 receptor) |
| 326 | A7901 | AA814380 | | |
| 327 | A8156 | BQ010373 | HEG | HEG homolog 1 (zebrafish) |
| 328 | A9282 | AF086912 | OGN | Osteoglycin (osteoinductive factor, mimecan) |
| 329 | A9451 | NM_018950 | HLA-F | Major histocompatibility complex, class I, F |
| 330 | B4086 | M21574 | PDGFRA | Platelet-derived growth factor receptor, alpha polypeptide |
| 331 | C4095 | NM 002122 | BLA-DQA1 | Major histocompatibility complex, class II, DQ alpha 1 |
| 332 | A6322 | BU623850 | BZRP | Benzodiazapine receptor (peripheral) |
| 333 | A6358 | AK056079 | ATP5J | ATP synthase, H+ transporting, mitochondrial F0 complex, subunit F6 |
| 334 | A6683 | AB088477 | PER1 | Period homolog 1 (Drosophila) |
| 335 | A7230 | NM_001845 | COL4A1 | Collagen, type IV, alpha 1 |
| 336 | A8607 | AK021632 | LOC91526 | Hypothetical protein DKFZp434D2328 |
| 337 | A8682 | AL713770 | FAM31C | Family with sequence similarity 31, member C |
| 338 | A9373 | AK128695 | COL6A2 | Collagen, type VI, alpha 2 |
| 339 | B0563 | AB209058 | HLA-DPA1 | Major histocompatibility complex, class II, DP alpha 1 |
| 340 | B0350 | BM981167 | LOC132671 | Spermatogenesis associated 18 homolog (rat) |
| 341 | B1004 | NM_00453 | MMP2 | Matrix metalloproteinase 2 (gelatinase A, 72kDa gelatinase, 72kDa type IV collagenase) |
| 342 | B1227 | W900*09 | BAZ2A | Fucosyltransferase 1 (galactoside 2-alpha-L-fucosyltransferase) |
| 343 | B4674 | AA149429 | ATP10D | ATPase, Class V, type 10D |
| 344 | B5483 | BC018897 | SLC2A9 | Solute carrier family 2 (facilitated glucose transporter), member 9 |
| 345 | B4111 | BC017059 | IFI16 | Interferon, gamma-inducible protein 16 |
| 346 | B8113 | BC020848 | RNASE6 | Ribonuclease, RNase A family, k6 |
| 347 | A6750 | BQ052434 | NKG7 | Natural killer cell group 7 sequence |
| 348 | A7222 | NM_001911 | CTSG | Cathepsin G |
| 349 | A7809 | N63706 | | Hypothetical LOC201484 |
| 350 | B1676 | BC025985 | IGHG4 | Immunoglobulin heavy constant gamma 4 (G4m marker) |
| 351 | B4060 | NM 001953 | ECGF1 | Endothelial cell growth factor 1 (platelet-derived) |
| 352 | B4288 | AK092766 | OLFML3 | Olfactomedin-like 3 |
| 353 | B5138 | BM678311 | FCN3 | Ficolin (collagen/fibrinogen domain containing) 3 (Hakata antigen) |
| 354 | A6447 | Auk127088 | EPB41L2 | Erythrocyte membrane protein band 4.1-like 2 |
| 355 | A6617 | AF182316 | FER1L3 | Fer-1-like 3, myoferlin (C. elegans) |
| 356 | A8209 | AK090439 | NOD27 | Nucleotide-binding oligomerization domains 27 |
| 357 | A8525 | W67837 | EMP2 | Epithelial membrane protein 2 |
| 358 | A9042 | NM_022349 | MS4A6A | Membrane-spanning 4-domains, subfamily A, member 6A |
| 359 | C4268 | BM975803 | MGC26610 | Hypothetical protein MGC26610 |
| 360 | B6888 | AI347378 | URP2 | UNC-112 related protein 2 |
| 361 | A6751 | NM_002258 | KLRB1 | Killer cell lectin-like receptor subfamily B, member 1 |
| 362 | A6530 | NM_006988 | ADAMTS1 | A disintegrin-like and metalloprotease (reprolysin type) with thrombospondin type 1 motif, 1 |
| 363 | A6731 | AK024428 | PSCD4 | Pleckstrin homology, Sec7 and coiled-coil domains 4 |
| 364 | A7147 | NM_006435 | IFITM2 | Interferon induced transmembrane protein 2(1-8D) |
| 365 | A8152 | NM_002119 | HLA-DOA | Major histocompatibility complex, class II, DO alpha |
| 366 | A8744 | NM_001233 | CAV2 | Caveolin 2 |
| 367 | B4206 | CR594594 | STK17B | Serine/threonine kinase 17b (apoptosis-inducing) |
| 368 | A2257N | BC052998 | DDR2 | Discoidin domain receptor family, member 2 |
| 369 | A3161N | CR597101 | ZFP36 | Zinc finger protein 36, C3H type, homolog (mouse) |
| 370 | A3496 | BC028129 | HK3 | Hexokinase 3 (white cell) |
| 371 | B4130 | BC059394 | LYN | V-yes-1 Yamaguchi sarcoma viral related oncogene homolog |
| 372 | B4750 | NM_004665 | VNN2 | Vanin 2 |
| 373 | B5421 | AA648414 | MS4A1 | Membrane-spanning 4-domains, subfamily A, member 1 |
| 374 | B5842N | AF545852 | MK2S4 | Protein kinase substrate MK2S4 |
| 375 | B6305 | H03606 | NPL | N-acetylneuraminate pyruvate lyase (dihydrodipicolinate synthase) |
| 376 | B7289N | AF146761 | SLAMF8 | SLAM family member 8 |
| 377 | A1871N | NM_198235 | RNASE1 | Ribonuclease, RNase A family, 1 (pancreatic) |
| 378 | A2547N | BM017946 | S100A10 | S100 calcium binding protein A10 (annexin II ligand, calpactin I, light polypeptide (p11)) |
| 379 | A4385N | BC039031 | IL1R2 | Interleukin 1 receptor, type II |
| 380 | A0560N | NM_000618 | IGF1 | Insulin-like growth factor 1 (somatomedin C) |
| 381 | A3439N | BM994174 | HBB | Hemoglobin, beta |
| 382 | A7760N | BC047390 | ARID5A | AT rich interactive domain 5A (MRF1-like) |
| 383 | B3893 | AY549722 | ITLN1 | Intelectin 1 (galactofuranose binding) |
| 384 | B4440 | AB040120 | SLC39A8 | Solute carrier family 39 (zinc transporter), member 8 |
| 385 | B4597 | AK125090 | | CDNA FLJ43100 fis, clone CTONG2003100 |
| 386 | B7122 | AA480009 | DEPDC2 | DEP domain containing 2 |
| 387 | B2559 | CA426475 | HBE1 | Hemoglobin, epsilon 1 |
| 388 | B4852N | BC010954 | CXCL10 | Chemokine (C-X-C motif) ligand 10 |
| 389 | B5372 | BM995690 | YME1L1 | YME1-like 1 (S. cerevisiae) |
| 390 | B5699 | NM_033515 | ARHGAP18 | Rho GTPase activating protein 18 |
| 391 | B6688 | NM_003042 | SLC6A1 | Solute carrier family 6 (neurotransmitter transporter, GABA), member 1 |
| 392 | B7741 | NM_177551 | GPR109A | G protein-coupled receptor 109A |
| 393 | B9533 | W44970 | ATXN7 | Ataxin 7 |
| 394 | A0327N | NM_002421 | MMP1 | Matrix metalloproteinase 1 (interstitial collagenase) |
| 395 | A0774N | BC012613 | CPA3 | Carboxypeptidase A3 (mast cell) |
| 396 | A7247N | AL133118 | EMCN | Endomucin |
| 397 | B4598 | AK130136 | DAB2 | Disabled homolog 2, mitogen-responsive phosphoprotein (Drosophila) |
| 398 | B6414N | AB023171 | C11orf9 | Chromosome 11 open reading frame 9 |
| 399 | B8090 | BC043352 | ZBTB4 | Zinc finger and BTB domain containing 4 |
| 400 | B8265 | AA156792 | HEYL | Hairy/enhancer-of-split related with YRPW motif-like |
| 401 | B8366 | AI342255 | SYNPO2 | Synaptopodin 2 |
| 402 | A0702N | BQ189297 | FLT1 | Fms-related tyrosine kinase 1 (vascular endothelial growth factor/vascular permeability factor receptor) |
| 403 | A7184 | Z18951 | CAV1 | Caveolin 1, caveolae protein, 22kDa |
| 404 | B4137 | NM_053025 | MYLK | Myosin, light polypeptide kinase |
| 405 | B4942 | T79183 | TAX1BP1 | Tax1 (human T-cell leukemia virus type I) binding protein 1 |
| 406 | B5172N | NM_001289 | CLIC2 | Chloride intracellular channel 2 |
| 407 | B5623 | AA505359 | MYO1F | Myosin IF |
| 408 | B7105 | AK055782 | PDLIM2 | PDZ and LIM domain 2 (mystique) |
| 409 | B8036 | R20340 | ATP5S | ATP synthase, H+ transporting, mitochondrial F0 complex, subunit s (factor B) |
| 410 | B9524 | H84724 | | Transcribed locus, strongly similar to XP_213346.2 PREDICTED: similar to 605 ribosomal protein L26 [Rattus norvegicus] |
| 411 | A2632N | NM_003816 | ADAM9 | A disintegrin and metalloproteinase domain 9 (meltrin gamma) |
| 412 | A8786N | NM_003725 | RODH | Hydroxysteroid (17-beta) dehydrogenase 6 |
| 413 | B4396 | W58589 | DDR2 | Discoidin domain receptor family, member 2 |
| 414 | B4603 | BU739773 | AVPI1 | Arginine vasopressin-induced 1 |
| 415 | B5866N | AB040902 | FLRT3 | Fibronectin leucine rich transmembrane protein 3 |
| 416 | B7171 | H75419 | CYBRD1 | Cytochrome b reductase 1 |
| 417 | A0225N | M93426 | PTPRZ1 | Protein tyrosine phosphatase, receptor-type, Z polypeptide 1 |
| 418 | A1818N | NM_033138 | CALD1 | Caldesmon 1 |
| 419 | A3200N | AK122763 | COL5A1 | Collagen, type V, alpha 1 |
| 420 | A0704N | NM_005204 | MAP3K8 | Mitogen-activated protein kinase kinase kinase 8 |
| 421 | B5012 | AA725828 | SOX18 | SRY (sex determining region Y)-box 18 |
| 422 | B8029 | AK092472 | FLT1 | Fms-related tyrosine kinase 1 (vascular endothelial growth factor/vascular permeability factor receptor) |
| 423 | A1779N | AK223296 | LILRB5 | Leukocyte immunoglobulin-like receptor, subfamily B (with TM and ITIM domains), member 5 |
| 424 | A4829N | NM_001650 | AQP4 | Aquaporin 4 |
| 425 | A1146N | NM_001909 | CTSD | Cathepsin D (lysosomal aspartyl protease) |
| 426 | A1471N | M83772 | FMO3 | Flavin containing monooxygenase 3 |
| 427 | A2633N | BX648814 | ANGPT1 | Angiopoietin 1 |
| 428 | A6777 | BQ276959 | LGALS2 | Lectin, galactoside-binding, soluble, 2 (galectin 2) |
| 429 | B4215 | L06175 | HCP5 | HLA complex P5 |
| 430 | B4614 | AL833852 | WWTR1 WW | domain containing transcription regulator 1 |
| 431 | B5151 | BU627644 | 7h3 | Hypothetical protein FLJ13511 |
| 432 | B8924 | AI357442 | SPARC | Secreted protein, acidic, cysteine-rich (osteonectin) |
| 433 | B9790 | BC067746 | CLEC1 | C-type lectin domain family 1, member A |
| 434 | A0038N | W73825 | TCF21 | Transcription factor 21 |
| 435 | A3554 | AK130838 | HLA-DQA1 | Major histocompatibility complex, class II, DQ alpha 1 |
| 436 | A4375N | NM_003617 | RGS5 | Regulator of G-protein signalling 5 |
| 437 | B3232 | AK024979 | LDB2 | LIM domain binding 2 |
| 438 | B4364 | CD365397 | TRPV2 | Transient receptor potential cation channel, subfamily V, member 2 |
| 439 | B5459 | AA666119 | GBP3 | Guanylate binding protein 3 |
| 440 | B8627 | R39044 | RAB27B | RAB27B, member RAS oncogene family |
| 441 | B9616 | AK057418 | NYD-SP21 | Testes development-related NYD-SP21 |
| 442 | A1780N | CR606785 | ENPP2 | Ectonucleotide pyrophosphatase/phosphodiesterase 2 (autotaxin) |
| 443 | A2087N | BC012617 | ACTG2 | Actin, gamma 2, smooth muscle, enteric |
| 444 | A3417 | AK026432 | HCK | Hemopoietic cell kinase |
| 445 | A5148N | NM_006566 | CD226 | CD226 antigen |
| 446 | A0796N | M68891 | GATA2 | GATA binding protein 2 |
| 447 | A1151N | M55618 | TNC | Tenascin C (hexabrachion) |
| 448 | A1807N | BC018986 | HPGD | Hydroxyprostaglandin dehydrogenase 15-(NAD) |
| 449 | A9546N | BQ924772 | LOC124220 | Similar to common salivary protein 1 |
| 450 | B2696 | BC070085 | CSF2RB | Colony stimulating factor 2 receptor, beta, low-affinity (granulocyte-macrophage) |
| 451 | B3889 | BC013042 | MGC7036 | Hypothetical protein MGC7036 |
| 452 | B4643 | AI332375 | FSTL3 | Follistatin-like 3 (secreted glycoprotein) |
| 453 | B5721N | AK024116 | FLJ14054 | Hypothetical protein FLJ14054 |
| 454 | B5949 | M_016293 | BIN2 | Bridging integrator 2 |
| 455 | B7441 | AA994299 | C16orf30 | Chromosome 16 open reading frame 30 |
| 456 | B8656 | AY260577 | C14orf58 | Chromosome 14 open reading frame 58 |
| 457 | B9094 | AF084481 | WFS1 | Wolfram syndrome 1 (wolframin) |
| 458 | A0919N | J05550 | MRC1 | Mannose receptor, C type 1 |
| 459 | A1669 | M95787 | TAGLN | Transgelin |
| 460 | A1253N | X97229 | KIR2DL4 | Killer cell immunoglobulin-like receptor, two domains, long cytoplasmic tail, 4 |
| 461 | A9393N | W67577 | CD74 | CD74 antigen (invariant polypeptide of major histocompatibility complex, class II antigen-associated) |
| 462 | A7232N | BX648421 | IGJ | Immunoglobulin J polypeptide, linker protein for immunoglobulin alpha and mu polypeptides |
| 463 | B3063 | BU753099 | LY86 | Lymphocyte antigen 86 |
| 464 | B4922N | NM_014045 | LRP10 | Low density lipoprotein receptor-related protein 10 |
| 465 | B5081N | AL832416 | C9orf13 | Sushi, von Willebrand factor type A, EGF and pentraxin domain containing 1 |
| 466 | B7193N | BX109986 | | Transcribed locus |
| 467 | B9368 | AF504647 | | Cilia-associated protein (CYS1) |
| 468 | B9777 | NM_030781 | COLEC12 | Collectin sub-family member 12 |
| 469 | B9749 | BQ575959 | HTRA3 | HtrA serine peptidase 3 |
| 470 | A1981 | U58514 | CHI3L2 | Chitinase 3-like 2 |
| 471 | A6696 | NM_012072 | C1QR1 | Complement component 1, q subcomponent, receptor 1 |
| 472 | B1090N | AF361473 | ROBO4 | Roundabout homolog 4, magic roundabout (Drosophila) |
| 473 | B3933 | AY358360 | ELTD1 | EGF, latrophilin and seven transmembrane domain containing 1 |
| 474 | B3966 | BC047724 | C10orf128 | Chromosome 10 open reading frame 128 |
| 475 | B5205N | BC066121 | GPR116 | G protein-coupled receptor 116 |
| 476 | B7922 | NM_181844 BCL6B | | B-cell CLL/lymphoma 6, member B (zinc finger protein) |
| 477 | C4756 | BQ005590 | CCL19 | Chemokine (C-C motif) ligand 19 |
| 478 | C4973 | NM_002658 | PLAU | Plasminogen activator, urokinase |
| 479 | C7592 | NM_003974 | DOK2 | Docking protein 2, 56kDa |
| 480 | C8074 | X79204 | ATXN1 | Ataxin 1 |
| 481 | C8048 | NM_000458 | TCF2 | Transcription factor 2, hepatic; LF-B3; variant hepatic nuclear factor |
| 482 | C6675 | AY358677 | FAM3D | Family with sequence similarity 3, member D |
| 483 | C6882 | AF186022 | DAPP1 | Dual adaptor of phosphotyrosine and 3-phosphoinositides |
| 484 | C6547 | AA776821 | NXF3 | Nuclear RNA export factor 3 |
| 485 | C6721 | BC051881 | CXorf9 | Chromosome X open reading frame 9 |
| 486 | C1703 | W84753 | EPAS1 | Endothelial PAS domain protein 1 |
| 487 | C2019 | AF205940 | EMCN | Endomucin |
| 488 | C4328 | AK023966 | | CDNA FLJ13904 fis, clone THYRO1001895 |
| 489 | C4729 | N70455 | | Marker A mRNA, partial sequence |
| 490 | C7651 | BM560961 | PDLIM3 | PDZ and LIM domain 3 |
| 491 | C7512 | NM_000186 | CFH | Complement factor H |
| 492 | C7687 | CB119523 | IL6ST | Interleukin 6 signal transducer (gp130, oncostatin M receptor) |
| 493 | C8039 | Z22970 | CD163 | CD163 antigen |
| 494 | C9471 | AK090411 | RGPR | Regucalcin gene promotor region related protein |
| 495 | D0946 | BC025720 | KSP37 | Ksp37 protein |
| 496 | C0830 | AA012832 | | CDNA FLJ45341 fis, clone BRHIP3009672 |
| 497 | C1898 | AL713801 | SLAMF7 | SLAM family member 7 |
| 498 | C0893 | BC052210 | GARP | Leucine rich repeat containing 32 |
| 499 | C4116 | NM_001010919 | LOC441168 | Hypothetical protein LOC441168 |
| 500 | C6540 | AA010060 | FLJ33069 | Hypothetical protein FLJ33069 |
| 501 | C6900 | NM_138636 | TLR8 | Toll-like receptor 8 |
| 502 | C7721 | NM_000361 | THBD | Thrombomodulin |
| 503 | C0371 | CA431042 | | Transcribed locus, strongly similar to XP_549577.1 PREDICTED: hypothetical protein XP_549577 [Canis familiaris] |
| 504 | C0922 | AF378757 | PLXDC2 | Plexin domain containing 2 |
| 505 | C1602 | AK093513 | | CDNA FLJ3 6194 fis, clone TESTI2027615 |
| 506 | C3763 | AF480883 | PPAP2B | Phosphatidic acid phosphatase type 2B |
| 507 | C6234 | AI247176 | ABI3BP | ABI gene family, member 3 (NESH) binding protein |
| 508 | C8051 | BM685415 | C10orf116 | Chromosome 10 open reading frame 116 |
| 509 | C8088 | D87465 | SPOCK2 | Sparc/osteonectin, cwcv and kazal-like domains proteoglycan (testican) 2 |
| 510 | C8146 | BF697545 | MGP | Matrix Gla protein |
| 511 | D1273 | AJ001015 | RAMP2 | Receptor (calcitonin) activity modifying protein 2 |
| 512 | C5021 | AI352534 | CAV1 | Caveolin 1, caveolae protein, 22kDa |
| 513 | C6068 | AL831998 | ITGB6 | Integrin, beta 6 |
| 514 | C6974 | AK124567 | HIBCH | 3-hydroxyisobutyryl-Coenzyme A hydrolase |
| 515 | C6687 | BQ006452 | | |
| 516 | C7069 | U16307 | GLIPR1 | GLI pathogenesis-related 1 (glioma) |
| 517 | C8846 | AL023657 | SH2D1A | SH2 domain protein 1A, Duncan's disease (lymphoproliferative syndrome) |
| 518 | C9305 | AI080640 | AGR2 | Anterior gradient 2 homolog (Xenopus laevis) |
| 519 | C9513 | AA094308 | MK2S4 | Protein kinase substrate MK2S4 |
| 520 | C1412 | BX648776 | MSRB3 | Methionine sulfoxide reductase B3 |
| 521 | C1603 | BQ446275 | HBD | Hemoglobin, delta |
| 522 | C1660 | NM_001001927 | MTUS1 | Mitochondrialtumor suppressor 1 |
| 523 | C4979 | BC091497 | HLA-B | Major histocompatibility complex, class I, B |
| 524 | C8228 | AK124641 | CXCL12 | Chemokine (C-X-Cmotif) ligand 12 (stromal cell-derived factor 1) |
| 525 | C7997 | J03565 | CR2 | Complement component (3d/Epstein Barr virus) receptor 2 |
| 526 | C8052 | U28977 | CASP4 | Caspase 4, apoptosis-related cysteine protease |
| 527 | C8345 | NM_006889 | CD86 | CD86 antigen (CD28 antigen ligand 2, B7-2 antigen) |
| 528 | D0735 | AA740582 | | Transcribed locus |
| 529 | D1185 | AA451886 | CYP1B1 | Cytochrome P450, family 1, subfamily B, polypeptide 1 |
| 530 | D1274 | BF435815 | | MRNA; cDNA DKFZp56400862 (from clone DKFZp564O0862) |
| 531 | C1019 | NM_024997 | ATF7IP2 | Activating transcription factor 7 interacting protein 2 |
| 532 | C5025 | AA931221 | | Transcribed locus, strongly similar to XP_531118.1 PREDICTED: hypothetical protein XP_531118 [Pan troglodytes] |
| 533 | C7138 | BM678096 | TNA | C-type lectin domain family 3, member B |
| 534 | C7879 | NM_000688 | ALAS1 | Aminolevulinate, delta-, synthase 1 |
| 535 | C0629 | H16793 | C8orf4 | Chromosome 8 open reading frame 4 |
| 536 | C1604 | AA044381, | | |
| 537 | C4459 | M_012276 | ILT7 | Leukocyte immunoglobulin-like receptor, subfamily A (without TM domain), member 4 |
| 538 | C5014 | AI185804 | FN1 | Fibronectin 1 |
| 539 | C5174 | AL832259 | LOC284749 | Hypothetical protein LOC284749 |
| 540 | C6386 | W05570 | C1QTNF5 | C1q and tumor necrosis factor related protein 5 |
| 541 | C7847 | BM696919 | CRYAB | Crystallin, alpha B |
| 542 | C8044 | NM_004430 | EGR3 | Early growth response 3 |
| 543 | C8786 | AA215586 | LOC389119 | Similar to RIKEN cDNA 6530418L21 |
| 544 | C0335 | CR590615 | ACTA2 | Actin, alpha 2, smooth muscle, aorta |
| 545 | C3746 | NM_199511 | URB | Steroid sensitive gene 1 |
| 546 | C4184 | NM_020482 | FHL5 | Four and a half LIM domains 5 |
| 547 | C7674 | AA148213 | WWTR1 WW | domain containing transcription regulator 1 |
| 548 | C8158 | CR616676 | HP | Haptoglobin |
| 549 | C7773 | AF430643 | GBP5 | Guanylate binding protein 5 |
| 550 | B9924 | CA432542 | ESAM | Endothelial cell adhesion molecule |
| 551 | C4971 | NM_006169 | NNMT | Nicotinamide N-methyltransferase |
| 552 | C4981 | AK074480 | ANXA1 | Annexin A1 |
| 553 | C5016 | BC093009 | PPGB | Protective protein for beta-galactosidase (galactosialidosis) |
| 554 | C6826 | X52203 | LOC91316 | Similar to bK246H3.1 (immunoglobulin lambda-like polypeptide 1, pre-B-cell specific) |
| 555 | C6387 | AI022180 | | Transcribed locus |
| 556 | C7370 | BC037568 | EOMES | Eomesodermin homolog (Xenopus laevis) |
| 557 | C8046 | NM_002864 | PZP | Pregnancy-zone protein |
| 558 | C8006 | M28128 | RNASE3 | Ribonuclease, RNase A family, 3 (eosinophil cationic protein) |
| 559 | C9503 | AA621124 | LOC338773 | Hypothetical protein LOC338773 |
| 560 | C0250 | NM_016730 | FOLR1 | Folate receptor 1 (adult) |
| 561 | C0724 | NM_002725 | PRELP | Proline arginine-rich end leucine-rich repeat protein |
| 562 | C4068 | NM_002621 | PFC | Properdin P factor, complement |
| 563 | C4960 | AI185825 | B2M | Beta-2-microglobulin |
| 564 | C6906 | AK122672 | GPCR5A | G protein-coupled receptor, family C, group 5, member A |
| 565 | C7886 | AI270402 | INHBA | Inhibin, beta A (activin A, activin AB alpha polypeptide) |
| 566 | C8023 | M81141 | HLA-DQB1 | Major histocompatibility complex, class II, DQ beta 1 |
| 567 | C8119 | NM_002775 | PRSS11 | Protease, serine, 11 (IGF binding) |
| 568 | E0507 | BM994142 | HLA-C | Major histocompatibility complex, class I, C |
| 569 | D3727 | AA843148 | LANCL1 | LanC lantibiotic synthetase component C-like 1 (bacterial) |
| 570 | D5261 | BC033490 | LOC285016 | Hypothetical protein LOC285016 |
| 571 | D7152 | NM_003387 | WASPIP | Wiskott-Aldrich syndrome protein interacting protein |
| 572 | D7468 | BC010943 | OSMR | Oncostatin M receptor |
| 573 | D1727 | M59911 | ITGA3 | Integrin, alpha 3 (antigen CD49C, alpha 3 subunit of VLA-3 receptor) |
| 574 | D3149 | AF338109 | PACAP | Proapoptotic caspase adaptor protein |
| 575 | D9799 | AI074177 | C1QA | Complement component 1, q subcomponent, alpha polypeptide |
| 576 | E0691 | BC067086 | BTN3A2 | Butyrophilin, subfamily 3, member A2 |
| 577 | D4511 | AW402154 | | Similar to MHC HLA-SX-alpha |
| 578 | D9839 | BE855441 | | Hypothetical LOC401131 |
| 579 | D4936 | NM_000908 | NPR3 | Natriuretic peptide receptor C/guanylate cyclase C (atrionatriuretic peptide receptor C) |
| 580 | D4503 | NM_144673 | CKLFSF2 | Chemokine-like factor super family 2 |
| 581 | D4978 | BG622766 | C6orf189 | Chromosome 6 open reading frame 189 |
| 582 | D8491 | NM_001122 | ADFP | Adipose differentiation-related protein |
| 583 | E0733 | NM_004684 | SPARCL1 | SPARC-like 1 (mast9, hevin) |
| 584 | D1758 | U14394 | TIMP3 | Tissue inhibitor of metalloproteinase 3 (Sorsby fundus dystrophy, pseudoinflammatory) |
| 585 | D8910 | AF455138 | STEAP2 | Six transmembrane epithelial antigen of the prostate 2 |
| 586 | E0176 | AI090671 | FLJ12057 | Hypothetical protein FLJ12057 |
| 587 | D3351 | BC072670 | MGC16044 | Hypothetical protein MGC16044 |
| 588 | D6472 | AI160370 | MGC26963 | Hypothetical protein MGC26963 |
| 589 | D8933 | BX538309 | MAMDC2 | MAM domain containing 2 |
| 590 | B0289 | AK098225 | R-spondin | Likely ortholog of mouse roof plate-specific spondin |
| 591 | E0644 | NM_000610 | CD44 | CD44 antigen (homing function and Indian blood group system) |
| 592 | E1622 | NM_001753 | CAV1 | Caveolin 1, caveolae protein, 22kDa |
| 593 | D3086 | AK123160 | MGC24133 | Hypothetical protein MGC24133 |
| 594 | D3831 | AW978852 | | Transcribed locus |
| 595 | D4744 | AW504569 | | Transcribed locus, moderately similar to XP_522527.1 PREDICTED: similar to carnitine deficiency-associated gene expressed in ventricle 1 [Pan troglodytes] |
| 596 | D5083 | BM673802 | ACE | Angiotensin I converting enzyme (peptidyl-dipeptidase A) 1 |
| 597 | D8527 | CR613409 | CA2 | Carbonic anhydrase II |
| 598 | D9397 | BX360819 | IQSEC3 | IQ motif and Sec7 domain 3 |
| 599 | D3194 | AA634405 | | Transcribed locus, weakly similar to NP_908973.1 ring finger protein 29 isoform 1; muscle specific ring finger 2 [Homo sapiens] |
| 600 | D4050 | C06094 | LRAP | Leukocyte-derived arginine aminopeptidase |
| 601 | D4885 | AI139813 | | Similar to polycystin 1-like 3 |
| 602 | D4980 | AA919126 | MHC2TA | MHC class II transactivator |
| 603 | D7349 | AI016360 | FLJ40873 | Hypothetical protein FLJ40873 |
| 604 | D8515 | NM_002345 | LUM | Lumican |
| 605 | D1767 | BC014357 | CCND2 | Cyclin D2 |
| 606 | D6360 | NM_021233 | DNASE2B | Deoxyribonuclease II beta |
| 607 | D9290 | NM_022153 | PP2135 | Chromosome 10 open reading frame 54 |
| 608 | E0706 | A298180 | MMP7 | Matrix metalloproteinase 7 (matrilysin, uterine) |
| 609 | E0716 | BG012035 | NPC2 | Niemann-Pick disease, type C2 |
| 610 | D4128 | NM_173060 | CAST | Calpastatin |
| 611 | D4189 | W93113 | WNT2 | Wingless-type MMTV integration site family member 2 |
| 612 | D4428 | BM992880 | NF1 | Neurofibromin 1 (neurofibromatosis, von Recklinghausen disease, Watson disease) |
| 613 | D4731 | BX648450 | T3JAM | TRAF3-interacting Jun N-terminal kinase (JNK)-activating modulator |
| 614 | D7150 | AB037886 | ABI3 | ABI gene family, member 3 |
| 615 | D8412 | NM_024508 | ZBED2 | Zinc finger, BED domain containing 2 |
| 616 | E0336 | AI097529 | EPAS1 | Endothelial PAS domain protein 1 |
| 617 | D4035 | BC005839 | FSTL3 | Follistatin-like 3 (secreted glycoprotein) |
| 618 | D4622 | AK127644 | | Homo sapiens, Similar to AD038, clone IMAGE:3838464, mRNA |
| 619 | D8827 | BQ030224 | CERKL | Ceramide kinase-like |
| 620 | D9990 | BQ717155 | | Transcribed locus |
| 621 | E0358 | AK021543 | DNM3 | Dynamin 3 |
| 622 | E0593 | NM_017458 | MVP | Major vault protein |
| 623 | E1436 | AK123803 | DAB2 | Disabled homolog 2, mitogen-responsive phosphoprotein (Drosophila) |
| 624 | A6232N | NM_005795 | CALCRL | Calcitonin receptor-like |
| 625 | A0834N | X06948 | FCER1A | Fc fragment of IgE, high affinity I, receptor for; alpha polypeptide |
| 626 | A1040 | BQ432639 | CCL2 | Chemokine (C-C motif) ligand 2 |
| 627 | C8476 | R59552 | CHRDL1 | Chordin-like 1 |
| 628 | C9661 | NM_005771 | DHRS9 | Dehydrogenase/reductase (SDR family) member 9 |
| 629 | F0049 | NM_007289 | MME CALLA, | Membrane metallo-endopeptidase (neutral endopeptidase, enkephalinase, CD10) |
| 630 | D6878 | AI002365 | PDGFRB | Platelet-derived growth factor receptor, beta polypeptide |
| 631 | F0916 | NM_000686 | AGTR2 | Angiotensin II receptor, type 2 |
| 632 | F0496 | NM_006926 | SFTPA2 A2 | Surfactant, pulmonary-associated protein |
| 633 | F1046 | NM_014583 | LMCD1 | LIM and cysteine-rich domains 1 |
| 634 | F1457 | M16006 | SERPINE1 | Serine (or cysteine) proteinase inhibitor, clade E (nexin, plasminogen activator inhibitor type 1), member 1 |
| 635 | F6208 | XM_058513 | LRRK2 | Leucine-rich repeat kinase 2 |
| 636 | A7428 | NM_002121 | HLA-DPB1 | Major histocompatibility complex, class II, DP beta 1 |
| 637 | A3096 | CR601701 | ANXA3 | Annexin A3 |
| 638 | A8575 | NM_145641 | APOL3 | ApolipoproteinL,3 |
| 639 | B7430N | AA522674 | LIMPS2 | G protein-coupled receptor 17 |
| 640 | B4689 | AB183546 | GPR126 | G protein-coupled receptor 126 |
| 641 | B9057 | AF361494 | SOSTDC1 | Sclerostin domain containing 1 |
| 642 | F0169 | NM_178445 | CCRL1 | Chemokine (C-C motif) receptor-like 1 |
| 643 | F0352 | NM_018414 | SIAT7A | ST6 (alpha-N-acetyl-neuraminyl-2,3-beta-galactosyl-1,3)-N-acetylgalactosaminide alpha-2,6-sialyltransferase 1 |
| 644 | F1974 | M36634 | VIP | Vasoactive intestinal peptide |
| 645 | F2686 | CR616854 | EVI2B | Ecotropic viral integration site 2B |
| 646 | F2724 | AK024275 | FLJ14213 | Hypothetical protein FLJ14213 |
| 647 | A0203N | AB209361 | FAS | Fas (TNF receptor superfamily, member 6) |
| 648 | F0018 | NM_000963 | PTGS2 | Prostaglandin-endoperoxide synthase 2 (prostaglandin G/H synthase and cyclooxygenase) |
| 649 | A0359N | BC015753 | CXCL2 | Chemokine (C-X-C motif) ligand 2 |
| 650 | A6274 | Y13710 | CCL18 | Chemokine (C-C motif) ligand 18 (pulmonary and activation-regulated) |
| 651 | C0081 | NM_182485 | CPEB2 | Cytoplasmic polyadenylation element binding protein 2 |
| 652 | F1429 | AK021639 | CXorf21 | Chromosome X open reading frame 21 |
| 653 | F1750 | AK022379 | B2M | Beta-2-microglobulin |
| 654 | F2986 | AK027232 | LBH | Likely ortholog of mouse limb-bud and heart gene |
| 655 | F3562 | AK001862 | FLJ11000 | Hypothetical protein FLJ11000 |
| 656 | F4498 | AK023683 | KIAA0635 | Centrosomal protein 4 |
| 657 | F6249 | AL117617 | RBMS1 | RNA binding motif, single stranded interacting protein 1 |
| 658 | A1122N | D90402 | EDNRB | Endothelin receptor type B |
| 659 | A7284N | AF297711 | NTN4 | Netrin 4 |
| 660 | A3258 | U19487 | PTGER2 | Prostaglandin E receptor 2 (subtype EP2), 53kDa |
| 661 | C6124 | NM_002989 | CCL21 | Chemokine (C-C motif) ligand 21 |
| 662 | F1976 | AB029496 | LOC56920 | Semaphorin sem2 |
| 663 | F4950 | NM_194430 | RNASE4 | Angiogenin, ribonuclease, RNase A family, 5 |
| 664 | F6595 | AW938336 | | CDNA FLJ26188 fis, clone ADG04821 |
| 665 | A0005N | NM_153683 | KL | Klotho |
| 666 | A4037N | AF159456 | DMBT1 | Deleted in malignant brain tumors 1 |
| 667 | G2548 | NM_001430 | EPAS1 | Endothelial PAS domain protein 1 |
| 668 | B6183N | NM_172200 | IL15RA | Interleukin 15 receptor, alpha |
| 669 | B2304N | BI832920 | HCST | Hematopoietic cell signal transducer |
| 670 | F3564 | CR749667 | PDE4B | Phosphodiesterase 4B, cAMP-specific (phosphodiesterase E4 dunce homolog, Drosophila) |
| 671 | F5634 | XM_376412 | KIAA0825 | KIAA0825 protein |
| 672 | F6116 | BC030244 | TNNC1 | Troponin C, slow |
| 673 | F7457 | BQ276976 | PIP | Prolactin-induced protein |
| 674 | F1252 | AB023193 | NTNG1 | Netrin G1 |
| 675 | A3453 | BC041790 | TNFAIP3 | Tumor necrosis factor, alpha-induced protein 3 |
| 676 | B5089N | AA828067 | C1QB | Complement component 1, q subcomponent, beta polypeptide |
| 677 | B7331 | W15232 | EHD2 | EH-domain containing 2 |
| 678 | B9086 | BC032365 | SH2D3C | SH2 domain containing 3C |
| 679 | F0196 | AL050224 | PTRF | Polymerase I and transcript release factor |
| 680 | F0266 | NM_000878 | IL2RB | Interleukin 2 receptor, beta |
| 681 | F1121 | CR621445 | IFI44 | Interferon-induced protein 44 |
| 682 | F1134 | NM_001460 | FMO2 | Flavin containing monooxygenase 2 |
| 683 | F2465 | U88878 | TLR2 | Toll-like receptor 2 |
| 684 | B3745 | N92541 | | Transcribed locus |
| 685 | B2139 | NM_020530 | OSM | Oncostatin M |
| 686 | F0035 | NM_000779 | CYP4B1 | Cytochrome P450, family 4, subfamily B, polypeptide 1 |
| 687 | F0121 | AF089854 | TU3A | TU3A protein |
| 688 | F0911 | L08177 | EBI2 | Epstein-Barr virus induced gene 2 (lymphocyte-specific G protein-coupled receptor) |
| 689 | F2245 | AY198414 | PRDM1 | PR domain containing 1, with ZNF domain |
| 690 | F2392 | NM_001901 | CTGF | Connective tissue growth factor |
| 691 | F7458 | BC089435 | ADAMTS8 | A disintegrin-like and metalloprotease (reprolysin type) with thrombospondin type 1 motif, 8 |
| 692 | F3558 | AB033030 | CDGAP | KIAA1204 protein |
| 693 | A7140N | BX103455 | CCL3 | Chemokine (C-C motif) ligand 3 |
| 694 | A7440 | BC053585 | CSF3R | Colony stimulating factor 3 receptor (granulocyte) |
| 695 | B7499 | BX641020 | ARID5B | AT rich interactive domain 5B (MRF1-like) |
| 696 | B9118 | NM_018384 | GIMAP5 | GTPase, IMAP family member 5 |
| 697 | F0267 | NM_007312 | HYAL1 | Hyaluronoglucosaminidase 1 |
| 698 | F0566 | M32315 | TNFRSF1B | Tumor necrosis factor receptor superfamily, member 1B |
| 699 | F2076 | AL162032 | GPR133 | G protein-coupled receptor 133 |
| 700 | F3313 | AK025164 | FLJ21511 | Hypothetical protein FLJ21511 |
| 701 | F5985 | AF011333 | LY75 | Lymphocyte antigen 75 |
| 702 | F5702 | AK024358 | MPEG1 | Macrophage expressed gene 1 |
| 703 | A0279 | NM_005257 | GATA6 | GATA binding protein 6 |
| 704 | F0004 | NM_005252 | FOS | V-fos FBJ murine osteosarcoma viral oncogene homolog |
| 705 | B6424 | AL049313 | CLIC5 | Chloride intracellular channel 5 |
| 706 | C8355 | NM_006762 | LAPTM5 | Lysosomal associated multispanning membrane protein 5 |
| 707 | C0484 | NM_005472 | KCNE3 | Potassium voltage-gated channel, Isk-related family, member 3 |
| 708 | F0528 | AK025661 | LIMS1 | LIM and senescent cell antigen-like domains 1 |
| 709 | F0471 | AK025015 | FLJ13955 | Hypothetical protein FLJ13955 |
| 710 | F1525 | M24736 | SELE | selection E (endothelial adhesion molecule 1) |
| 711 | F2253 | U52513 | IFIT3 | Interferon-induced protein with tetratricopeptide repeats 3 |
| 712 | F3502 | X05409 | ALDH2 | Aldehyde dehydrogenase 2 family (mitochondrial) |
| 713 | F5638 | NM_004669 | CLIC3 | Chloride intracellular channel 3 |
| 714 | F5279 | L76566 | HLA-DRB6 | Major histocompatibility complex, class II, DR beta 6 (pseudogene) |
| 715 | F4556 | AF151978 | SLC6A14 | Solute carrier family 6 (amino acid transporter), member 14 |
| 716 | F6365 | AL080114 | C10orf72 | Chromosome 10 open reading frame 72 |
| 717 | A2762N | BI819219 | SCGB1A1 | Secretoglobin, family 1A, member 1 (uteroglobin) |
| 718 | F0200 | AL832950 | FLJ31033 | Hypothetical protein FLJ31033 |
| 719 | F0213 | NM_002908 | REL | V-reI reticuloendotheliosis viral oncogene homolog (avian) |
| 720 | E0382 | AF178930 | CARD15 | Caspase recruitment domain family, member 15 |
| 721 | F0288 | BC080187 | LMOD1 | Leiomodin 1 (smooth muscle) |
| 722 | F0671 | XM_047357 | LBA1 | Lupus brain antigen 1 |
| 723 | F4989 | AK023309 | LOC286126 | Hypothetical protein LOC286126 |
| 724 | A1022N | M98399 | CD36 | CD36 antigen (collagen type I receptor, thrombospondin receptor) |
| 725 | F0915 | M55284 | PRKCH | Protein kinase C, eta |
| 726 | F2335 | AK001832 | FLJ10970 | Hypothetical protein FLJ10970 |
| 727 | F2918 | AF376061 | CARD12 | Caspase recruitment domain family, member 12 |
| 728 | F5669 | NM_004585 | RARRES3 | Retinoic acid receptor responder (tazarotene induced) 3 |
| 729 | F6994 | BM920112 | PSMB9 | Proteasome (prosome, macropain) subunit, beta type, 9 (large multifunctional protease 2) |
| 730 | A7263 | XM_039877 | MUC5B | Mucin 5, subtype B, tracheobronchial |
| 731 | B4276 | AK056725 | ACVRL1 | Activin A receptor type II-like 1 |
| 732 | C6775 | AA708738 | | Transcribed locus |
| 733 | D9503 | AA928598 | | |
| 734 | F0304 | X76534 | GPNMB | Glycoprotein (transmembrane) nmb |
| 735 | F1343 | BC032404 | EVE1 | SH3 domain protein D19 |
| 736 | F1225 | AF118108 | XLKD1 | Extracellular link domain containing 1 |
| 737 | F3459 | AF046888 | TNFSF13 | Tumor necrosis factor (ligand) superfamily, member 12 |
| 738 | F6844 | AK023947 | | |
| 739 | F0238 | AK001872 | PDCD1LG2 | Programmed cell death 1 ligand 2 |
| 740 | B1756 | NM_017520 | HSMPP8 | M-phase phosphoprotein, mpp8 |
| 741 | F0890 | AK022272 | PRKCE | Protein kinase C, epsilon |
| 742 | F2909 | AK021490 | | |
| 743 | G2841 | AI271559 | SYT1 | Synaptotagmin I |
| 744 | G6276 | NM_178456 | C20orf85 | Chromosome 20 open reading frame 85 |
| 745 | B2509 | R85681 | | |
| 746 | G0040 | NM_147156 | TMEM23 | Transmembrane protein 23 |
| 747 | G2128 | AL080208 | DMXL1 | Dmx-like 1 |
| 748 | G8306 | AK124472 | CPVL | Carboxypeptidase, vitellogenic-like |
| 749 | F1349 | AK001903 | INHBA | Inhibin, beta A (activin A, activin AB alpha polypeptide) |
| 750 | G3717 | BM977979 | | Transcribed locus |
| 751 | G8521 | U27109 | MMRN1 | Multimerin 1 |
| 752 | F2950 | AK000865 | NPAS3 | Neuronal PAS domain protein 3 |
| 753 | F4504 | U85992 | BMPER | BMP-binding endothelial regulator precursor protein |
| 754 | G2260 | NM_182920 | ADAMTS9 | A disintegrin-like and metalloprotease (reprolysin type) with thrombospondin type 1 motif, 9 |
| 755 | G2274 | BC047894 | ATXN1 | Ataxin 1 |
| 756 | G3573 | AK054824 | NCAM1 | Neural cell adhesion molecule 1 |
| 757 | G3585 | BC022570 | MGC27121 | MGCZ7121 gene |
| 758 | G8708 | AJ315733 | ADAMTS15 | A disintegrin-Iike and metalloprotease (reprolysin type) with thrombospondin type 1 motif, 15 |
| 759 | B2314N | R41489 | | Hypothetical LOC388617 |
| 760 | G2622 | AF378754 | ARHGEF17 | Rho guanine nucleotide exchange factor (GEF) 17 |
| 761 | G4063 | NM_001010923 | C6orf190 | chromosome 6 open reading frame 190 |
| 762 | G8295 | AF301016 | CXCL16 | Chemokine (C-X-C motif) ligand 16 |
| 763 | G4051 | AL832347 | CMYA5 | Cardiomyopathy associated 5 |
| 764 | G6000 | BC075802 | ARC | Activity-regulated cytoskeleton-associated protein |
| 765 | F6698 | NM_001295 | CCR1 | Chemokine (C-C motif) receptor 1 |
| 766 | G2317 | AL512703 | | |
| 767 | F1371 | AJ271684 | CLECSF5 | C-type lectin domain family 5, member A |
| 768 | G3132 | AL713738 | IL7R | Interleukin 7 receptor |
| 769 | G3911 | AK097866 | CDH4 | Cadherin 4, type 1, R-cadherin (retinal) |
| 770 | G6001 | H87471 | KYNU | Kynureninase (L-kynurenine hydrolase) |
| 771 | G8416 | CR626671 | TFPI | Tissue factor pathway inhibitor (lipoprotein-associated coagulation inhibitor) |
| 772 | F0889 | AK022052 | EPHA6 | EPH receptor A6 |
| 773 | G1752 | AL390176 | | FP6778 |
| 774 | G2698 | CA306377 | ALOX5AP | Arachidonate 5-lipoxygenase-activating protein |
| 775 | G3001 | NM_003956 | CH25H | Cholesterol 25-hydroxylase |
| 776 | G8762 | AA778816 | CD36 | CD36 antigen (collagen type I receptor, thrombospondin receptor) |

**Table 3 Up-regulated genes in SCLC**

| Asignment NO | LMMID | GenBank ID | Symbol | Gene name |
|---|---|---|---|---|
| 777 | A0289 | U46838 | MCM6 | MCM6 minichromosome maintenance deficient 6 (MIS5 homolog, S. pombe) (S. cerevisiae) |
| 778 | A0692 | X57548 | CDH2 | Cadherin 2, type 1, N-cadherin (neuronal) |
| 779 | A0627 | NM_003185 | TAF4 | TAF4 RNA polymerase II, TATA box binding protein (TBP)-associated factor, 135kDa |
| 780 | A0777 | M58583 | CBLN1 | Cerebellin 1 precursor |
| 781 | A1957 | U20979 | CHAFIA | Chromatin assembly factor 1, subunit A (p150) |
| 782 | A2361 | NM_003362 | UNG | Uracil-DNA glycosylase |
| 783 | A6175 | AI967994 | LOC81691 | Exonuclease NEF-sp |
| 784 | A2955 | BM921123 | TFF3 | Trefoil factor 3 (intestinal) |
| 785 | A3526 | BQ423966 | RQCD1 | RCD1 required for cell (S. pombe) differentiation1 homolog (S. pombe) |
| 786 | A3565 | L10678 | PFN2 | Profilin 2 |
| 787 | A3700 | U87864 | NEURL | Neuralized-like (Drosophila) |
| 788 | A4513 | Z21488 | CNTN1 | Contactin 1 |
| 789 | A4616 | AJ007669 | FANCG | Fanconi anemia, complementation group G |
| 790 | A4831 | D83017 | NELL1 | NEL-like 1 (chicken) |
| 791 | A5243 | AF070541 | LOC284244 | Hypothetical protein LOC284244 |
| 792 | A5313 | BM462481 | KIF1A | Kinesin family member 1A |
| 793 | A5821 | AI671006 | DKFZP564B167 | DKFZP564B167 protein |
| 794 | A0167 | NM 001790 | CDC25C | Cell division cycle 25C |
| 795 | A0238 | U01828 | MAP2 | Microtubule-associated protein 2 |
| 796 | A0748 | M76180 | DDC | Dopa decarboxylase (aromatic L-amino acid decarboxylase) |
| 797 | A6111 | NM 018105 | THAP1 | THAP domain containing, apoptosis associated protein 1 |
| 798 | A0833 | BC021085 | SORD | Sorbitol dehydrogenase |
| 799 | A1286 | AF034633 | GPR39 | G protein-coupled receptor 39 |
| 800 | A1589 | U97188 | IMP-3 | IGF-II mRNA-binding protein 3 |
| 801 | A1294 | BC041382 | CAPON | C-terminal PDZ domain ligand of neuronal nitric oxide synthase |
| 802 | A2466 | AJ223728 | CDC45L | CDC45 cell division cycle 45-like (S. cerevisiae) |
| 803 | A2755 | BC011262 | PHGDH | Phosphoglycerate dehydrogenase |
| 804 | A3315 | BQ876913 | NPY | Neuropeptide Y |
| 805 | A6223 | AF456477 | MAPT | Microtubule-associated protein tau |
| 806 | A3477 | NM_000920 | PC | Pyruvate carboxylase |
| 807 | A3717 | U93869 | POLR3F | Polymerase (RNA) III (DNA directed) polypeptide F, 39 kDa |
| 808 | A4024 | AK091336 | STMN2 | Stathmin-like 2 |
| 809 | A4873 | BC017723 | MAGEA4 | Melanoma antigen family A, 4 |
| 810 | A5324 | AI357641 | CDKN2C | Cyclin-dependentkinase inhibitor 2C (p18, inhibits CDK4) |
| 811 | A6102 | R71596 | | Transcribed locus |
| 812 | A0547 | NM_001527 | HDAC2 | Histone deacetylase 2 |
| 813 | A1605 | NM_203401 | STMN1 | Stathmin 1/oncoprotein 18 |
| 814 | A1550 | NM_198700 | CUGBP1 | CUG triplet repeat, RNA binding protein 1 |
| 815 | A2593 | BC093053 | SGNE1 | Secretory granule, neuroendocrine protein 1 (7B2 protein) |
| 816 | A6158 | NM_005909 | MAP1B | Microtubule-associated protein 1B |
| 817 | A2670 | X74142 | FOXG1B | Forkhead box G1B |
| 818 | A2735 | BC036811 | PTHR2 | Parathyroid hormone receptor 2 |
| 819 | A2978 | X04741 | UCHL1 | Ubiquitin carboxyl-terminal esterase L1 (ubiquitin thiolesterase) |
| 820 | A3058 | NM_202002 | FOXM1 | Forkhead box M1 |
| 821 | A2708 | NM 005513 | GTF2E1 | General transcription factor IIE, polypeptide 1, alpha 56kDa |
| 822 | A3095 | U26726 | HSD11B2 | Hydroxysteroid (11-beta) dehydrogenase 2 |
| 823 | A3341 | AB209177 | PAX6 | Paired box gene 6 (aniridia, keratitis) |
| 824 | A3668 | U76362 | SLC1A7 | Solute carrier family 1 (glutamate transporter), member 7 |
| 825 | A4966 | NM_001389 | DSCAM | Down syndrome cell adhesion molecule |
| 826 | A5282 | AW975611 | | Transcribed locus |
| 827 | A5544 | BC070049 | LANCL2 | LanC lantibiotic synthetase component C-like 2 (bacterial) |
| 828 | A0303 | U79240 | PASK | PAS domain containing serine/threonine kinase |
| 829 | A0024 | AF017790 | KNTC2 | Kinetochore associated 2 |
| 830 | A1198 | U61849 | NPTX1 | Neuronal pent-axin I |
| 831 | A2029 | BC034227 | D4S234E | DNA segment on chromosome 4 (unique) 234 expressed sequence |
| 832 | A2610 | NM_020546 | ADCY2 | Adenylate cyclase 2 (brain) |
| 833 | A2796 | NM_006681 | NMU | Neuromedin U |
| 834 | A2827 | X51698 | TFF2 | Trefoil factor 2 (spasmolytic protein 1) |
| 835 | A3243 | CR624652 | TTK | TTK protein kinase |
| 836 | A3272 | K02054 | GRP | Gastrin-releasing peptide |
| 837 | A6247 | L10374 | | (clone CTG-A4) mRNA sequence |
| 838 | A4128 | CB530031 | GNAS | GNAS complex locus |
| 839 | A4273 | NM_017495 | RNPC1 | RNA-binding region (RNP1, RRM) containing 1 |
| 840 | A4906 | NM_025263 | PRR3 | Proline rich 3 |
| 841 | A4914 | NM_000841 | GRM4 | Glutamate receptor, metabotropic 4 |
| 842 | A5325 | R20639 | DPYSL5 | Dihydropyrimidinase-like 5 |
| 843 | A5456 | AL833943 | MGC8407 | CaM kinase-like vesicle-associated |
| 844 | A5403 | AK023284 | TEX27 | Testis expressed sequence 27 |
| 845 | A5623 | AF044588 | PRC1 | Protein regulator of cytokinesis 1 |
| 846 | A0333 | NM 002466 | MYBL2 oncogene | V-myb myeloblastosis viral homolog (avian)-like 2 |
| 847 | A0397 | U04641 | PC | Pyruvate carboxylase |
| 848 | A0812 | M16937 | HOXB7 | Homeo box B7 |
| 849 | A1209 | NM 001071 | TYMS | Thymidylate synthetase |
| 850 | A6122 | AB040529 | MAGED4 | Melanoma antigen family D, 4 |
| 851 | A1683 | U16954 | AF1Q | ALL1-fused gene from chromosome |
| 852 | A2254 | NM_006845 | KIF2C | Kinesin family member'2C |
| 853 | A3088 | A046378 | DNTT | Deoxynucleotidyltransferase, terminal |
| 854 | A3117 | NM_000412 | HRG | Histidine-rich glycoprotein |
| 855 | A3669 | U76388 | NR5A1 | Nuclear receptor subfamily 5, group A, member 1 |
| 856 | A3765 | X60673 | AK3 | Adenylate kinase 3-like 1 |
| 857 | A5601 | H19339 | | MRNA; cDNA DKFZp547G036 (from clone PKFZp547G036) |
| 858 | A5513 | BC000567 | SEZ6L2 | Seizure related 6 homolog (mouse)-like 2 |
| 859 | A6027 | AK095553 | CACNG3 | Calcium channel, voltage-dependent, gamma subunit 3 |
| 860 | A0018 | NM_198433 | STK6 | Serine/threonine kinase 6 |
| 861 | A0245 | BC010044 | CDC20 | CDC20 cell division cycle 20 homolog (S. cerevisiae) |
| 862 | A0499 | BM912233 | CKS2 | CDC28 protein kinase regulatory subunit 2 |
| 863 | A1223 | X73502 | KRT20 | Keratin 20 |
| 864 | A1564 | U70370 | PITX1 | Paired-like homeodomain transcription factor 1 |
| 865 | A6127 | AI356291 | GPT2 | Glutamic pyruvate transaminase (alanine aminotransferase) 2 |
| 866 | A1766 | S78296 | INA | Internexin neuronal intermediate filament protein, alpha |
| 867 | A1966 | X81438 | AMPH | Amphiphysin (Stiff-Man syndrome with breast cancer 128kDa autoantigen) |
| 868 | A1841 | NM_004203 | PKMYT1 | Protein kinase, membrane associated tyrosine/threonine 1 |
| 869 | A4468 | NM 206900 | RTN2 | Reticulon 2 |
| 870 | A5367 | BX537405 | RANBP6 | RAN binding protein 6 |
| 871 | A5653 | AA455657 | ZNF184 | Zinc finger protein 184 (Kruppel-like) |
| 872 | A5404 | NM_004438 | EPHA4 | EPH receptor A4 |
| 873 | A5787 | CA313915 | KIAA0460 | KIAA0460 protein |
| 874 | A5916 | AA536187 | SLC24A5 | Solute carrier family 24, member 5 |
| 875 | A0004 | AB003698 | CDC7 | CDC7 cell division cycle 7 (S. cerevisiae) |
| 876 | A0269 | NM_000465 | BARD1 | BRCA1 associated RING domain 1 |
| 877 | A0813 | S82986 | HOXC6 | Homeo box C6 |
| 878 | A1618 | X70683 | SOX4 | SRY (sex determining region Y)-box 4 |
| 879 | A2673 | X16135 | HNRPL | Heterogeneous nuclear ribonucleoprotein L |
| 880 | A3539 | NM 001275 | CHGA | Chromogranin A (parathyroid secretory protein 1) |
| 881 | A3677 | U79666 | CACNA1A | Calcium channel, voltage-dependent, P/Q type, alpha 1A subunit |
| 882 | A4193 | BU737730 | RBP1 | Retinol binding protein 1, cellular |
| 883 | A4345 | BC039257 | NUP155 | Nucleoporin 155kDa |
| 884 | A4546 | M92299 | HOXB5 | Homeo box B5 |
| 885 | A4553 | NM_004111 | FEN1 | Flap 1 structure-specific endonuclease |
| 886 | A4959 | AF042282 | EXO1 | Exonuclease 1 |
| 887 | A4900 | NM 004725 | BUB3 | BUB3 budding uninhibited by benzimidazoles 3 homolog (yeast) |
| 888 | A0437 | AF047002 | THOC4 | THO complex 4 |
| 889 | A0917 | AF036268 | SH3GL2 | SH3-domain GRB2-like 2 |
| 890 | A0895 | D78012 | CRMP1 | Collapsin response mediator protein 1 |
| 891 | A1231 | X83957 | NEB | Nebulin |
| 892 | A1767 | M93107 | BDH | 3-hydroxybutyrate dehydrogenase (heart, mitochondrial) |
| 893 | A1912 | BC052996 | CTNNA2 | Catenin (cadherin-associated protein), alpha 2 |
| 894 | A2154 | NM_001033 | RRM1 | Ribonucleotide reductase M1 polypeptide |
| 895 | A2382 | AB208895 . | EZH2 | Enhancer of zeste homolog 2 (Drosophila) |
| 896 | A2420 | D38073 | MCM3 | MCM3 minichromosome maintenance deficient 3 (S. cerevisiae) |
| 897 | A3077 | NM_000921 | PDE3A | Phosphodiesterase 3A, cGMP-inhibited |
| 898 | A2807 | X02404 | CALCB | Calcitonin-related polypeptide, beta |
| 899 | A3378 | L20814 | GRIA2 | Glutamate receptor, ionotropic, AMPA 2 |
| 900 | A6229 | CR613811 | SNRPD1 | Small nuclear ribonucleoprotein D1 polypeptide 16kDa |
| 901 | A3274 | NM_001809 | CENPA | Centromere protein A, 17kDa |
| 902 | A3298 | M91670 | UBE2S | Ubiquitin-conjugating enzyme E2S |
| 903 | A4792 | NM_005723 | TM4SF9 | Tetraspanin 5 |
| 904 | A4854 | BC000442 | AURKB | Aurora kinase B |
| 905 | A5048 | BC014941 | ID4 | Inhibitor of DNA binding 4, dominant negative helix-loop-helix protein |
| 906 | A6036 | AJ132932 | DCT | Dopachrome tautomerase (dopachrome delta-isomerase, tyrosine-related protein 2) |
| 907 | A5713 | AK074119 | ZZZ3 | Zinc finger, ZZ domain containing 3 |
| 908 | A5884 | BC065204 | FLJ35348 | FLJ35348 |
| 909 | A0157 | NM_031966 | CCNB1 | Cyclin B1 |
| 910 | A0429 | BM554470 | UBE2C | Ubiquitin-conjugating enzyme E2C |
| 911 | A6139 | BU730831 | PAFAH1B3 | Platelet-activating factor acetylhydrolase, isoform Ib, gamma subunit 29kDa |
| 912 | A2282 | BC014039 | MELK | Maternal embryonic leucine zipper kinase |
| 913 | A2596 | BC000375 | CHGB | Chromogranin B (secretogranin 1) |
| 914 | A2934 | CR621534 | NUTF2 | Nuclear transport factor 2 |
| 915 | A3151 | M83712 | CHRNA5 | Cholinergic receptor, nicotinic, alpha polypeptide 5 |
| 916 | A4110 | NM_001976 | ENO3 | Enolase 3 (beta, muscle) |
| 917 | A4383 | Z97029 | RNASEH2A | Ribonuclease H2, large subunit |
| 918 | A4417 | BC025381 | CLULI | Clusterin-like 1 (retinal) |
| 919 | A5207 | CA422300 | MAC30 | Hypothetical protein MAC30 |
| 920 | A5157 | AF027153 | | EST |
| 921 | A5579 | R41754 | KIAA1906 | KIAA1906 protein |
| 922 | A5696 | BC050464 | MGC16824 | Esophageal cancer associated protein |
| 923 | A0207 | M73812 | CCNE1 | Cyclin El |
| 924 | A0724 | NM_001520 | GTF3C1 | General transcription factor IIIC, polypetide 1, alpha 220kDa. |
| 925 | A1257 | BC006992 | RAD51AP1 | RAD51 associated protein 1 |
| 926 | A1641 | NM_002968 | SALL1 | Sal-like I (Drosophila) |
| 927 | A1835 | U18018 | ETV4 | Ets variant gene 4 (E1A enhancer binding protein, E1AF) |
| 928 | A6152 | XM_376018 | KIAA1644 | KIAA1644 protein |
| 929 | A2498 | L11932 | SHMT2 | Serine hydroxymethyltransferase 2 (mitochondrial) |
| 930 | A2448 | AF010314 | ENC1 | Ectodermal-neural cortex (with BTB-like domain) |
| 931 | A2996 | U11287 | GRIN2B | Glutamate receptor, ionotropic, N-methyl D-aspartate 2B |
| 932 | A4021 | D38081 | | EST |
| 933 | A4563 | J04088 | TOP2A | Topoisomerase (DNA) II alpha 170kDa |
| 934 | A4849 | NM_000555 | DCX | Doublecortex; lissencephaly, X-linked (doublecortin) |
| 935 | A4946 | NM 005284 | GPR6 | G protein-coupled receptor 6 |
| 936 | A5400 | AK122818 | BTBD11 | BTB (POZ) domain containing 11 |
| 937 | A6073 | AI290541 | | CDNAFLJ11723 fis, clone HEMBA1005314 |
| 938 | A5918 | BX648117 | ZNF6 | Zinc finger protein 6 (CMPX1) |
| 939 | A1787 | U30872 | CENPF | Centromere protein F,350/400ka (mitosin) |
| 940 | A1995 | M14745 | BCL2 | B-cell CLL/lymphoma 2 |
| 941 | A4259 | BC073991 | ENO1 | Enolase 1, (alpha) |
| 942 | A4807 | AJ001515 | RYR3 | Ryanodine receptor 3 |
| 943 | A4814 | NM 004209 | SYNGR3 | Synaptogyrin 3 |
| 944 | A0098 | NM 016841 | MAPT | Microtubule-associated protein tau |
| 945 | A0763 | NM_001478 | GALGT | UDP-N-acetyl-alpha-D-galactosamine:(N-acetylneuraminyl)-galactosylglucosylceramide N-acetylgalactosaminyltransferase (GaINAe-T) |
| 946 | A1970 | BC000356 | MAD2L1 | MAD2 mitotic arrest deficient-like 1 (yeast) |
| 947 | A2450 | NM_001740 | CALB2 | Calbindin 2, 29kDa (calretinin) |
| 948 | A2837 | BU618918 | CDKN3 | Cyclin-dependent kinase inhibitor 3 (CDK2-associated dual specificity phosphatase) |
| 949 | A3004 | NM_000037 | ANK1 | Ankyrin 1, erythrocytic |
| 950 | A3885 | AF117758 | SFRP5 | Secreted frizzled-related protein 5 |
| 951 | A4492 | NM_152246 | CPT1B | Carnitine palmitoyltransferase 1B (muscle) |
| 952 | A4438 | AF055015 | EYA2 | Eyes absent homolog 2 (Drosophila) |
| 953 | A5589 | H24317 | | EST |
| 954 | A5657 | BQ219156 | FiSPC150 | Ubiquitin-conjugating enzyme E2T (putative) |
| 955 | A5911 | AK125888 | FBXO32 | F-box protein 32 |
| 956 | A5919 | AL117393 | KIF5C | Kinesin family member 5C |
| 957 | A6618 | BC040293 | | Clone 23555 mRNA sequence |
| 958 | A7608 | NM_005189 | MGC10561 | Chromobox homolog 2 (Pc class homolog, Drosophila) |
| 959 | A7112 | D83699 | | Harakiri, BCL2 interacting protein (contains only BH3 domain) |
| 960 | A8287 | R87657 | DKFZp762E1312 | Hypothetical protein DKFZp762E1312 |
| 961 | A9172 | AB037848 | SYT13 | Synaptotagmin XIII |
| 962 | B1221 | BU076403 | MOSPD1 | Motile sperm domain containing 1 |
| 963 | B2003 | AA676866 | CIT | Citron (rho-interacting, serine/threonine kinase 21) |
| 964 | B4069 | NM_004415 | DSP | Desmoplakin |
| 965 | B4861 | X14850 | H2AFX | H2A histone family, member X |
| 966 | B4239 | NM_058179 | PSAT1 | Phosphoserine aminotransferase 1 |
| 967 | C4276 | NM_001407 | CELSR3 | Cadherin, EGF LAG seven-pass G-type receptor 3 (flamingo homolog, Drosophila) |
| 968 | A6625 | BX538010 | NRCAM | Neuronal cell adhesion molecule |
| 969 | A6661 | CR737409 | | Transcribed locus |
| 970 | A6891 | BU616541 | PIAS2 | Protein inhibitor of activated STAT, 2 |
| 971 | A8458 | AA490987 | SLC35B3 | Solute carrier family 35, member B3 |
| 972 | A8624 | XM_087225 | | Similar to male-specific lethal 3-like 1 isoform a; drosophila MSL3-like 1 |
| 973 | A9538 | AA564637 | SMC2L1 | SMC2 structural maintenance of chromosomes 2-like 1 (yeast) |
| 974 | B2699 | AA702785 | HMGN3 | High mobility group nucleosomal binding domain 3 |
| 975 | B4113 | BC044591 | WASF1 | WAS protein family, member 1 |
| 976 | B6579 | AK126500 | APEG1 | Aortic 1 preferentially expressed gene |
| 977 | A6384 | NM_004378 | CRABP1 | Cellular retinoic acid binding protein |
| 978 | A7787 | BC066913 | RAB26 | RAB26, member RAS oncogene family |
| 979 | A8928 | R38549 | | Hypothetical gene supported by AK098833 |
| 980 | A9139 | AF056085 | GPR51 | G protein-coupled receptor 51 |
| 981 | A9820 | AI215798 | SPIRE2 | Spire homolog 2 (Drosophila) |
| 982 | B0811 | AW183154 | KIF14 | Kinesin family member 14 |
| 983 | B1303 | AI674977 | SR140 | U2-associated SR140 protein |
| 984 | B2004 | AW085193 | KCNK9 | Potassium channel, subfamily K, member 9 |
| 985 | B4864 | NM_002145 | HOXB2 | Homeo box B2 |
| 986 | B9222 | AF450487 | KIF21A | Kinesin family member 21A |
| 987 | A7143 | BM473942 | NME1 | Non-metastatic cells 1, protein (NM23A) expressed in |
| 988 | A7780 | NM_005650 | TCF20 | Transcription factor 20 (AR1) |
| 989 | A9047 | NM_004626 | WNT11 | Wingless-type MMTV integration site family, member 11 |
| 990 | B4211 | AI142644 | HSF2 | Heat shock transcription factor 2 |
| 991 | A6670 | AB018279 | SV2A | Synaptic vesicle glycoprotein 2A |
| 992 | A7908 | AA490691 | HOXD11 | Homeo box D11 |
| 993 | A8066 | AL136588 | DKFZp761D112 | Hypothetical protein DKFZp761D112 |
| 994 | A8922 | AK090707 | KCNK9 | Potassium channel, subfamily K, member 9 |
| 995 | B0812 | AF306679 | ESCO2 | Establishment of cohesion 1 homolog 2 (S. cerevisiae) |
| 996 | B2515 | BG674807 | HCN3 | Hyperpolarization activated cyclic nucleotide-gated potassium channel 3 |
| 997 | B6769 | AF032862 | HMMR | Hyaluronan-mediated motility receptor (RHAMM) |
| 998 | A9304 | AA812940 | MLLT6 | Myeloid/lymphoid or mixed-lineage leukemia (trithorax homolog, Drosophila); translocated to, 6 |
| 999 | A9280 | AW136599 | HUNK | Hormonally upregulated Neu-associated kinase |
| 1000 | B0978 | AA633743 | GNG3 | Guanine nucleotide binding protein (G protein), gamma 3 |
| 1001 | B2909 | CR625760 | TOP2A | Topoisomerase (DNA) II alpha 170kDa |
| 1002 | B2185 | AA678952 | SUV420H2 | Suppressor of variegation 4-20 homolog 2 (Drosophila) |
| 1003 | B2346 | AA669023 | PCDH9 | Protocadherin 9 |
| 1004 | A6842 | AB043585 | RPRM | Reprimo, TP53 dependant G2 arrest mediator candidate |
| 1005 | A9165 | AB209499 | CACNA1E | Calcium channel, voltage-dependent, alpha 1E subunit |
| 1006 | C2323 | AB011127 | KIAA0555 | Jak and microtubule interacting protein 2 |
| 1007 | C4885 | BM977947 | CIB2 | Calcium and integrin binding family member 2 |
| 1008 | A6598 | BM677885 | RASL11B | RAS-like, family 11, member B |
| 1009 | B2824 | BC050557 | TIMELESS | Timeless homolog (Drosophila) |
| 1010 | A6900 | R58925 | RUFY2 | RUN and FYVE domain containing 2 |
| 1011 | A7024 | BU734286 | RBP1 | Retinol binding protein 1, cellular |
| 1012 | A6869 | BC011665 | TCF3 | Transcription factor 3 (E2A immunoglobulin enhancer binding factors E12/E47) |
| 1013 | B0075 | BM671360 | BRUNOL4 | Bruno-like 4, RNA binding protein (Drosophila) |
| 1014 | B0657 | BC000336 | SCGN | Secretagogin, EF-hand calcium binding protein |
| 1015 | B0286 | BC069280 | HIST1H4D | Histone 1, H4d |
| 1016 | B0979 | BC030666 | MGC33993 | Ring finger protein 182 |
| 1017 | B3668 | AA004208 | KIF4A | Kinesin family member 4A |
| 1018 | B8243 | Cm_031689 | MGA | MAX gene associated |
| 1019 | C3700 | BC080569 | GTF2IRD1 | GTF2I repeat domain containing 1 |
| 1020 | A6615 | AL833942 | SEPT3 | Septin 3 |
| 1021 | A7432 | M32313 | SRD5A1 | Steroid-5-alpha-reductase, alpha polypeptide 1 (3-oxo-5 alpha-steroid delta 4-dehydrogenase alpha 1) |
| 1022 | A7870 | NM_018492 | PBK | PDZ binding kinase |
| 1023 | B0296 | AA025738 | RPIP8 | RaP2 interacting protein 8 |
| 1024 | A6759 | CR605190 | CBX5 | Chromobox homolog 5 (HP1 alpha homolog, Drosophila) |
| 1025 | A7027 | AY037298 | ELOVL4 | Elongation of very long chain fatty acids (FEN1/Elo2, SUR4/Elo3, yeast)-like 4 |
| 1026 | A7146 | X53655 | NTF3 | Neurotrophin 3 |
| 1027 | A7724 | BC004988 | FEM1A | Fem-1 homolog a (C.elegans) |
| 1028 | A8598 | NM 001005389 | NFASC | Neurofascin |
| 1029 | B4097 | CR596974 | MARCKSL1 | MARCKS-likel 1 |
| 1030 | C0565 | BC025725 | CXorf50 | Chromosome X open reading frame 50 |
| 1031 | A6673 | AL137430 | LOC283070 | Hypothetical protein LOC283070 |
| 1032 | A6769 | M_002594 | PCSK2 | Protein convertase subtilisin/kexin type 2 |
| 1033 | A7991 | AA858368 | TUBB | Tubulin, beta polypeptide |
| 1034 | B1119 | AI215478 | HMMR | Hyaluronan-mediated motility receptor (RHAMM) |
| 1035 | C3611 | BC025714 | PPP3CA | Protein phosphatase 3 (formerly 2B), catalytic subunit, alpha isoform (calcineurin A alpha) |
| 1036 | C4066 | NM_013259 | TAGLN3 | Transgelin 3 |
| 1037 | A6666 | BU728456 | RIMS2 | Regulating synaptic membrane exocytosis 2 |
| 1038 | A6914 | R61693 | SUV420H2 | Suppressor of variegation 4-20 homolog 2 (Drosophila) |
| 1039 | A8381 | AA766314 | RASSF6 | Ras association (RalGDS/AF-6) domain family 6 |
| 1040 | B0164 | NM_001012271 | BIRC5 | Baculoviral IAP repeat-containing 5 (survivin) |
| 1041 | A5678N | BC037346 | TMPO | Thymopoietin |
| 1042 | B3027 | AL832036 | FLJ40629 | Hypothetical protein FLJ40629 |
| 1043 | B3467 | AK127169 | FLJ14624 | Hypothetical protein FLJ14624 |
| 1044 | B5461 | R56840 | MCM8 | MCM8 minichromosome maintenance deficient 8 (S. cerevisiae) |
| 1045 | B8296 | AA192306 | TRDN | Triadin |
| 1046 | B8658 | CA429220 | SKP2 | S-phase kinase-associated protein 2 (p45) |
| 1047 | A6636 | NM_138967 | SCAMP5 | Secretory carrier membrane protein 5 |
| 1048 | A9044 | BC003186 | Pfs2 | DNA replication complex GINS protein PSF2 |
| 1049 | A8913N | CA427305 | SMAD2 | SMAD, mothers against DPP homolog 2 (Drosophila) |
| 1050 | B3206 | AI492066 | JMJD1A | Jumonji domain containing 1A |
| 1051 | B4566 | BC056909 | DDA3 | Differential display and activated by p53 |
| 1052 | B4535 | BC007217 | BRD9 | Bromodomain containing 9 |
| 1053 | B5904 | BC008947 | C10orf3 | Chromosome 10 open reading frame |
| 1054 | B6570N | BX57I741 | KIF3C | Kinesin family member 3C |
| 1055 | B6723 | AK096415 | KLHL11 | Kelch-like 11 (Drosophila) |
| 1056 | B6813 | BX092653 | | Transcribed locus, strongly similar to NP_002137.3 homeo box B3; homeo box 2G; homeobox protein Hox-B3 [Homo sapiens] |
| 1057 | B7534 | A298501 | SDK1 | Sidekick homolog 1 (chicken) |
| 1058 | B8870 | NM_018685 | ANLN | Anillin, actin binding protein (scraps homolog, Drosophila) |
| 1059 | B9850 | N63620 | | CDNAFLJ39261 fis, clone OCBBF2009391 |
| 1060 | A0220 | BC017452 | RFC4 | Replication factor C (activator 1) 4, 37kDa |
| 1061 | A3529N | D89016 | ARHGEF16 | Rho guanine exchange factor (GEF) 16 |
| 1062 | A8047 | BU187168 | TP53BP1 | Tumor protein p53 binding protein, 1 |
| 1063 | A5346N | AA747005 | WNK2 | WNK lysine deficient protein kinase 2 |
| 1064 | A9236N | BX117945 | | Transcribed locus, strongly similar to NP_000843.1 glutathione transferase; deafness, X-linked 7; fatty acid ethyl ester synthase III [Homo sapiens] |
| 1065 | B1253N | NM_005915 | MCM6 | MCM6 minichromosome maintenance deficient 6 (MIS5 homolog, S. pombe) (S. cerevisiae) |
| 1066 | B4821N | BC008366 | DDC | Dopa decarboxylase (aromatic L-amino acid decarboxylase) |
| 1067 | B5169 | AF177227 | CKAP2 | Cytoskeleton associated protein 2 |
| 1068 | B5412N | CR590914 | FLJ10156 | Family with sequence similarity 64, member A |
| 1069 | B6599 | AW299854 | PFKFB2 | 6-phosphofnzcto-2-kinase/fructose-2,6-biphosphatase 2 |
| 1070 | B8035 | AL834240 | KIAA1576 | KIAA1576 protein |
| 1071 | B9480 | AB018345 | KIAA0802 | KIAA0802 |
| 1072 | A2059N | M81883 | GAD1 | Glutamate decarboxylase 1 (brain, 67kDa) |
| 1073 | B3049 | BC009333 | UNC5A | Unc-5 homolog A (C. elegans) |
| 1074 | B3536 | BX091598 BX091598 | | Homo sapiens, clone IMAGE:5750475, mRNA |
| 1075 | B4168 | AA665612 | HSPA4 | Heat shock 70kDa protein 4 |
| 1076 | B4925N | AI168314 | NBEA | Neurobeachin |
| 1077 | B5865 | AJ249900 | SMOC1 | SPARC related modular calcium binding 1 |
| 1078 | B7303 | H10302 | KIAA1853 | KIAA1853 protein |
| 1079 | B7475 | R49594 | | Transcribed locus, moderately similartoNP_775622.1 expressed sequence AW121567[Mus musculus] |
| 1080 | B8043 | AK124568 | | CDNA FLJ37441 fis, clone . BRAWH2006543 |
| 1081 | A0061 | AF053306 | BUB1B | BUB1 budding uninhibited by benzimidazoles 1 homolog beta (yeast) |
| 1082 | A6224 | U55970 | LOC147343 | Hypothetical protein LOC147343 |
| 1083 | A9617N | BX109949 | FAM24A | Family with sequence similarity 24, member A |
| 1084 | B2980 | AA858174 | | Homo sapiens, clone IMAGE:3839141, mRNA |
| 1085 | B2863 | NM_178155 | FUT8 | Fucosyltansferase 8 (alpha (1,6) fucosyltransferase) |
| 1086 | B4456 | BX537652 | FLJ12892 | Coiled-coil domain containing 14 |
| 1087 | B4512 | AK123362 | COCH | Coagulation factor C homolog, cochlin (Limulus polyphemus) |
| 1088 | B7281 | NM_058186 | FAM3B | Family with sequence similarity 3, member B |
| 1089 | B7113 | AF061573 | PCDH8 | Protocadherin 8 |
| 1090 | B7197N | R07614 | | EST |
| 1091 | B8070 | AL110252 | GDAP1 | Gangliosule-induced differentiation-associated protein 1 |
| 1092 | B8213 | AA729769 | LOC112476 | Similar to lymphocyte antigen 6 complex, locus G5B; G5b protein; open reading frame 31 |
| 1093 | B8756 | D84294 | TTC3 | Tetratricopeptide repeat domain 3 |
| 1094 | B9060 | AB028641 | SOX11 | SRY (sex determining region Y)-box 11 |
| 1095 | A2065N | AK124656 | ENO2 | Enolase 2 (gamma, neuronal) |
| 1096 | A2574N | NM_213621 | HTR3A | 5-hydroxytryptamine (serotonin) receptor 3A |
| 1097 | A9518N | AA570186 | | Hypothetical gene supported by AK096951;BC066547 |
| 1098 | B5150N | NM_016065 | MRPS 16 | Mitochondrial ribosomal protein S16 |
| 1099 | B6180 | AF052098 | LGI2 | Leucine-rich repeat LGI family, member 2 |
| 1100 | B6283 | AY257469 | CIT | Citron (rho-interacting, serine/threonine kinase 21) |
| 1101 | B6539 | NM_198270 | NHS | Nance-Horan syndrome (congenital cataracts and dental anomalies) |
| 1102 | B7439 | N51406 | FLJ14503 | Hypothetical protein FLJ14503 |
| 1103 | B8194 | BX112665 | NOL4 | Nucleolar protein 4 |
| 1104 | B8448 | AK025598 | FLJ21945 | Hypothetical protein FLJ21945 |
| 1105 | B8631 | AB075826 | KIAA1946 | KIAA1946 |
| 1106 | B8367 | BC036011 | PKIB | Protein kinase (cAMP-dependent, catalytic) inhibitor beta |
| 1107 | B9340 | T78186 | DNMT3A | DNA (cytosine-5-)-methyltransferase 3 alpha |
| 1108 | A2000 | BC014564 | MEST | Mesoderm specific transcript homolog (mouse) |
| 1109 | A1221N | AA714394 | HMGB2 | High-mobility group box 2 |
| 1110 | A7506N | AF124726 | ACIN1 | Apoptotic chromatin condensation inducer 1 |
| 1111 | A8318N | CR602279 | ENC1 | Ectodermal-neural cortex (with BTB-like domain) |
| 1112 | B2587 | BC038986 | REV3L | REV3-like, catalytic subunit of DNA polymerase zeta (yeast) |
| 1113 | B3640 | BM668692 | MGC2654 | Hypothetical protein MGC2654 |
| 1114 | B3425 | CB051043 | | Transcribed locus |
| 1115 | B4513 | AB033078 | SGPL1 | Sphingosine-1-phosphate lyase 1 |
| 1116 | B6353 | R19310 | RELN | Reelin |
| 1117 | B6383 | R39854 | SLC35F1 | Solute carrier family 35, member F1 |
| 1118 | B8889 | T10122 | T1 | Tularik gene 1 |
| 1119 | B8503 | AF225426 | FMN2 | Formin 2 |
| 1120 | B8902 | AI280015 | FLJ25555 | Hypothetical protein FLJ25555 |
| 1121 | B9303 | AK129960 | LOC92558 | Hypothetical protein LOC92558 |
| 1122 | B3010 | BX537920 | SENP1 | SUMO1/sentrin specific protease |
| 1123 | B3732 | BC014851 | LFNG | Lunatic fringe homolog (Drosophila) |
| 1124 | B3942 | AA191573 | SYNJ2 | Synaptojanin 2 |
| 1125 | B3971 | AF290612 | NUSAP1 | Nucleolar and spindle associated protein 1 |
| 1126 | B4030 | AK055793 | C20orf129 | Chromosome 20 open reading frame 129 |
| 1127 | B5434N | NM 152329 | PPIL5 | Peptidylprolyl isomerase (cyclophilin)-like 5 Solute carrier family 7 (cationic |
| 1128 | B5992 | NM_003045 | SLC7A1 | amino acid transporter, y+ system), member 1 |
| 1129 | B8059 | BCO11000 | CDCA5 | Cell division cycle associated 5 |
| 1130 | B8234 | AF070632 | | Clone 24405 mRNA sequence |
| 1131 | B9542 | AA001410 | DKFZP434I2117 | Family with sequence similarity 57, member B |
| 1132 | B9855 | F10439 | | EST |
| 1133 | A0907N | NM_016083 | CNR1 | Cannabinoid receptor 1 (brain) |
| 1134 | A2515 | X16396 | MTHFD2 | Methylenetetrahydrofolate dehydrogenase (NADP+ dependent) 2, methenyltetrahydrofolate cyclohydrolase |
| 1135 | A6857N | BC015152 | MGC33584 | Hypothetical protein MGC33584 |
| 1136 | B3950 | AK023245 | FLJ21144 | Hypothetical protein FLJ21144 |
| 1137 | B3876 | BG354581 | CDCA8 | Cell division cycle associated 8 |
| 1138 | B4587 | AB096683 | MGC57827 | Similar to RIKEN cDNA 2700049P18 gene |
| 1139 | B5013 | T90472 | TBC1D7 | TBC1 domain family, member 7 |
| 1140 | B5281 | BC050525 | USP1 | Ubiquitin specific protease 1 |
| 1141 | B6647 | XM 350880 | PPMLH | Protein phosphatase 1H (PP2C domain containing) |
| 1142 | B6693 | AW968496 | PAX5 | Paired box gene 5 (B-cell lineage specific activator) |
| 1143 | B7367 | CR616479 | AMACR | Alpha-methylacyl-CoA racemase |
| 1144 | B7889N | AB051529 | DISP2 | Dispatched homolog 2 (Drosophila) |
| 1145 | A4045N | BE538546 | PMCH | Pro-melanin-concentrating hormone |
| 1146 | A7820 | AK091904 | | CDNA FLJ34585 fis, clone KIDNE2008758 |
| 1147 | A8297N | BX648236 | BHC80 | PHD finger protein 21A |
| 1148 | B3781 | AK056473 | FAM33A | Family with sequence similarity 33, member A |
| 1149 | B4447 | NM_032287 | LDOC1L | Leucine zipper, down-regulated in cancer 1-like |
| 1150 | B4688 | BC036521 | ASF1B | ASF1 anti-silencing function 1 homolog B (S. cerevisiae) |
| 1151 | B5478 | AA018042 | PARI | Prader-Willi/Angelman region-1 |
| 1152 | B5765 | CR617576 | | Hypothetical LOC400813 |
| 1153 | B5860N | BM683578 | DEPDC1 | DEP domain containing 1 |
| 1154 | B6369 | AU152505 | MAPK8 | Mitogen-activated protein kinase 8 |
| 1155 | B6190 | AF169675 | FLRT1 | Fibronectin leucine rich transmembrane protein 1 |
| 1156 | B6595N | BC009493 | DOLPP1 | Dolichyl pyrophosphate phosphatase 1 |
| 1157 | B6968 | BC016950 | MGC2610 | Phosphatase, orphan 2 |
| 1158 | B7805 | R91157 | KIAA1467 | Serotonin-7 receptor pseudogene |
| 1159 | B8597 | H05706 | | EST |
| 1160 | B9234 | NM_173582 | PGM2L1 | Phosphoglucomutase 2-like 1 |
| 1161 | B9322 | R61893 | MAP3K4 | Mitogen-activated protein kinase kinase kinase 4 |
| 1162 | A0969N | NM_001873 | CPE | Carboxypeptidase E |
| 1163 | A2753N | BC009924 | NPTX2 | Neuronal pentraxin II |
| 1164 | A6574N | NM 032932 | RAB11FIP4 | RAB11 family interacting protein 4 (class II) |
| 1165 | A6909 | NM_018667 | SMPD3 | Sphingomyelin phosphodiesterase 3, neutral membrane (neutral sphingomyelinase II) |
| 1166 | B3160N | AA778238 | LOC374654 | Kinesin family member 7 |
| 1167 | B4319N | NM_017934 | PHIP | Pleckstrin homology domain interacting protein |
| 1168 | B4915N | NM_1 75864 | CBFA2T2 | Core-binding factor, runt domain, alpha subunit 2; translocated to, 2 |
| 1169 | B4788N | AA776829 | | Transcribed locus, strongly similar to XP_496265.1 PREDICTED: hypothetical protein XP_496265 [Homo sapiens] |
| 1170 | B5382N | AK125194 | MAP1B | Microtubule-associated protein 1B |
| 1171 | B6357 | BC000157 | LOC51058 | Hypothetical protein LOC51058 |
| 1172 | B6264 | H70605 | FLJ21148 | Hypothetical protein FLJ21148 |
| 1173 | B9393 | BC067362 | SAMD10 | Sterile alpha motif domain containing 10 |
| 1174 | B9353 | AF429308 | TSGA14 | Testis specific, 14 |
| 1175 | B9838 | AA018510 | Cl8orf54 | Chromosome 18 open reading frame |
| 1176 | A2603N | Z46629 | SOX9 | SRY (sex determining region Y)-rbox 9 (campomelic dysplasia, autosomal sex-reversal) |
| 1177 | A7435N | BC004312 | IGFBP2 | Insulin-like growth factor binding protein 2, 36kDa |
| 1178 | A5656N | CR624273 | DSCR2 | Down syndrome critical region gene 2 |
| 1179 | B1221N | CX166508 | MOSPD1 | Motile sperm domain containing 1 |
| 1180 | B3542N | AA804242 | FLJ12973 | Hypothetical protein FLJ12973 |
| 1181 | B6082 | BX537781 | FNDC5 | Fibronectin type III domain containing 5 |
| 1182 | B6346 | BC044632 | TCF19 | Transcription factor 19 (SC1) |
| 1183 | B6379 | NM_033512 | TSPYL5 | TSPY-like5 |
| 1184 | B6879 | BG260518 | | Arsenic transactivated protein 1 |
| 1185 | B7622 | AB051490 | ZNF407 | Zinc finger protein 407 |
| 1186 | B8105 | AI023320 | | Hypothetical LOC387790 |
| 1187 | B8716 | AY376439 | ECT2 | Epithelial cell transforming sequence 2 oncogene |
| 1188 | B8415 | BC053858 | ZNF550 | Zinc finger protein 550 |
| 1189 | B8882 | BC005832 | KIAA0101 | KIAA0101 |
| 1190 | B9324 | AI192179 | | Transcribed locus |
| 1191 | B9860 | AA921341 | LPGAT1 | Lysophosphatidylglycerol acyltransferase 1 |
| 1192 | B9973 | BC035561 | FLJ23825 | Hypothetical protein FLJ23825 |
| 1193 | C0213 | BX110085 | | Transcribed locus |
| 1194 | C0468 | BX648336 | ZNF451 | KIAA1702 protein |
| 1195 | C0741 | AK090555 | KIAA0676 | KIAA0676 protein |
| 1196 | C1701 | H60869 | | EST |
| 1197 | C1730 | BU682808 | GNAS | GNAS complex locus |
| 1198 | C4591 | N66152 | | Transcribed locus |
| 1199 | C4641 | BF115786 | ZCCHC11 | Zinc finger, CCHC domain containing 11 |
| 1200 | C4825 | BX106774 | DMXL1 | Dmx-like |
| 1201 | C4786 | N72266 | LOC90110 | Hypothetical protein LOC90110 |
| 1202 | C5144 | F22544 | ANK1 | Ankyrin 1, erythrocytic |
| 1203 | C5431 | AW080025 | TEBP | Unactive progesterone receptor, 23 kD |
| 1204 | C6986 | NM_020946 | KIAA1608 | KIAA1608 |
| 1205 | C6425 | W94690 | | Full length insert cDNA clone ZE04G11 |
| 1206 | C6915 | AW016811 | | cDNA: FLJ22648 fis, clone HSI07329 |
| 1207 | C7152 | AI338356 | SPPL3 | Signal peptide peptidase 3 |
| 1208 | C7252 | AB037820 | MARCH-IV | Membrane-associated ring finger (C3HC4) 4 |
| 1209 | C7658 | AA143060 | MUM1 | Melanoma associated antigen (mutated) 1 |
| 1210 | C7977 | AL833463 | LOC283658 | Hypothetical protein LOC283658 |
| 1211 | C8621 | AW195492 | | Transcribed locus, weakly similar to NP_000541.1 tyrosinase-related protein 1 [Homo sapiens] |
| 1212 | D0470 | BC011873 | MTRF1L | Mitochondrial translational release factor 1-like |
| 1213 | D0952 | AI014551 | ACTR1B | ARP1 actin-related protein 1 homolog B, centractin beta (yeast) |
| 1214 | D1223 | C609058 | DLX5 | Distal-less homeo box 5 |
| 1215 | C0715 | BE620837 | KLP1 | K562 cell-derived leucine-zipper-like protein 1 |
| 1216 | C1372 | BP386622 | PCSK1N | Proprotein convertase subtilisin/kexin type 1 inhibitor |
| 1217 | C1609 | NM_000315 | PTH | Parathyroid hormone |
| 1218 | C2050 | BF060678 | C14orf118 | Chromosome 14 open reading frame 118 |
| 1219 | C4149 | AI668649 | | Transcribed locus |
| 1220 | C4539 | AK025455 | C14orfl69 | Chromosome 14 open reading frame 169 |
| 1221 | C5971 | BG396731 | TMSNB | Thymosin-like 8 |
| 1222 | C7573 | AA781731 | FLJ20364 | Hypothetical protein FLJ20364 |
| 1223 | C7706 | BC008667 | PANK2 | Pantothenate kinase 2 (Hallervorden-Spatz syndrome) |
| 1224 | C8802 | AA436403 | FZD3 | Frizzled homolog 3 (Drosophila) |
| 1225 | D0715 | AK126649 | | CDNA FLJ44692 fis, clone BRACE3013986 |
| 1226 | D1311 | AA461492 | SPINK5L3 | Serine PI Kazal type 5-like 3 |
| 1227 | D1320 | AK131393 | WTAP | Wilms tumor 1 associated protein |
| 1228 | C0227 | N49962 | BCL2 | B-cell CLL/lymphoma 2 |
| 1229 | C0743 | H23209 | | CDNA FLJ37694 fis, clone BRHIP2015224 |
| 1230 | C1928 | A310956 | | Transcribed locus, weakly similar to XP_543946.1 PREDICTED: similar to chromosome 10 open reading frame 12 [Canis familiaris] |
| 1231 | C4099 | N37039 | CHMP1.5 | Chromatin modifying protein 1B |
| 1232 | C4284 | AL834247 | MYPN | Myopalladin |
| 1233 | C4464 | AA514648 | LAMA1 | Laminin, alpha 1 |
| 1234 | C4909 | BM665681 | C6orfl29 | Chromosome 6 open reading frame 129 |
| 1235 | C7393 | BU169416 | SEC11L3 | SEC11-like 3 (S. cerevisiae) |
| 1236 | C8084 | U36448 | CADPS | Ca2+-dependent secretion activator |
| 1237 | C8701 | AA195938 | | Full-length cDNA clone CS0DI011YD16 of Placenta Cot 25-normalized of Homo sapiens (human) |
| 1238 | C8825 | AA706627 | | Transcribed locus |
| 1239 | C9858 | NM_006892 | DNMT3B | DNA (cytosine-5-)-methyltransferase 3 beta |
| 1240 | C1063 | BC035771 | RAD1 | RAD1 homolog (S. pombe) |
| 1241 | C2259 | CA436350 | | Transcribed locus |
| 1242 | C3711 | AU253494 | FARP1 | FERM, RhoGEF (ARHGEF) and pleckstrin domain protein 1 (chondrocyte-derived) |
| 1243 | C3688 | BC075836 | RBBP4 | Retinoblastoma binding protein 4 |
| 1244 | C4303 | BC014476 | GKAP1 | G kinase anchoring protein 1 |
| 1245 | C4541 | AI701591 | | Transcribed locus |
| 1246 | C7766 | NM_021174 | KIAA1967 | KIAA1967 |
| 1247 | D0006 | NM_145697 | CDCA1 | Cell division cycle associated 1 |
| 1248 | D1350 | AK022625 | LOC92270 | Hypothetical protein LOC92270 |
| 1249 | C0911 | BU728526 | FLJ14768 | Hypothetical protein FLJ14768 |
| 1250 | C2290 | XM_044178 | KIAA1211 | KIAA1211 protein |
| 1251 | C4175 | BM683457 | EPHA7 | EPH receptor A7 |
| 1252 | C5153 | AK093996 | C9orf52 | Chromosome 9 open reading frame 52 |
| 1253 | C6909 | BX537704 | ALS2CR13 | Amyotrophic lateral sclerosis 2 (juvenile) chromosome region, candidate 13 |
| 1254 | C8624 | NM_005858 | AKAP8 | A kinase (PRKA) anchor protein 8 |
| 1255 | D0919 | BC030692 | ELAVL2 | ELAV (embryonic lethal, abnormal vision, Drosophila)-like 2 (Hu antigen B) |
| 1256 | D1058 | BX105057 | BSN | Bassoon (presynaptic cytomatrix protein) |
| 1257 | C1388 | BM675070 | HIST1H2BD | Histone1, H2bd |
| 1258 | C1869 | BC046365 | LOC253012 | Hypothetical protein LOC253012 |
| 1259 | C2298 | AF260237 | HES6 | Hairy and enhancer of split 6 (Drosophila) |
| 1260 | C4573 | CR599655 | TIGD3 | Tigger transposable element derived |
| 1261 | C7230 | BC009563 | | Homo sapiens, clone IMAGE:3901628, mRNA |
| 1262 | C7529 | AF311339 | C6orf162 | Chromosome 6 open reading frame 162 |
| 1263 | C8428 | NM_003884 | PCAF | P300/CBP-associated factor |
| 1264 | C8633 | BM480220 | MGC10911 | Hypothetical protein MGC10911 |
| 1265 | C9998 | NM 004316 | ASCL1 | Achaete-scute complex-like 1 (Drosophila) |
| 1266 | D1322 | BX647857 | ASB5 | Ankyrin repeat and SOCS box-containing 5 |
| 1267 | C0532 | H09657 | MGC39900 | Hypothetical protein MGC39900 |
| 1268 | C1982 | AI076840 | MGC33926 | Hypothetical protein MGC33926 |
| 1269 | C2021 | AL118812 | UGT8 | UDP glycosyltransferase 8 (UDP-galactose ceramide galactosyltransferase) |
| 1270 | C6875 | AA043381 | HOXD10 | Homeo box D10 |
| 1271 | C7114 | BU738386 | LOC284352 | Hypothetical protein LOC284352 |
| 1272 | C7048 | AK127778 | CXXC4 | CXXC finger 4 |
| 1273 | C9473 | AK127016 | PDZK4 | PDZ domain containing 4 |
| 1274 | D0393 | AA400194 | | Transcribed locus, weakly similar to XP_496793.1 PREDICTED: similar to signal-transducing adaptor protein-2; brk kinase substrate [Homo sapiens] |
| 1275 | D1366 | NM_001008393 | LOC201725 | Hypothetical protein LOC201725 |
| 1276 | C0589 | AF161506 | HSPC1-57 | HSPC157 protein |
| 1277 | C0824 | AI474181 | AHI1 | Abelson helper integration site |
| 1278 | C0453 | AW205849 | PIAS2 | Protein inhibitor of activated STAT, 2 |
| 1279 | C0651 | BM666770 | ADNP | Activity-dependent neuroprotector |
| 1280 | C9030 | AK129763 | | Hypothetical gene supported by AK000477 |
| 1281 | C8776 | AA766028 | AF15Q14 | Cancer susceptibility candidate 5 |
| 1282 | D1432 | AB023144 | SEZ6L | Seizure related 6 homolog (mouse)-like |
| 1283 | C1093 | AW976357 | CDCA1 | Cell division cycle associated 1 |
| 1284 | C5005 | BX648571 | FLJ38736 | Hypothetical protein FLJ38736 |
| 1285 | C5869 | NM_003447 | ZNF165 | Zinc finger protein 165 |
| 1286 | C5994 | BX117516 | | Homo sapiens, clone IMAGE:5271474,mRNA |
| 1287 | C7862 | AK002107 | RAB3B | RAB3B, member RAS oncogene family |
| 1288 | D0694 | BC015867 | SDCCAG8 | Serologically defmed colon cancer antigen 8 |
| 1289 | D0657 | AB058780 | SIAT2 | ST6 beta-galactosamide alpha-2,6-sialyltranferase 2 |
| 1290 | C0247 | BG390319 | LSM7 | LSM7 homolog, U6 small nuclear RNA associated (S. cerevisiae), |
| 1291 | C1624 | CA310876 | TULP4 | Tubby like protein 4 |
| 1292 | C2005 | AV702357 | | Transcribed locus |
| 1293 | C1399 | AA129217 | FLJ34048 | Hypothetical protein FLJ34048 |
| 1294 | C4221 | M_030913 | SEMA6C domain, | Sema domain, transmembrane domain (TM), and cytoplasmic (semaphorin) 6C |
| 1295 | C4588 | AA016977 | | MRNA;cDNADKFZp686F1844 (from clone DKFZp686F1844) |
| 1296 | C8107 | NM 031890 | CECR6 | Cat eye syndrome chromosome region, candidate 6 |
| 1297 | C8118 | BC048799 | SYN1 | Synapsin I |
| 1298 | C9517 | H73947 | POLR2J | Polymerase (RNA) II (DNA directed) polypeptide J, 13.3kDa |
| 1299 | C9571 | N36794 | TRIM67 | Tripartite motif-containing 67 |
| 1300 | D0010 | AA358397 | | Transcribed locus, weakly similar to XP_517655.1 PREDICTED: similar to KIAA0825 protein [Pan troglodytes] |
| 1301 | B9876 | R42862 | | Transcribed locus, moderately similar to XP_531995.1 PREDICTED: similar to calicin [Canis familiaris] |
| 1302 | C1890 | CR621991 | PLEK2 | Pleckstrin 2 |
| 1303 | C5995 | AL137736 | ARHGEF 19 | Rho guanine nucleotide exchange factor (GEF) 19 |
| 1304 | C6914 | AB037753 | FBXO42 | F-box protein 42 |
| 1305 | C6634 | AA398740 | | MRNA, chromosome 1 specific transcript KIAA0504 |
| 1306 | C7318 | BM677716 | ATP8A2 | ATPase, aminophospholipid transporter-like, Class I, type 8A, member 2 |
| 1307 | C9638 | CB129979 NM_033132 | ZIC5 | Zic family member 5 (odd-paired homolog, Drosophila) |
| 1308 | D1113 | AA939201 | MGC51082 | Hypothetical protein MGC51082 |
| 1309 | C0162 | CX865705 | WHSC1 | Wolf-Hirsehhorn syndrome candidate 1 |
| 1310 | C0827 | BC012568 | FLJ20364 | Hypothetical protein FLJ20364 |
| 1311 | C1590 | BU172301 | SMC4L1 | SMC4 structural maintenance of chromosomes 4-like (yeast) |
| 1312 | C2132 | AW134658 | MSI2 | Musashi homolog 2 (Drosophila) |
| 1313 | C3642 | BX648749 | SYNJ2 | Synaptojanin 2 |
| 1314 | C4633 | NM_152380 | TBX15 | T-box15 |
| 1315 | C4821 | BC01.8841 | C20orf20 | Chromosome 20 open reading frame 20 |
| 1316 | C6086 | BG029496 | RPL4 | Ribosomal protein L4 |
| 1317 | C7616 | NM_001015049 | BAG5 | BCL2-associated athanogene 5 |
| 1318 | C7757 | AK024506 | C14orf80 | Chromosome 14 open reading frame 80 |
| 1319 | C9520 | NM_033428 | C9orf123 | Chromosome 9 open reading frame 123 |
| 1320 | D0729 | AL365454 | INSR | Insulin receptor |
| 1321 | D1222 | AY190526 | B3GTL | Beta 3-glycosyltransferase-like |
| 1322 | D3205 | AY024361 | MLL3 | B melanoma antigen family, member 4 |
| 1323 | D4260 | BX648541 | | Homo sapiens, clone IMAGE:5270438, mRNA |
| 1324 | D4500 | AL833102 | CEPT1 | Choline/ethanolamine phosphotransferase 1 |
| 1325 | D6683 | NM_003106 | SOX2 | SRY (sex determining region Y)-box |
| 1326 | D6996 | AA928117 | ATP8A2 | ATPase, aminophospholipid transporter-like, Class I, type 8A, member 2 |
| 1327 | D8807 | BU730306 | MGC39606 | Hypothetical protein MGC39606 |
| 1328 | E0002 | BF195994 | PIAS2 | Protein inhibitor of activated STAT, 2 |
| 1329 | D9482 | AI049911 | ZNF643 | Zinc finger protein 643 |
| 1330 | E0371 | BC051333 | FLJ38944 | Hypothetical protein FLJ38944 |
| 1331 | E0912 | CR606585 | FLJ20345 | Hypothetical protein FLJ20345 |
| 1332 | D3851 | BF512494 | AGTPBP1 | ATP/GTP binding protein 1 |
| 1333 | D4284 | AI217674 | ZNF516 | Zinc finger protein 516 |
| 1334 | D4789 | AW070371 | SIMP | Source of immunodominant MHC-associated peptides |
| 1335 | D5753 | AA971042 | RHPN1 | Rhophilin, Rho GTPase binding protein 1 |
| 1336 | D6248 | AW295407 | FLJ25078 | Hypothetical protein FLJ25078 |
| 1337 | D7209 | AA058578 | | CDNA FLJ34585 fis, clone KIDNE2008758 |
| 1338 | D7481 | BX392279 | | Transcribed locus, strongly similar to XP_496781.1 PREDICTED: transposon-derived Buster3 transposase-like [Homo sapiens] |
| 1339 | D9991 | AK001720 | FLJ10858 | Nei endonuclease VIII-like 3 (E. coli) |
| 1340 | E0702 | BE045592 | SLC7A1 | Solute carrier family 7 (cationic amino acid transporter, y+ system), member 1 |
| 1341 | E0898 | NM_182551 | LYCAT | Lysocardiolipin acyltransferase |
| 1342 | E1118 | BX648933 | CLASP1 | Cytoplasmic linker associated protein |
| 1343 | D5565 | AK055216 | QTRT1 | Queuine tRNA-ribosyltransferase 1 (tRNA-guanine transglycosylase) |
| 1344 | D5692 | AL133031 | MLR1 | Transcription factor MLR1 |
| 1345 | D6407 | AA992705 | B4GALT6 | UDP-Gal:betaGlcNAc beta 1,4-galactosyltransferase, polypeptide 6 |
| 1346 | D6549 | BC004888 | FLJ10052 | Sushi domain containing 4 |
| 1347 | D7184 | CA948670 | XPR1 | Xenotropic and polytropic retrovirus receptor |
| 1348 | D8071 | BU786809 | | Transcribed locus |
| 1349 | D8457 | AA830551 | FLJ13848 | Hypothetical protein FLJ13848 |
| 1350 | D4077 | BC030960 | FLJ20225 | Ring finger protein 186 |
| 1351 | D5316 | H89599 | USP33 | Ubiquitin specific protease 33 |
| 1352 | D5081 | BX111010 | | XK-related protein 7 |
| 1353 | D5263 | NM 199355 | ADAMTS18 | A disintegrin-like and metalloprotease (reprolysin type) with thrombospondin type 1 motif, 18 |
| 1354 | D6309 | BU608866 | KIF5A | Kinesin family member 5A |
| 1355 | D6165 | AK124850 | RUTBC2 | RUN and TBC1 domain containing 2 |
| 1356 | D8019 | AA502265 | EXOSC2 | Exosome component 2 |
| 1357 | E0128 | AI089023 | FXYD7 | FXYD domain containing ion transport regulator 7 |
| 1358 | D2965 | BU622474 | | Similar to D(1B) dopamine receptor (D(5) dopamine receptor) (D1beta dopamine receptor) |
| 1359 | D4164 | BC068567 | MGC99813 | Similar to RIKEN cDNA A230078I05 gene |
| 1360 | D6767 | BM312795 | | Transcribed locus |
| 1361 | D8239 | AK026765 | C6orf59 | Chromosome 6 open reading frame 59 |
| 1362 | D7512 | AI066545 | ADAM12 | A disintegrin and metalloproteinase domain 12 (meltrin alpha) |
| 1363 | D9544 | H05758 | | Transcribed locus, moderately similar to NP_775735.11(3)mbt-like 4 [Homo sapiens] |
| 1364 | E0573 | BC020516 | IRF2BP2 | Interferon regulatory factor 2 binding protein 2 |
| 1365 | E1412 | AI989840 | | EST |
| 1366 | D3125 | AA761702 | | EST |
| 1367 | D4920 | AI247180 | GUCY1B2 | Guanylate cyclase 1, soluble, beta 2 |
| 1368 | D7212 | AA132702 | XTP2 | BAT2 domain containing 1 |
| 1369 | D7652 | AA976388 | | EST |
| 1370 | D8822 | AI052358 | BACE2 | Beta-site APP-cleaving enzyme 2 |
| 1371 | D9500 | AI361654 | | EST |
| 1372 | D3142 | AA767335 | PAX5 | Paired box gene 5 (B-cell lineage specific activator) |
| 1373 | D3190 | AK124869 | LOC400745 | Hypothetical gene supported by AK124869 gene supported by |
| 1374 | D4225 | BC028087 | VMD2L3 | Vitelliform macular dystrophy 2-like 3 |
| 1375 | D5415 | AW135928 | HOXB3 | Homeo box B3 |
| 1376 | D5556 | CR605673 | CBX5 | Chromobox homolog 5 (HP1 alpha homolog, Drosophila) |
| 1377 | D5349 | AI025236 | | Similar to asparagine synthetase; glutamine-dependent asparagine synthetase; TS11 cell cycle control protein |
| 1378 | D7443 | AI017753 | AHI1 | Abelson helper integration site |
| 1379 | D6707 | AA885838 | | Transcribed locus |
| 1380 | E1260 | BF793356 | XPO5 | Exportin 5 |
| 1381 | D4203 | AA781829 | | Similar to hypothetical protein BC009489 |
| 1382 | D4215 | AB096175 | SP5 | Sp5 transcription factor |
| 1383 | D4968 | BG054785 | | Transcribed locus, weakly similar to NP_997360.1 FLJ27365 protein [Homo sapiens] |
| 1384 | D5491 | AA947258 | | Transcribed locus |
| 1385 | D5898 | BX091406 | RAB6IP2 | RAB6 interacting protein 2 |
| 1386 | D5941 | AF293337 | SLC4A5 | Solute carrier family 4, sodium bicarbonate cotransporter, member 5 |
| 1387 | D6154 | AK123297 | ZNF37B | Zinc finger protein 37b (KOX 21) |
| 1388 | D6320 | XM_086879 | | Hypothetical LOC150371 |
| 1389 | D8901 | AI262277 | PFN2 | Profilin 2 |
| 1390 | D5416 | AF209747 | KCNMB2 | Potassium large conductance calcium-activated channel, subfamily M, beta member 2 |
| 1391 | D6708 | BC036529 | EPC1 | Enhancer of polycomb homolog 1 (Drosophila) |
| 1392 | D8294 | CD675645 | CSMD2 | CUB and Sushi multiple domains 2 |
| 1393 | E1507 | NM_013286 | RBM15B | RNA binding motif protein 15B |
| 1394 | D2882 | AA777954 | | EST |
| 1395 | D3959 | CR742308 | KLF12 | Kruppel-like factor 12 |
| 1396 | D4459 | AI553756 | PSMA3 | Proteasome (prosome, macropain) subunit, alpha type, 3 |
| 1397 | D4961 | AW972234 | | Transcribed locus |
| 1398 | D5370 | AA907927 | MDS009 | X 009 protein |
| 1399 | D7159 | AF317392 | BCOR | BCL6 co-repressor |
| 1400 | D7669 | BE348434 | | Transcribed locus |
| 1401 | D8876 | AL110204 | | MRNA; cDNA DKFZp586K1922 (from clone DKFZp586K1922) |
| 1402 | D9621 | NM_178229 | IQGAP3 | IQ motif containing GTPase activating protein 3 |
| 1403 | E0087 | BX484485 | MLL3 | B melanoma antigen family, member 4 |
| 1404 | E0623 | AL162079 | SLC16A1 | Solute carrier family 16 (monocarboxylic acid transporters), member 1 |
| 1405 | E0228 | H93431 | MYEF2 | Myelin expression factor 2 |
| 1406 | E1227 | NM_182964 | NAV2 | Neuron navigator 2 |
| 1407 | E1349 | BC041395 | | Homo sapiens, Similar to diaphanous homolog 3 (Drosophila), clone TMAGE:5277415, mRNA |
| 1408 | D3016 | AA781633 | LOC96610 | Hypothetical protein similar to KIAA0187 gene product |
| 1409 | D4168 | BM665164 | AP1S2 | Adaptor-related protein complex 1, sigma 2 subunit |
| 1410 | D5785 | AI553802 | | Transcribed locus |
| 1411 | D7831 | N66442 | CACNB2 | Calcium channel, voltage-dependent, beta 2 subunit |
| 1412 | D4532 | BM681974 | HSPC129 | Hypothetical protein HSPC129 |
| 1413 | D4971 | AA918686 | PFKFB2 | 6-phosphofructo-2-kinase/fructose-2,6-biphosphatase 2 |
| 1414 | D4999 | AA971400 | MGC47816 | Hypothetical protein MGC47816 |
| 1415 | D8440 | AA826148 | NRCAM | Neuronal cell adhesion molecule |
| 1416 | D8905 | AI021894 | MAP4K3 | Mitogen-activated protein kinase kinase kinase kinase 3 |
| 1417 | D9505 | BX100129 | | LOC440048 |
| 1418 | E0506 | NM_006904 | PRKDC | Protein kinase, DNA-activated, catalytic polypeptide |
| 1419 | A3896 | BC015050 | OIP5 | Opa interacting protein 5 |
| 1420 | C8129 | R42281 | | Hypothetical LOC147975 |
| 1421 | E2104 | CN280172 | YWHAQ | Tyrosine 3-monooxygenase/tryptophan 5-monooxygenase activation protein, theta polypeptide |
| 1422 | F0411 | AW898615 | | EST |
| 1423 | F2358 | AK021481 | GPC6 | Glypican 6 |
| 1424 | F4579 | AK022347 | PRKG1 | Protein kinase, cGMP-dependent, |
| 1425 | F7162 | AK000364 | CHD7 | Chromodomain helicase DNA binding protein 7 |
| 1426 | F8390 | AL831863 | | Full length insert cDNA clone YY86C01 |
| 1427 | F1471 | AB209394 | TNFRSF21 | Tumor necrosis factor receptor superfamily, member 21 |
| 1428 | D0740 | AA425325 | FLJ13305 | Hypothetical protein FLJ13305 |
| 1429 | D8310 | AA772401 | | EST |
| 1430 | D8441 | AA826176 | ATRX | Alpha thalassemia/mental retardation syndrome X-linked (RAD54 homolog, S. cerevisiae) |
| 1431 | F1227 | BX648495 | SLC38A1 | Solute carrier family 38, member 1 |
| 1432 | F2779 | BC001226 | PLEK2 | Pleckstrin 2 |
| 1433 | F3387 | AB020704 | PPFIA4 | Protein tyrosine phosphatase, receptor type, f polypeptide (PTPRF), interacting protein (liprin), alpha 4 |
| 1434 | F6592 | AY358353 | STK32B | Serine/threonine kinase 32B |
| 1435 | F8155 | AA935795 | | Similar to RIKEN cDNA 9930021J17 |
| 1436 | F8586 | AA579871 | SMARCC1 | SWI/SNF related, matrix associated, actin dependent regulator of chromatin, subfamily c, member 1 |
| 1437 | F8672 | AI291049 | PEX14 | Peroxisomal biogenesis factor 14 |
| 1438 | A5933 | XM_059689 | | Similar to CG4502-PA |
| 1439 | B6582 | R41184 | C13orf7 | Chromosome 13 open reading frame 7 |
| 1440 | C0640 | BC026307 | C18orf9 | Chromosome 18 open reading frame 9 |
| 1441 | F2230 | AK000112 | FLJ20105 | FLJ20105 protein |
| 1442 | F6998 | AF188703 | TBX4 | T-box 4 |
| 1443 | A8190 | AB011102 | ZNF292 | Zinc finger protein 292 |
| 1444 | B4853N | CD013889 | CHRNA1 | Cholinergic receptor, nicotinic, alpha polypeptide 1 (muscle) |
| 1445 | C7707 | AA152312 | LRFN5 | Leucine rich repeat and fibronectin type III domain containing 5 |
| 1446 | F1112 | AF107203 | A2BP1 | Ataxin 2-binding protein 1 |
| 1447 | F3847 | AK027006 | TNRC9 | Trinucleotide repeat containing 9 |
| 1448 | F4080 | NM_004523 | KIF11 | Kinesin family member 11 |
| 1449 | F4620 | AK021722 | AGPAT5 | 1-acylglycerol-3-phosphate O-acyltransferase 5 (lysophosphatidic acid acyltransferase, epsilon) |
| 1450 | F6507 | AL046246 | PGAP1 | GPI deacylase |
| 1451 | F7407 | AF095288 | PTTG2 | Pituitary tumor-transforming 2 |
| 1452 | F7399 | AI928242 | TFCP2L1 | Transcription factor CP2-like 1 |
| 1453 | F7652 | AK023043 | E2F7 | E2F transcription factor 7 |
| 1454 | A0576N | NM_138555 | KIF23 | Kinesin family member 23 |
| 1455 | E2082 | BX537667 | FARP1 | FERM, RhoGEF (ARHGEF) and pleckstrin domain protein 1 (chondrocyte-derived) |
| 1456 | F0164 | AB002362 | IGSF1 | Immunoglobulin superfamily, member 1 |
| 1457 | F2316 | AB033090 | PAK7 | P21(CDKN1A)-activated kinase 7 |
| 1458 | F3465 | AY033998 | ELAVL4 | ELAV (embryonic lethal, abnormal vision, Drosophila)-like 4 (Hu antigen D) |
| 1459 | F2938 | AK021734 | LOC153811 | Hypothetical protein LOC153811 |
| 1460 | F6193 | AK026280 | | CDNA: FLJ22627 fis, clone HSI06152 |
| 1461 | F7647 | AW241714 | TOX | Thymus high mobility group box protein TOX |
| 1462 | B7594N | AL045782 | | Transcribed locus |
| 1463 | E1632 | BU633335 | SMAD4 | SMAD, mothers against DPP homolog 4 (Drosophila) |
| 1464 | F0283 | AK123311 | GAP43 | Growth associated protein 43 |
| 1465 | F0331 | AL050002 | OLFML2A | Olfactomedin-like 2A |
| 1466 | F2073 | NM 020990 | | Creatine kinase, mitochondrial 1A |
| 1467 | F2807 | AL080146 | CCNB2 | Cyclin B2 |
| 1468 | F3374 | AF195765 | RAMP | RA-regulated nuclear matrix-associated protein |
| 1469 | F3431 | AK021954 | NRCAM | Neuronal cell adhesion molecule |
| 1470 | F5930 | NM_002509 | NKX2-2 | NK2 transcription factor related, locus 2 (Drosophila) |
| 1471 | F4952 | AL080082 | | MRNA; cDNA DKFZp564G1162 (from clone DKFZp564G1162) |
| 1472 | F4987 | AK000053 | MCLC | Mid-1-related chloride channel 1 |
| 1473 | F6022 | AK022479 | HDHD1A | Haloacid dehalogenase-like hydrolase domain containing 1A |
| 1474 | F6910 | BF940192 | KIAA0776 | KIAA0776 |
| 1475 | F7918 | A124726 | NRXN1 | Neurexin 1 |
| 1476 | B5456 | N62789 | DPP10 | Dipeptidylpeptidase 10 |
| 1477 | C9358 | AI126777 | FLJ45455 | FLJ45455 protein |
| 1478 | F0134 | AL833269 | LRRIQ2 | Leucine-rich repeats and IQ motif containing 2 |
| 1479 | F0983 | AL832106 | MLR2 | Ligand-dependent corepressor |
| 1480 | F1653 | BC011621 | HOOK1 | Hook homolog 1 (Drosophila) |
| 1481 | A2921 | NM_001012334 | MDK | Midkine (neurite growth-promoting factor 2) |
| 1482 | B9628 | BM449624 | | EST |
| 1483 | E1638 | CA447923 | ZBTB10 | Zinc finger and BTB domain containing 10 |
| 1484 | F1394 | AB046773 | KIAA1553 | KIAA1553 |
| 1485 | F2445 | AK022644 | MGC3101 | Hypothetical protein MGC3101 |
| 1486 | F2861 | C598555 | KIF20A | Kinesin family member 20A |
| 1487 | F4025 | AK021428 | C6orf210 | Chromosome 6 open reading frame 210 |
| 1488 | F4070 | NM_020897 | HCN3 | Hyperpolarization activated cyclic nucleotide-gated potassium channel 3 |
| 1489 | F3361 | AK090857 | SNAP25 | SynaPtosomal-associated protein, 25kDa |
| 1490 | F5806 | AF000381 | | EST |
| 1491 | A6212 | T35708 | PAK1 | P21/Cdc42/Racl-activated kinase 1 (STE20 homolog, yeast) |
| 1492 | A6689 | BU741863 | SPOCK | Sparc/osteonectin, cwcv and kazal-like domains proteoglycan (testican) |
| 1493 | C9981 | AI961235 | FLJ12505 | Hypothetical protein FLJ12505 |
| 1494 | F2294 | AK024900 | AP2B1 | Adaptor-related protein complex 2, beta 1 subunit |
| 1495 | F2376 | AK021714 | | CDNA FLJ11652 fis, clone HEMBA1004461 |
| 1496 | F2929 | AF022109 | CDC6 | CDC6 cell division cycle 6 homolog (S. cerevisiae) |
| 1497 | F3624 | AF319045 | CNTNAP2 | Contactin associated protein-like 2 |
| 1498 | F5215 | AL049314 | LOC92482 | Hypothetical protein LOC92482 |
| 1499 | F7562 | AI146812 | | EST |
| 1500 | F7685 | AV699624 | | Transcribed locus |
| 1501 | A3339 | M93119 | INSM1 | Insulinoma-associated 1 |
| 1502 | C4168 | W33155 | | EST |
| 1503 | D3317 | AA884583 | | Transcribed locus |
| 1504 | F1332 | CR592757 | BRRN1 | Barren homolog (Drosophila) |
| 1505 | F2556 | U91641 | SIAT8E | ST8 alpha-N-acetyl-neuraminide alpha-2,8-sialyltransferase 5 |
| 1506 | F3349 | AL109706 | | MRNA full length insert cDNA clone EUROIMAGE 362430 |
| 1507 | F6689 | AK021848 | | EST |
| 1508 | B8706 | R52614 | CDK5R1 | Cyclin-dependent kinase 5, regulatory subunit 1 (p35) |
| 1509 | F4976 | AF165527 | DGCR8 | DiGeorge syndrome critical region gene 8 |
| 1510 | A0636 | Z29066 | NEK2 | NIMA (never in mitosis gene a)-related kinase 2 |
| 1511 | F0967 | AB006000 | LECT1 | Leukocyte cell derived chemotaxin 1 |
| 1512 | F2228 | X51688 | CCNA2 | Cyclin A2 |
| 1513 | F6269 | AY327407 | C2orf10 | Chromosome 2 open reading frame 10 |
| 1514 | B4408 | AK074029 | FLJ20255 | Hypothetical protein FLJ20255 |
| 1515 | F4649 | L19183 | MAC30 | Hypothetical protein MAC30 |
| 1516 | F5946 | AL137529 | ACPL2 | Acid phosphatase-like 2 |
| 1517 | F5974 | AF070581 | PAK3 | P21 (CDKN1A) activated kinase 3 |
| 1518 | F4158 | BC047767 | APOBEC2 | Apolipoprotein B mRNA editing enzyme, catalytic polypeptide-like 2 |
| 1519 | C1813 | NM_133372 | KIAA1961 | KIAA1961 gene |
| 1520 | G2326 | BI496673 | BAI3 | Brain-specific angiogenesis inhibitor 3 |
| 1521 | B7569 | T66310 | SCUBE3 | Signal peptide, CUB domain, EGF-like 3 |
| 1522 | G3673 | BM677658 | PHIP | Pleckstrin homology domain interacting protein |
| 1523 | G5139 | A077841 | PURG | Purine-rich element binding protein G |
| 1524 | G5155 | BF055352 | SEC11L3 | SEC11-like 3 (S. cerevisiae) |
| 1525 | F4405 | NM_003540 | | EST |
| 1526 | G3375 | AW300826 | | Transcribed locus |
| 1527 | B3505 | AA725827 | | Transcribed locus |
| 1528 | C1747 | H63387 | | MRNA; cDNA DKFZp761I2317 (from clone DKFZp761I2317) |
| 1529 | F1579 | AK021717 | | CDNA FLJ11655 fis, clone HEMBA1004554 |
| 1530 | F6220 | AW976075 | C7orf24 | Chromosome 7 open reading frame 24 |
| 1531 | G3363 | AK094436 | KIAA0802 | KIAA0802 |
| 1532 | F6572 | NM_003545 | | EST |
| 1533 | G2316 | AJ412030 | DLEU1 | Deleted in lymphocytic leukemia, 1 |
| 1534 | G3606 | BM680332 | | EST |
| 1535 | F8619 | AI632567 | TFCP2L1 | Transcription factor CP2-like 1 |
| 1536 | G2535 | AI700987 | C11orf23 | Chromosome 11 open reading frame 23 |
| 1537 | G2892 | AI024536 | | Transcribed locus |
| 1538 | G2797 | BC033114 | LOC144501 | Hypothetical protein LOC144501 |
| 1539 | G3676 | BM669634 | | EST |
| 1540 | G5950 | H17455 | | EST |
| 1541 | A7040N | H53856 | | EST |
| 1542 | G2350 | AW134492 | C6orf31 | Chromosome 6 open reading frame 31 |
| 1543 | G3232 | BU619489 | TFAP2A | Transcription factor AP-2 alpha (activating enhancer binding protein 2 alpha) |
| 1544 | G3318 | NM_020686 | ABAT | 4-aminobutyrate aminotransferase |
| 1545 | G6544 | CR749603 | C6orf167 | Chromosome 6 open reading frame 167 |
| 1546 | G3342 | BE156543 | | EST |
| 1547 | G5799 | AA946808 | DEFB1 | Defensin, beta 1 |
| 1548 | G7085 | AW978782 | SYK | Spleen tyrosine kinase |
| 1549 | F4452 | AK001432 | | LOC440030 |
| 1550 | G2266 | AW805032 | IGSF4 | Immunoglobulin superfamily, member 4 |
| 1551 | G2192 | BC007393 | ZNF553 | Zinc finger protein 553 |
| 1552 | G2617 | BQ020506 | | Transcribed locus, moderately similar to NP_872301.1 hypothetical protein FLJ25224 [Homo sapiens] |
| 1553 | G2961 | N58198 | HSC20 | J-type co-chaperone HSC20 |
| 1554 | G3525 | AK055418 | | CDNA FLJ30856 fis, clone FEBRA2003258 |
| 1555 | F1303 | AK125482 | LOC92312 | Hypothetical protein LOC92312 |

### IDENTIFICATION OF GENES DIFFERENTIALLY UP-REGULATED IN SCLCS COMPARING WITH NSCLCS

To identify genes that characterize and distinguish the nature of SCLC from NSCLC, we compared the expression profiles of 15 advanced SCLC cases as well as 35 early-stage NSCLCs (ADC and SCC) and 27 advanced NSCLCs (ADC) (P-stages; IIIB or IV) previously obtained using the same cDNA microarray system (Kakiuchi S et al., Hum Mol Genet. 2004;13(24):3029-43, Kikuchi T et al., Oncogene 2003;22: 2192-2205) **(****Fig. 5*****A*).** Since the 62 NSCLC samples had been analyzed for a subset (27,648 genes) of the 32,256 genes on our present microarray-system, we analyzed the information of a subset of the 27,648 genes for which valid values could be obtained in more than 80% of the cases examined. We also excluded genes with observed standard deviations of < 1.7. The 475 genes that passed through this cut-off filter were analyzed further. In the sample axis (horizontal) in **Fig. 5*****A*,** 81 samples (four cases were examined in duplicate to validate the reproducibility and reliability of our experimental procedure) from 77 cases were clustered into two major groups on the basis of their expression profiles. The dendrogram shown at the top of **Fig. 5** represents similarities in expression patterns among individual cases; the shorter the branches are, the greater the similarities are. The four duplicated cases (No. 13, 20, K91, and LC12) that were labelled and hybridized in independent experiments were clustered most closely within the same group **(****Fig. 5*****B*).** The identical genes spotted on different positions on the slide glasses were also clustered into the adjacent rows **(****Fig. 5*****B*).** These results supported the high reproducibility and reliability of our experimental procedures. Of the 77 cases, the 15 SCLC clustered into one major group and the 20 early-stage ADC and 15 SCC as well as 27 advanced ADC clustered into individual groups. Clearly, SCLC and NSCLC appeared to have different gene expression profiles that reflect differences in the etiological and clinicopathological natures.

In this analysis, we obtained 34 genes which were expressed abundantly in SCLC, and some of which revealed the characteristics of certain neuronal functions, for example, neurogenesis and neuroprotection (Cluster-1 in **Fig. 5*****A*, 5*B*; Table 4**; *i.e. DPYSL2*, *ADNP etc*).

**Table 4 Up-regulated gene in SCLC comparing with NSCLC**

| Asignment NO | LMMID | GenBank ID | Symbol | Gene name |
|---|---|---|---|---|
| 1556 | | AI341170 | *Cep70* | P10-binding protein |
| 1557 | | AA788924 | *C5* | Complement component 5 |
| 1558 | | AL365454 | *INSR* | Insulin receptor |
| 1559 | | AK054999 | *FLJ30437* | CDNA FLJ30437 fis, clone BRACE2009045 |
| 1560 | | AI928242 | *TFCP2L1* | Transcription factor CP2-like 1 |
| 1561 | | NM_172164 | *NASP* | Nuclear autoantigenic sperm protein (histone-binding) |
| 1562 | | NM_001609 | *ACADSB* | Acyl-Coenzyme A dehydrogenase, short/branched chain |
| 1563 | | NM_015458 | *MTMR9* | Myotubularin related protein 9 |
| 1564 | | AA058578 | *FLJ34585* | CDNA FLJ34585 fis, clone KIDNE2008758 |
| 1565 | | AA921341 | *LPGAT1* | Lysophosphatidylglycerol acyltransferase 1 |
| 1566 | | CA503163 | *ADNP* | Activity-dependent neuroprotector |
| 1567 | | BC042688 | *RASD1* | RAS, dexamethasone-induced 1 |
| 1568 | | AK096960 | *RAD1* | RAD1 homolog (S. pombe) |
| 1569 | | AL832815 | *TMEM30A* | Transmembrane protein 30A |
| 1570 | | CR596214 | *HNRPA0* | Heterogeneous nuclear ribonucleoprotein A0 |
| 1571 | | BQ016211 | *FLJ10154* | Hypothetical protein FLJ10154 |
| 1572 | | BX647115 | *DPYSL2* | Dihydropyrimidinase-like 2 |
| 1573 | | AL137572 | *C1orf24* | Chromosome 1 open reading frame 24 |
| 1574 | | NM_133265 | *AMOT* | Angiomotin |
| 1575 | | AA602499 | *GLCCI1* | Glucocorticoid induced transcript 1 |
| 1576 | | U33749 | *TITF1* | Thyroid transcription factor 1 |
| 1577 | | BQ002875 | *PARP8* | Poly (ADP-ribose) polymerase family, member 8 |
| 1578 | | AK124953 | *FLJ36144* | Similar to hypothetical protein FLJ36144 |
| 1579 | | NM_033632 | *FBXW7* | F-box and WD-40 domain protein 7 (archipelago homolog, Drosophila) |
| 1580 | | AK096344 | *FLJ35220* | Hypothetical protein FLJ35220 |
| 1581 | | R42757 | *IGSF4* | Immunoglobulin superfamily, member 4 |
| 1582 | | AB209404 | *GLIS3* | GLIS family zinc finger 3 |
| 1583 | | AA418594 | *THRAP2* | Thyroid hormone receptor associated protein 2 |
| 1584 | | AB011124 | *ProSAPiP1* | ProSAPiP1 protein |
| 1585 | | AL110212 | *H2AFV* | H2A histone family, member V |
| 1586 | | N29574 | *RRAGD* | Ras-related GTP binding D |
| 1587 | | AF326917 | *AUTS2* | Autism susceptibility candidate 2 |
| 1588 | | AF059611 | *ENC1* | Ectodermal-neural cortex (with BTB-like domain) |
| 1589 | | AK022881 | *KIAA1272* | Chromosome 20 open reading frame 74 |

### Identification of Genes Related to Chemoresistance.

Since chemoresistance is a major obstacle for cancer treatment, identification of genes commonly up-regulated in cancer cells obtained from patients who had failed certain chemotherapy is one of effective approaches to understand the mechanism of chemoresistance and develop a novel cancer therapy that overcomes this problem. We obtained 68 genes expressed abundantly both in advanced SCLCs and advanced ADCs (Cluster-2 in **Fig. 5*****A***, **5C; Table 5**), both of which were obtained from chemotherapy-resistant lung cancer patients. "Chemotherapy-resistant lung cancer patient" refers to a living or dead lung cancer patient who have undergone chemotherapy treatments one or more times (although the chemotherapy protocols provided to these patients were not same). Some of them are known to be transcription factors and/or gene expression regulators including *TAF5L, TFCP2L4, PHF20, LMO4, TCF20, RFX2,* and *DKFZp547I048.* Moreover, some genes encoding nucleotide-binding proteins including *C9orf76, EHD3,* and *GIMAP4* were also found in the list.

In addition, we identified candidate genes as therapeutic targets of a chemotherapy-resistant lung cancer (Table 6) that were specifically up-regulated in advanced SCLCs compared with chemotherapy-sensitive lung cancer tissue.

The above-described chemotherapy-resistant lung cancer-associated genes were obtained by determining the gene expression levels in advanced SCLCs and advanced NSCLCs (Tables 5), or in advanced SCLCs (Tables 6), and selecting the genes whose expression level was increased compared to the control expression level in a chemotherapy-sensitive lung cancer. The control expression level can be obtained referring a known chemotherapy-sensitive lung cancer expression profile or simultaneously determined using as a template a control sample prepared from chemotherapy-resistant lung cancer patients.
Table 5 Up-regulated genes in advanced SCLCs and advanced NSCLC .compared with chemotherapy-sensitive lung cancer tissue

| Asignment NO | LMMID | GenBank ID | Symbol | Gene name |
|---|---|---|---|---|
| 1590 | C7072 | AB007952 | *FBXO28* | F-box protein 28 |
| 1591 | A6380 | NM_005141 | *FGB* | Fibrinogen beta chain |
| 1592 | D3853 | AA830326 | *EST* | |
| 1593 | B2655 | AA677491 | *STX8* | Syntaxin 8 |
| 1594 | B0828 | AK091100 | *LOC284591* | Hypothetical protein LOC284591 |
| 1595 | D0791 | AA464854 | *FAT3* | FAT tumor suppressor homolog 3 (Drosophila) |
| 1596 | A7111N | BC029858 | *B7* | B7 gene |
| 1597 | B6562 | CA306079 | *PLEKHJ1* | Pleckstrin homology domain containing, family J member 1 |
| 1598 | B1721 | NM_005650 | *TCF20* | Transcription factor 20 (AR1) |
| 1599 | A2343N | AK025742 | *UCP2* | Uncoupling protein 2 (mitochondrial, proton carrier) |
| 1600 | C6048 | AK075509 | *NRM* | Nurim (nuclear envelope membrane protein) |
| 1601 | F4090 | NM_001336 | *CTSZ* | Cathepsin Z |
| 1602 | B9465 | BC039999 | *C9orf76* | Chromosome 9 open reading frame 76 |
| 1603 | A0065N | AF502289 | *TRIP10* | Thyroid hormone receptor interactor 10 |
| 1604 | C9194 | BC041070 | *KRTHA4* | Keratin, hair, acidic, 4 |
| 1605 | C4127 | NM_001007094 | *ZNF37A* | Zinc finger protein 37a (KOX 21) |
| 1606 | C4205 | AA868706 | *KCTD15* | Potassium channel tetramerisation domain containing 15 |
| 1.607 | E0494 | CV424097 | *LMO4* | LIM domain only 4 |
| 1608 | C8848 | AF214736 | *EHD3* | EH-domain containing 3 |
| 1609 | B5323 | AA757392 | *EST* | |
| 1610 | C8152 | D87463 | *PHYHIP* | Phytanoyl-CoA hydroxylase interacting protein |
| 1611 | C8844 | BM916826 | *PHF20* | PHD finger protein 20 |
| 1612 | C8182 | H12117 | *MOBKL2B* | MOB1, Mps One Binder kinase activator-like 2B (yeast) |
| 1613 | B8435 | R32836 | *EST* | |
| 1614 | A9545 | AA563634 | *MGC29671* | Hypothetical protein MGC29671 |
| 1615 | C0829 | NM 203371 | *LOC387758* | Similar to RIKEN cDNA 1110018M03 |
| 1616 | A1092 | NM_002184 | *IL6ST* | Interleukin 6 signal transducer (gp130, oncostatin M receptor) |
| 1617 | B9769 | AK097664 | *LOC90557* | Hypothetical protein BC016861 |
| 1618 | A6912 | AA813719 | *DKFZp547I048* | Chromosome 1 open reading frame 173 |
| 1619 | D3154 | NM_182798 | *FLJ39155* | Hypothetical protein FLJ39155 |
| 1620 | C0465 | AK057053 | *METRN* | Meteorin, glial cell differentiation regulator |
| 1621 | C8310 | H11638 | *CHN2* | Chimerin (chimaerin) 2 |
| 1622 | C4220 | N93264 | *C9orf115* | Chromosome 9 open reading frame 115 |
| 1623 | D9015 | BC036890 | *TFCP2L4* | Grainyhead-like 3 (Drosophila) |
| 1624 | D0380 | BX109199 | *EST* | |
| 1625 | C4284 | AL834247 | *MYPN* | Myopalladin |
| 1626 | B1143 | NM_000692 | *ALDH1B1* | Aldehyde dehydrogenase 1 family, member B 1 |
| 1627 | C6026 | R49124 | *SLC2A9* | Solute carrier family 2 (facilitated glucose transporter), member 9 |
| 1628 | D1438 | AA828735 | *NMNAT2* | Nicotinamide nucleotide adenylyltransferase 2 |
| 1629 | F6820 | CR749297 | *SKIP* | SPHK1 (sphingosine kinase type 1) interacting protein |
| 1630 | B6115N | AF097431 | *LEPRE1* | Leucine proline-enriched proteoglycan (leprecan) 1 |
| 1631 | B6971 | BG209407 | *EST* | Transcribed locus |
| 1632 | D4018 | AI347994 | *TAF4B* | TAF4b RNA polymerase II, TATA box binding protein (TBP)-associated factor, 105kDa |
| 1633 | E0647 | BU628989 | *EST* | |
| 1634 | D0852 | AA429665 | *EST* | |
| 1635 | B8067 | BX648249 | *STN2* | Stonin 2 |
| 1636 | A4095N | N93656 | *RAMP2* | Receptor (calcitonin) activity modifying protein 2 |
| 1637 | A3977 | NM_014409 | *TAF5L* | TAF5-like RNA polymerase II, p300/CBP-associated factor (PCAF)-associated factor, 65kDa |
| 1638 | B2995 | W52081 | *LOC114926* | Hypothetical protein BC013035 |
| 1639 | B9222 | AF450487 | *KIF21A* | Kinesin family member 21A |
| 1640 | B2937 | BM472056 | *H2AFZ* | H2A histone family, member Z |
| 1641 | A3519 | CR606023 | *ATIC* | 5-aminoimidazole-4-carboxamide ribonucleotide formyltransferase/IMP cyclohydrolase |
| 1642 | C6040 | H05226 | *EST* | |
| 1643 | B7501 | AB014578 | *DNAJC13* | DnaJ (Hsp40) homolog, subfamily C, member 13 |
| 1644 | B9182 | AI288717 | *RFX2* | Regulatory factor X, 2 (influences HLA class II expression) |
| 1645 | C6846 | BC053521 | *SPTAN1* | Spectrin, alpha, non-erythrocytic 1 (alpha-fodrin) |
| 1646 | A1581 | U89942 | *LOXL2* | Lysyl oxidase-like 2 |
| 1647 | B9973 | BC035561 | *FLJ23825* | Hypothetical protein FLJ23825 |
| 1648 | A2593 | BC093053 | *SGNE1* | Secretory granule, neuroendocrine protein 1 (7B2 protein) |
| 1649 | E0836 | NM_032236 | *USP48* | Ubiquitin specific protease 48 |
| 1650 | F3362 | AK023995 | *FLJ12442* | Hypothetical protein FLJ12442 |
| 1651 | C0505 | NM_018326 | *GIMAP4* | GTPase, IMAP family member 4 |
| 1652 | D6314 | NM_018243 | *SEPT11* | Septin 11 |
| 1653 | B7487 | AA195424 | *C2orf22* | PQ loop repeat containing 3 |
| 1654 | F3351 | Y12735 | *DYRK3* | Dual-specificity tyrosine-(Y)-phosphorylation regulated kinase 3 |
| 1655 | B8166 | NM_182964 | *NAV2* | Neuron navigator 2 |
| 1656 | B3835 | NM_001695 | *ATP6V1C1* | ATPase, H+ transporting, lysosomal 42kDa, V1 subunit C, isoform 1 |
| 1657 | A5089 | U36501 | *SP100* | Nuclear antigen Sp100 |

**Table 6 Up-regulated genes in advanced SCLCs compared with chemotherapy-sensitive lung cancer tissue.**

| Asignment NO | GenBank ID | Symbol | Gene name |
|---|---|---|---|
| 1658 | BC035561 | FLJ23825 | Hypothetical protein FLJ23825 |
| 1659 | AF450487 | KIF21A | Kinesin family member 21A |
| 1660 | AL834247 | MYPN | Myopalladin |
| 1661 | NM_182964 | NAV2 | Neuron navigator 2 |
| 1662 | BC093053 | SGNE1 | Secretory granule, neuroendocrine protein 1 (7B2 protein) |
| 1663 | NM_005650 | TCF20 | Transcription factor 20 (AR1) |

### Discussion

Lung cancer is the most common cancer in the world. Chemotherapy remains the essential component for treatment of all patients with SCLC, regardless of stage (either LD or ED) or performance status. In LD, the addition of radiation therapy improves survival over chemotherapy alone. While SCLC is usually initially sensitive to chemotherapy and radiotherapy, responses are rarely long lasting. Frustratingly, most SCLC patients ultimately relapse with highly treatment-resistant disease and the final outcome of the patients is poor with an overall 5-year survival rate of less than 10%. Therefore it is urgently required to develop novel diagnostic tools for detection of early stage of primary cancer and/or relapse and molecular-targeted therapies involving small-molecule and antibody-based approaches as well as novel immunotherapies targeting cancer-specific antigens. Therefore, gene expression profile of SCLC is the first step to screen the drugable targets.

To analyze the gene expression profile of SCLC, we used genome-wide cDNA microarray containing 32,256 cDNAs. The advent of laser-microdissection technology has brought about a great improvement in the ability to isolate cancer cells from interstitial tissues. The proportion of contaminated surrounding non-cancerous cells using this method is estimated to be less than 0.3% (Yanagawa R et al., (2001) Neoplasia.;3:395-401, Kakiuchi et al., (2004) Hum Mol Genet.;13:3029-43 & (2003) Mol Cancer Res.;1:485-99), which is consistent with the conclusion that the data represents the expression profile of a highly pure population of SCLC cells.

We have established a detailed genome-wide database for sets of genes that are differentially expressed in SCLCs. To date, we identified 779 candidate genes and as tumor markers or therapeutic targets (*see* Table 3) that were specifically up-regulated in cancer. The up-regulated genes represented a variety of functions including genes associated with neuroendocrine functions or ones encoding cancer-testis or onco-fetal antigens as well as ones important for cell growth, proliferation, survival, and transformation. These genes encode proteins with a variety of functions that include transmembrane/secretory proteins, and cancer-testis or onco-fetal antigens as well as ones important in cell adhesion, cytoskeleton structure, signal transduction, and cell proliferation. Some of them are useful as diagnostic/prognostic markers and as therapeutic targets for development of new molecular-targeted agents or immunotherapy for lung-cancer treatment. Tumor-specific transmembrane/secretory proteins have significant advantages, because they are presented on the cell surface, making them easily accessible as molecular markers and therapeutic targets. Some tumor-specific markers available at present, including CYFRA or Pro-GRP, are transmembrane/secretory proteins (Miyake Y, et al., (1994) Cancer Res.; 54:2136-40.; Pujol JL, et al., (1993) Cancer Res.; 53:61-6.). An example of rituximab (Rituxan), a chimeric monoclonal antibody against CD20-positive lymphomas, provides proof of the concept that targeting specific cell-surface proteins can provide us significant clinical benefits (Hennessy BT, et al., (2004) Lancet Oncol.; 5:341-53.). On the other hand, among tumor antigens identified to date, cancer-testis antigens (CTAs) have been recognized as a group of highly attractive targets for cancer vaccine. Although other factors, for example, the *in vivo* immunogenicity of the protein are also important and further examination will be necessary, our candidate genes actually includes known CTA including TSGA14. Further study using this expression profile doubtlessly enables us to identify novel CTAs that are good targets for immunotherapy of SCLC. These targets are useful as diagnostic/prognostic markers and as therapeutic targets for development of new molecular-targeted agents or immunotherapy in lung-cancer treatment. Among the up-regulated genes, we selected 83 genes for validation by semi-quantitative RT-PCR experiments and confirmed their cancer-specific expression (Figures 2A).

And we discovered that *ZIC5* (SEQ ID NO.175, encoding SEQ ID NO.176) identified as an up-regulated gene was a cancer-testis antigen activated in the great majority of SCLCs, and was plays a pivotal role in cell growth/survival, as demonstrated by northern-blot analysis and siRNA experiments. This gene encodes a protein of 663 amino acids with five C2H2 ZNF domains. This molecule is structurally a nucleic acid binding Zinc ion binding protein. Among tumor antigens identified to date, cancer-testis antigens have been recognized as a group of highly attractive targets for cancer vaccine. Although other factors, for example, the *in vivo* immunogenicity of the protein are also important and further examination will be necessary, ZIC5 is a good target for immunotherapy as well as for development of new anti-cancer drugs.

Chemoresistance is a clinically very important issue that we need to overcome for the improvement in treatment of patients with an advanced or end-stage cancer. Our gene expression profile data obtained from the fifteen autopsy samples as well as advanced ADCs with the clinical history of chemotherapy (Cluster-2 in Fig. 5A, 5C; Table 5) were considered to reflect the characteristics of advanced lung cancers with acquired chemoresistance. Unsupervised cluster analysis of these subgroups identified up-regulated genes including *TAF5L, TFCP2L4, PHF20, LMO4, TCF20, and RFX2* that were known to have transcription factor activities. Some transcription factors were reported to be associated with acquired chemoresistance. For example, constitutive activation of NF-kappaB, a transcription factor involved in multiple cellular processes, appears to support cancer cell survival and to reduces the sensitivity against chemotherapeutic drugs (Arlt A & Schafer H. (2002) Int J Clin Pharmacol Ther.; 40:336-47.). On the other hand, some genes in the list included *C9orf76, EHD3,* and *GIMAP4* that were found to bind to the nucleotide. Since some DNA-binding proteins were known to play a critical role in the DNA-repair process, the genes shown above also have some functions in DNA repair and contribute to increase in chemoresistance. Further analysis of the genes in this group are important for development of novel therapies for chemoresistant tumors.

Neuroendocrine tumors of lung range from well differentiated neuroendocrine carcinoma (typical carcinoid) to intermediate grade (atypical carcinoma) or to very aggressive poorly differentiated lesions (large cell neuroendocrine carcinoma (LCNEC) and SCLC). SCLC is generally considered as a major neuroendocrine tumor of lung, and causes several paraneoplastic neuroendocrine syndromes. These syndromes represent clinically distinct symptoms in SCLC patients. Up-regulated genes included several genes which were related to the neuroendocrine function including insulinoma-associated 1 *(INSM1),* chromogranin A (parathyroid secretory protein 1; *CHGA*), and achaete-scute complex-like 1 (Drosophila; *ASCL1*), further supporting the strong relationship between SCLC and neuroendocrine syndromes at molecular levels. Our gene list also includes a set of genes related to some cancer-related syndromes including cachexia.

In summary, our cDNA microarray analysis combined with an LMM system revealed most comprehensive gene expression profiles of SCLC involving up-regulated genes that encode proteins with the function of cell cycle/growth, and signal transduction, or products with unknown function as well as transmembrane/secretory proteins and CTAs. Further analyses using animal models will narrow down the possible therapeutic targets as well as diagnostic ones for lung cancer. The combined use of the integrated gene-expression database of human cancers and normal organ tissues as well as siRNAs to select candidate genes like *ZIC5* offers a powerful strategy for rapid identification and further evaluation of target molecules for a personalized therapy.

### INDUSTRIAL APPLICABILITY

The gene-expression analysis of small cell lung cancer described herein, obtained through a combination of laser-capture dissection and genome-wide cDNA microarray, has identified specific genes as targets for cancer prevention and therapy. Based on the expression of a subset of these differentially expressed genes, the present invention provides molecular diagnostic markers for identifying and detecting small cell lung cancer.

The methods described herein are also useful in the identification of additional molecular targets for prevention, diagnosis and treatment of small cell lung cancer. The data reported herein add to a comprehensive understanding of small cell lung cancer, facilitate development of novel diagnostic strategies, and provide clues for identification of molecular targets for therapeutic drugs and preventative agents. Such information contributes to a more profound understanding of small cell lung tumorigenesis, and provides indicators for developing novel strategies for diagnosis, treatment, and ultimately prevention of small cell lung cancer.

The present inventors have also shown that the cell growth is suppressed by small interfering RNA (siRNA) that specifically targets the ZIC5 gene. Thus, this novel siRNA is useful target for the development of anti-cancer pharmaceuticals. For example, agents that block the expression of ZIC5 or prevent its activity find therapeutic utility as anti-cancer agents, particularly anti-cancer agents for the treatment of lung cancer, including small cell lung cancer.

Additionally, a clustering algorithm applied to the expression data of 34 genes identified by random-permutation test easily distinguished two major histological types of lung cancer, non-small cell lung cancer (NSCLC) and SCLC. These data provide valuable information for identifying novel diagnostic systems and therapeutic target molecules for this type of cancer. Chemotherapy for lung cnancer is completely different between small cell lung cancer and non-small cell lung cancer. Therefore, in order to decide a treating strategy for lung cancer, it is important to distinguish SCLC from NSCLC. However, conventional histopathological diagnosis requires specialized skills to distinguish them. Thus, the genes identified in the present invention are very useful for treating lung cancer.

The present invention further provides chemotherapy resistant lung cancer, or SCLC assosiated genes were identified. These genes were up-regulated in chemorestant lung cancer or SCLC. Accordingly, chemorestant lung cancer or SCLC can be predicted using expression level of the genes as diagnostic marker. As the result, any adverse effects caused by ineffective chemotherapy can be avoided, and more suitable and effective therapeutic strategy can be selected.

All patents, patent applications, and publications cited herein are incorporated by reference in their entirety.

Furthermore, while the invention has been described in detail and with reference to specific embodiments thereof, it is to be understood that the foregoing description is exemplary and explanatory in nature and is intended to illustrate the invention and its preferred embodiments. Through routine experimentation, one skilled in the art will readily recognize that various changes and modifications can be made therein without departing from the spirit and scope of the invention. Thus, the invention is intended to be defined not by the above description, but by the following claims and their equivalents.

Furthermore, the present invention relates to the following items:
1. A method of diagnosing small cell lung cancer or a predisposition for developing small cell lung cancer in a subject, comprising determining a level of expression of a small cell lung cancer-associated gene in a biological sample from a patient, wherein an increase or decrease in said sample expression level as compared to a normal control level of said gene indicates that said subject suffers from or is at risk of developing small cell lung cancer.
2. The method of item 1, wherein said small cell lung cancer-associated gene is selected from the group consisting of the genes of SCLC Nos. 777-1555, further wherein an increase in said sample expression level as compared to a normal control level indicates said subject suffers from or is at risk of developing small cell lung cancer.
3. The method of item 2, wherein said sample expression level is at least 10% greater than said normal control level.
4. The method of item 1, wherein said small cell lung cancer-associated gene is selected from the group consisting of the genes of SCLC Nos. 1-776, further wherein a decrease in said sample expression level as compared to a normal control level indicates said subject suffers from or is at risk of developing small cell lung cancer.
5. The method of item 4, wherein said sample expression level is at least 10% lower than said normal control level.
6. The method of item 1, wherein said method further comprises determining the level of expression of a plurality of small cell lung cancer-associated genes.
7. The method of item 1, wherein gene expression level is determined by a method selected from the group consisting of:
   a) detecting mRNA of the small cell lung cancer-associated gene,
   b) detecting a protein encoded by the small cell lung cancer-associated gene, and
   c) detecting a biological activity of a protein encoded by the small cell lung cancer-associated gene.
8. The method of item 7, wherein said detection is carried out on a DNA array.
9. The method of item 1, wherein said biological sample comprises an epithelial cell.
10. The method of item 1, wherein said biological sample comprises a small cell lung cancer cell.
11. The method of item 1 wherein said biological sample comprises an epithelial cell from a small cell lung cancer cell.
12. A small cell lung cancer reference expression profile comprising a pattern of gene expression of two or more small cell lung cancer-associated genes selected from the group consisting of the genes of SCLC Nos. 1-1555.
13. A small cell lung cancer reference expression profile comprising a pattern of gene expression for two or more small cell lung cancer-associated genes selected from the group consisting of the genes of SCLC Nos. 1-776.
14. A small cell lung cancer reference expression profile comprising a pattern of gene expression for two or more small cell lung cancer-associated genes selected from the group consisting of the genes of SCLC Nos.777-1555.
15. A method of screening for a compound for treating or preventing small cell lung cancer, said method comprising the steps of:
   a) contacting a test compound with a polypeptide encoded by a polynucleotide selected from the group consisting of the genes of SCLC Nos. 1-1555;
   b) detecting the binding activity between the polypeptide and the test compound; and
   c) selecting the test compound that binds to the polypeptide.
16. A method of screening for a compound for treating or preventing small cell lung cancer, said method comprising the steps of:
   a) contacting a candidate compound with a cell expressing one or more marker genes, wherein the one or more marker genes are selected from the group consisting of the genes of SCLC Nos. 1-1555; and
   b) selecting the candidate compound that reduces the expression level of one or more marker genes selected from the group consisting of the genes of SCLC Nos.777-1555, or elevates the expression level of one or more marker genes selected from the group consisting of the genes of SCLC Nos. 1-776, as compared to an expression level detected in the absence of the candidate compound.
17. The method of item 16, wherein said cell comprises a small cell lung cancer cell.
18. A method of screening for a compound for treating or preventing small cell lung cancer, said method comprising the steps of:
   a) contacting a test compound with a polypeptide encoded by a polynucleotide selected from the group consisting of the genes of SCLC Nos. 1-1555;
   b) detecting the biological activity of the polypeptide of step (a); and
   c) selecting the test compound that suppresses the biological activity of the polypeptide encoded by the polynucleotide selected from the group consisting of the genes of SCLC Nos.777-1555 as compared to the biological activity of said polypeptide detected in the absence of the test compound, or enhances the biological activity of the polypeptide encoded by the polynucleotide selected from the group consisting of the genes of SCLC Nos. 1-776 as compared to the biological activity of said polypeptide detected in the absence of the test compound.
19. A method of screening for compound for treating or preventing small cell lung cancer, said method comprising the steps of:
   a) contacting a candidate compound with a cell into which a vector, comprising the transcriptional regulatory region of one or more marker genes and a reporter gene that is expressed under the control of the transcriptional regulatory region, has been introduced, wherein the one or more marker genes are selected from the group consisting of the genes of SCLC Nos.1-1555;
   b) measuring the expression or activity of said reporter gene; and
   c) selecting the candidate compound that reduces the expression or activity of said reporter gene when said marker gene is an up-regulated marker gene selected from the group consisting of the genes of SCLC Nos.777-1555, or that enhances the expression or activity level of said reporter gene when said marker gene is a down-regulated marker gene selected from the group consisting of the genes of SCLC Nos. 1-776, as compared to an expression or activity level detected in the absence of the test compound.
20. A kit comprising a detection reagent which binds to (a) two or more nucleic acid sequences selected from the group consisting of the genes of SCLC Nos. 1-1555, or (b) polypeptides encoded thereby.
21. An array comprising two or more nucleic acids which bind to one or more nucleic acid sequences selected from the group consisting of the genes of SCLC Nos. 1-1555.
22. A method of treating or preventing small cell lung cancer in a subject comprising administering to said subject an antisense composition, said antisense composition comprising a nucleotide sequence complementary to a coding sequence selected from the group consisting of the genes of SCLC Nos.777-1555.
23. A method of treating or preventing small cell lung cancer in a subject comprising administering to said subject an siRNA composition, wherein said siRNA composition reduces the expression of a nucleic acid sequence selected from the group consisting of the genes of SCLC Nos. 777-1555.
24. A method of treating or preventing small cell lung cancer in a subject comprising the step of administering to said subject a pharmaceutically effective amount of an antibody, or immunologically active fragment thereof, that binds to a protein encoded by any one gene selected from the group consisting of the genes of SCLC Nos. 777-1555.
25. A method of treating or preventing small cell lung cancer in a subject comprising administering to said subject a vaccine comprising (a) a polypeptide encoded by a nucleic acid selected from the group consisting of the genes of SCLC Nos. 777-1555, (b) an immunologically active fragment of said polypeptide, or (c) a polynucleotide encoding the polypeptide.
26. A method of inducing an anti-tumor immunity, said method comprising the step of contacting with an antigen presenting cell a polypeptide, a polynucleotide encoding the polypeptide or a vector comprising the polynucleotide, wherein the polypeptide is encoded by a gene selected from the group consisting of SCLC No. 777-1555, or the fragment thereof.
27. The method of inducing an anti-tumor immunity of item 26, wherein the method further comprises the step of administering the antigen presenting cell to a subject
28. A method of treating or preventing small cell lung cancer in a subject, said method comprising the step of administering a compound obtained by a method according to any one of items 15-19.
29. A method of treating or preventing small cell lung cancer in a subject comprising administering to said subject a pharmaceutically effective amount of an agent comprising (a) a polynucleotide selected from the group consisting of the genes of SCLC Nos. 1-776, or (b) a polypeptide encoded thereby.
30. A composition for treating or preventing small cell lung cancer, said composition comprising a pharmaceutically effective amount of an antisense polynucleotide or siRNA against a polynucleotide selected from the group consisting of the genes of SCLC Nos. 777-1555.
31. A composition for treating or preventing small cell lung cancer, said composition comprising a pharmaceutically effective amount of an antibody or fragment thereof that binds to a protein encoded by a gene selected from the group consisting of the genes of SCLC Nos. 777-1555.
32. A composition for treating or preventing small cell lung cancer, said composition comprising as an active ingredient a pharmaceutically effective amount of a compound selected by a method of any one of items 15-19, and a pharmaceutically acceptable carrier.
33. A method of discriminating small-cell lung cancer and non-small cell lung cancer, the method comprising the steps of:
   a) detecting an expression level of one or more marker genes in a specimen collected from a subject to be diagnosed, wherein the one or more marker genes is selected from the group consisting of the genes of SCLC Nos. 1556-1589; and
   (b) comparing the expression level of the one or more marker genes to that of non-small cell lung cancer, wherein a high expression level of the marker gene, as compared to non-small cell lung cancer, is indicative of small-cell lung cancer.
34. A method of treating or preventing small cell lung cancer in a subject comprising administering to said subject a composition comprising a small interfering RNA (siRNA) that inhibits expression of *ZIC5.*
35. The method of item 34, wherein said siRNA comprises a sense nucleic acid sequence and an anti-sense nucleic acid sequence that specifically hybridizes to a sequence from *ZIC5.*
36. The method of item 35, wherein said siRNA comprises a ribonucleotide sequence corresponding to a sequence consisting of SEQ ID NO: 171 as the target sequence.
37. The method of item 36, wherein said siRNA has the general formula 5'-[A]-[B]-[A']-3', wherein [A] is a ribonucleotide sequence corresponding to a sequence consisting of nucleotides of SEQ ID NO: 171
   [B] is a ribonucleotide loop sequence consisting of 3 to 23 nucleotides, and
   [A'] is a ribonucleotide sequence consisting of the complementary sequence of [A].
38. The method of item 34, wherein said composition comprises a transfection-enhancing agent.
39. A double-stranded molecule comprising a sense strand and an antisense strand, wherein the sense strand comprises a ribonucleotide sequence corresponding to a target sequence consisting of SEQ ID NO: 171, and wherein the antisense strand comprises a ribonucleotide sequence which is complementary to said sense strand, wherein said sense strand and said antisense strand hybridize to each other to form said double-stranded molecule, and wherein said double-stranded molecule, when introduced into a cell expressing the *ZIC5* gene, inhibits expression of said gene.
40. The double-stranded molecule of item 39, wherein said target sequence comprises at least about 10 contiguous nucleotides from the nucleotide sequences of SEQ ID NO: 175.
41. The double-stranded molecule of item 40, wherein said target sequence comprises from about 19 to about 25 contiguous nucleotides from the nucleotide sequences of SEQ ID NO: 175.
42. The double-stranded molecule of item 41, wherein said double-stranded molecule is a single ribonucleotide transcript comprising the sense strand and the antisense strand linked via a single-stranded ribonucleotide sequence.
43. The double-stranded molecule of item 40, wherein the double-stranded molecule is an oligonucleotide of less than about 100 nucleotides in length.
44. The double-stranded molecule of item 43, wherein the double-stranded molecule is an oligonucleotide of less than about 75 nucleotides in length.
45. The double-stranded molecule of item 44, wherein the double-stranded molecule is an oligonucleotide of less than about 50 nucleotides in length.
46. The double-stranded molecule of item 45, wherein the double-stranded molecule is an oligonucleotide of less than about 25 nucleotides in length.
47. The double-stranded polynucleotide of item 46, wherein the double stranded molecule is an oligonucleotide of between about 19 and about 25 nucleotides in length.
48. A vector encoding the double-stranded molecule of item 40.
49. The vector of item 48, wherein the vector encodes a transcript having a secondary structure and comprises the sense strand and the antisense strand.
50. The vector of item 49, wherein the transcript further comprises a single-stranded ribonucleotide sequence linking said sense strand and said antisense strand.
51. A vector comprising a polynucleotide comprising a combination of a sense strand nucleic acid and an antisense strand nucleic acid, wherein said sense strand nucleic acid comprises nucleotide sequence of SEQ ID NO: 171, and said antisense strand nucleic acid consists of a sequence complementary to the sense strand.
52. The vector of item 51, wherein said polynucleotide has the general formula 5'-[A]-[B]_[A']-3'
   wherein [A] is a nucleotide sequence of SEQ ID NO: 171; [B] is a nucleotide sequence consisting of 3 to 23 nucleotides; and [A'] is a nucleotide sequence complementary to [A].
53. A pharmaceutical composition for treating or preventing small cell lung cancer comprising a pharmaceutically effective amount of a small interfering RNA (siRNA) that inhibits expression of *ZIC5* as an active ingredient, and a pharmaceutically acceptable carrier.
54. The pharmaceutical composition of item 53, wherein the siRNA comprises a nucleotides sequence consisting of SEQ ID NO: 171 as the target sequence.
55. The composition of item 54, wherein the siRNA has the general formula 5'-[A]-[B]-[A']-3'
   wherein [A] is a ribonucleotide sequence corresponding to a nucleotide sequence of SEQ ID NO: 171; [B] is a ribonucleotide sequence consisting of 3 to 23 nucleotides; and [A'] is a ribonucleotide sequence complementary to [A].
56. A method of diagnosing chemotherapy-resistant lung cancer or a predisposition for developing chemotherapy-resistant lung cancer in a subject, comprising determining a level of expression of a chemotherapy-resistant lung cancer-associated gene selected from the group consisting of the genes listed in Table 5 in a biological sample from a patient, wherein an increase in said sample expression level as compared to a control level of said gene indicates that said subject suffers from or is at risk of developing chemotherapy-resistant lung cancer.
57. The method of item 56, wherein said sample expression level is at least 10% greater than said control level.
58. The method of item 56, wherein the chemotherapy-resistant lung cancer is small cell lung cancer, and the chemotherapy-resistant lung cancer-associated gene is selected from the group consisting of the genes listed in Table 6.
59. The method of item 56, wherein said method further comprises determining the level of expression of a plurality of chemotherapy-resistant lung cancer-associated genes.
60. The method of item 56, wherein gene expression level is determined by a method selected from the group consisting of:
   (a) detecting mRNA of the chemotherapy-resistant lung cancer-associated gene,
   (b) detecting a protein encoded by the chemotherapy-resistant lung cancer-associated gene, and
   (c) detecting a biological activity of a protein encoded by the chemotherapy-resistant lung cancer-associated gene.
61. The method of item 60, wherein said detection is carried out on a DNA array.
62. The method of item 56, wherein said biological sample comprises a lung cancer cell.
63. The method of :item 56, wherein said biological sample comprises an epithelial cell from a lung cancer cell.
64. A chemotherapy-resistant lung cancer reference expression profile comprising a pattern of gene expression for two or more chemotherapy-resistant lung cancer-associated genes selected from the group consisting of the genes listed in Table 5.
65. The expression profile of item 64, wherein the chemotherapy-resistant lung cancer is small cell lung cancer, and the chemotherapy-resistant lung cancer-associated gene is selected from the group consisting of the genes listed in Table 6.
66. A method of screening for a compound for treating or preventing chemotherapy-resistant lung cancer, said method comprising the steps of:
   a) contacting a test compound with a polypeptide encoded by a polynucleotide selected from the group consisting of the genes listed in Table 5;
   b) detecting the binding activity between the polypeptide and the test compound; and
   c) selecting the test compound that binds to the polypeptide.
67. A method of screening for a compound for treating or preventing chemotherapy-resistant lung cancer, said method comprising the steps of:
   a) contacting a candidate compound with a cell expressing one or more marker genes, wherein the one or more marker genes are selected from the group consisting of the genes listed in Table 5; and
   b) selecting the candidate compound that reduces the expression level of one or more marker genes selected from the group consisting of the genes listed in Table 5, as compared to an expression level detected in the absence of the candidate compound.
68. The method of item 67, wherein said cell comprises a chemotherapy-resistant lung cancer cell.
69. A method of screening for a compound for treating or preventing chemotherapy-resistant lung cancer, said method comprising the steps of:
   a) contacting a test compound with a polypeptide encoded by a polynucleotide selected from the group consisting of the genes listed in Table 5;
   b) detecting the biological activity of the polypeptide of step (a); and
   c) selecting the test compound that suppresses the biological activity of the polypeptide encoded by the polynucleotide selected from the group consisting of the genes listed in Table 5 as compared to the biological activity of said polypeptide detected in the absence of the test compound.
70. A method of screening for compound for treating or preventing chemotherapy-resistant lung cancer, said method comprising the steps of:
   a) contacting a candidate compound with a cell into which a vector, comprising the transcriptional regulatory region of one or more marker genes and a reporter gene that is expressed under the control of the transcriptional regulatory region, has been introduced, wherein the one or more marker genes are selected from the group consisting of the genes listed in Table 5;
   b) measuring the expression or activity of said reporter gene; and
   c) selecting the candidate compound that reduces the expression or activity of said reporter gene as compared to an expression or activity level detected in the absence of the test compound.
71. The method of any one of items 66-70, wherein the chemotherapy-resistant lung cancer is small cell lung cancer, and the chemotherapy-resistant lung cancer-associated gene is selected from the group consisting of the genes listed in Table 6.
72. A kit for detecting a chemotherapy-resistant lung cancer, the kit comprising a detection reagent which binds to (a) two or more nucleic acid sequences selected from the group consisting of the genes listed in Table 5, or (b) polypeptides encoded thereby.
73. The kit of item 72, wherein the chemotherapy-resistant lung cancer is small cell lung cancer, and the chemotherapy-resistant lung cancer-associated gene is selected from the group consisting of the genes listed in Table 6.
74. An array for detecting a chemotherapy-resistant lung cancer, the array comprising two or more nucleic acids which bind to one or more nucleic acid sequences selected from the group consisting of the genes listed in Table 5.
75. The array of item 74, wherein the chemotherapy-resistant lung cancer is small cell lung cancer, and the chemotherapy-resistant lung cancer-associated gene is selected from the group consisting of the genes listed in Table 6.
76. A method of treating or preventing chemotherapy-resistant lung cancer in a subject comprising administering to said subject an antisense composition, said antisense composition comprising a nucleotide sequence complementary to a coding sequence selected from the group consisting, of the genes listed in Table 5.
77. A method of treating or preventing chemotherapy-resistant lung cancer in a subject comprising administering to said subject an siRNA composition, wherein said siRNA composition reduces the expression of a nucleic acid sequence selected from the group consisting of the genes listed in Table 5.
78. A method of treating or preventing chemotherapy-resistant lung cancer in a subject comprising the step of administering to said subject a pharmaceutically effective amount of an antibody, or immunologically active fragment thereof, that binds to a protein encoded by any one gene selected from the group consisting of the genes listed in Table 5.
79. A method of treating or preventing chemotherapy-resistant lung cancer in a subject comprising administering to said subject a vaccine comprising (a) a polypeptide encoded by a nucleic acid selected from the group consisting of the genes listed in Table 5, (b) an immunologically active fragment of said polypeptide, or (c) a polynucleotide encoding the polypeptide.
80. A method of inducing an anti-tumor immunity, said method comprising the step of contacting with an antigen presenting cell a polypeptide, a polynucleotide encoding the polypeptide or a vector comprising the polynucleotide, wherein the polypeptide is encoded by a gene selected from the group consisting of the genes listed in Table 5, or the fragment thereof.
81. The method of inducing an anti-tumor immunity of item 80, wherein the method further comprises the step of administering the antigen presenting cell to a subject.
82. The method of any one of items 75-81, wherein the chemotherapy-resistant lung cancer is small cell lung cancer, and the chemotherapy-resistant lung cancer-associated gene is selected from the group consisting of the genes listed in Table 6.
83. A method of treating or preventing chemotherapy-resistant lung cancer in a subject, said method comprising the step of administering a compound obtained by a method according to any one of items 66-70.
84. A method of treating or preventing chemotherapy resistant small cell lung cancer in a subject, said method comprising the step of administering a compound obtained by a method according to item 71.
85. A composition for treating or preventing chemotherapy-resistant lung cancer, said composition comprising a pharmaceutically effective amount of an antisense polynucleotide or siRNA against a polynucleotide selected from the group consisting of the genes listed in Table 5.
86. A composition for treating or preventing chemotherapy-resistant lung cancer, said composition comprising a pharmaceutically effective amount of an antibody or fragment thereof that binds to a protein encoded by a gene selected from the group consisting of the genes listed in Table 5.
87. The composition of items 85 or 86, wherein the chemotherapy-resistant lung cancer is small cell lung cancer, and the chemotherapy-resistant lung cancer-associated gene is selected from the group consisting of the genes listed in Table 6.
88. A composition for treating or preventing chemotherapy-resistant lung cancer, said composition comprising as an active ingredient a pharmaceutically effective amount of a compound selected by a method of any one of items 66-70, and a pharmaceutically acceptable carrier.
89. A composition for treating or preventing chemotherapy resistant small cell lung cancer, said composition comprising as an active ingredient a pharmaceutically effective amount of a compound selected by a method of items 71, and a pharmaceutically acceptable carrier.

## Claims

1. A method for screening for a compound for treating or preventing small cell lung cancer, said method comprising the steps (a)-(c) of set A, (a)-(c) of set B, (1)-(2) of set C, or (a)-(c) of set D as follows:
Set A;
(a) contacting a test compound with a polypeptide encoded by a polynucleotide of the gene of SCLC No. 1445 (LRFN5);
(b) detecting the biological activity of the polypeptide of step (a); and
(c) selecting the test compound that suppresses the biological activity of the polypeptide encoded by the polynucleotide of the gene of SCLC No. 1445 (LRFN5) as compared to the biological activity of said polypeptide detected in the absence of the test compound,
Set B;
(a) contacting a test compound with a polypeptide encoded by a polynucleotide of the gene of SCLC No. 1445 (LRFN5);
(b) detecting the binding activity between the polypeptide and the test compound; and
(c) selecting the test compound that binds to the polypeptide,
Set C;
(1) contacting a candidate compound with a cell expressing the gene of SCLC No. 1445 (LRFN5); and
(2) selecting the candidate compound that reduces the expression level of the gene of SCLC No. 1445 (LRFN5), as compared to an expression level detected in the absence of the candidate compound, or
Set D;
(a) contacting a candidate compound with a cell into which a vector, comprising the transcriptional regulatory region of SCLC No. 1445 (LRFN5) and a reporter gene that is expressed under the control of the transcriptional regulatory region, has been introduced;
(b) measuring the expression or activity of said reporter gene; and
(c) selecting the candidate compound that reduces the expression or activity of said reporter gene, as compared to an expression or activity level detected in the absence of the test compound.

2. The method of claim 1, wherein the method comprises steps (a)-(c) of set A, and the biological activity is a cell proliferative activity.

3. The method of claim 1, wherein the method comprises steps (1)-(2) of set C, and said cell comprises a small cell lung cancer cell.
